(19) Europäisches Patentamt
European Patent Office
Office européen des brevets

(11) **EP 3 492 454 A1**

(12) **EUROPEAN PATENT APPLICATION**

(43) Date of publication:
**05.06.2019 Bulletin 2019/23**

(21) Application number: **17204796.1**

(22) Date of filing: **30.11.2017**

(51) Int Cl.:
***C07D 241/12*** *(2006.01)* ***C07D 401/04*** *(2006.01)*
***C07D 417/12*** *(2006.01)* ***C07D 417/14*** *(2006.01)*
***C07D 277/52*** *(2006.01)* ***C07D 213/22*** *(2006.01)*
***C07D 213/38*** *(2006.01)* ***C07C 211/45*** *(2006.01)*
***A61P 35/00*** *(2006.01)* ***A61P 37/00*** *(2006.01)*
***A61K 31/425*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Step Pharma S.A.S.**
**75272 Paris, Cedex 06 (FR)**

(72) Inventor: **The designation of the inventor has not yet been filed**

(74) Representative: **Kinch, Alison**
**Sagittarius IP**
**Marlow International**
**Parkway**
**Marlow SL7 1YL (GB)**

(54) **COMPOUNDS**

(57) Compounds of formula (I):

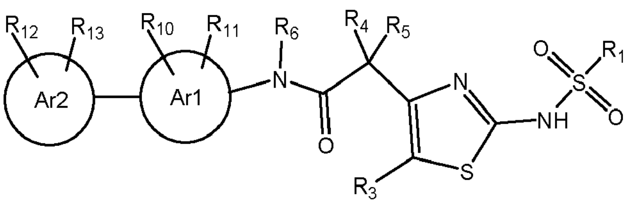

(I)

and related aspects.



Printed by Jouve, 75001 PARIS (FR)

## Description

### Field of the invention

The invention relates to novel aminothiazole compounds, processes for the manufacture of such compounds, related intermediates, compositions comprising such compounds and the use of such compounds as cytidine triphosphate synthase 1 inhibitors, particularly in the treatment or prophylaxis of disorders associated with cell proliferation.

### Background of the invention

Nucleotides are a key building block for cellular metabolic processes such as deoxyribonucleic acid (DNA) and ribonucleic acid (RNA) synthesis. There are two classes of nucleotides, purines and pyrimidines and both classes are important for metabolic processes. Based on this, many therapies have been developed to target different aspects of nucleotide synthesis, with some inhibiting generation of purine nucleotides and some pyrimidine nucleotides.

The pyrimidine nucleotide cytidine 5' triphosphate (CTP) is a precursor required not just for the metabolism of DNA and RNA but also phospholipids and sialyation of proteins. CTP originates from two sources: a salvage pathway and a *de novo* synthesis pathway that depends on two enzymes, the CTP synthases (or synthetases) 1 and 2 (CTPS1 and CTPS2) (Evans and Guy 2004; Higgins, *et al.* 2007; Ostrander, *et al.* 1998).

CTPS1 and CTPS2 catalyse the conversion of uridine triphosphate (UTP) and glutamine into cytidine triphosphate (CTP):

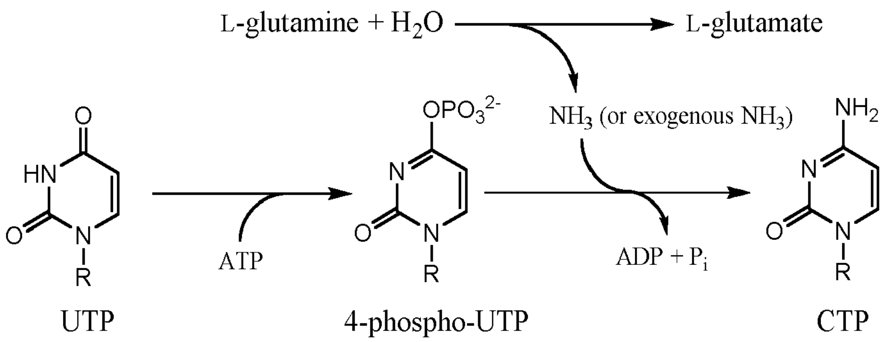


Both enzymes have two domains, an N-terminal synthetase domain and a C-terminal glutaminase domain (Kursula, *et al.* 2006). The synthetase domain transfers a phosphate from adenosine triphosphate (ATP) to the 4-position of UTP to create an activated intermediate, 4-phospho-UTP. The glutaminase domain generates ammonia from glutamine, via a covalent thioester intermediate with a conserved active site cysteine, generating glutamate. This ammonium is transferred from the glutaminase domain to the synthetase domain *via* a tunnel or can be derived from external ammonium. This ammonium is then used by the synthetase domain to generate CTP from the 4-phospho-UTP (Lieberman, 1956).

Although CTPS exists as two isoforms in humans and other eukaryotic organisms, CTPS1 and CTPS2, functional differences between the two isoforms are not yet fully elucidated (van Kuilenburg, *et al.* 2000).

The immune system provides protection from infections and has therefore evolved to rapidly respond to the wide variety of pathogens that the individual may be exposed to. This response can take many forms, but the expansion and differentiation of immune populations is a critical element and is hence closely linked to metabolic processes. Within this, CTP synthase activity appears to play an important role in DNA synthesis and the rapid expansion of lymphocytes following activation (Fairbanks, *et al.* 1995; van den Berg, *et al.* 1995).

Strong validation that CTPS1 is the critical enzyme in human lymphocyte proliferation came with the identification of a loss-of-function homozygous mutation (rs145092287) in this enzyme that causes a distinct and life-threatening immunodeficiency, characterized by an impaired capacity of activated T- and B-cells to proliferate in response to antigen receptor-mediated activation. Activated CTPS1-deficient cells were shown to have decreased levels of CTP. Normal T-cell proliferation was restored in CTPS1-deficient cells by expressing wild-type CTPS1 or by addition of exogenous CTP or its nucleoside precursor, cytidine. CTPS1 expression was found to be low in resting lymphocytes, but rapidly upregulated following activation of these cells. Expression of CTPS1 in other tissues was generally low. CTPS2 seems to be ubiquitously expressed in a range of cells and tissues but at low levels, and the failure of CTPS2, which is still intact in the patients, to compensate for the mutated CTPS1, supports CTPS1 being the critical enzyme for the immune populations affected in the patients (Martin, *et al.* 2014).

Overall, these findings suggest that CTPS1 is a critical enzyme necessary to meet the demands for the supply

of CTP required by several important immune populations.

Normally the immune response is tightly regulated to ensure protection from infection, whilst controlling any response targeting host tissues. In certain situations, the control of this process is not effective, leading to immune-mediated pathology. A wide range of human diseases are thought to be due to such inappropriate responses mediated by different elements of the immune system.

Given the role that cell populations, such as T and B lymphocytes, are thought to play in a wide range of autoimmune and other diseases, CTPS1 represent a target for a new class of immunosuppressive agents. Inhibition of CTPS1 therefore provides a novel approach to the inhibition of activated lymphocytes and selected other immune cell populations such as Natural Killer cells, Mucosal-Associated Invariant T (MAIT) and Invariant Natural Killer cells, highlighted by the phenotype of the human mutation patients (Martin, *et al.* 2014).

As far as is known to date, no selective CTPS1 inhibitors have been developed. Several (presumed) non-selective CTPS inhibitors have been used for oncology indications up to phase I/II clinical trials, but were stopped due to toxicity and efficacy issues. More recently, the CTPS1 selective inhibitory peptide CTpep-3 has been identified. The inhibitory effects of CTpep-3 however, were seen in cell free assays but not in the cellular context. This was not unexpected though, since the peptide is unlikely to enter the cell and hence is not easily developable as a therapeutic (Sakamoto, *et al.* 2017).

In summary, the available information and data strongly suggest that inhibitors of CTPS1 will reduce the proliferation of a number of immune cell populations. Inhibitors of CTPS1 may therefore be expected to have utility for treatment or prophylaxis in a wide range of indications where the pathology is driven by these populations.

CTPS1 inhibitors represent a novel approach for inhibiting selected components of the immune system in various tissues, and the related pathologies or pathological conditions such as, in general terms, rejection of transplanted cells and tissues, Graft-related diseases or disorders, allergies and autoimmune diseases.

**Summary of the Invention**

The invention provides to a compound of formula (I):

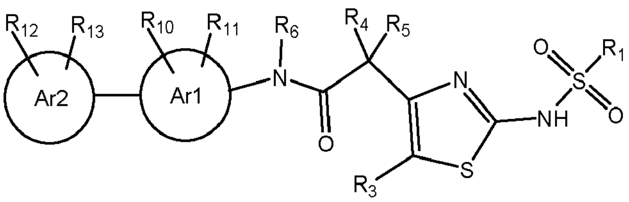

(I)

wherein

$R_1$ is $C_{1-5}$alkyl, $C_{0-2}$alkylene$C_{3-5}$cycloalkyl which cycloalkyl is optionally substituted by $CH_3$, or $CF_3$;

$R_3$ is H, halo, $CH_3$ or $CF_3$,

or $R_3$ together with $R_5$ forms a 5- or 6-membered cycloalkyl;

$R_4$ and $R_5$ are each independently H, F, $C_{1-6}$alkyl, $C_{0-2}$alkylene$C_{3-6}$cycloalkyl, $OC_{1-6}$alkyl, $OC_{0-2}$alkylene$C_{3-6}$cycloalkyl, $C_{1-3}$alkylene$OC_{1-3}$alkyl, $C_{1-6}$haloalkyl or $OC_{1-6}$haloalkyl,

or $R_4$ is H and $R_5$ together with $R_3$ form a 5- or 6-membered cycloalkyl,

or $R_4$ and $R_5$ together with the carbon atom to which they are attached form a $C_{3-6}$spirocycloalkyl,

or $R_4$ is O and $R_5$ is absent;

$R_6$ is H or $C_{1-3}$alkyl,

or $R_6$ together with $R_{11}$ in the ortho-position to the amide are a $C_2$alkylene chain forming a 5-membered ring;

Ar1 is 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $C_{1-2}$haloalkyl, $OC_{1-2}$haloalkyl or CN;

$R_{11}$ is H, F, Cl, $CH_3$, $OCH_3$, $CF_3$, $OCF_3$ or CN,

or $R_{11}$, when in the ortho-position to the amide, together with $R_6$ are a $C_2$alkylene chain forming a 5-membered ring;

$R_{12}$ is attached to Ar2 in the ortho or meta position relative to Ar1 and $R_{12}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, $C_{1-4}$alkylOH, CN, $C_{1-3}$alkylene$OC_{1-3}$alkyl, $C_{1-4}$haloalkyl, $OC_{1-4}$haloalkyl, $N(CH_3)_2$, $SO_2CH_3$, $C(O)N(CH_3)_2$, $NHC(O)C_{1-3}$alkyl or a $C_{3-6}$heterocycloalkyl comprising one nitrogen located at the point of attachment to Ar2; and

$R_{13}$ is H, F, $CH_3$ or $OCH_3$.

A compound of formula (I) may be provided in the form of a salt and/or solvate thereof and/or derivative thereof. Suitably, the compound of formula (I) may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Also provided is a compound of formula (I), or a pharmaceutically acceptable salt and/or solvate thereof and/or derivative thereof, for use as a medicament, in particular for use in the inhibition of CTPS1 in a subject or the prophylaxis or treatment of associated diseases or disorders, such as those in which a reduction in T-cell and/or B-cell proliferation would be beneficial.

Further, there is provided a method for the inhibition of CTPS1 in a subject or the prophylaxis or treatment of associated diseases or disorders, such as those in which a reduction in T-cell and/or B-cell proliferation would be beneficial, by administering to a subject in need thereof a compound of formula (I) or a pharmaceutically acceptable salt and/or solvate thereof and/or derivative thereof.

Additionally provided is the use of a compound of formula (I), or a pharmaceutically acceptable salt and/or solvate thereof and/or derivative thereof, in the manufacture of a medicament for the the inhibition of CTPS1 in a subject or the prophylaxis or treatment of associated diseases or disorders, such as those in which a reduction in T-cell and/or B-cell proliferation would be beneficial.

Suitably the disease or disorder is selected from: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; and transplantation.

Also provided are pharmaceutical compositions containing a compound of formula (I), or a pharmaceutically acceptable salt and/or solvate thereof and/or derivative thereof, and a pharmaceutically acceptable carrier or excipient.

Also provided are processes for preparing compounds of formula (I) and novel intermediates of use in the preparation of compounds of formula (I).

## Detailed description of the Invention

The invention provides to a compound of formula (I):

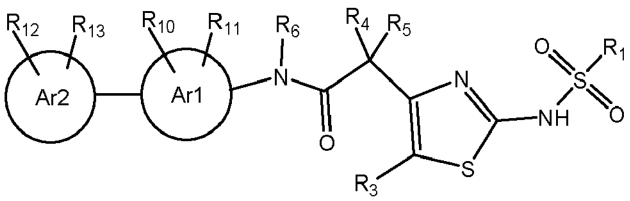

(I)

wherein

$R_1$ is $C_{1-5}$alkyl, $C_{0-2}$alkylene$C_{3-5}$cycloalkyl which cycloalkyl is optionally substituted by $CH_3$, or $CF_3$;

$R_3$ is H, halo, $CH_3$ or $CF_3$,

or $R_3$ together with $R_5$ forms a 5- or 6-membered cycloalkyl;

$R_4$ and $R_5$ are each independently H, F, $C_{1-6}$alkyl, $C_{0-2}$alkylene$C_{3-6}$cycloalkyl, $OC_{1-6}$alkyl, $OC_{0-2}$alkylene$C_{3-6}$cycloalkyl, $C_{1-3}$alkylene$OC_{1-3}$alkyl, $C_{1-6}$haloalkyl or $OC_{1-6}$haloalkyl,

or $R_4$ is H and $R_5$ together with $R_3$ form a 5- or 6-membered cycloalkyl,

or $R_4$ and $R_5$ together with the carbon atom to which they are attached form a $C_{3-6}$spirocycloalkyl,

or $R_4$ is O and $R_5$ is absent;

$R_6$ is H or $C_{1-3}$alkyl,

or $R_6$ together with $R_{11}$ in the ortho-position to the amide are a $C_2$alkylene chain forming a 5-membered ring;

Ar1 is 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $C_{1-2}$haloalkyl, $OC_{1-2}$haloalkyl or CN;

$R_{11}$ is H, F, Cl, $CH_3$, $OCH_3$, $CF_3$, $OCF_3$ or CN,

or $R_{11}$, when in the ortho-position to the amide, together with $R_6$ are a $C_2$alkylene chain forming a 5-membered ring;

$R_{12}$ is attached to Ar2 in the ortho or meta position relative to Ar1 and $R_{12}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, $C_{1-4}$alkylOH, CN, $C_{1-3}$alkylene$OC_{1-3}$alkyl, $C_{1-4}$haloalkyl, $OC_{1-4}$haloalkyl, $N(CH_3)_2$, $SO_2CH_3$, $C(O)N(CH_3)_2$, $NHC(O)C_{1-3}$alkyl or a $C_{3-6}$heterocycloalkyl comprising one nitrogen located at the point of attachment to Ar2; and

$R_{13}$ is H, F, $CH_3$ or $OCH_3$;

or a salt and/or solvate thereof and/or derivative thereof.

The term 'alkyl' as used herein, such as in $C_{1-3}$alkyl, $C_{1-4}$alkyl or $C_{1-5}$alkyl, whether alone or forming part of a larger group such as an Oalkyl group (e.g. $OC_{1-3}$alkyl, $OC_{1-4}$alkyl and $OC_{1-5}$alkyl), is a straight or a branched fully saturated hydrocarbon chain containing the specified number of carbon atoms. Examples of alkyl groups include the $C_{1-5}$alkyl groups methyl, ethyl, *n*-propyl, *iso*-propyl, *n*-butyl, *iso*-butyl, *sec*-butyl, *tert*-butyl and *n*-pentyl, *sec*-pentyl and 3-pentyl, in particular the $C_{1-3}$alkyl groups methyl, ethyl, *n*-propyl and *iso*-propyl. Reference to "propyl" includes *n*-propyl and *iso*-propyl, and reference to "butyl" includes *n*-butyl, isobutyl, *sec*-butyl and *tert*-butyl. Examples of Oalkyl groups include the $OC_{1-4}$alkyl groups methoxy, ethoxy, propoxy (which includes *n*-propoxy and *iso*-propoxy) and butoxy (which includes *n*-butoxy, *iso*-butoxy, sec-butoxy and *tert*-butoxy).

The term 'alkylene' as used herein, such as in $C_{0-2}$alkylene$C_{3-5}$cycloalkyl or $C_{1-2}$alkylene$OC_{1-2}$alkyl is a bifunctional straight or a branched fully saturated hydrocarbon chain containing the specified number of carbon atoms. Examples of $C_{0-2}$alkylene groups are where the group is absent (i.e. Co), methylene ($C_1$) and ethylene ($C_2$).

The term 'alkynyl' as used herein, such as in $C_{2-4}$alkynyl is a branched or unbranched unsaturated hydrocarbon chain containing the specified number of carbon atoms, two of which are linked by a carbon-carbon triple bond.

The term 'cycloalkyl' as used herein, such as in $C_{3-5}$cycloalkyl or $C_{3-6}$cycloalkyl, whether alone or forming part of a larger group such as $OC_{3-5}$cycloalkyl or $C_{0-2}$alkylene$C_{3-5}$cycloalkyl is a fully saturated hydrocarbon ring containing the specified number of carbon atoms. Examples of cycloalkyl groups include the $C_{3-6}$cycloalkyl groups cyclopropyl, cyclobutyl, cyclopentyl and cyclohexyl, in particular the $C_{3-5}$cycloalkyl groups cyclopropyl, cyclobutyl and cyclopentyl.

The term 'heterocycloalkyl' as used herein, such as in $C_{3-6}$heterocycloalkyl is a fully saturated hydrocarbon ring containing the specified number of carbon atoms wherein at least one of the carbon atoms is replaced by a heteroatom such as N, S or O. Examples of $C_{3-6}$heterocycloalkyl include those comprising one nitrogen atom such as containing one heteroatom (i.e. nitrogen) or containing two heteroatoms (e.g. two nitrogen atoms or one nitrogen atom and one oxygen atom). Particular examples of $C_{3-6}$heterocycloalkyl comprising one nitrogen atom include pyrrolidinyl, pyrazolidinyl, piperidinyl, piperazinyl, morpholinyl and thiomorpholinyl, especially, pyrrolidinyl and morpholinyl.

The term 'spirocycloalkyl' as used herein, such as in $C_{3-6}$spirocycloalkyl, is a fully saturated hydrocarbon ring containing the specified number of carbon atoms including the secondary carbon atom through which the spirocycloalkyl group is attached. Example of spirocycloalkyl groups include the $C_{3-6}$spirocycloalkyl groups cyclopropyl, cyclobutyl, cyclopentyl or cyclohexyl, in particular the $C_{3-5}$spirocycloalkyl groups cyclopropyl, cyclobutyl or cyclopentyl:

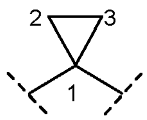
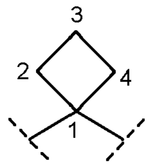
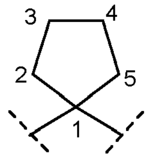

$C_3$ spirocycloalkyl    $C_4$ spirocycloalkyl    $C_5$ spirocycloalkyl

The term 'halo' or 'halogen' as used herein, refers to fluorine, chlorine, bromine or iodine. Particular examples of halo are fluorine and chlorine, especially fluorine.

The term 'haloalkyl' as used herein, such as in $C_{1-4}$haloalkyl, whether alone or forming part of a larger group such as an Ohaloalkyl group, such as in $OC_{1-4}$haloalkyl, is a straight or a branched fully saturated hydrocarbon chain containing the specified number of carbon atoms and at least one halogen atom, such as fluoro or chloro, especially fluoro. An example of haloalkyl is $CF_3$. Examples of Ohaloalkyl include $OCF_3$, $OCHF_2$ and $OCH_2CF_3$.

The term '6-membered aryl' as used herein refers to a phenyl ring.

The term '6-membered heteroaryl' as used herein refers to 6-membered aromatic rings containing at least one heteroatom (e.g. nitrogen). Exemplary 6-membered heteroaryls include one nitrogen atom (pyridinyl), two nitrogen atoms (pyridazinyl, pyrimidinyl or pyrazinyl) and three nitrogen atoms (triazinyl).

The phrase 'in the para position relative to the amide' as used herein, such as in relation to the position of Ar2, means that compounds with the following substructure are formed:

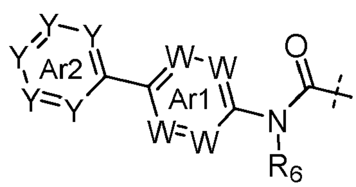

wherein W may be N, CH, $CR_{10}$ or $CR_{11}$, and Y may be N, CH, $CR_{12}$ or $CR_{13}$ as required by the definitions provided for compounds of formula (I).

The terms 'ortho' and 'meta' as used herein, such as when used in respect of defining the position of $R_{12}$ on Ar2 is with respect to Ar1:

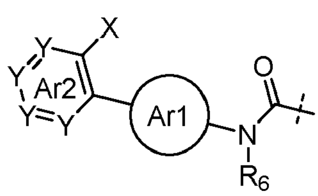

*ortho*

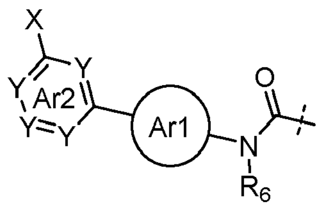

*meta*

In one embodiment of the invention $R_1$ is $C_{1-5}$alkyl. When $R_1$ is $C_{1-5}$alkyl, $R_1$ may be methyl, ethyl, propyl (n-propyl or isopropyl), butyl (n-butyl, isobutyl, sec-butyl or tert-butyl) or pentyl (e.g. n-pentyl, sec-pentyl or 3-pentyl).

In a second embodiment of the invention $R_1$ is $C_{0-2}$alkylene$C_{3-5}$cycloalkyl which cycloalkyl is optionally substituted by $CH_3$. In some embodiments, $R_1$ is $C_{0-2}$alkylene$C_{3-5}$cycloalkyl. In other embodiments, $R_1$ is $C_{0-2}$alkylene$C_{3-5}$cycloalkyl which cycloalkyl is substituted by $CH_3$. $R_1$ may be $C_{3-5}$cycloalkyl, which cycloalkyl is optionally substituted by $CH_3$. $R_1$ may be $C_1$alkylene$C_{3-5}$cycloalkyl, which cycloalkyl is optionally substituted by $CH_3$. $R_1$ may be $C_2$alkylene$C_{3-5}$cycloalkyl, which cycloalkyl is optionally substituted by $CH_3$. $R_1$ may be $C_{0-2}$alkylene$C_3$cycloalkyl, which cycloalkyl is optionally substituted by $CH_3$. $R_1$ may be $C_{0-2}$alkylene$C_4$cycloalkyl, which cycloalkyl is optionally substituted by $CH_3$. $R_1$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene.

In a third embodiment of the invention $R_1$ is $CF_3$.

Suitably $R_1$ is cyclopropyl, cyclopropyl substituted by $CH_3$ at the point of attachment, cyclobutyl, $CH_3$, isopropyl, sec-butyl, tert-butyl or $CF_3$. In particular $R_1$ is cyclopropyl, cyclopropyl substituted by $CH_3$ at the point of attachment, cyclobutyl, $CH_3$, isopropyl, sec-butyl or tert-butyl, especially cyclopropyl, cyclopropyl substituted by $CH_3$ at the point of attachment, cyclobutyl or isopropyl, such as cyclopropyl or cyclopropyl substituted by $CH_3$ at the point of attachment.

In one embodiment $R_3$ is H. In a second embodiment $R_3$ is halo, in particular chloro or fluoro, especially chloro. In a third embodiment $R_3$ is $CH_3$. In a fourth embodiment $R_3$ is $CF_3$. In a fifth embodiment $R_3$ together with $R_5$ forms a 5- or 6-membered cycloalkyl, in particular a 5-membered cycloalkyl.

The phrase '$R_3$ together with $R_5$ forms a 5- or 6-membered cycloalkyl' means that compounds with the following substructure are formed:

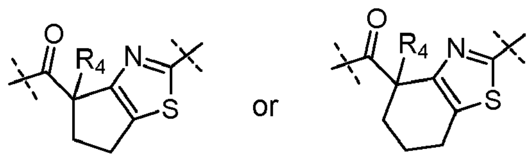

In particular $R_3$ is H, $CH_3$ or $R_3$ together with $R_5$ forms a 5-or 6-membered cycloalkyl, especially H, $CH_3$ or $R_3$ together with $R_5$ forms a 5-membered cycloalkyl, such as $R_3$ is H or $CH_3$.

In one embodiment $R_4$ is O and $R_5$ is absent. In a second embodiment, $R_4$ and $R_5$ together with the carbon atom to which they are attached form a $C_{3-6}$spirocycloalkyl, such as spirocyclopropyl, spirocyclobutyl or spirocyclopentyl. In a third embodiment $R_4$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a fourth embodiment $R_4$ is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl. In a fifth embodiment $R_4$ is $OC_{1-6}$alkyl, in particular $OC_{1-4}$alkyl, such as methoxy, ethoxy, propoxy (n-propoxy or isopropoxy) or butoxy (n-butoxy, isobutoxy, sec-butoxy or tert-butoxy). In a sixth embodiment $R_4$ is $OC_{0-2}$alkylene$C_{3-6}$cycloalkyl, such as $OC_{3-6}$cycloalkyl, $OC_1$alkylene$C_{3-6}$cycloalkyl or $OC_2$alkylene$C_{3-6}$cycloalkyl. In a seventh embodiment $R_4$ is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl. In an eighth embodiment $R_4$ is $C_{1-6}$haloalkyl, in particular $C_{1-4}$haloalkyl. In a ninth embodiment $R_4$ is $OC_{1-6}$haloalkyl, in particular $OC_{1-4}$haloalkyl. In a tenth embodiment $R_4$ is H. In an eleventh embodiment $R_4$ is fluoro.

Suitably $R_4$ is H, $CH_3$, ethyl, isopropyl, fluoro, $OCH_3$, isopropoxy or $CH_2CH_2OCH_3$, in particular H, $CH_3$, ethyl, fluoro, $OCH_3$, isopropoxy or $CH_2CH_2OCH_3$, especially H, $CH_3$, ethyl, $OCH_3$ or $CH_2CH_2OCH_3$.

Suitably $R_4$ may be C=O and $R_5$ is absent.

Suitably $R_4$ and $R_5$ together with the carbon atom to which they are attached form a spirocyclopropyl or spirocyclopentyl, in particular a spirocyclopentyl.

Suitably $R_4$ is H and $R_3$ together with $R_5$ forms a 5- or 6-membered cycloalkyl, in particular a 5-membered cycloalkyl, especially $R_4$ is H and $R_3$ together with $R_5$ forms a 5- or 6-membered cycloalkyl, such as a 5-membered cycloalkyl.

In one embodiment $R_5$ is $C_{1-6}$alkyl, in particular $C_{1-4}$alkyl, such as methyl, ethyl, propyl (n-propyl or isopropyl) or butyl (n-butyl, isobutyl, sec-butyl or tert-butyl). In a second embodiment $R_5$ is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl. In a third embodiment $R_5$ is $OC_{1-6}$alkyl, in particular $OC_{1-4}$alkyl, such as methoxy, ethoxy, propoxy (n-propoxy or isopropoxy) or butoxy (n-butoxy, isobutoxy, sec-butoxy or tert-butoxy). In a fourth embodiment $R_5$ is $OC_{0-2}$alkylene$C_{3-6}$cycloalkyl, such as $OC_{3-6}$cycloalkyl, $OC_1$alkylene$C_{3-6}$cycloalkyl or $OC_2$alkylene$C_{3-6}$cycloalkyl. In a fifth embodiment $R_5$ is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl. In a sixth embodiment $R_5$ is $C_{1-6}$haloalkyl, in particular $C_{1-4}$haloalkyl. In a seventh embodiment $R_5$ is $OC_{1-6}$haloalkyl, in particular $OC_{1-4}$haloalkyl. In an eighth embodiment $R_5$ is H. In a ninth embodiment $R_5$ is fluoro.

Suitably $R_5$ is H, $CH_3$, ethyl, isopropyl or fluoro, in particular $R_5$ is H, methyl or ethyl.

Suitably $R_4$ is H, $CH_3$, ethyl, fluoro, $OCH_3$, propoxy or $CH_2CH_2OCH_3$ and $R_5$ is H, $CH_3$, ethyl or fluoro, in particular $R_4$ is H, $CH_3$, ethyl or $OCH_3$ and $R_5$ is H, methyl or ethyl. For example, $R_4$ and $R_5$ are H, $R_4$ and $R_5$ are methyl, $R_4$ and $R_5$ are ethyl, $R_4$ is $CH_2CH_2OCH_3$ and $R_5$ is H or $R_4$ and $R_5$ are fluoro.

Suitably, when $R_4$ is other than H, methyl, ethyl or fluoro, then $R_5$ is H.

In one embodiment $R_6$ is H. In a second embodiment $R_6$ is $C_{1-3}$alkyl, in particular methyl. In a third embodiment $R_6$ together with $R_{11}$ in the ortho-position to the amide are a $C_2$alkylene chain forming a 5-membered ring.

Suitably $R_6$ is H, methyl or $R_6$ together with $R_{11}$ in the ortho-position to the amide are a $C_2$alkylene chain forming a 5-membered ring. In particular $R_6$ is H or $R_6$ together with $R_{11}$ in the ortho-position to the amide are a $C_2$alkylene chain forming a 5-membered ring, especially $R_6$ is H.

The term '$R_6$ together with $R_{11}$ in the ortho-position to the amide are a $C_2$alkylene chain forming a 5-membered ring' as used herein means that compounds with the following substructure are formed:

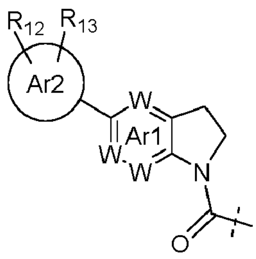

wherein W may be N or $CR_{10}$.

In one embodiment Ar1 is a 6-membered aryl, i.e. phenyl. In a second embodiment Ar1 is a 6-membered heteroaryl, in particular containing one nitrogen atom (pyridyl) or two nitrogen atoms (pyridazinyl, pyrimidinyl or pyrazinyl).

In particular Ar1 is phenyl, 2-pyridyl, 3-pyridyl or 2,6-pyrimidinyl, especially phenyl, 2-pyridyl or 3-pyridyl, such as phenyl or 2-pyridyl. The position numbering for Ar1 is in respect of the amide, with the carbon at the point of attachment designated position 1 and other numbers providing the relative location of the nitrogen atoms, for example:

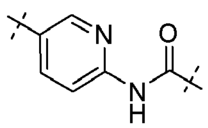
2-pyridyl

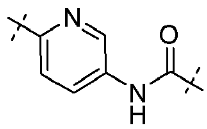
3-pyridyl

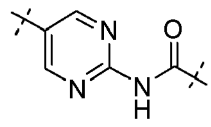
2,6-pyrimidinyl .

In one embodiment $R_{10}$ is H. In a second embodiment $R_{10}$ is halo, for example fluoro or chloro. In a third embodiment $R_{10}$ is $C_{1-2}$alkyl, such as $CH_3$ or ethyl. In a fourth embodiment $R_{10}$ is $OC_{1-2}$alkyl, such as $OCH_3$ or ethoxy. In a fifth embodiment $R_{10}$ is $C_{1-2}$haloalkyl, such as $CF_3$. In a sixth embodiment $R_{10}$ is $OC_{1-2}$haloalkyl, such as $OCF_3$. In a seventh embodiment $R_{10}$ is CN.

Suitably $R_{10}$ is H, fluoro, chloro, $CH_3$, $OCH_3$, ethoxy, $OCF_3$ or CN, in particular H, fluoro, chloro, $CH_3$, $OCH_3$, ethoxy or $OCF_3$, especially or H, fluoro, chloro, $CH_3$, $OCH_3$ or $OCF_3$, such as H, fluoro or $CH_3$.

In one embodiment $R_{11}$ is H. In a second embodiment $R_{11}$ is F. In a third embodiment, $R_{11}$ is $CH_3$. In a fourth embodiment $R_6$ together with $R_{11}$ in the ortho-position to the amide are a $C_2$alkylene chain forming a 5-membered ring.

In one embodiment Ar2 is a 6-membered aryl, i.e. phenyl. In a second embodiment Ar2 is a 6-membered heteroaryl, in particular containing one nitrogen atom (pyridyl) or two nitrogen atoms (pyridazinyl, pyrimidinyl or pyrazinyl).

The position numbering for Ar2 is in respect of the point of attachment to Ar1, for example:

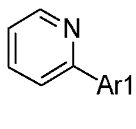
2-pyridyl

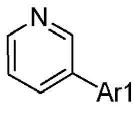
3-pyridyl

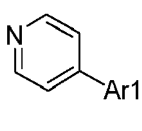
4-pyridyl

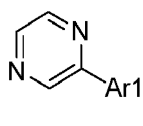
2,5-pyrazinyl

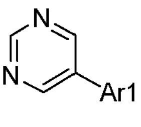
3,5-pyrimidinyl

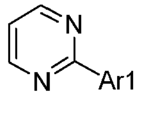
2,6-pyrimidinyl

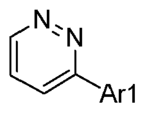
2,3-pyridazinyl

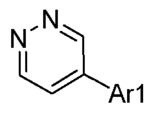
3,4-pyridazinyl .

In particular Ar2 is phenyl, 2-pyridyl, 3-pyridyl, 4-pyridyl, 2,3-pyridazinyl, 3,4-pyridazinyl, 3,5-pyrimidinyl or 2,5-pyrazinyl, especially 3-pyridyl, 3,5-pyrimidinyl or 2,5-pyrazinyl, such as 3-pyridyl or 2,5-pyrazinyl.

In one embodiment $R_{12}$ is H. In a second embodiment $R_{12}$ is halo, for example fluoro or chloro. In a third embodiment $R_{12}$ is $C_{1-4}$alkyl, such as methyl, ethyl, propyl (n-propyl or isopropyl) or butyl (n-butyl, isobutyl, sec-butyl or tert-butyl). In a fourth embodiment $R_{12}$ is $C_{2-4}$alkynyl, such as C≡CH. In a fifth embodiment $R_{12}$ is $C_{0-2}$alkylene$C_{3-5}$cycloalkyl, such as $C_{3-5}$cycloalkyl (e.g. cyclopropyl), $C_1$alkylene$C_{3-5}$cycloalkyl or $C_2$alkylene$C_{3-5}$cycloalkyl. In a sixth embodiment $R_{12}$ is $OC_{1-4}$alkyl, such as $OCH_3$, ethoxy, isopropoxy or n-propoxy. In a seventh embodiment $R_{12}$ is $OC_{0-2}$alkylene$C_{3-5}$cycloalkyl, such as $OC_{3-5}$cycloalkyl (e.g. cyclopropoxy or cyclobutoxy),

$OC_1$alkylene$C_{3-5}$cycloalkyl or $OC_2$alkylene$C_{3-5}$cycloalkyl. In an eighth embodiment $R_{12}$ is $OCH_2CH_2N(CH_3)_2$. In a ninth embodiment $R_{12}$ is $C_{1-4}$alkylOH, such as $CH_2OH$ or $C(CH_3)_2OH$. In a tenth embodiment $R_{12}$ is CN. In an eleventh embodiment $R_{12}$ is $C_{1-3}$alkylene$OC_{1-3}$alkyl. In a twelfth embodiment $R_{12}$ is $C_{1-4}$haloalkyl, such as $CF_3$. In a thirteenth embodiment $R_{12}$ is $OC_{1-4}$haloalkyl, such as $OCF_3$, $OCHF_2$ or $OCH_2CF_3$. In a fourteenth embodiment $R_{12}$ is $N(CH_3)_2$. In a fifteenth embodiment $R_{12}$ is $SO_2CH_3$. In a sixteenth embodiment $R_{12}$ is $C(O)N(CH_3)_2$. In a seventeenth embodiment $R_{12}$ is $NHC(O)C_{1-3}$alkyl such as $NHC(O)CH_3$. In an eighteenth embodiment $R_{12}$ is a $C_{3-6}$heterocycloalkyl comprising one nitrogen located at the point of attachment to Ar2, such as a $C_5$heterocycloalkyl, in particular pyrrolidinyl, or a $C_6$heterocycloalkyl such as morpholinyl.

$R_{12}$ is suitably H, fluoro, chloro, $CH_3$, cyclopropyl, C≡CH, $OCH_3$, ethoxy, *n*-propoxy, isopropoxy, cyclopropoxy, cyclobutoxy, CN, $CF_3$, $OCF_3$, $OCHF_2$, $OCH_2CF_3$, $CH_2OH$, $N(CH_3)_2$, $NHC(O)CH_3$, $SO_2CH_3$, $C(O)N(CH_3)_2$ or pyrrolidinyl, in particular H, fluoro, chloro, $CH_3$, cyclopropyl, C≡CH, $OCH_3$, ethoxy, *n*-propoxy, isopropoxy, cyclopropoxy, cyclobutoxy, CN, $CF_3$, $OCF_3$, $OCHF_2$, $OCH_2CF_3$, $CH_2OH$, $C(O)N(CH_3)_2$ or pyrrolidinyl, especially H, fluoro, chloro, $CH_3$, cyclopropyl, C≡CH, $OCH_3$, ethoxy, *n*-propoxy, isopropoxy, cyclopropoxy, CN, $CF_3$, $OCHF_2$, $OCH_2CF_3$ or pyrrolidinyl, such as H, fluoro, chloro, $CH_3$, C≡CH, $OCH_3$, ethoxy, *n*-propoxy, isopropoxy, cyclopropoxy, CN, $CF_3$, $OCHF_2$ or $OCH_2CF_3$.

Suitably $R_{12}$ is in the meta position of Ar2. Alternatively, $R_{12}$ is in the ortho position of Ar2.

In one embodiment $R_{13}$ is methyl. In a second embodiment $R_{13}$ is H. In a third embodiment $R_{13}$ is methoxy. In a fourth embodiment $R_{13}$ is fluoro.

In one embodiment, Ar1 is 2-pyridyl:

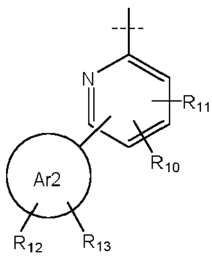

;

$R_1$ is cyclopropyl or sec-butyl; $R_3$ is H or forms a 5-membered ring with $R_5$; $R_4$ and $R_5$ are independently H, $CH_3$, ethyl, $OCH_3$, isopropoxy or $CH_2CH_2OCH_3$, or $R_4$ is H and $R_5$ and $R_3$ join to form a 5-membered cycloalkyl ring; $R_6$ is H; Ar2 is 3-pyridyl, phenyl, 2,5-pyrazinyl or 3,5-pyrimidinyl; $R_{10}$ is H or F and $R_{11}$ is H; $R_{12}$ is in the meta position and is selected from the group consisting of H, F, Cl, $CH_3$, CN, $CF_3$, $OCF_3$, $OCH_2CF_3$, $OCH_3$, ethoxy, n-propoxy, isopropoxy, cyclopropoxy and cyclobutoxy; and $R_{13}$ is H.

Suitably, Ar2 is 3-pyridyl or 2,5-pyrazinyl (e.g. 2,5-pyrazinyl); and $R_{12}$ is selected from the group consisting of H, F, Cl, $CH_3$, CN, $CF_3$, $OCH_2CF_3$, $OCH_3$, ethoxy, n-propoxy, isopropoxy, cyclopropoxy and cyclobutoxy.

More suitably, $R_3$ is H; $R_4$ and $R_5$ are independently H, $CH_3$, ethyl, $OCH_3$, isopropoxy or $CH_2CH_2OMe$ (e.g. H, $CH_3$, ethyl, $CH_2CH_2OCH_3$ or $OCH_3$); and $R_{12}$ is selected from the group consisting of F, Cl, $CH_3$, CN, $CF_3$, $OCH_2CF_3$, $OCH_3$, ethoxy, n-propoxy, isopropoxy and cyclopropoxy (e.g. $CF_3$, $OCH_3$, OEt, O*n*Pr, O*i*Pr and OCyclethoxy, n-propoxy, isopropoxy and cyclopropoxy).

In one embodiment, Ar1 is 3-pyridyl:

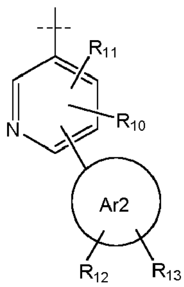

;

$R_1$ is cyclopropyl; $R_3$ is H; $R_4$ and $R_5$ are independently H or $CH_3$ (e.g. $CH_3$); $R_6$ is H; Ar2 is 3-pyridyl, phenyl or 3,5-pyrimidinyl (e.g. 3-pyridyl or 3,5-pyrimidinyl); and $R_{10}$, $R_{11}$, $R_{12}$ and $R_{13}$ are H.

In one embodiment, Ar1 is phenyl:

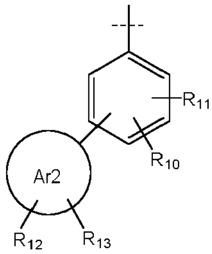

;

$R_1$ is cyclopropyl, cyclopropyl substituted by $CH_3$ at the point of attachment, cyclobutyl, $CH_3$, isopropyl, sec-butyl, tert-butyl or $CF_3$; $R_3$ is H, $CH_3$ or forms a 5- or 6-membered ring with $R_5$; Ar2 is selected from the group consisting of 3-pyridyl, phenyl, 2,5-pyrazinyl, 2-pyridyl, 3,5-pyrimidinyl and 2,3-pyridazinyl; and
when Ar2 is 3-pyridyl,

$R_1$ is cyclopropyl, cyclopropyl substituted by $CH_3$ at the point of attachment, cyclobutyl or $CH_3$;
$R_4$ and $R_5$ are independently selected from the list consisting of H, $CH_3$, ethyl, isopropyl, F, $OCH_3$, isopropoxy and $CH_2CH_2OCH_3$; or

$R_4$ is H and $R_5$ and $R_3$ join to form a 5- or 6-membered cycloalkyl ring; or
$R_4$ is O and $R_5$ is absent;

$R_6$ is H or $CH_3$;
$R_{10}$ is H, F, $CH_3$, Cl, $OCH_3$, ethoxy, $OCF_3$ or CN;
$R_{11}$ is H, F or $CH_3$;
$R_{12}$ is selected from the group consisting of H, $CH_3$, F, Cl, $OCH_3$, ethoxy, n-propoxy, isopropoxy, CN, $CH_2OH$, $CF_3$, $OCHF_2$, $OCH_2CF_3$, $N(CH_3)_2$ and $SO_2CH_3$; and
$R_{13}$ is H; or

when Ar2 is phenyl,

$R_1$ is cyclopropyl;
$R_4$ and $R_5$ are independently H, $CH_3$, ethyl or F;
$R_6$ is H;
$R_{12}$ is H, $OCH_3$, CN and Cl; and
$R_{10}$, $R_{11}$ and $R_{13}$ are H; or

when Ar2 is 2,5-pyrazinyl,

$R_1$ is cyclopropyl, cyclopropyl substituted by $CH_3$ at the point of attachment, cyclobutyl, isopropyl, sec-butyl, tert-butyl or $CF_3$;
$R_4$ and $R_5$ are independently H, $CH_3$, ethyl, isopropyl, $OCH_3$ and $CH_2CH_2OCH_3$ or join together to form a $C_5$spirocycloalkyl ring;
$R_6$ is H or $R_6$ together with $R_{11}$, when in the ortho-position to the amide, is covalently linked by an alkylene chain to form a 5-membered ring;
$R_{10}$ is H, F, $OCH_3$ or CN;
$R_{11}$ is H or when in the ortho-position to the amide, together with $R_6$ is covalently linked by an alkylene chain to form a 5-membered ring; and
$R_{12}$ is selected from the group consisting of H, $CH_3$, cyclopropyl, C≡CH, Cl, $CF_3$, $OCH_2CF_3$, $CH_2OH$, CN, $OCH_3$, ethoxy, isopropoxy, cyclopropoxy, n-propoxy, cyclobutoxy, $C(O)N(CH_3)_2$, $NHC(O)CH_3$ and pyrrolidinyl;
$R_{13}$ is H or $CH_3$; or

when Ar2 is 2-pyridyl,

$R_1$ is cyclopropyl;
$R_4$ and $R_5$ are $CH_3$;
$R_6$ is H;
$R_{12}$ is $OCH_3$, Cl or $CF_3$; and

$R_{10}$ and $R_{11}$ are H;
$R_{13}$ is H; or

when Ar2 is 3,5-pyrimidinyl,

$R_1$ is cyclopropyl;
$R_4$ and $R_5$ are independently H, $CH_3$ or isopropyl; and
$R_6$, $R_{10}$, $R_{11}$, $R_{12}$ and $R_{13}$ are H; or

when Ar2 is 2,3-pyridazinyl,

$R_1$ is cyclopropyl;
$R_4$ and $R_5$ are $CH_3$; and
$R_6$, $R_{10}$, $R_{11}$, $R_{12}$ and $R_{13}$ are H.

In this embodiment, suitably Ar2 is selected from the group consisting of 3-pyridyl, 2,5-pyrazinyl, 2-pyridyl and 3,5-pyrimidinyl; and
when Ar2 is 3-pyridyl,

$R_4$ and $R_5$ are independently selected from the group consisting of H, $CH_3$, ethyl, isopropyl, F, $OCH_3$, isopropoxy and $CH_2CH_2OCH_3$;

or $R_4$ is H and $R_5$ and $R_3$ join to form a 5- or 6-membered cycloalkyl ring;

$R_{10}$ is H, F, Cl, $CH_3$, $OCH_3$ or $OCF_3$ (e.g. H, F or $CH_3$); and
$R_{12}$ is selected from the group consisting of H, $CH_3$, F, Cl, $CF_3$, $OCHF_2$, $OCH_2CF_3$, CN, $OCH_3$, ethoxy, n-propoxy and isopropoxy; or

when Ar2 is 2-pyridyl,

$R_{12}$ is Cl or $CF_3$; or

when Ar2 is 3,5-pyrimidinyl,

$R_4$ and $R_5$ are independently H or $CH_3$; or

when Ar2 is 2,5-pyrazinyl,

$R_{10}$ is H, F or $OCH_3$;
$R_{12}$ is selected from the group consisting of H, $CH_3$, cyclopropyl, C≡CH, Cl, $CF_3$, $OCH_2CF_3$, $CH_2OH$, CN, $OCH_3$, ethoxy, isopropoxy, cyclopropoxy, n-propoxy, cyclobutoxy, $C(O)N(CH_3)_2$ and pyrrolidinyl;
$R_{13}$ is H or $CH_3$.

More suitably, $R_1$ is cyclopropyl, cyclopropyl substituted by $CH_3$ at the point of attachment, cyclobutyl or isopropyl (e.g. cyclopropyl or cyclopropyl substituted by $CH_3$ at the point of attachment); $R_3$ is H or forms a 5- or 6-membered ring with $R_5$ (e.g. H); Ar2 is 3-pyridyl, 2,5-pyrazinyl or 3,5-pyrimidinyl (e.g. 3-pyridyl or 2,5-pyrazinyl); and
when Ar2 is 3-pyridyl,

$R_4$ and $R_5$ are independently selected from the group consisting of H, F, $CH_3$, ethyl, $OCH_3$, isopropoxy and $CH_2CH_2OCH_3$ (e.g. H, $CH_3$, ethyl or $OCH_3$),

or $R_4$ is H and $R_5$ and $R_3$ join to form a 5-membered cycloalkyl ring; and

$R_6$ is H; and
$R_{11}$ is H or $CH_3$ (e.g. H);
$R_{12}$ is selected from the group consisting of H, $CH_3$, F, Cl, $CF_3$, $OCHF_2$, CN, $OCH_3$, ethoxy, isopropoxy and n-propoxy (e.g. H, F, Cl, $CF_3$, $OCHF_2$, CN, $CH_3$, $OCH_3$, ethoxy or isopropoxy); or

when Ar2 is 3,5-pyrimidinyl,

$R_4$ and $R_5$ are $CH_3$; or

when Ar2 is 2,5-pyrazinyl,

$R_4$ and $R_5$ are independently H, $CH_3$, ethyl, $OCH_3$ and $CH_2CH_2OCH_3$ or join together to form a $C_5$spirocycloalkyl ring (e.g. H, $CH_3$, ethyl or $OCH_3$);
$R_6$ is H or $R_6$ together with $R_{11}$, when in the ortho-position to the amide, is covalently linked by an alkylene chain to form a 5-membered ring (e.g. H);
$R_{10}$ is H or F;
$R_{12}$ is selected from the group consisting of H, $CH_3$, cyclopropyl, C=CH, Cl, $CF_3$, $OCH_2CF_3$, $OCH_3$, ethoxy, isopropoxy, cyclopropoxy, n-propoxy and pyrrolidinyl (e.g. H, C=CH, Cl, $CF_3$, $OCH_2CF_3$, $OCH_3$, ethoxy, isopropoxy or cyclopropoxy); and
$R_{13}$ is H.

In one embodiment, Ar1 is 2,6-pyrimidinyl:

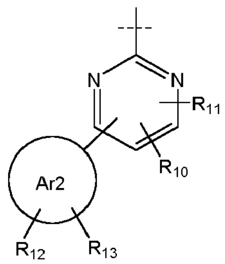
;

$R_1$ is cyclopropyl; $R_3$ is H; $R_4$ and $R_5$ are $CH_3$; $R_6$ is H; $R_{10}$ is H; $R_{11}$ is H; Ar2 is 3-pyridyl; $R_{12}$ is H, F, CN, $CF_3$ or $OCH_3$; and $R_{13}$ is H.

Throughout the specification Ar1 and Ar2 may be depicted as follows:

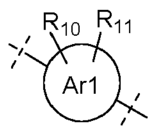 ; 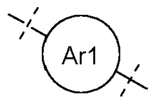 ; 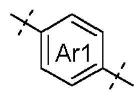 ; Ar1

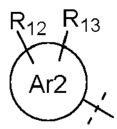 ; 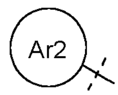 ; 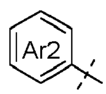 ; Ar2

All depictions with respect to Ar1 are equivalent and all depictions with respect to Ar2 are equivalent, unless the context requires otherwise, depictions of Ar1 and Ar2 should not be taken to exclude the presence of heteroatoms or substitutions.

The present invention provides the compounds described in any one of Examples T1 to T322.

The present invention provides the following compounds:

N-([1,1'-biphenyl]-4-yl)-2-(2-(methylsulfonamido)thiazol-4-yl)acetamide;
N-([1,1'-biphenyl]-4-yl)-2-(2-(cyclopropanesulfonamido)thiazol-4-yl)acetamide;
2-(2-(cyclopropanesulfonamido)thiazol-4-yl)-2-ethyl-N-(5-(pyrazin-2-yl)pyridin-2-yl)butanamide;
2-(2-(cyclopropanesulfonamido)thiazol-4-yl)-2-methyl-N-(4-(pyrimidin-2-yl)phenyl)propanamide;
2-(2-(cyclopropanesulfonamido)thiazol-4-yl)-N-(4-(pyridin-3-yl)phenyl)butanamide (racemic);
2-(2-(cyclopropanesulfonamido)thiazol-4-yl)-N-(4-(pyridin-3-yl)phenyl)butanamide (enantiomer 1);
2-(2-(cyclopropanesulfonamido)thiazol-4-yl)-N-(4-(pyridin-3-yl)phenyl)butanamide (enantiomer 2);
2-(2-(cyclopropanesulfonamido)thiazol-4-yl)-N-(4-(5-fluoropyridin-3-yl)phenyl)butanamide (racemic);

2-(2-(cyclopropanesulfonamido)thiazol-4-yl)-N-(4-(5-fluoropyridin-3-yl)phenyl)butanamide (enantiomer 1);
2-(2-(cyclopropanesulfonamido)thiazol-4-yl)-N-(4-(5-fluoropyridin-3-yl)phenyl)butanamide (enantiomer 2);
2-(2-(cyclopropanesulfonamido)thiazol-4-yl)-3-methyl-N-(4-(pyrimidin-5-yl)phenyl)butanamide;
2-(2-(cyclopropanesulfonamido)thiazol-4-yl)-3-methyl-N-(4-(pyridin-3-yl)phenyl)butanamide;
2-(2-(cyclopropanesulfonamido)thiazol-4-yl)-N-(4-(5-methoxypyridin-3-yl)phenyl)-2-methylpropanamide;
N-(2-chloro-4-(pyridin-3-yl)phenyl)-2-(2-(cyclopropanesulfonamido)thiazol-4-yl)acetamide;
2-(2-(cyclopropanesulfonamido)-5-methylthiazol-4-yl)-2-methyl-N-(4-(6-(trifluoromethyl)pyrazin-2-yl)phenyl)propenamide;
2-(2-(cyclopropanesulfonamido)thiazol-4-yl)-2-methyl-N-(4-(pyrimidin-5-yl)phenyl)propenamide;
6-(4-(2-(2-(cyclopropanesulfonamido)thiazol-4-yl)-2-methylpropanamido)phenyl)-N,N-dimethylpyrazine-2-carboxamide;
N-(5-(5-cyanopyridin-3-yl)pyrimidin-2-yl)-2-(2-(cyclopropanesulfonamido)thiazol-4-yl)-2-methylpropanamide;
N-([1,1'-biphenyl]-4-yl)-2-(5-chloro-2-(cyclopropanesulfonamido)thiazol-4-yl)acetamide;
2-(2-(cyclopropanesulfonamido)thiazol-4-yl)-N-(4-(6-ethynylpyrazin-2-yl)phenyl)butanamide;
2-(2-(cyclopropanesulfonamido)thiazol-4-yl)-N-(6-(pyrimidin-5-yl)pyridin-3-yl)acetamide;
2-(2-(cyclopropanesulfonamido)thiazol-4-yl)-N-(5-phenylpyridin-2-yl)acetamide;
2-(2-(cyclopropanesulfonamido)thiazol-4-yl)-N-(4'-fluoro-[1,1'-biphenyl]-4-yl)acetamide;
2-(2-(cyclopropanesulfonamido)thiazol-4-yl)-N-methyl-N-(4-(pyridin-3-yl)phenyl)acetamide;
N-([2,3'-bipyridin]-5-yl)-2-(2-(cyclopropanesulfonamido)thiazol-4-yl)propenamide;
2-(2-(cyclopropanesulfonamido)thiazol-4-yl)-N-(3'-methoxy-[1,1'-biphenyl]-4-yl)-2-methylpropanamide;
2-(2-(cyclopropanesulfonamido)thiazol-4-yl)-2-methyl-N-(4-(pyridin-3-yl)phenyl)propenamide;
2-(2-(cyclopropanesulfonamido)thiazol-4-yl)-2-methyl-N-(4-(5-methylpyridin-3-yl)phenyl)propenamide;
2-(2-(cyclopropanesulfonamido)thiazol-4-yl)-2-methyl-N-(4-(pyridazin-4-yl)phenyl)propenamide;
2-(2-(cyclopropanesulfonamido)thiazol-4-yl)-2-methyl-N-(4-(pyrazin-2-yl)phenyl)propenamide;
2-(2-(cyclopropanesulfonamido)thiazol-4-yl)-N-(4-(6-methoxypyrazin-2-yl)phenyl)butanamide;
N-(3-cyano-4-(pyrazin-2-yl)phenyl)-2-(2-(cyclopropanesulfonamido)thiazol-4-yl)-2-methylpropanamide;
2-(2-(cyclopropanesulfonamido)thiazol-4-yl)-2-methyl-N-(5-(6-(trifluoromethyl)pyrazin-2-yl)pyridin-2-yl)propenamide;
2-(2-(cyclopropanesulfonamido)thiazol-4-yl)-N-(2,3-difluoro-4-(pyridin-3-yl)phenyl)-2-methylpropanamide;
2-(2-(cyclopropanesulfonamido)thiazol-4-yl)-2-methyl-N-(5-(pyridin-3-yl)pyrimidin-2-yl)propenamide;
2-(2-(cyclopropanesulfonamido)thiazol-4-yl)-2-methyl-N-(5-(6-propoxypyrazin-2-yl)pyridin-2-yl)propenamide;
2-(2-(cyclopropanesulfonamido)thiazol-4-yl)-N-(3-fluoro-4-(pyrazin-2-yl)phenyl)-2-methylpropanamide;
2-(2-(cyclopropanesulfonamido)thiazol-4-yl)-2-methyl-N-(4-(pyridin-3-yl)-2-(trifluoromethoxy)phenyl)propenamide;
N-(2-chloro-4-(pyridin-3-yl)phenyl)-2-(2-(cyclopropanesulfonamido)thiazol-4-yl)-2-methylpropanamide;
2-(2-(cyclopropanesulfonamido)thiazol-4-yl)-N-(2-fluoro-4-(pyridin-3-yl)phenyl)-2-methylpropanamide ;
2-(2-(cyclopropanesulfonamido)thiazol-4-yl)-N-(3-methoxy-4-(pyrazin-2-yl)phenyl)-2-methylpropanamide;
2-(2-(cyclopropanesulfonamido)thiazol-4-yl)-N-(4-(2-methoxypyridin-3-yl)phenyl)-2-methylpropanamide;
2-(2-(cyclopropanesulfonamido)thiazol-4-yl)-N-(4-(5-(hydroxymethyl)pyridin-3-yl)phenyl)acetamide;
2-(2-(cyclopropanesulfonamido)thiazol-4-yl)-N-(4-(6-methoxypyridin-3-yl)phenyl)-2-methylpropanamide;
N-(4-(5-cyanopyridin-3-yl)phenyl)-2-(2-(cyclopropanesulfonamido)thiazol-4-yl)-2-methylpropanamide;
2-(2-(cyclopropanesulfonamido)thiazol-4-yl)-2-methyl-N-(4-(5-(trifluoromethyl)pyridin-3-yl)phenyl)propenamide;
2-(2-(cyclopropanesulfonamido)thiazol-4-yl)-2-methyl-N-(4-(5-(methylsulfonyl)pyridin-3-yl)phenyl)propenamide;
2-(2-(cyclopropanesulfonamido)thiazol-4-yl)-N-(2-methoxy-4-(5-methoxypyridin-3-yl)phenyl)-2-methylpropanamide;
2-(2-(cyclopropanesulfonamido)thiazol-4-yl)-2-methyl-N-(5'-(trifluoromethyl)-[3,3'-bipyridin]-6-yl)propenamide;
2-(2-(cyclopropanesulfonamido)thiazol-4-yl)-N-(4-(6-ethoxypyrazin-2-yl)phenyl)-2-methylpropanamide;
2-(2-(cyclopropanesulfonamido)thiazol-4-yl)-2-methyl-N-(4-(6-morpholinopyrazin-2-yl)phenyl)propenamide;
N-(4-(6-cyclobutoxypyrazin-2-yl)phenyl)-2-(2-(cyclopropanesulfonamido)thiazol-4-yl)-2-methylpropanamide;
2-(2-(cyclopropanesulfonamido)thiazol-4-yl)-2-methyl-N-(4-(6-propoxypyrazin-2-yl)phenyl)propenamide;
2-(2-(cyclopropanesulfonamido)thiazol-4-yl)-N-(2-fluoro-4-(5-methoxypyridin-3-yl)phenyl)-2-methylpropanamide;
2-(2-(cyclopropanesulfonamido)thiazol-4-yl)-2-methoxy-N-(4-(6-methoxypyrazin-2-yl)phenyl)acetamide;
N-(4-(5-chloropyridin-3-yl)phenyl)-2-(2-(cyclopropanesulfonamido)thiazol-4-yl)-2-isopropoxyacetamide;
2-(2-(cyclopropanesulfonamido)thiazol-4-yl)-4-methoxy-N-(5-(6-(trifluoromethyl)pyrazin-2-yl)pyridin-2-yl)butanamide;
N-([1,1'-biphenyl]-4-yl)-2-(2-(cyclopropanesulfonamido)thiazol-4-yl)-2,2-difluoroacetamide;
2-(2-(cyclobutanesulfonamido)thiazol-4-yl)-N-(4-(6-methoxypyrazin-2-yl)phenyl)acetamide;
N-([3,3'-bipyridin]-6-yl)-2-(2-(cyclopropanesulfonamido)thiazol-4-yl)butanamide;
2-(2-(cyclopropanesulfonamido)thiazol-4-yl)-2-methyl-N-(5-phenylpyridin-2-yl)propenamide;

2-(2-(cyclopropanesulfonamido)thiazol-4-yl)-N-(5-(pyrimidin-5-yl)pyridin-2-yl)acetamide;
N-([3,3'-bipyridin]-6-yl)-2-(2-(cyclopropanesulfonamido)thiazol-4-yl)acetamide;
2-(2-(cyclopropanesulfonamido)thiazol-4-yl)-N-(6-phenylpyridin-3-yl)acetamide;
N-([2,3'-bipyridin]-5-yl)-2-(2-(cyclopropanesulfonamido)thiazol-4-yl)acetamide;
2-(2-(cyclopropanesulfonamido)thiazol-4-yl)-2-methyl-N-(4-(pyridazin-3-yl)phenyl)propenamide;
2-(2-(cyclopropanesulfonamido)thiazol-4-yl)-N-(4-(pyridazin-4-yl)phenyl)butanamide;
2-(2-(cyclopropanesulfonamido)thiazol-4-yl)-N-(4-(pyrazin-2-yl)phenyl)butanamide;
N-(4-(5-chloropyridin-3-yl)phenyl)-2-(2-(cyclopropanesulfonamido)thiazol-4-yl)-4-methoxybutanamide;
2-(2-(cyclopropanesulfonamido)thiazol-4-yl)-N-(4-(5-fluoropyridin-3-yl)phenyl)butanamide;
2-(2-(cyclopropanesulfonamido)thiazol-4-yl)-N-(4-(6-(trifluoromethyl)pyrazin-2-yl)phenyl)butanamide;
2-(2-(cyclopropanesulfonamido)thiazol-4-yl)-3-methyl-N-(4-(pyrazin-2-yl)phenyl)butanamide;
2-(2-(cyclopropanesulfonamido)thiazol-4-yl)-N-(4-(6-propoxypyrazin-2-yl)phenyl)butanamide;
2-(2-(cyclopropanesulfonamido)thiazol-4-yl)-N-(4-(6-isopropoxypyrazin-2-yl)phenyl)butanamide;
2-(2-(cyclopropanesulfonamido)thiazol-4-yl)-N-(4-(6-cyclopropoxypyrazin-2-yl)phenyl)butanamide;
N-(4-(6-chloropyrazin-2-yl)phenyl)-2-(2-(cyclopropanesulfonamido)thiazol-4-yl)butanamide;
N-(4-(6-cyanopyrazin-2-yl)phenyl)-2-(2-(cyclopropanesulfonamido)thiazol-4-yl)butanamide;
2-(2-(cyclopropanesulfonamido)thiazol-4-yl)-N-(4-(6-(trifluoromethyl)pyrazin-2-yl)phenyl)butanamide;
2-(2-(cyclopropanesulfonamido)thiazol-4-yl)-N-(4-(6-methoxypyrazin-2-yl)phenyl)acetamide;
2-(2-(cyclopropanesulfonamido)thiazol-4-yl)-N-(4-(pyrazin-2-yl)phenyl)acetamide;
N-([1,1'-biphenyl]-4-yl)-2-(cyclopropanesulfonamido)-4,5,6,7-tetrahydrobenzo[d]thiazole-4-carboxamide;
2-(cyclopropanesulfonamido)-N-(4-(pyridin-3-yl)phenyl)-4,5,6,7-tetrahydrobenzo[d]thiazole-4-carboxamide;
N-([1,1'-biphenyl]-4-yl)-2-(2-(cyclopropanesulfonamido)thiazol-4-yl)butanamide;
N-([1,1'-biphenyl]-4-yl)-2-(2-(cyclopropanesulfonamido)thiazol-4-yl)-3-methylbutanamide;
N-(3'-chloro-[1,1'-biphenyl]-4-yl)-2-(2-(cyclopropanesulfonamido)thiazol-4-yl)-2-methylpropanamide;
N-(3'-cyano-[1,1'-biphenyl]-4-yl)-2-(2-(cyclopropanesulfonamido)thiazol-4-yl)-2-methylpropanamide;
2-(2-(cyclopropanesulfonamido)thiazol-4-yl)-2,2-difluoro-N-(4-(pyridin-3-yl)phenyl)acetamide;
N-(4-(5-fluoropyridin-3-yl)phenyl)-2-(2-(cyclopropanesulfonamido)thiazol-4-yl)butanamide;
2-(2-(cyclopropanesulfonamido)thiazol-4-yl)-2-ethyl-N-(4-(pyridin-3-yl)phenyl)butanamide;
N-(4-(5-cyanopyridin-3-yl)phenyl)-2-(2-(cyclopropanesulfonamido)thiazol-4-yl)-2-ethylbutanamide;
2-(2-(cyclopropanesulfonamido)thiazol-4-yl)-N-(4-(5-ethoxypyridin-3-yl)phenyl)propenamide;
N-(4-(5-chloropyridin-3-yl)phenyl)-2-(2-(cyclopropanesulfonamido)thiazol-4-yl)propenamide;
2-(2-(cyclopropanesulfonamido)thiazol-4-yl)-N-(4-(5-ethoxypyridin-3-yl)phenyl)butanamide;
N-([1,1'-biphenyl]-4-yl)-2-(2-(cyclopropanesulfonamido)thiazol-4-yl)-2-methylpropanamide;
2-(2-(cyclopropanesulfonamido)thiazol-4-yl)-N-(4-(4-methylpyridin-3-yl)phenyl)acetamide;
2-(2-(cyclopropanesulfonamido)thiazol-4-yl)-N-(4-(5-methylpyridin-3-yl)phenyl)acetamide;
2-(2-(cyclopropanesulfonamido)thiazol-4-yl)-N-(4-(2-methylpyridin-3-yl)phenyl)acetamide;
2-(2-(cyclopropanesulfonamido)thiazol-4-yl)-N-(4-(6-methylpyridin-3-yl)phenyl)acetamide;
2-(2-(cyclopropanesulfonamido)thiazol-4-yl)-N-(4-(pyridin-3-yl)phenyl)propenamide;
2-(2-(cyclopropanesulfonamido)thiazol-4-yl)-2-methyl-N-(4-(2-methylpyridin-3-yl)phenyl)propenamide;
2-(2-(cyclopropanesulfonamido)thiazol-4-yl)-2-oxo-N-(4-(pyridin-3-yl)phenyl)acetamide;
2-(2-(cyclopropanesulfonamido)thiazol-4-yl)-2-methyl-N-(4-(6-methylpyridin-3-yl)phenyl)propenamide;
2-(2-(cyclopropanesulfonamido)thiazol-4-yl)-N-(4-(6-(trifluoromethyl)pyrazin-2-yl)phenyl)butanamide;
2-(2-(cyclopropanesulfonamido)thiazol-4-yl)-2-methoxy-N-(4-(6-methoxypyrazin-2-yl)phenyl)acetamide (enantiomer 1);
2-(2-(cyclopropanesulfonamido)thiazol-4-yl)-2-methoxy-N-(4-(6-methoxypyrazin-2-yl)phenyl)acetamide (enantiomer 2);
2-(2-(cyclopropanesulfonamido)thiazol-4-yl)-2-methyl-N-(5-(6-(2,2,2-trifluoroethoxy)pyrazin-2-yl)pyridin-2-yl)propenamide;
2-(2-(cyclopropanesulfonamido)thiazol-4-yl)-N-(3-fluoro-5-(pyrazin-2-yl)pyridin-2-yl)-2-methylpropanamide;
2-(2-(cyclopropanesulfonamido)thiazol-4-yl)-N-(5-(6-ethoxypyrazin-2-yl)-3-fluoropyridin-2-yl)-2-methylpropanamide;
2-(2-(cyclopropanesulfonamido)thiazol-4-yl)-N-(3-fluoro-5-(6-(trifluoromethyl)pyrazin-2-yl)pyridin-2-yl)-2-methylpropanamide;
N-(5-(6-cyanopyrazin-2-yl)-3-fluoropyridin-2-yl)-2-(2-(cyclopropanesulfonamido)thiazol-4-yl)-2-methylpropanamide;
2-(2-(cyclopropanesulfonamido)thiazol-4-yl)-2-methyl-N-(5'-(2,2,2-trifluoroethoxy)-[3,3'-bipyridin]-6-yl)propenamide;
2-(2-(cyclopropanesulfonamido)thiazol-4-yl)-N-(5'-(difluoromethoxy)-[3,3'-bipyridin]-6-yl)-2-methylpropanamide;

N-([2,3'-bipyridin]-5-yl)-2-(2-(cyclopropanesulfonamido)thiazol-4-yl)-2-methylpropanamide;
2-(2-(cyclopropanesulfonamido)thiazol-4-yl)-2-methyl-N-(6-(pyrimidin-5-yl)pyridin-3-yl)propenamide;
2-(2-(cyclopropanesulfonamido)thiazol-4-yl)-N-(4-(5-(difluoromethoxy)pyridin-3-yl)phenyl)-2-methylpropanamide;
2-(2-(cyclopropanesulfonamido)thiazol-4-yl)-2-ethyl-N-(4-(6-methoxypyrazin-2-yl)phenyl)butanamide;
N-(4-(5-chloropyridin-3-yl)phenyl)-2-(2-(cyclopropanesulfonamido)thiazol-4-yl)-2-ethylbutanamide;
2-(2-(cyclopropanesulfonamido)thiazol-4-yl)-2-ethyl-N-(2-fluoro-4-(pyridin-3-yl)phenyl)butanamide;
2-(2-(cyclopropanesulfonamido)thiazol-4-yl)-2-ethyl-N-(4-(pyrazin-2-yl)phenyl)butanamide;
2-(2-(cyclopropanesulfonamido)thiazol-4-yl)-2-ethyl-N-(4-(6-propoxypyrazin-2-yl)phenyl)butanamide;
2-(2-(cyclopropanesulfonamido)thiazol-4-yl)-N-(2-fluoro-4-(6-(trifluoromethyl)pyrazin-2-yl)phenyl)-2-methylpropanamide;
2-(2-(cyclopropanesulfonamido)thiazol-4-yl)-N-(4-(5-ethoxypyridin-3-yl)-2-fluorophenyl)-2-methylpropanamide;
2-(2-(cyclopropanesulfonamido)thiazol-4-yl)-N-(2-fluoro-4-(5-fluoropyridin-3-yl)phenyl)-2-methylpropanamide;
2-(2-(cyclopropanesulfonamido)thiazol-4-yl)-2-methyl-N-(4-(6-(2,2,2-trifluoroethoxy)pyrazin-2-yl)phenyl)propenamide;
N-(4-(5-chloropyridin-3-yl)-2-fluorophenyl)-2-(2-(cyclopropanesulfonamido)thiazol-4-yl)-2-methylpropanamide;
N-(4-(5-cyanopyridin-3-yl)-2-fluorophenyl)-2-(2-(cyclopropanesulfonamido)thiazol-4-yl)-2-methylpropanamide;
1-(2-(cyclopropanesulfonamido)thiazol-4-yl)-N-(4-(6-methoxypyrazin-2-yl)phenyl)cyclopentane-1-carboxamide;
2-(2-(cyclopropanesulfonamido)thiazol-4-yl)-N-(2-fluoro-4-(6-(2,2,2-trifluoroethoxy)pyrazin-2-yl)phenyl)-2-methylpropanamide;
2-(2-(cyclopropanesulfonamido)thiazol-4-yl)-N-(2-fluoro-4-(5-(2,2,2-trifluoroethoxy)pyridin-3-yl)phenyl)-2-methylpropanamide;
2-(2-(cyclopropanesulfonamido)thiazol-4-yl)-N-(2-fluoro-4-(5-(trifluoromethyl)pyridin-3-yl)phenyl)-2-methylpropanamide;
2-(2-(cyclopropanesulfonamido)thiazol-4-yl)-N-(4-(6-ethoxypyrazin-2-yl)-2-fluorophenyl)-2-methylpropanamide;
2-(2-(cyclopropanesulfonamido)thiazol-4-yl)-2-methyl-N-(4-(5-(2,2,2-trifluoroethoxy)pyridin-3-yl)phenyl)propenamide;
2-(2-(cyclopropanesulfonamido)thiazol-4-yl)-N-(4-(6-ethynylpyrazin-2-yl)phenyl)-2-methylpropanamide;
2-(2-(cyclopropanesulfonamido)thiazol-4-yl)-N-(4-(6-ethoxypyrazin-2-yl)-2-methylphenyl)-2-methylpropanamide;
N-(4-(6-chloropyrazin-2-yl)-2-methylphenyl)-2-(2-(cyclopropanesulfonamido)thiazol-4-yl)-2-methylpropanamide;
2-(2-(cyclopropanesulfonamido)thiazol-4-yl)-N-(4-(5-(difluoromethoxy)pyridin-3-yl)-2-fluorophenyl)-2-methylpropanamide;
2-(2-(cyclopropanesulfonamido)thiazol-4-yl)-2-methyl-N-(5-(pyrazin-2-yl)pyridin-2-yl)propenamide;
N-(5-(6-cyclobutoxypyrazin-2-yl)pyridin-2-yl)-2-(2-(cyclopropanesulfonamido)thiazol-4-yl)-2-methylpropanamide;
2-(2-(cyclopropanesulfonamido)thiazol-4-yl)-N-(5-(6-cyclopropoxypyrazin-2-yl)pyridin-2-yl)-2-methylpropanamide;
2-(2-(cyclopropanesulfonamido)thiazol-4-yl)-N-(5-(6-isopropoxypyrazin-2-yl)pyridin-2-yl)-2-methylpropanamide;
N-([3,3'-bipyridin]-6-yl)-2-(2-(cyclopropanesulfonamido)thiazol-4-yl)-2-ethylbutanamide;
2-(2-(cyclopropanesulfonamido)thiazol-4-yl)-N-(5'-ethoxy-[3,3'-bipyridin]-6-yl)-2-ethylbutanamide;
2-(2-(cyclopropanesulfonamido)thiazol-4-yl)-2-methyl-N-(5'-propoxy-[3,3'-bipyridin]-6-yl)propenamide;
2-(2-(cyclopropanesulfonamido)thiazol-4-yl)-2-ethyl-N-(5-(6-(trifluoromethyl)pyrazin-2-yl)pyridin-2-yl)butanamide;
N-([3,3'-bipyridin]-6-yl)-2-(2-(cyclopropanesulfonamido)thiazol-4-yl)-2-methylpropanamide;
2-(2-(cyclopropanesulfonamido)thiazol-4-yl)-N-(2-methoxy-4-(pyridin-3-yl)phenyl)-2-methylpropanamide;
2-(2-(cyclopropanesulfonamido)thiazol-4-yl)-N-(3-methoxy-4-(pyridin-3-yl)phenyl)-2-methylpropanamide;
2-(2-(cyclopropanesulfonamido)thiazol-4-yl)-N-(3-fluoro-4-(pyridin-3-yl)phenyl)-2-methylpropanamide;
N-(3-cyano-4-(pyridin-3-yl)phenyl)-2-(2-(cyclopropanesulfonamido)thiazol-4-yl)-2-methylpropanamide;
N-(3-chloro-4-(pyridin-3-yl)phenyl)-2-(2-(cyclopropanesulfonamido)thiazol-4-yl)-2-methylpropanamide;
N-(4-(6-cyanopyrazin-2-yl)phenyl)-2-(2-(cyclopropanesulfonamido)thiazol-4-yl)-2-methylpropanamide;
N-(4-(6-chloropyrazin-2-yl)phenyl)-2-(2-(cyclopropanesulfonamido)thiazol-4-yl)-2-methylpropanamide;
2-(2-(cyclopropanesulfonamido)thiazol-4-yl)-2-ethyl-N-(4-(5-fluoropyridin-3-yl)phenyl)butanamide;
2-(2-(cyclopropanesulfonamido)thiazol-4-yl)-2-methyl-N-(4-(5-propoxypyridin-3-yl)phenyl)propenamide;
2-(2-(cyclopropanesulfonamido)thiazol-4-yl)-N-(4-(5-isopropoxypyridin-3-yl)phenyl)-2-methylpropanamide;
2-(2-(cyclopropanesulfonamido)thiazol-4-yl)-N-(2-fluoro-4-(5-isopropoxypyridin-3-yl)phenyl)-2-methylpropanamide;
N-(4-(6-chloropyrazin-2-yl)phenyl)-2-(2-(cyclopropanesulfonamido)thiazol-4-yl)-2-ethylbutanamide;
N-(4-(6-cyanopyrazin-2-yl)phenyl)-2-(2-(cyclopropanesulfonamido)thiazol-4-yl)-2-ethylbutanamide;
4-(6-chloropyrazin-2-yl)-N-(1-(2-(cyclopropanesulfonamido)thiazol-4-yl)propyl)benzamide;
2-methyl-2-(2-(methylsulfonamido)thiazol-4-yl)-N-(4-(pyridin-3-yl)phenyl)propenamide;
2-(2-(cyclopropanesulfonamido)thiazol-4-yl)-N,2-dimethyl-N-(4-(pyridin-3-yl)phenyl)propenamide;
N-(1-(2-(cyclopropanesulfonamido)thiazol-4-yl)propyl)-4-(5-(trifluoromethyl)pyridin-3-yl)benzamide (enantiomer 1);

N-(1-(2-(cyclopropanesulfonamido)thiazol-4-yl)propyl)-4-(5-(trifluoromethyl)pyridin-3-yl)benzamide (enantiomer 2);
N-(1-(2-(cyclopropanesulfonamido)thiazol-4-yl)propyl)-4-(5-fluoropyridin-3-yl)benzamide (enantiomer 1);
N-(1-(2-(cyclopropanesulfonamido)thiazol-4-yl)propyl)-4-(5-fluoropyridin-3-yl)benzamide (enantiomer 2);
2-(cyclopropanesulfonamido)-N-(5-(6-(trifluoromethyl)pyrazin-2-yl)pyridin-2-yl)-5,6-dihydro-4H-cyclopenta[d]thiazole-4-carboxamide;
2-(2-(cyclopropanesulfonamido)thiazol-4-yl)-4-methoxy-N-(5-(pyrazin-2-yl)pyridin-2-yl)butanamide;
2-(2-(cyclopropanesulfonamido)thiazol-4-yl)-4-methoxy-N-(5'-methoxy-[3,3'-bipyridin]-6-yl)butanamide;
2-(2-(cyclopropanesulfonamido)thiazol-4-yl)-2-isopropoxy-N-(5-(6-(trifluoromethyl)pyrazin-2-yl)pyridin-2-yl)acetamide;
2-(2-(cyclopropanesulfonamido)thiazol-4-yl)-N-(5-(6-propoxypyrazin-2-yl)pyridin-2-yl)butanamide;
2-(2-(cyclopropanesulfonamido)thiazol-4-yl)-N-(5-(6-isopropoxypyrazin-2-yl)pyridin-2-yl)butanamide;
2-(2-(cyclopropanesulfonamido)thiazol-4-yl)-N-(5-(6-(trifluoromethyl)pyrazin-2-yl)pyridin-2-yl)butanamide;
2-(2-(cyclopropanesulfonamido)thiazol-4-yl)-N-(5-(6-methoxypyrazin-2-yl)pyridin-2-yl)butanamide;
2-(2-(cyclopropanesulfonamido)thiazol-4-yl)-N-(5-(6-ethoxypyrazin-2-yl)pyridin-2-yl)butanamide;
N-(5-(6-cyanopyrazin-2-yl)pyridin-2-yl)-2-(2-(cyclopropanesulfonamido)thiazol-4-yl)butanamide;
2-(2-(cyclopropanesulfonamido)thiazol-4-yl)-N-(5'-fluoro-[3,3'-bipyridin]-6-yl)butanamide;
N-(5'-cyano-[3,3'-bipyridin]-6-yl)-2-(2-(cyclopropanesulfonamido)thiazol-4-yl)butanamide;
2-(2-(cyclopropanesulfonamido)thiazol-4-yl)-N-(5-phenylpyridin-2-yl)butanamide;
2-(2-(cyclopropanesulfonamido)thiazol-4-yl)-N-(5-(6-(2,2,2-trifluoroethoxy)pyrazin-2-yl)pyridin-2-yl)butanamide;
2-(2-(cyclopropanesulfonamido)thiazol-4-yl)-N-(5-(6-ethoxypyrazin-2-yl)-3-fluoropyridin-2-yl)butanamide;
N-(5-(6-cyanopyrazin-2-yl)-3-fluoropyridin-2-yl)-2-(2-(cyclopropanesulfonamido)thiazol-4-yl)butanamide;
2-(2-(cyclopropanesulfonamido)thiazol-4-yl)-N-(5'-(2,2,2-trifluoroethoxy)-[3,3'-bipyridin]-6-yl)butanamide;
2-(2-(cyclopropanesulfonamido)thiazol-4-yl)-N-(5'-(difluoromethoxy)-[3,3'-bipyridin]-6-yl)butanamide;
N-(5-(6-chloropyrazin-2-yl)pyridin-2-yl)-2-(2-(cyclopropanesulfonamido)thiazol-4-yl)butanamide;
2-(2-(cyclopropanesulfonamido)thiazol-4-yl)-N-(2,3-difluoro-4-(pyridin-3-yl)phenyl)acetamide;
2-(2-(cyclopropanesulfonamido)thiazol-4-yl)-N-(4-(6-ethoxypyrazin-2-yl)phenyl)butanamide (racemic);
2-(2-(cyclopropanesulfonamido)thiazol-4-yl)-4-methoxy-N-(4-(6-methoxypyrazin-2-yl)phenyl)butanamide;
2-(2-(cyclopropanesulfonamido)thiazol-4-yl)-N-(4-(5-fluoropyridin-3-yl)phenyl)-4-methoxybutanamide;
2-(2-(cyclopropanesulfonamido)thiazol-4-yl)-N-(4-(6-ethoxypyrazin-2-yl)phenyl)propenamide;
2-(2-(cyclopropanesulfonamido)thiazol-4-yl)-N-(4-(5-(trifluoromethyl)pyridin-3-yl)phenyl)butanamide;
N-(4-(5-cyanopyridin-3-yl)phenyl)-2-(2-(cyclopropanesulfonamido)thiazol-4-yl)butanamide;
2-(2-(cyclopropanesulfonamido)thiazol-4-yl)-N-(4-(6-(2,2,2-trifluoroethoxy)pyrazin-2-yl)phenyl)butanamide;
2-(2-(cyclopropanesulfonamido)thiazol-4-yl)-N-(2-fluoro-4-(pyrazin-2-yl)phenyl)butanamide;
2-(2-(cyclopropanesulfonamido)thiazol-4-yl)-N-(2-fluoro-4-(6-(trifluoromethyl)pyrazin-2-yl)phenyl)butanamide;
2-(2-(cyclopropanesulfonamido)thiazol-4-yl)-N-(4-(6-ethoxypyrazin-2-yl)-2-fluorophenyl)butanamide;
2-(2-(cyclopropanesulfonamido)thiazol-4-yl)-N-(4-(5-ethoxypyridin-3-yl)-2-fluorophenyl)butanamide;
2-(2-(cyclopropanesulfonamido)thiazol-4-yl)-N-(2-fluoro-4-(5-fluoropyridin-3-yl)phenyl)butanamide;
2-(2-(cyclopropanesulfonamido)thiazol-4-yl)-N-(2-fluoro-4-(pyridin-3-yl)phenyl)butanamide;
N-(4-(5-cyanopyridin-3-yl)-2-fluorophenyl)-2-(2-(cyclopropanesulfonamido)thiazol-4-yl)butanamide;
N-(4-(5-chloropyridin-3-yl)-2-fluorophenyl)-2-(2-(cyclopropanesulfonamido)thiazol-4-yl)butanamide;
2-(2-(cyclopropanesulfonamido)thiazol-4-yl)-N-(4-(6-ethoxypyrazin-2-yl)phenyl)butanamide (enantiomer 1);
2-(2-(cyclopropanesulfonamido)thiazol-4-yl)-N-(4-(6-ethoxypyrazin-2-yl)phenyl)butanamide (enantiomer 2);
N-(4-(1-(5-(6-ethoxypyrazin-2-yl)indolin-1-yl)-1-oxobutan-2-yl)thiazol-2-yl)cyclopropanesulfonamide;
2-(2-(cyclopropanesulfonamido)thiazol-4-yl)-N-(2-fluoro-4-(6-(2,2,2-trifluoroethoxy)pyrazin-2-yl)phenyl)butanamide;
2-(2-(cyclopropanesulfonamido)thiazol-4-yl)-N-(2-fluoro-4-(6-methoxypyrazin-2-yl)phenyl)butanamide;
2-(2-(cyclopropanesulfonamido)thiazol-4-yl)-N-(4-(5-(difluoromethoxy)pyridin-3-yl)-2-fluorophenyl)butanamide;
2-(2-(cyclopropanesulfonamido)thiazol-4-yl)-N-(4-(5-(difluoromethoxy)pyridin-3-yl)phenyl)butanamide;
2-(2-(cyclopropanesulfonamido)thiazol-4-yl)-N-(4-(5-(2,2,2-trifluoroethoxy)pyridin-3-yl)phenyl)butanamide;
2-(2-(cyclopropanesulfonamido)thiazol-4-yl)-N-(2-fluoro-4-(5-(2,2,2-trifluoroethoxy)pyridin-3-yl)phenyl)butanamide;
2-(cyclopropanesulfonamido)-N-(4-(pyridin-3-yl)phenyl)-5,6-dihydro-4H-cyclopenta[d]thiazole-4-carboxamide;
2-(2-(cyclopropanesulfonamido)thiazol-4-yl)-2-methoxy-N-(5-(6-(trifluoromethyl)pyrazin-2-yl)pyridin-2-yl)acetamide;
2-(2-(cyclopropanesulfonamido)thiazol-4-yl)-N-(2-methoxy-4-(pyridin-3-yl)phenyl)acetamide;
2-(2-(cyclopropanesulfonamido)thiazol-4-yl)-N-(2-fluoro-4-(pyridin-3-yl)phenyl)acetamide;
N-(4-(5-cyanopyridin-3-yl)phenyl)-2-(cyclopropanesulfonamido)-5,6-dihydro-4H-cyclopenta[d]thiazole-4-carboxamide;

2-(cyclopropanesulfonamido)-N-(4-(5-fluoropyridin-3-yl)phenyl)-5,6-dihydro-4H-cyclopenta[d]thiazole-4-carboxamide;
2-(2-(cyclopropanesulfonamido)thiazol-4-yl)-2-methoxy-N-(4-(pyridin-3-yl)phenyl)acetamide;
2-(2-(cyclopropanesulfonamido)thiazol-4-yl)-N-(2-fluoro-4-(pyridin-3-yl)phenyl)-2-methoxyacetamide;
N-(2-chloro-4-(pyridin-3-yl)phenyl)-2-(2-(cyclopropanesulfonamido)thiazol-4-yl)butanamide;
2-(2-(cyclopropanesulfonamido)thiazol-4-yl)-N-(4-(6-(2,2,2-trifluoroethoxy)pyrazin-2-yl)phenyl)butanamide;
2-(2-(cyclopropanesulfonamido)thiazol-4-yl)-N-(4-(6-(2,2,2-trifluoroethoxy)pyrazin-2-yl)phenyl)butanamide;
2-(2-(cyclopropanesulfonamido)thiazol-4-yl)-N-(5'-methoxy-[3,3'-bipyridin]-6-yl)-2-methylpropanamide;
N-(5'-chloro-[3,3'-bipyridin]-6-yl)-2-(2-(cyclopropanesulfonamido)thiazol-4-yl)-2-methylpropanamide;
N-(5'-cyano-[3,3'-bipyridin]-6-yl)-2-(2-(cyclopropanesulfonamido)thiazol-4-yl)-2-methylpropanamide;
2-(2-(cyclopropanesulfonamido)thiazol-4-yl)-N-(5-fluoro-[3,3'-bipyridin]-6-yl)-2-methylpropanamide;
N-(5'-cyano-5-fluoro-[3,3'-bipyridin]-6-yl)-2-(2-(cyclopropanesulfonamido)thiazol-4-yl)-2-methylpropanamide;
N-(5'-chloro-5-fluoro-[3,3'-bipyridin]-6-yl)-2-(2-(cyclopropanesulfonamido)thiazol-4-yl)-2-methylpropanamide;
2-(2-(cyclopropanesulfonamido)thiazol-4-yl)-N-(5,5'-difluoro-[3,3'-bipyridin]-6-yl)-2-methylpropanamide;
N-(5-(3-chloro-5-methylphenyl)pyridin-2-yl)-2-(2-(cyclopropanesulfonamido)thiazol-4-yl)-2-methylpropanamide;
2-(2-(cyclopropanesulfonamido)thiazol-4-yl)-N-(5-(3-methoxyphenyl)pyridin-2-yl)-2-methylpropanamide;
2-(2-(cyclopropanesulfonamido)thiazol-4-yl)-N-(5-(3-fluoro-5-methoxyphenyl)pyridin-2-yl)-2-methylpropanamide;
2-(2-(cyclopropanesulfonamido)thiazol-4-yl)-N-(5-(3,5-dimethoxyphenyl)pyridin-2-yl)-2-methylpropanamide;
2-(2-(cyclopropanesulfonamido)thiazol-4-yl)-2-methyl-N-(5-(3-(trifluoromethyl)phenyl)pyridin-2-yl)propenamide;
2-(2-(cyclopropanesulfonamido)thiazol-4-yl)-2-methyl-N-(5-(3-(trifluoromethoxy)phenyl)pyridin-2-yl)propenamide;
2-(2-(cyclopropanesulfonamido)thiazol-4-yl)-N-(5-(3-(2-hydroxypropan-2-yl)phenyl)pyridin-2-yl)-2-methylpropanamide;
2-(2-(cyclopropanesulfonamido)thiazol-4-yl)-2-methyl-N-(5-(3-morpholinophenyl)pyridin-2-yl)propenamide;
2-(2-(cyclopropanesulfonamido)thiazol-4-yl)-2-methyl-N-(6-phenylpyridin-3-yl)propenamide; 2-(2-(cyclopropanesulfonamido)thiazol-4-yl)-N-(4-(2-fluoropyridin-3-yl)phenyl)-2-methylpropanamide;
2-(2-(cyclopropanesulfonamido)thiazol-4-yl)-N-(4-(5-(hydroxymethyl)pyridin-3-yl)phenyl)-2-methylpropanamide;
2-(2-(cyclopropanesulfonamido)thiazol-4-yl)-N-(4-(2-methoxypyrimidin-5-yl)phenyl)acetamide;
N-(4'-(tert-butyl)-[1,1'-biphenyl]-4-yl)-2-(2-(cyclopropanesulfonamido)thiazol-4-yl)acetamide;
2-(2-(cyclopropanesulfonamido)thiazol-4-yl)-N-(4-(pyridin-3-yl)phenyl)acetamide;
2-(2-(cyclopropanesulfonamido)thiazol-4-yl)-N-(4-(pyridin-4-yl)phenyl)acetamide;
2-(2-(cyclopropanesulfonamido)thiazol-4-yl)-N-(2'-methoxy-[1,1'-biphenyl]-4-yl)acetamide;
2-(2-(cyclopropanesulfonamido)thiazol-4-yl)-N-(4-(pyrimidin-5-yl)phenyl)acetamide;
2-(2-(cyclopropanesulfonamido)thiazol-4-yl)-2-methyl-N-(4-(2-(trifluoromethyl)pyridin-3-yl)phenyl)propenamide;
2-(2-(cyclopropanesulfonamido)thiazol-4-yl)-2-methyl-N-(5'-methyl-[3,3'-bipyridin]-6-yl)propenamide;
2-(2-(cyclopropanesulfonamido)thiazol-4-yl)-N-(4-(2-methoxy-4-methylpyridin-3-yl)phenyl)-2-methylpropanamide;
2-(2-(cyclopropanesulfonamido)thiazol-4-yl)-N-(4-(6-methoxy-5-methylpyridin-3-yl)phenyl)-2-methylpropanamide;
N-(4-(5-chloropyridin-3-yl)phenyl)-2-(2-(cyclopropanesulfonamido)thiazol-4-yl)-2-methylpropanamide;
2-(2-(cyclopropanesulfonamido)thiazol-4-yl)-N-(4-(5-fluoropyridin-3-yl)phenyl)-2-methylpropanamide;
2-(2-(cyclopropanesulfonamido)thiazol-4-yl)-2-methyl-N-(4-(4-methylpyridin-3-yl)phenyl)propenamide;
2-(2-(cyclopropanesulfonamido)thiazol-4-yl)-2-methyl-N-(4-(4-(trifluoromethyl)pyridin-3-yl)phenyl)propenamide;
N-(4-(5-chloropyridin-3-yl)-2-methoxyphenyl)-2-(2-(cyclopropanesulfonamido)thiazol-4-yl)-2-methylpropanamide;
2-(2-(cyclopropanesulfonamido)thiazol-4-yl)-N-(4-(5-(dimethylamino)pyridin-3-yl)phenyl)-2-methylpropanamide;
2-(2-(cyclopropanesulfonamido)thiazol-4-yl)-N-(2-methoxy-4-(5-methylpyridin-3-yl)phenyl)-2-methylpropanamide;
2-(2-(cyclopropanesulfonamido)thiazol-4-yl)-N-(2-methoxy-4-(5-(trifluoromethyl)pyridin-3-yl)phenyl)-2-methylpropanamide;
2-(2-(cyclopropanesulfonamido)thiazol-4-yl)-N-(4-(6-methoxypyridin-3-yl)phenyl)acetamide;
2-(2-(cyclopropanesulfonamido)thiazol-4-yl)-N-(5'-fluoro-[3,3'-bipyridin]-6-yl)-2-methylpropanamide;
N-(5-(6-chloropyrazin-2-yl)pyridin-2-yl)-2-(2-(cyclopropanesulfonamido)thiazol-4-yl)-2-methylpropanamide;
N-(5-(6-cyanopyrazin-2-yl)pyridin-2-yl)-2-(2-(cyclopropanesulfonamido)thiazol-4-yl)-2-methylpropanamide;
2-(2-(cyclopropanesulfonamido)thiazol-4-yl)-2-methyl-N-(5-(pyrimidin-5-yl)pyridin-2-yl)propenamide;
2-(2-(cyclopropanesulfonamido)thiazol-4-yl)-N-(4-(6-methoxypyrazin-2-yl)phenyl)-2-methylpropanamide;
2-(2-(cyclopropanesulfonamido)thiazol-4-yl)-2-methyl-N-(4-(6-methylpyrazin-2-yl)phenyl)propenamide;
2-(2-(cyclopropanesulfonamido)thiazol-4-yl)-2-methyl-N-(4-(6-(trifluoromethyl)pyrazin-2-yl)phenyl)propenamide;
N-(4-(6-chloropyridin-2-yl)phenyl)-2-(2-(cyclopropanesulfonamido)thiazol-4-yl)-2-methylpropanamide;
2-(2-(cyclopropanesulfonamido)thiazol-4-yl)-N-(4-(6-methoxypyridin-2-yl)phenyl)-2-methylpropanamide;
2-(2-(cyclopropanesulfonamido)thiazol-4-yl)-2-methyl-N-(4-(6-(trifluoromethyl)pyridin-2-yl)phenyl)propenamide;
2-(2-(cyclopropanesulfonamido)thiazol-4-yl)-N-(4-(4-methoxypyridin-2-yl)phenyl)-2-methylpropanamide;
2-(2-(cyclopropanesulfonamido)thiazol-4-yl)-N-(4-(6-isopropoxypyrazin-2-yl)phenyl)-2-methylpropanamide;

2-(2-(cyclopropanesulfonamido)thiazol-4-yl)-N-(4-(6-cyclopropoxypyrazin-2-yl)phenyl)-2-methylpropanamide;
2-(2-(cyclopropanesulfonamido)thiazol-4-yl)-N-(2-fluoro-4-(pyrazin-2-yl)phenyl)-2-methylpropanamide;
2-(2-(cyclopropanesulfonamido)thiazol-4-yl)-N-(2-fluoro-4-(6-methoxypyrazin-2-yl)phenyl)-2-methylpropanamide;
N-(4-(6-chloro-3-methylpyrazin-2-yl)phenyl)-2-(2-(cyclopropanesulfonamido)thiazol-4-yl)-2-methylpropanamide;
N-(4-(6-chloro-5-methylpyrazin-2-yl)phenyl)-2-(2-(cyclopropanesulfonamido)thiazol-4-yl)-2-methylpropanamide;
2-(2-(cyclopropanesulfonamido)thiazol-4-yl)-2-methyl-N-(4-(6-(pyrrolidin-1-yl)pyrazin-2-yl)phenyl)propenamide;
2-(2-(cyclopropanesulfonamido)thiazol-4-yl)-N-(4-(6-(2-(dimethylamino)ethoxy)pyrazin-2-yl)phenyl)-2-methylpropanamide;
2-(2-(cyclopropanesulfonamido)thiazol-4-yl)-2-methyl-N-(4-(3-methylpyrazin-2-yl)phenyl)propenamide;
N-(4-(6-acetamidopyrazin-2-yl)phenyl)-2-(2-(cyclopropanesulfonamido)thiazol-4-yl)-2-methylpropanamide;
2-(2-(cyclopropanesulfonamido)thiazol-4-yl)-N-(4-(5,6-dimethylpyrazin-2-yl)phenyl)-2-methylpropanamide;
2-(2-(cyclopropanesulfonamido)thiazol-4-yl)-N-(4-(6-(hydroxymethyl)pyrazin-2-yl)phenyl)-2-methylpropanamide;
2-(2-(cyclopropanesulfonamido)thiazol-4-yl)-N-(4-(3,6-dimethylpyrazin-2-yl)phenyl)-2-methylpropanamide;
2-(2-(cyclopropanesulfonamido)thiazol-4-yl)-N-(4-(5-methoxypyridin-3-yl)-2-methylphenyl)-2-methylpropanamide;
N-(4-(5-cyanopyridin-3-yl)-2-methylphenyl)-2-(2-(cyclopropanesulfonamido)thiazol-4-yl)-2-methylpropanamide;
2-(2-(cyclopropanesulfonamido)thiazol-4-yl)-N-(4-(5-fluoropyridin-3-yl)-2-methylphenyl)-2-methylpropanamide;
N-(4-(5-chloropyridin-3-yl)-3-methylphenyl)-2-(2-(cyclopropanesulfonamido)thiazol-4-yl)-2-methylpropanamide;
N-(4-(5-cyanopyridin-3-yl)-3-ethoxyphenyl)-2-(2-(cyclopropanesulfonamido)thiazol-4-yl)-2-methylpropanamide;
2-(2-(cyclopropanesulfonamido)thiazol-4-yl)-N-(4-(5-ethoxypyridin-3-yl)phenyl)-2-methylpropanamide;
2-(2-(cyclopropanesulfonamido)thiazol-4-yl)-N-(4-(6-cyclopropylpyrazin-2-yl)phenyl)butanamide;
2-(2-(cyclopropanesulfonamido)thiazol-4-yl)-N-(5-(5-methoxypyridin-3-yl)pyrimidin-2-yl)-2-methylpropanamide;
2-(2-(cyclopropanesulfonamido)thiazol-4-yl)-N-(5-(5-fluoropyridin-3-yl)pyrimidin-2-yl)-2-methylpropanamide;
2-(2-(cyclopropanesulfonamido)thiazol-4-yl)-2-methyl-N-(5-(5-(trifluoromethyl)pyridin-3-yl)pyrimidin-2-yl)propenamide;
N-(5-(6-ethoxypyrazin-2-yl)pyridin-2-yl)-2-methyl-2-(2-((2-methylpropyl)sulfonamido)thiazol-4-yl)propenamide;
N-(4-(6-ethoxypyrazin-2-yl)phenyl)-2-methyl-2-(2-((trifluoromethyl)sulfonamido)thiazol-4-yl)propenamide;
2-methyl-2-(2-((1-methylethyl)sulfonamido)thiazol-4-yl)-N-(4-(pyridin-3-yl)phenyl)propenamide;
N-(4-(6-ethoxypyrazin-2-yl)phenyl)-2-methyl-2-(2-((1-methylethyl)sulfonamido)thiazol-4-yl)propenamide;
2-methyl-2-(2-((1-methylcyclopropane)-1-sulfonamido)thiazol-4-yl)-N-(4-(pyridin-3-yl)phenyl)propenamide;
N-(4-(5-chloropyridin-3-yl)phenyl)-2-methyl-2-(2-((1-methylcyclopropane)-1-sulfonamido)thiazol-4-yl)propenamide;
N-(4-(6-ethoxypyrazin-2-yl)phenyl)-2-methyl-2-(2-((1-methylcyclopropane)-1-sulfonamido)thiazol-4-yl)propenamide;
2-methyl-2-(2-((1-methylcyclopropane)-1-sulfonamido)th iazol-4-yl)-N-(4-(6-(trifluoromethyl)pyrazin-2-yl)phenyl)propenamide;
2-(2-((1,1-dimethylethyl)sulfonamido)thiazol-4-yl)-2-methyl-N-(4-(pyridin-3-yl)phenyl)propenamide;
2-(2-((1,1-dimethylethyl)sulfonamido)thiazol-4-yl)-N-(4-(6-ethoxypyrazin-2-yl)phenyl)-2-methylpropanamide;
2-(2-((1,1-dimethylethyl)sulfonamido)thiazol-4-yl)-2-methyl-N-(4-(6-(trifluoromethyl)pyrazin-2-yl)phenyl)propenamide;
2-(2-(cyclobutanesulfonamido)thiazol-4-yl)-2-methyl-N-(4-(pyridin-3-yl)phenyl)propenamide;
2-(2-(cyclobutanesulfonamido)thiazol-4-yl)-N-(4-(6-ethoxypyrazin-2-yl)phenyl)-2-methylpropanamide;
2-(2-(cyclobutanesulfonamido)thiazol-4-yl)-2-methyl-N-(4-(6-(trifluoromethyl)pyrazin-2-yl)phenyl)propenamide;
N-(4-(5-cyanopyridin-3-yl)phenyl)-2-(2-(cyclopropanesulfonamido)thiazol-4-yl)-N,2-dimethylpropanamide;
2-(2-(cyclopropanesulfonamido)thiazol-4-yl)-N,2-dimethyl-N-(4-(6-(trifluoromethyl)pyrazin-2-yl)phenyl)propenamide;
2-methyl-2-(2-((2-methylpropyl)sulfonamido)thiazol-4-yl)-N-(4-(pyridin-3-yl)phenyl)propenamide;
N-(4-(6-ethoxypyrazin-2-yl)phenyl)-2-methyl-2-(2-((2-methylpropyl)sulfonamido)thiazol-4-yl)propenamide;
2-methyl-2-(2-((2-methylpropyl)sulfonamido)thiazol-4-yl)-N-(4-(6-(trifluoromethyl)pyrazin-2-yl)phenyl)propenamide;
N-(4-(5-chloropyridin-3-yl)phenyl)-2-(2-(cyclopropanesulfonamido)-5-methylthiazol-4-yl)-2-methylpropanamide;
2-(2-(cyclopropanesulfonamido)thiazol-4-yl)-N-methyl-N-(4-(pyridin-3-yl)phenyl)butanamide;
N-(4-(5-cyanopyridin-3-yl)phenyl)-2-(2-(cyclopropanesulfonamido)thiazol-4-yl)-N-methylbutanamide;
2-(2-(cyclopropanesulfonamido)-5-methylthiazol-4-yl)-N-(4-(6-ethoxypyrazin-2-yl)phenyl)-2-methylpropanamide;
N-(4-(5-chloropyridin-3-yl)phenyl)-2-(2-(cyclopropanesulfonamido)thiazol-4-yl)-N,2-dimethylpropanamide;
2-(2-(cyclopropanesulfonamido)-5-methylthiazol-4-yl)-2-methyl-N-(4-(pyridin-3-yl)phenyl)propenamide;
N-(4-(5-cyanopyridin-3-yl)-2,6-dimethylphenyl)-2-(2-(cyclopropanesulfonamido)thiazol-4-yl)-2-methylpropanamide;
N-(4-(5-chloropyridin-3-yl)-2,6-dimethylphenyl)-2-(2-(cyclopropanesulfonamido)thiazol-4-yl)-2-methylpropanamide; and
N-(4-(5-cyanopyridin-3-yl)-3-methylphenyl)-2-(2-(cyclopropanesulfonamido)thiazol-4-yl)-2-methylpropanamide.

The compounds of the invention may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Compounds of the invention of particular interest are those demonstrating an $IC_{50}$ of 1uM or lower, especially 100nM or lower, in respect of CTPS1 enzyme, using the methods of the examples (or comparable methods).

It will be appreciated that for use in medicine the salts of the compounds of formula (I) should be pharmaceutically acceptable. Non-pharmaceutically acceptable salts of the compounds of formula (I) may be of use in other contexts. Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid or base addition salts with one or more equivalents of the acid or base. The present invention includes within its scope all possible stoichiometric and non-stoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It will be understood that the invention includes pharmaceutically acceptable derivatives of compounds of formula (I) and that these are included within the scope of the invention.

As used herein "pharmaceutically acceptable derivative" includes any pharmaceutically acceptable prodrug such as an ester or salt of such ester of a compound of formula (I) which, upon administration to the recipient is capable of providing (directly or indirectly) a compound of formula (I) or an active metabolite or residue thereof.

It is to be understood that the present invention encompasses all isomers of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exists as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or> 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^{2}H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{37}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Unnatural variant isotopic forms which incorporate deuterium i.e $^{2}H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples, and modifications thereof.

**General Routes:**

Generic routes by which compound examples of the invention may be conveniently prepared are summarised below.

## Scheme 1

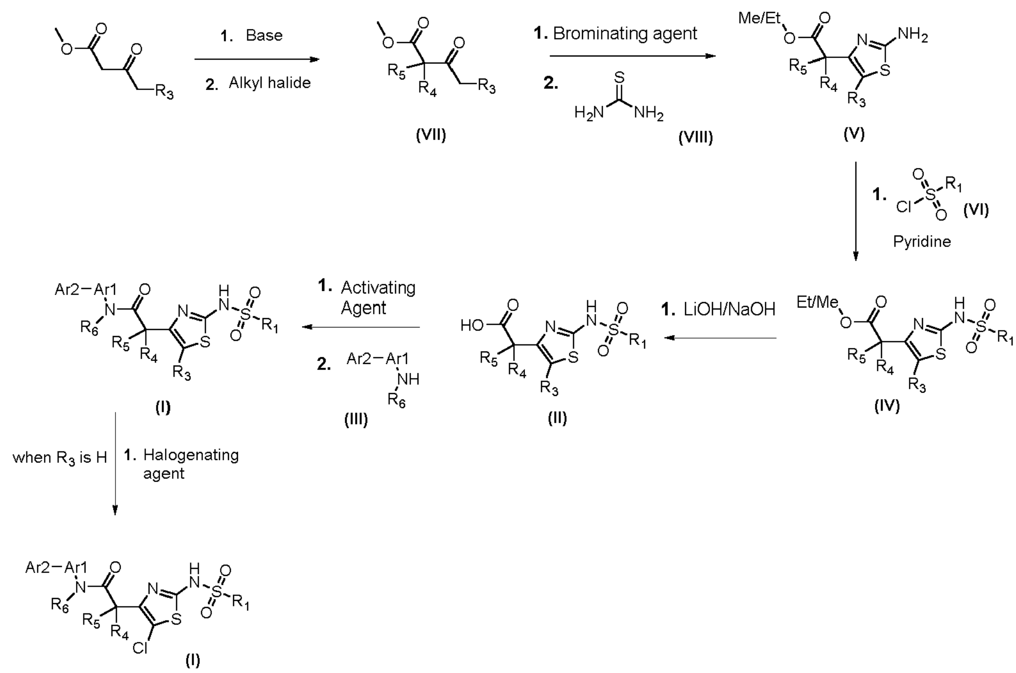


Compounds of general formula **(I),** where $R_1$, $R_2$, $R_3$, $R_4$, $R_5$, $R_6$, $R_{10}$, $R_{11}$, $R_{12}$, $R_{13}$, Ar1 and Ar2 are defined above, may be synthesised by the general scheme (Scheme 1). Ketoesters of formula **(VII)** may be prepared by alkylation of an unsubstituted ketoester, which is well established in the literature with many simple derivatives being commercially available.

Intermediates of formula **(V)** are readily prepared from ketoesters of formula **(VII)** using a two step procedure. Firstly, bromination using bromine or pyrimidium tribromide can afford the alpha bromoketone ester. This intermediate

may be isolated but is routinely used directly without characterisation or purification in the subsequent step. Thiourea **(VIII)** may be added to form thiazoles of the formula **(V)** via cyclisation. Such reactions may be subject to gentle heating to, for example, 40 °C.

The compound intermediates of formula **(IV)** can be obtained by sulfonylation of amines of formula **(V)** with a suitable sulfonyl chloride **(VI)** in pyridine. Such reactions may be subject to gentle heating to, for example, 30 - 60 °C.

The alkyl esters of formula **(IV)** may be conveniently hydrolysed by exposure to a suitable inorganic base, for example lithium hydroxide, in an aqueous mixture of aprotic and protic solvents, such as THF:methanol:water. Such reactions may be subject to gentle heating to, for example, 30 - 50 °C.

Compounds of formula **(I)** may be obtained by a general process whereby a carboxylic acid precursor **(II),** or a suitably protected derivative thereof, is reacted with an activating agent, to generate a reactive, electrophilic carboxylic acid derivative, followed by subsequent reaction with an amine of formula **(III),** or a suitably protected derivative thereof. It will be understood by persons skilled in the art that, in some instances, the activated carboxylic acid derivative, such as an acid chloride, may be isolated or in other cases may be a transient intermediate that is not isolated, but generated *in situ* and used directly. Reagents suitable for the activation of the carboxylate group include carbonyl diimidazole, 1-chloro-*N,N*,2-trimethylprop-1-en-1-amine (Ghosez reagent) and a wide selection of peptide coupling agents such as 1-[bis(dimethylamino)methylene]-1H-1,2,3-triazolo[4,5-b]pyridinium 3-oxid hexafluorophosphate (HATU) and the like. Such reactions are conveniently carried out in a non-polar, aprotic solvent, such as DCM at or below ambient temperature.

If $R_3$ = H in compounds of the general formula **(I),** substitution can be undertaken using a halogenating reagent, such as *N*-chlorosuccinimide, in an organic solvent such as MeCN to generate compounds of the general formula **(I)** wherein $R_3$ = Cl.

## Scheme 2

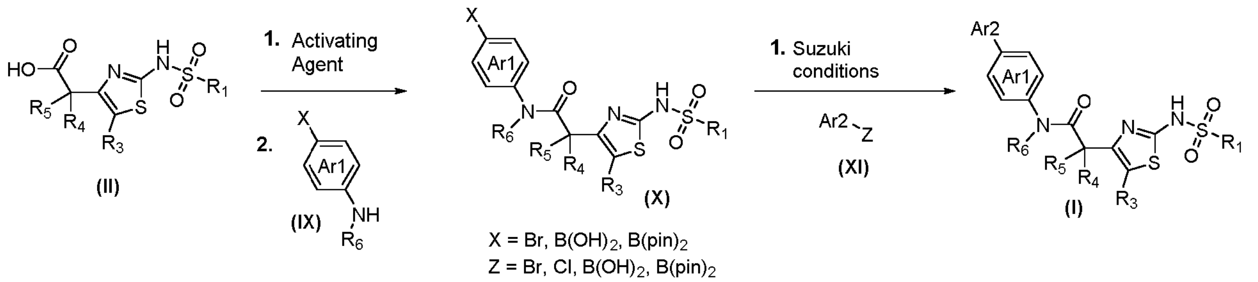


Compounds of formula **(I)** may be obtained by a general process whereby a carboxylic acid precursor **(II),** or a suitably protected derivative thereof, is reacted with an activating agent, to generate a reactive, electrophilic carboxylic acid derivative, followed by subsequent reaction with an amine of formula **(IX).** Intermediates of formula **(X)** are then converted to a compound of the invention of general formula **(I)** by coupling under Suzuki conditions with an aromatic halide or boronate of general formula **(XI),** of which **X** is defined above and represents usually a bromide, a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as bis(diphenylphosphino)ferrocene]dichloropalladium(II) and an inorganic base such as potassium carbonate in a solvent mixture of dioxane and water. It will be understood by persons skilled in the art that many catalysts and conditions can be employed for such couplings.

## Scheme 3

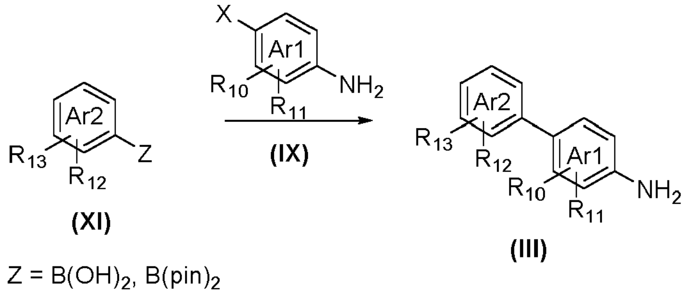


Z = $B(OH)_2$, $B(pin)_2$
X = Br, Cl

Intermediates of formula **(III)** where Ar2 is an unsubstituted or substituted 3-pyridyl ring, may be synthesised by coupling under Suzuki conditions of a boronate of general formula **(XI),** of which $R^{12}$ and $R^{13}$ are defined above and **Z** represents a dihydroxyboryl or dialkyloxyboryl group, usually a 4,4,5,5-tetramethyl-1,3,3,2-dioxaborolan-2-yl group, to a substituted pyridine of formula **(IX)** of which $R^{10}$ and $R^{11}$ are defined above and where **X** denotes a halide. The couplings according to the Suzuki method are performed, for example, by heating in the presence of a catalyst such as [1,1'-bis(diphenylphosphino)ferrocene]dichloropalladium(II) complex with dichloromethane and an inorganic base such as potassium carbonate in a solvent mixture of 1,4-dioxane and water.

## Scheme 4

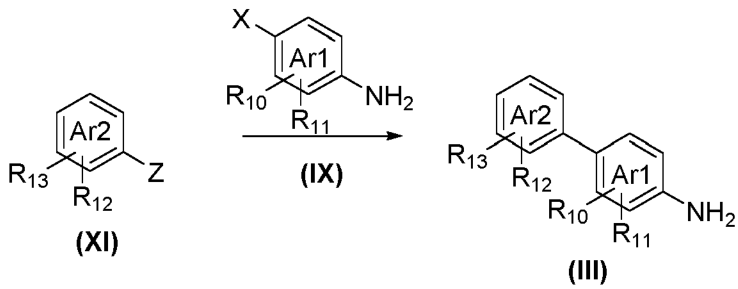


(XI) (III)

Z = Br, Cl
X = $B(OH)_2$, $B(pin)_2$

Intermediates of formula **(III)** where Ar2 is an unsubstituted or substituted 2-pyrazine ring, may be synthesised by coupling under Suzuki conditions of an aromatic halide of general formula **(XI),** of which $R^{12}$ and $R^{13}$ are defined above and **Z** represents a halide, to a boronate of general formula **(IX)** of which $R^{10}$ and $R^{11}$ are defined above and where **X** denotes a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as tetrakis(triphenylphosphine)palladium or [1,1'-bis(diphenylphosphino) ferrocene]dichloropalladium(II) and an inorganic base such as potassium carbonate in a solvent mixture of dioxane and water.

## Scheme 5

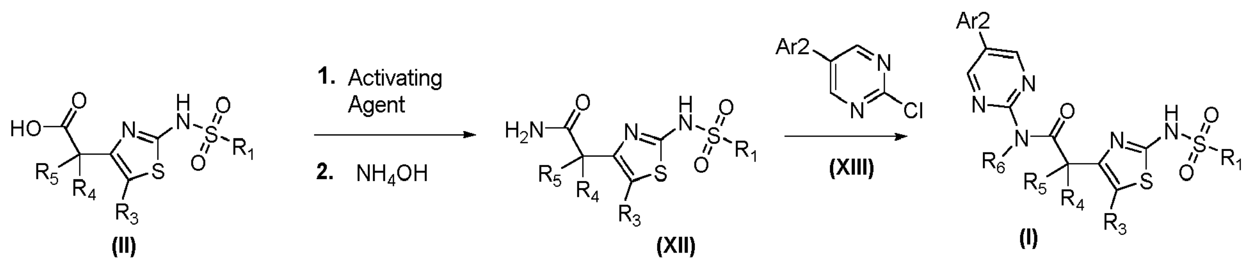


(II) (XII) (I)

The compound intermediates of formula **(I)** where Ar1 is a 2-amino pyrimidine, can be obtained by activating the acid **(II)** with a suitable activating agent, to generate a reactive, electrophilic carboxylic acid derivative, followed by subsequent reaction with an ammonia source such as ammonium hydroxide. The primary amide **(XII)** can then be coupled with a 2-chloropyrimidine derivative **(XIII)** using a palladium catalyst such as Pd 177 (Xantphos precatalyst) and inorganic base, for example cesium carbonate in 1.4-dioxane to yield compounds of the general formula **(I).** It will be understood by persons skilled in the art that traditional coupling conditions directly from an electrophilic carboxylic acid derivative and 2-aminopyrimidine derivative are difficult due to the unreactive nature of the nucleophile.

## Scheme 6

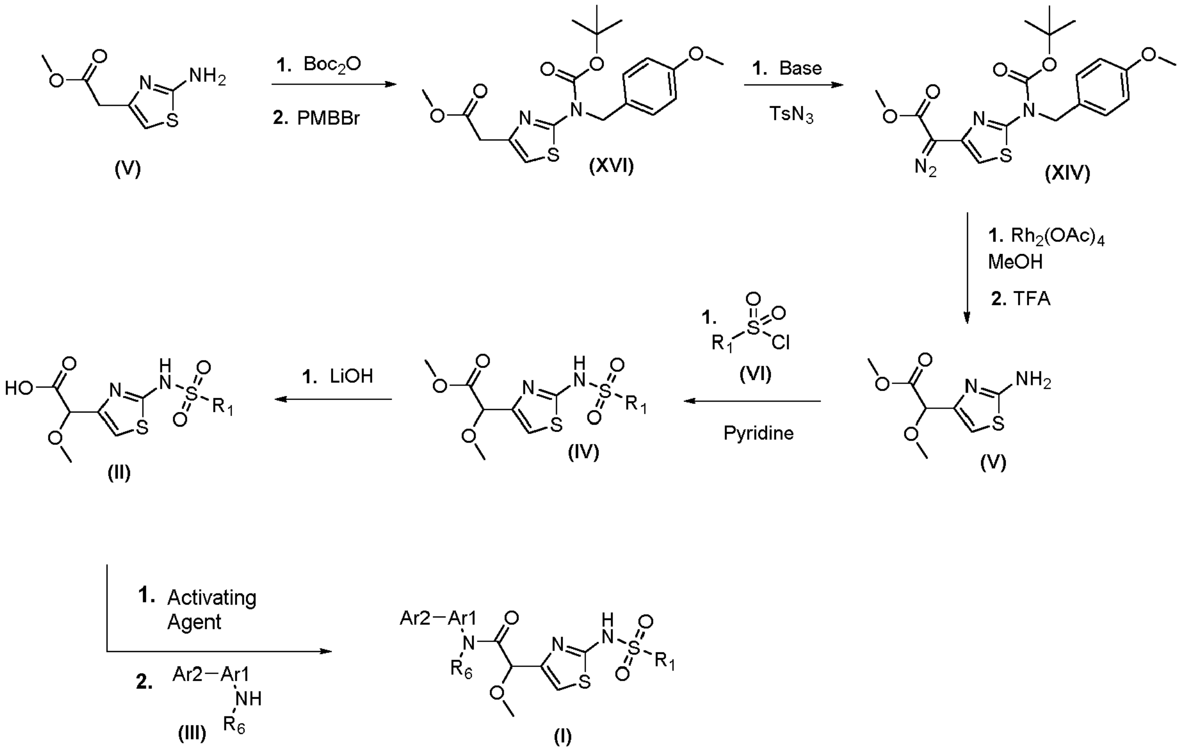


In general and as illustrated in Scheme 6, the 2-(2-(alkylsulfonamido)thiazol-4-yl) amide derivatives of general formula **(I)** in which $R_1$, $R_6$, Ar1 and Ar2 are defined above may be prepared in several steps, starting from methyl 2-(2-aminothiazol-4-yl)acetate. Intermediates of formula **(XVI)** can be formed by sequential protection of the amine **(V)** with Boc and PMB protecting groups. Addition of a base, such as 1,8-diazabicyclo[5.4.0]undec-7-ene, and tosyl azide leads to the selective introduction of the diazo functional group on the alpha-position relative to the ester to yield intermediates of formula **(XIV).** Under rhodium catalysis and in the presence of a nucleophilic source, such as methanol, followed by deprotection of both protecting groups at elevated temperatures, derivatives of formula **(V)** may be prepared. The compound intermediates of formula **(IV)** can be obtained by sulfonylation of amines of formula **(V)** with a suitable sulfonyl chloride **(VI)** in pyridine. Such reactions may be subject to gentle heating to, for example, 30 - 50 °C. The alkyl esters of formula (IV) may be conveniently converted to compounds of formula **(I)** according to synthetic steps reported in Scheme 1.

## Scheme 7

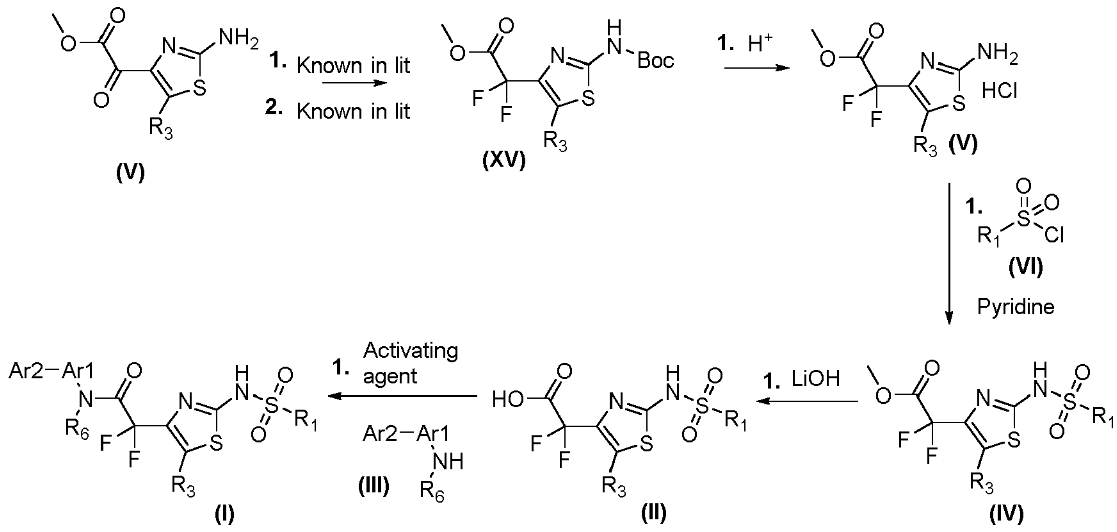


(V) (XV) (V) (VI) (IV) (II) (III) (I)

The difluoro intermediate of formula **(XV)** may be prepared in two steps by firstly Boc protection using Boc-anhydride in a polar solvent such as dichloroethane, followed by utilisation of a fluorinating agent such as bis(2-methoxyethyl)aminosulfur trifluoride. These fluorination reactions are, for example, performed at RT and in a solvent such as toluene. The following deprotection to yield the free aminothiazole **(V)** is performed, for example, by exposure to acid in an organic solvent, such as HCl in 1,4-dioxane. The compound intermediates of formula **(IV)** can be obtained by sulfonylation of amines of formula **(V)** with a suitable sulfonyl chloride **(VI)** in pyridine. Such reactions may be subject to gentle heating to, for example, 30-50 °C. The alkyl esters of formula **(IV)** may be conveniently converted to compounds of formula **(I)** according to synthetic steps reported in Scheme 1.

## Scheme 8

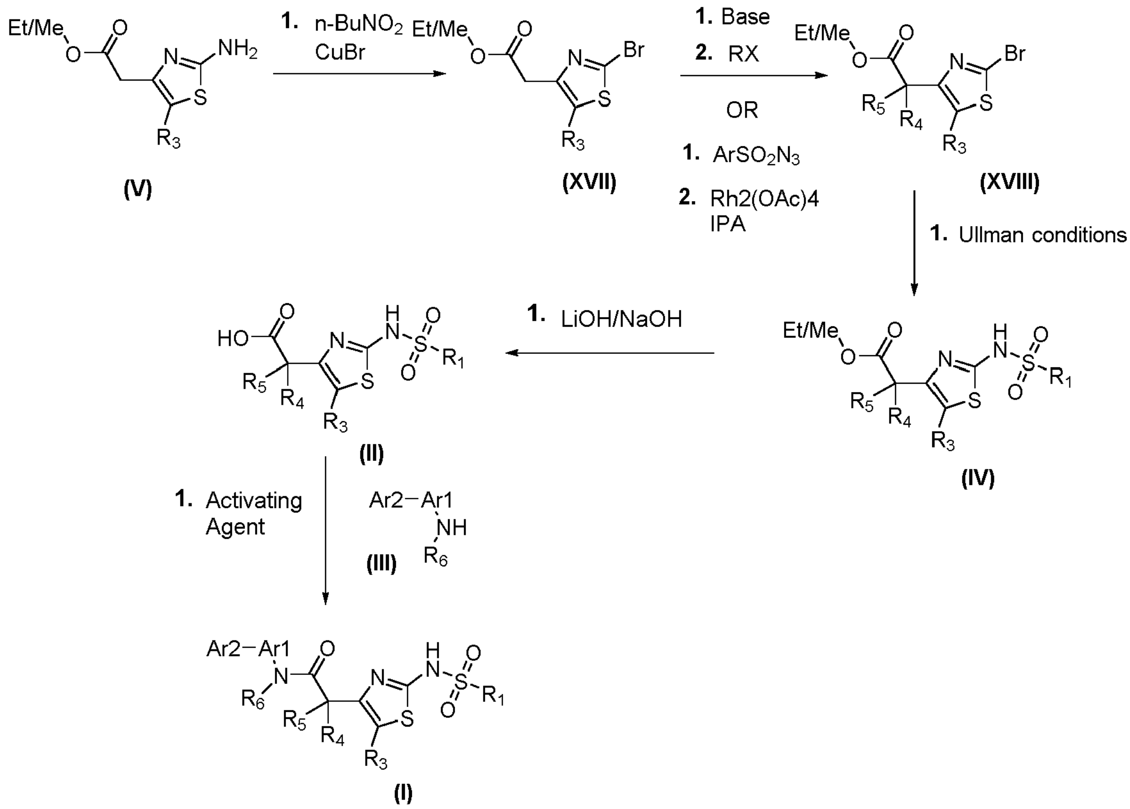


Compound of general formula **(XVII)** may be prepared by conversion of a methyl 2-(2-aminothiazol-4-yl)acetate derivative by a Sandmeyer type reaction using an organic nitrite, such as n-butylnitrite, in the presence of a halide source, such as Cu(I)Br in acetonitrile. Such reactions can be undertaken at temperatures of RT to 60 °C. Introduction of $R_4/R_5$ can be undertaken by two alternative methods at this stage. Firstly, alkylation of compounds of general formula **(XVII)** can be undertaken by addition of a suitable base, for example, LiHMDS, together with an alkylating agent, such as iodomethane which results in dialkylation alpha to the ester moiety to yield compounds of formula **(XVIII),** where $R_4$ = $R_5$ = Me. Secondly, diazotisation of compounds of general formula **(XVII)** with the use of an diazo transfer reagent, such as 4-acetamidobenzenesulfonyl azide, under basic conditions, followed by treatment with rhodium and subsequent insertion of the corresponding nucleophile, such as isopropyl alcohol, gives intermediates of general formula **(XVIII)** where $R_4$ = Oisopropoxy and $R_5$ = H.

Introduction of the sulfonamide group in the preparation of compounds of formula **(IV)** may be achieved by an Ullmann coupling reaction using a copper catalyst, such as Cu(I)I, in the presence of an inorganic base, potassium carbonate, and a diamine ligand in dioxane. Such reactions are typically carried out at elevated temperatures such as 80 °C. The alkyl esters of formula **(IV)** may be conveniently converted to compounds of formula **(I)** according to synthetic steps reported in Scheme 1.

**Intermediates of the Invention**

The present invention also relates to novel intermediates in the synthesis of compounds of formula (I) such as compounds of formula (II)-(XVIII). Particular intermediates of interest are those of the following general formulae, wherein the variable groups and associated preferences are as defined previously for compounds of formula (I):

- Compounds of formula (II)

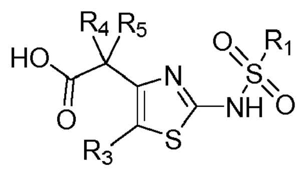

wherein $R_1$, $R_3$, $R_4$ and $R_5$ are as defined herein;

- Compounds of formula (III)

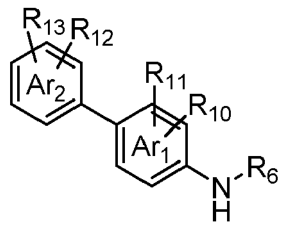

wherein Ar1, Ar2, $R_6$, $R_{10}$, $R_{11}$, $R_{12}$ and $R_{13}$ are as defined herein;

- Compounds of formula (IV)

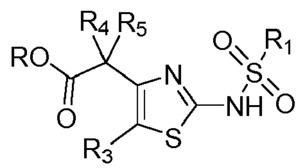

wherein R is $C_{1-6}$alkyl (e.g. methyl, ethyl) or benzyl;

- Compounds of formula (X)

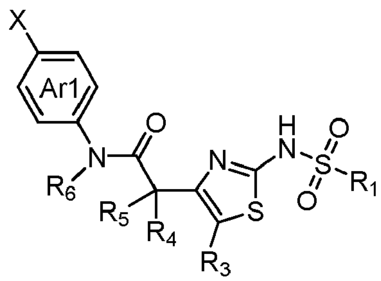

X = Cl, Br, $B(OH)_2$, $B(pin)_2$ ;
wherein Ar1, $R_1$, $R_3$, $R_4$, $R_5$ and $R_6$ are as defined herein;

- Compounds of formula (XI I)

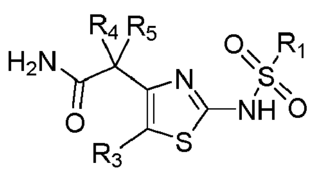

wherein $R_1$, $R_3$, $R_4$ and $R_5$ are as defined herein; and

- Compounds of formula (XIII)

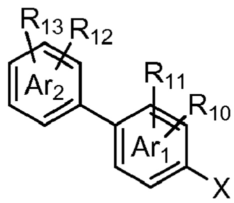

wherein Ar1, Ar2, $R_{10}$, $R_{11}$, $R_{12}$ and $R_{13}$ are as defined herein and X is halo such as Cl.

Included as an aspect of the invention are all novel intermediates described in the examples, including:

- Intermediates A1 to A75; and
- Intermediates B1 to B73.

**Therapeutic Methods**

Compounds of formula (I) of the present invention have utility as inhibitors of CTPS1.

Therefore, the invention also provides a compound of formula (I), or a pharmaceutically acceptable salt and/or solvate (e.g. salt) and/or derivative thereof, for use as a medicament, in particular in the treatment or prophylaxis of a disease or disorder wherein an inhibitor of CTPS1 is beneficial, for example those diseases and disorders mentioned herein below.

The invention provides a method for the treatment or prophylaxis of a disease or disorder wherein an inhibitor of CTPS1 is beneficial, for example those diseases and disorders mentioned herein below, which comprises administering to a subject in need thereof an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the treatment or prophylaxis of a disease or disorder wherein an inhibitor of CTPS1 is beneficial, for example those diseases and disorders mentioned herein below.

More suitably, the disease or disorder wherein an inhibitor of CTPS1 is beneficial is a disease or disorder wherein a reduction in T-cell and/or B-cell proliferation would be beneficial.

The invention also provides a compound of formula (I), or a pharmaceutically acceptable salt and/or solvate (e.g. salt) and/or derivative thereof, for use in the inhibition of CTPS1 in a subject.

The invention provides a method for the inhibition of CTPS1 in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the inhibition of CTPS1 in a subject.

The invention also provides a compound of formula (I), or a pharmaceutically acceptable salt and/or solvate (e.g. salt) and/or derivative thereof, for use in the reduction of T-cell and/or B-cell proliferation in a subject.

The invention provides a method for the reduction of T-cell and/or B-cell proliferation in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the reduction of T-cell and/or B-cell proliferation in a subject.

More suitably, the disease or disorder wherein an inhibitor of CTPS1 is beneficial is a disease or disorder wherein a reduction in T-cell and/or B-cell proliferation would be beneficial.

The term 'treatment' or 'treating' as used herein includes the control, mitigation, reduction, or modulation of the disease state or its symptoms.

The term 'prophylaxis' or 'preventing' is used herein to mean preventing symptoms of a disease or disorder in a subject or preventing recurrence of symptoms of a disease or disorder in an afflicted subject and is not limited to complete prevention of an affliction.

Suitably, the disease or disorder is selected from rejection of transplanted cells and tissues, Graft-related diseases or disorders, allergies and autoimmune diseases.

In one embodiment the disease or disorder is the rejection of transplanted cells and tissues. The subject may have been transplanted with a graft selected from the group consisting of heart, kidney, lung, liver, pancreas, pancreatic islets, brain tissue, stomach, large intestine, small intestine, cornea, skin, trachea, bone, bone marrow (or any other source of hematopoietic precursor cells and stem cells including hematopoietic cells mobilized from bone marrow into peripheral blood or umbilical cord blood cells), muscle, or bladder. The compounds of the invention may be of use in preventing or suppressing an immune response associated with rejection of a donor tissue, cell, graft or organ transplant in a subject.

In a further embodiment the disease or disorder is a Graft-related disease or disorder. Graft-related diseases or disorders include graft versus host disease (GVHD), such as GVHD associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc, and Host-Versus-Graft-Disease (HVGD). The compounds of the invention may be of use in preventing or suppressing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes). Thus the compounds of the invention have utility in preventing Host-Versus-Graft-Disease (HVGD) and Graft-Versus-Host-Disease (GVHD).

A CTPS1 inhibitor may be administered to the subject before, after transplantation and/or during transplantation. In some embodiments, the CTPS1 inhibitor may be administered to the subject on a periodic basis before and/or after transplantation.

In another embodiment, the disease or disorder is an allergy.

In additional embodiments the immune related disease or disorder is an autoimmune disease. As used herein, an "autoimmune disease" is a disease or disorder directed at a subject's own tissues. Examples of autoimmune diseases include, but are not limited to Addison's Disease, Adult-onset Still's disease, Alopecia Areata, Alzheimer's disease, Anti-neutrophil Cytoplasmic Antibodies (ANCA)-Associated Vasculitis, Ankylosing Spondylitis, Anti-phospholipid Syndrome (Hughes' Syndrome), Aplastic Anemia, Arthritis, Asthma, Atherosclerosis, Atherosclerotic plaque, Atopic Dermatitis, Autoimmune Hemolytic Anemia, Autoimmune Hepatitis, Autoimmune Hypophysitis (Lymphocytic Hypophysitis), Autoimmune Inner Ear Disease, Autoimmune Lymphoproliferative Syndrome, Autoimmune Myocarditis, Autoimmune Neutropenia, Autoimmune Oophoritis, Autoimmune Orchitis, Auto-Inflammatory Diseases requiring an immunosuppressive treatment, Azoospermia, Bechet's Disease, Berger's Disease, Bullous Pemphigoid, Cardiomyopathy, Cardiovascular disease, Celiac disease including Refractory Celiac Disease (type I and type II), Chronic Fatigue Immune Dysfunction Syndrome (CFIDS), Chronic Idiopathic Polyneuritis, Chronic Inflammatory Demyelinating Polyneuropathy (CIPD), Chronic Relapsing Polyneuropathy (Guillain-Barré syndrome), Churg-Strauss Syndrome (CSS), Cicatricial Pemphigoid, Cold Agglutinin Disease (CAD), chronic obstructive pulmonary disease (COPD), CREST Syndrome, Cryoglobulin Syndromes, Cutaneous Lupus, Dermatitis Herpetiformis, Dermatomyositis, Eczema, Epidermolysis Bullosa Acquisita, Essential Mixed Cryoglobulinemia, Evan's Syndrome, Exophthalmos, Fibromyalgia, Goodpasture's Syndrome, Grave's disease, Hemophagocytic Lymphohistiocytosis (HLH) (including Type 1 Hemophagocytic Lymphohistiocytosis), Histiocytosis/Histiocytic Disorders, Hashimoto's Thyroiditis, Idiopathic Pulmonary Fibrosis, Idiopathic Thrombocytopenia Purpura (ITP), IgA Nephropathy, Immunoproliferative Diseases or Disorders, Inflammatory Bowel Disease (IBD), Interstitial Lung Disease, Juvenile Arthritis, Juvenile Idiopathic Arthritis (JIA), Kawasaki's Disease, Lambert-Eaton Myasthenic Syndrome, Lichen Planus, Localized Scleroderma, Lupus Nephritis, Ménière's Disease, Microangiopathic Hemoytic Anemia, Microscopic Polyangitis, Miller Fischer Syndrome/Acute Disseminated Encephalomyeloradiculopathy, Mixed Connective Tissue Disease, Multiple Sclerosis (MS), Muscular Rheumatism, Myalgic Encephalomyelitis (ME), Myasthenia Gravis, Ocular Inflammation, Pemphigus Foliaceus, Pemphigus Vulgaris, Pernicious Anemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune Cholangiopathy, Primary Glomerulonephritis, Primary Sclerosing Cholangitis, Psoriasis, Psoriatic Arthritis, Pure Red Cell Anemia, Raynaud's Phenomenon, Reiter's Syndrome/Reactive Arthritis, Relapsing Polychondritis, Restenosis, Rheumatic Fever, Rheumatic Disease, Rheumatoid Arthritis, Sarcoidosis, Schmidt's Syndrome, Scleroderma/Systemic Sclerosis, Sjörgen's Syndrome, Stiff-Man Syndrome, The Sweet Syndrome (Febrile Neutrophilic Dermatosis), Systemic Lupus Erythematosus (SLE), Systemic Scleroderma, Takayasu Arteritis, Temporal Arteritis/Giant Cell Arteritis, Thyroiditis, Type 1 diabetes, Type 2 diabetes, Uveitis, Vasculitis, Vitiligo, Wegener's Granulomatosis, and X-linked lymphoproliferative disease.

Of particular interest are diseases and disorders which are mainly driven by T-cell activation and proliferation, including:

- diseases and disorders which are not linked to alloreactivity including:

    - Alopecia areata, atopic dermatitis, eczema, psoriasis, lichen planus, psoriatic arthritis, vitiligo;

- Uveitis;
- Ankylosing spondylitis, Reiter's syndrome/reactive arthritis;
- Aplastic anemia, autoimmune lymphoproliferative syndrome/disorders, hemophagocytic lymphohistiocytosis;
- Type 1 diabetes; and
- Refractory celiac disease;

- Acute rejection of grafted tissues and transplanted organs; acute graft versus host disease (GVHD) after transplantation of bone marrow cells or any other source of allogenic cells including hematopoietic precursors cells and/or stem cells.

Also of interest are diseases and disorders which are driven by both T- and B-cell activation and proliferation, with an important involvement of B-cells, including:

- diseases and disorders for which the involvement of pathogenic auto-antibodies is well characterized, including:
  - Allergy;
  - Cicatricial pemphigoid, bullous pemphigoid, epidermolysis bullosa acquisita, pemphigus foliaceus, pemphigus vulgaris, dermatitis herpetiformis;
  - ANCA-associated vasculitis and microscopic polyangitis, vasculitis, Wegener's granulomatosis; Churg-Strauss syndrome (CSS), polyarteritis nodosa, cryoglobulin syndromes and essential mixed cryglobulinemia;
  - Systemic lupus erythematosus (SLE), antiphospholipid syndrome (Hughes' syndrome), cutaneous lupus, lupus nephritis, mixed connective tissue disease;
  - Thyroiditis, Hashimoto thyroiditis, Grave's disease, exophthalmos;
  - Autoimmune hemolytic anemia, autoimmune neutropenia, ITP, pernicious anaemia, pure red cell anaemia, micro-angiopathic hemolytic anemia;
  - Primary glomerulonephritis, Berger's disease, Goodpasture's syndrome, IgA nephropathy; and
  - Chronic idiopathic polyneuritis, chronic inflammatory demyelinating polyneuropathy (CIPD), chronic relapsing polyneuropathy (Guillain-Barré syndrome), Miller Fischer syndrome, Stiff man syndrome, Lambert-Eaton myasthenic syndrome, myasthenia gravis.

- diseases and disorders for which the involvement of B-cells is less clearly characterized (although sometimes illustrated by the efficacy of anti-CD20 monoclonal antibodies or intravenous immunoglobulin infusions) and may not correspond or be limited to the production of pathogenic antibodies (nevertheless, non-pathogenic antibodies are sometimes described or even often present and used as a diagnosis biomarker), including:
  - Addison's disease, autoimmune oophoritis and azoospermia, polyglandular syndromes (Whitaker's syndrome), Schmidt's syndrome;
  - Autoimmune myocarditis, cardiomyopathy, Kawasaki's disease;
  - Rheumatoid arthritis, Sjögren's syndrome, mixed connective tissue disease, polymyositis and dermatomyositis; polychondritis;
  - Primary glomerulonephritis;
  - Multiple sclerosis;

• Autoimmune hepatitis, primary biliary cirrhosis/ autoimmune cholangiopathy,

  ▪ Hyper acute rejection of transplanted organs;

  ▪ Chronic rejection of graft or transplants;

  ▪ Chronic Graft versus Host reaction / disease after transplantation of bone marrow cells or hematopoietic precursor cells.

Additionally of interest are diseases and disorders for which the mechanism is shared between activation/proliferation of T-cells and activation/proliferation of innate immune cells and other inflammatory cellular subpopulations (including myeloid cells such as macrophages or granulocytes) and resident cells (such as fibroblasts and endothelial cells), including:

▪ COPD, idiopathic pulmonary fibrosis, interstitial lung disease, sarcoidosis;

▪ Adult onset Still's disease, juvenile idiopathic arthritis, Systemic sclerosis, CREST syndrome where B cells and pathogen antibodies may also play a role; the Sweet syndrome; Takayasu arteritis, temporal arteritis/ giant cell arteritis;

▪ Ulcerative cholangitis, inflammatory bowel disease (IBD) including Crohn's disease and ulcerative colitis, primary sclerosing cholangitis.

Also of interest are diseases and disorders for which the mechanism remains poorly characterized but involves the activation and proliferation of T-cells, including:

▪ Alzheimer's disease, cardiovascular syndrome, type 2 diabetes, restenosis, chronic fatigue immune dysfuntion syndrome (CFIDS).

- Autoimmune Lymphoproliferative disorders, including:

  ◦ Autoimmune Lymphoproliferative Syndrome and X-linked lymphoproliferative disease.

Suitably the disease or disorder is selected from: inflammatory skin diseases such as psoriasis or lichen planus; acute and/or chronic GVHD such as steroid resistant acute GVHD; acute lymphoproliferative syndrome; systemic lupus erythematosus, lupus nephritis or cutaneous lupus; or transplantation..

Suitably the subject is a mammal, in particular the subject is a human.

**Pharmaceutical Compositions**

For use in therapy the compounds of the invention are usually administered as a pharmaceutical composition. The invention also provides a pharmaceutical composition comprising a compound of formula (I), or a pharmaceutically acceptable salt and/or solvate (e.g. salt) and/or derivative thereof, and a pharmaceutically acceptable carrier or excipient.

In one embodiment, there is provided a pharmaceutical composition comprising a compound of formula (I), or a pharmaceutically acceptable salt and/or solvate (e.g. salt) and/or derivative thereof, for use in the treatment or prophylaxis of a disease or disorder as described herein.

In a further embodiment, there is provided a method for the prophylaxis or treatment of a disease or disorder as described herein, which comprises administering to a subject in need thereof an effective amount of a pharmaceutical composition comprising a compound of formula (I) or a pharmaceutically acceptable salt and/or solvate (e.g. salt) and/or derivative thereof.

The invention also provides the use of a pharmaceutical composition comprising a compound of formula (I), or a pharmaceutically acceptable salt and/or solvate thereof (e.g. salt) and/or derivative thereof, in the manufacture of a medicament for the treatment or prophylaxis of a disease or disorder as described herein.

The compounds of formula (I) or their pharmaceutically acceptable salts and/or solvates and/or derivatives thereof may be administered by any convenient method, e.g. by oral, parenteral, buccal, sublingual, nasal, rectal or transdermal administration, and the pharmaceutical compositions adapted accordingly.

The compounds of formula (I) or their pharmaceutically acceptable salts and/or solvates and/or derivatives thereof which are active when given orally can be formulated as liquids or solids, e.g. as syrups, suspensions, emulsions,

tablets, capsules or lozenges.

A liquid formulation will generally consist of a suspension or solution of the active ingredient (such as a compound of formula (I) or a pharmaceutically acceptable salt and/or solvate (e.g. salt) and/or derivative thereof) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient (such as a compound of formula (I) or a pharmaceutically acceptable salt and/or solvate (e.g. salt) and/or derivative thereof) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient (such as a compound of formula (I) or a pharmaceutically acceptable salt and/or solvate (e.g. salt) and/or derivative thereof) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluoro-chloro-hydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Suitably, the composition is in unit dose form such as a tablet, capsule or ampoule.

The composition may for example contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05 mg to 2000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment or prophylaxis of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg, and such unit doses may be administered more than once a day, for example two or three a day. Such therapy may extend for a number of weeks or months.

The invention provides, in a further aspect, a combination comprising a compound of formula (I) or a pharmaceutically acceptable, salt, solvate and/or derivative thereof (e.g. a combination comprising a compound of formula (I) or a pharmaceutically acceptable derivative thereof) together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered separately, sequentially or simultaneously by any convenient route.

Optimal combinations may depend on the disease or disorder. Possible combinations include those with one or more active agents selected from the list consisting of: 5-aminosalicylic acid, or a prodrug thereof (such as sulfasalazine, olsalazine or bisalazide); corticosteroids (e.g. prednisolone, methylprednisolone, or budesonide); immunosuppressants (e.g. cyclosporin, tacrolimus, sirolimus, methotrexate, azathioprine mycophenolate mofetil, leflunomide, cyclophosphamide, 6-mercaptopurine or anti-lymphocyte (or thymocyte) globulins); anti-TNF-alpha antibodies (e.g., infliximab, adalimumab, certolizumab pegol or golimumab); anti-IL12/IL23 antibodies (e.g., ustekinumab); anti-IL6 or anti-IL6R antibodies, anti-IL17 antibodies or small molecule IL12/IL23 inhibitors (e.g., apilimod); Anti-alpha-4-beta-7 antibodies (e.g., vedolizumab); MAdCAM-1 blockers (e.g., PF-00547659); antibodies against the cell adhesion molecule alpha-4-integrin (e.g., natalizumab); antibodies against the IL2 receptor alpha subunit (e.g., daclizumab or basiliximab); JAK inhibitors including JAK1 and JAK3 inhibitors (e.g., tofacitinib, baricitinib, R348); Syk inhibitors and prodrugs thereof (e.g., fostamatinib and R-406); Phosphodiesterase-4 inhibitors (e.g., tetomilast); HMPL-004; probiotics; Dersalazine; semapimod/CPSI-2364; and protein kinase C inhibitors (e.g. AEB-071).

Some of the combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

The invention is further exemplified by the following non-limiting examples.

## EXAMPLES

Abbreviations used herein are defined below. Any abbreviations not defined are intended to convey their generally accepted meaning.

**Table 1:** Abbreviations

| | |
|---|---|
| AcOH | glacial acetic acid |
| $AlMe_3$ | trimethylaluminium |
| aq | aqueous |
| BEH | ethylene bridged hybrid |
| Boc | *tert*-butyloxycarbonyl |
| br | broad |
| CatCart® | catalytic cartridge |
| $CS_2CO_3$ | cesium carbonate |
| CSH | charged surface hybrid |
| d | doublet |
| DBU | 1,8-diazabicyclo[5.4.0]undec-7-ene |
| DCM | dichloromethane |
| DIPEA | N,N-diisopropylethylamine |
| dioxane | 1,4-dioxane |
| DMAP | 4-(dimethylamino)pyridine |
| DMF | N,N-dimethylformamide |
| DMSO (-d6) | dimethyl sulfoxide (hexadeuterodimethyl sulfoxide) |
| dppf | 1,1'-bis(diphenylphosphino)ferrocene |
| ee | Enantiomeric excess |
| $(ES^+)$ | electrospray ionisation, positive mode |
| $(ES^-)$ | electrospray ionisation, negative mode |
| ESI | electrospray ionisation |
| Et | ethyl |
| EtOAc | ethyl acetate |
| EtOH | ethanol |
| g | grams |
| HATU | 1-[bis(dimethylamino)methylene]-1*H*-1,2,3-triazolo[4,5-*b*]pyridinium 3-oxid hexafluorophosphate |
| HPLC | high performance liquid chromatography |
| hr(s) | hour(s) |
| $IC_{50}$ | 50% inhibitory concentration |
| IPA | isopropyl alcohol |
| $^iPr$ | isopropyl |
| $K_2CO_3$ | potassium carbonate |
| LCMS | liquid chromatography-mass spectrometry |
| LiOH | lithium hydroxide |

(continued)

| | |
|---|---|
| LiHMDS | lithium bis(trimethylsilyl)amide |
| $(M+H)^+$ | protonated molecular ion |
| $(M-H)^-$ | unprotonated molecular ion |
| M | molar concentration |
| mL | millilitre |
| mmol | millimole |
| $MgSO_4$ | magnesium sulfate |
| Me | methyl |
| MeCN | acetonitrile |
| MeI | iodomethane |
| MeOH | methanol |
| MHz | megahertz |
| min(s) | minute(s) |
| MSD | mass selective detector |
| m/z | mass-to-charge ratio |
| $NaHCO_3$ | sodium bicarbonate |
| $Na_2S_2O_3$ | sodium thiosulfate |
| NCS | *N*-chlorosuccinimide |
| $NH_4OH$ | ammonium hydroxide |
| nm | nanometre |
| nM | nanomolar |
| NMR | nuclear magnetic resonance (spectroscopy) |
| OD | optical density |
| PDA | photodiode array |
| Pd 170 | chloro(crotyl)(2-dicyclohexylphosphino-2',4',6'-triisopropybiphenyl)palladium(II) or XPhos Pd (crotyl)Cl |
| Pd 174 | allyl(2-di-*tert*-butylphosphino-2',4',6'-triisopropyl-1,1'-biphenyl)palladium(II) triflate or [*t*BuXPhosPd(allyl)]OTf |
| Pd 177 | (4,5-bis(diphenylphosphino)-9,9-dimethylxanthene)(allyl)palladium(II) chloride or [XantPhosPd (allyl)]Cl |
| $PdCl_2$(dppf) | [1,1'-bis(diphenylphosphino)ferrocene]dichloropalladium(II) |
| $Pd(PPh_3)_4$ | tetrakis(triphenylphosphine)palladium(0) |
| $Pd(t\text{-}Bu_3P)_2$ | bis(tri-*tert*-butylphosphine)palladium(0) |
| prep HPLC | preparative high performance liquid chromatography |
| Ph | phenyl |
| PMB | para-methoxybenzyl |
| q | quartet |
| RT | room temperature |
| Rt | retention time |
| RP | reverse phase |
| s | singlet |
| sat | saturated |
| SCX | solid supported cation exchange (resin) |
| SDS | sodium dodecyl sulphate |
| t | triplet |
| T3P | 2,4,6-tripropyl-1,3,5,2,4,6-trioxatriphosphorinane-2,4,6-trioxide |
| *t*BuXPhos-Pd-G3 | [(2-di-*tert*-butylphosphino-2',4',6'-triisopropyl-1,1'-biphenyl)-2-(2'-amino-1,1'-biphenyl)] palladium(II) methanesulfonate |
| THF | tetrahydrofuran |
| UPLC | ultra performance liquid chromatography |
| UV | ultraviolet |

(continued)

| | |
|---|---|
| v/v | volume/volume |
| VWD | variable wave detector |
| um | micrometre |
| uM | micromolar |
| uL | microlitre |
| °C | degrees Celsius |

**General Procedures**

All starting materials and solvents were obtained either from commercial sources or prepared according to the literature citation. Unless otherwise stated all reactions were stirred. Organic solutions were routinely dried over anhydrous magnesium sulfate. Hydrogenations were performed on a Thales H-cube flow reactor under the conditions stated.

Column chromatography was performed on pre-packed silica (230-400 mesh, 40-63 um) cartridges using the amount indicated. SCX was purchased from Supelco and treated with 1 M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 0.7M $NH_3$ in MeOH.

*Preparative Reverse Phase High Performance Liquid Chromatography*

**Prep HPLC**

***Acidic prep***

Waters X-Select CSH column C18, 5 um (19 x 50 mm), flow rate 28 mL $min^{-1}$ eluting with a $H_2O$-MeCN gradient containing 0.1 % v/v formic acid over 6.5 min using UV detection at 254 nm.

***Basic prep***

Waters X-Bridge Prep column C18, 5 um (19 x 50 mm), flow rate 28 mL $min^{-1}$ eluting with a 10 mM $NH_4HCO_3$-MeCN gradient over 6.5 min using UV detection at 254 nm.

**Analytical Methods**

*Reverse Phase HPLC Conditions for the LCMS Analytical Methods*

**HPLC acidic:** Acidic LCMS 4 minute (5-95%)

Analytical LCMS was carried out using a Waters X-Select CSH C18, 2.5 um, 4.6x30 mm column eluting with a gradient of 0.1% Formic acid in MeCN in 0.1% Formic acid in water. The gradient from 5-95% 0.1% Formic acid in MeCN occurs between 0.00-3.00 minutes at 2.5ml/min with a flush from 3.01-3.5 minutes at 4.5ml/min. A column re-equilibration to 5% MeCN is from 3.60-4.00 minutes at 2.5ml/min. UV spectra of the eluted peaks were measured using an Agilent 1260 Infinity VWD at 254nm. Mass spectra were measured using an Agilent 6120 MSD running with positive/negative switching.

**HPLC basic:** Basic LCMS 4 minute (5-95%)

Analytical LCMS was carried out using a Waters X-Select BEH C18, 2.5 um, 4.6x30 mm column eluting with a gradient of MeCN in aqueous 10mM ammonium bicarbonate. The gradient from 5-95% MeCN occurs between 0.00-3.00 minutes at 2.5ml/min with a flush from 3.01-3.5 minutes at 4.5ml/min. A column re-equilibration to 5% MeCN is from 3.60-4.00 minutes at 2.5ml/min. UV spectra of the eluted peaks were measured using an Agilent 1260 Infinity VWD at 254nm. Mass spectra were measured using an Agilent 6120 MSD running with positive/negative switching.

*Reverse Phase HPLC Conditions for the UPLC Analytical Methods*

**UPLC acidic:** Acidic UPLC 3 minute

Analytical UPLC/MS was carried out using a Waters Acquity CSH C18, 1.7 um, 2.1x30 mm column eluting with a gradient of 0.1% Formic acid in MeCN in0.1% Formic acid in water. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**UPLC basic:** Basic UPLC 3 minute

Analytical UPLC/MS was carried out using a Waters Acquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

Column temperature is 40 °C in all runs. Injection volume is 3ul and the flow rate is 0.77ml/min. PDA scan from 210-400nm on all runs.

*Normal Phase HPLC Conditions for the Chiral Analytical Methods*

**Chiral IA method:** Chiral HPLC (Diacel Chiralpak IA, 5 um, 4.6x250 mm, 1.0 ml/min, 30% EtOH in [4:1 isohexane (0.2% TFA): DCM].

**Chiral IC method:** Chiral HPLC (Diacel Chiralpak IC, 5 um, 4.6x250 mm, 1.0 ml/min, 30% EtOH (0.2% TFA) in [4:1 isohexane (0.2% TFA):$CHCl_3$.

*$^1H$ NMR Spectroscopy*

$^1H$ NMR spectra were acquired on a BrukerAvance III spectrometer at 400 MHzorBrukerAvance III HD spectrometer at 500 MHz using residual undeuterated solvent as reference and unless specified otherwise were run in DMSO-d6.

**Preparation of Intermediates**

Known synthetic intermediates were procured from commercial sources or were obtained using published literature procedures. Additional intermediates were prepared by the representative synthetic processes described herein.

**Thiazole intermediate preparation**

Ethyl 2-(2-aminothiazol-4-yl)propanoate **INTB1**

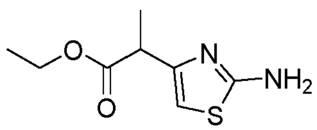

To a solution of ethyl 2-methyl-3-oxobutanoate (3.93 mL, 27.7 mmol) in chloroform (40 mL) protected from light was added pyridinium tribromide (9.34 g, 27.7 mmol). The reaction was heated to 40 °C for 2 hrs. The reaction mixture was concentrated in *vacuo.* This was then taken up in EtOH (40 mL) and thiourea (2.11 g, 27.7 mmol) was added. The reaction was heated to 70 °C and after 2 hrs the reaction mixture was allowed to cool to RT. This was then concentrated and heated to reflux in EtOAc (10 mL) for 30 min before being allowed to cool to RT. This was then concentrated onto silica. The crude product was purified by chromatography on silica gel (120 g column, 10-70% EtOAc/iso-hexane) to

afford ethyl 2-(2-aminothiazol-4-yl)propanoate (2.8 g, 12.58 mmol, 45 % yield) as a yellow gum; Rt 0.83 min (UPLC basic); m/z 201 $(M+H)^+$ $(ES^+)$; $^1H$ NMR (400 MHz, DMSO-d6) δ 6.91 (s, 2H), 6.28 (d, J = 0.8 Hz, 1 H), 4.13 - 3.93 (m, 2H), 3.62 - 3.46 (m, 1 H), 1.32 (d, J = 7.1 Hz, 3H), 1.16 (t, J = 7.1 Hz, 3H).

Methyl 2-(2-aminothiazol-4-yl)-2-methylpropanoate **INTB2**

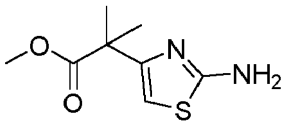

To a solution of methyl 2,2-dimethyl-3-oxobutanoate (9.01 mL, 62.4 mmol) in MeOH (70 mL) at 0 °C was added bromine (4.82 mL, 94 mmol) dropwise over 10 min. The reaction was stirred at 0 °C for 10 min. The reaction mixture was then heated at 30 °C for 2 hrs. After cooling to RT, the reaction mixture was diluted with water (100 mL) was concentrated in *vacuo.* The product was extracted using EtOAc (3 x 100 mL). The combined organics were dried ($Na_2SO_4$) and concentrated in *vacuo.* The resulting orange oil was dissolved in MeOH (50 mL) and thiourea (4.75 g, 62.4 mmol) was added. The reaction mixture was heated at 40 °C for 1 hr, then stirred at RT for 18 hrs. The reaction mixture was concentrated *vacuo* to give a pale yellow solid. The residue was recrystallised from MeOH (40 mL). The resultant solid was filtered, rinsed with MeOH (10 mL), and dried in *vacuo* to afford methyl 2-(2-aminothiazol-4-yl)-2-methylpropanoate (10.4 g, 50.8 mmol, 81 % yield) as a colourless solid; Rt 0.85 min (UPLC basic); m/z 201 $(M+H)^+$ $(ES^+)$; $^1H$ NMR (400 MHz, DMSO-d6) δ 6.92 (s, 2H), 6.28 (s, 1 H), 3.55 (s, 3H), 1.40 (s, 6H).

Ethyl 2-(2-aminothiazol-4-yl)butanoate **INTB3**

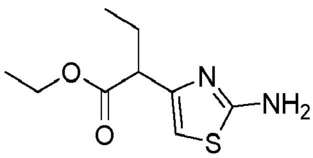

Bromine (5.13 mL, 100 mmol) was added dropwise (over 10 min) to a solution of ethyl 2-ethyl-3-oxobutanoate (10.5 g, 66.4 mmol) in EtOH (70 mL) at 0 °C. The reaction was stirred at 0 °C for 10 mins then heated to 30 °C for 2 hrs. The reaction mixture was cooled to RT. The solvent was removed in *vacuo* and then diluted with water (100 mL) and EtOAc (100 mL). The aqueous phase was extracted further with EtOAc (2 x 100 mL) and the combined organic phases were passed through a phase separator and solvent removed in *vacuo.* This was dissolved in EtOH (50 mL) and thiourea (5.05 g, 66.4 mmol) was added. The reaction mixture was heated at 40 °C for 1 hr, then stirred at RT for 18 hrs. The reaction mixture was concentrated in *vacuo,* then EtOAc (100 mL) was added. The organic phase was washed with a solution of $Na_2S_2O_3$ (aq. 100 mL), $NaHCO_3$ (aq. 100 mL), passed through a phase separator and solvent removed in *vacuo.* The crude product was purified by chromatography on silica gel (120 g column, 0-100% EtOAc/iso-hexane) to afford ethyl 2-(2-aminothiazol-4-yl)butanoate (6.18 g, 28.0 mmol, 42 % yield) as a pale yellow solid; Rt 0.88 min (HPLC acidic); m/z 215 $(M+H)^+$ $(ES^+)$; $^1H$ NMR (400 MHz, DMSO-d6) δ 6.91 (s, 2H), 6.28 (s, 1 H), 4.15 - 3.97 (m, 2H), 3.37 (t, J = 7.3 Hz, 1 H), 1.92 - 1.70 (m, 2H), 1.16 (t, J = 7.1 Hz, 3H), 0.83 (t, J = 7.4 Hz, 3H).

**Method A: Formation of thiazole amines from ketoesters**

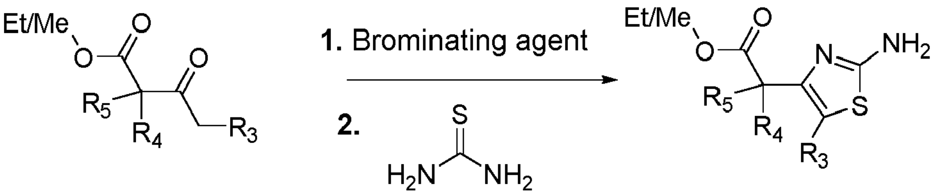


To a solution of ketoester (1 eq) in an alcoholic solvent such as MeOH or EtOH (1 volume) at 0 °C was added bromine (1.5 eq) dropwise over 10 mins. The reaction was stirred at 0 °C for 10 mins. The reaction mixture was then heated at 30 °C for 2 hrs. After cooling to RT the reaction mixture was diluted with water. The product was extracted using an appropriate solvent such as EtOAc. The combined organics were dried ($Na_2SO_4$) and concentrated in *vacuo.* The resulting compound was dissolved in alcoholic solvent such as MeOH or EtOH (1 volume) and thiourea (1 eq) was added. The reaction mixture was heated at 40 °C for 1 hr, then stirred at RT for 18 hrs. The reaction mixture was concentrated in *vacuo* and purified by normal phase chromatography or *via* trituration with an appropriate solvent.

**Table 2:** Additional intermediates made according to Method A.

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB4 | ethyl 2-(2-am inothiazol-4-yl)-3-methylbutanoate 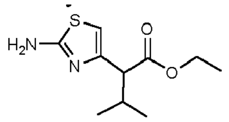 | Method A, [HPLC basic], 299, (1.77). | 6.91 (s, 2H), 6.31 (s, 1H), 4.14 - 3.96 (m, 2H), 3.16 (d, J = 9.7 Hz, 1H), 2.25 (dp, J = 9.6, 6.7 Hz, 1H), 1.20 - 1.11 (m, 3H), 0.91 (d, J = 6.7 Hz, 3H), 0.77 (d, J = 6.7 Hz, 3H). |
| INTB5 | methyl 2-(2-amino-5-methylthiazol-4-yl)-2-methylpropanoate 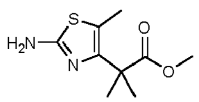 | Method A, [UPLC acidic], 215, (0.46). | 6.60 (s, 2H), 3.61 (s, 3H), 2.00 (s, 3H), 1.41 (s, 6H). |
| INTB6 | ethyl 2-(2-am inothiazol-4-yl)-4-m ethoxybutanoate 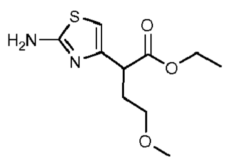 | Method A, [UPLC acidic], 245, (0.44). | 6.96 (s, 2H), 6.30 (s, 1H), 4.15 - 3.90 (m, 2H), 3.57 (t, J = 7.4 Hz, 1H), 3.29 - 3.23 (m, 2H), 3.19 (s, 3H), 2.17 - 1.83 (m, 2H), 1.15 (t, J = 7.1 Hz, 3H). |
| INTB61 | ethyl 2-(2-aminothiazol-4-yl)-2-ethylbutanoate 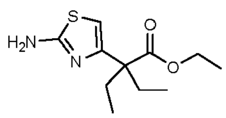 | Method A, [UPLC basic], 243, (1.24). | 8.58 (v. br, 2H), 6.73 (s, 1H), 4.20 - 4.00 (m, 2H), 1.89 (q, J = 7.0, 5.7 Hz, 4H), 1.22 1.13 (m, 3H), 0.71 (t, J = 7.1 Hz, 6H). |

Methyl 2-(2-((tert-butoxycarbonyl)(4-methoxybenzyl)amino)thiazol-4-yl)acetate **INTB7**

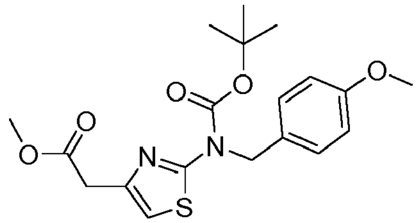

A solution of methyl 2-(2-((tert-butoxycarbonyl)amino)thiazol-4-yl)acetate (7.3 g, 26.8 mmol) in DCM (100 mL) was treated with DBU (7.84 mL, 52.0 mmol) and 1-(bromomethyl)-4-methoxybenzene (3 mL, 20.8 mmol). The solution was then stirred at RT for 18 hrs. 1 M HCl (aq, 100 mL) was added and the mixture was passed through a phase separator, further extracting with DCM (50 mL). The organic phases were combined and concentrated on to silica (20 g). The crude product was purified by chromatography on silica gel (80 g column, 0-20% EtOAc/isohexane) to afford methyl 2-(2-((tert-butoxycarbonyl)(4-methoxybenzyl)amino)thiazol-4-yl)acetate (2.5 g, 6.24 mmol, 30.0 % yield) as an off-white gum; Rt 1.76 mins (UPLC acidic); m/z 415 $(M+Na)^+$$(ES^+)$; $^1$H NMR (400 MHz, DMSO-d6) δ 7.31 - 7.22 (m, 2H), 7.02 (s, 1 H), 6.93 - 6.76 (m, 2H), 5.13 (s, 2H), 3.71 (s, 3H), 3.71 - 3.70 (m, 2H), 3.62 (s, 3H), 1.47 (s, 9H).

Methyl 2-(2-((tert-butoxycarbonyl)(4-methoxybenzyl)amino)thiazol-4-yl)-2-diazoacetate **INTB8**

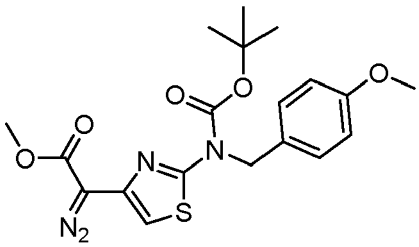

A solution of methyl 2-(2-((tert-butoxycarbonyl)(4-methoxybenzyl)amino)thiazol-4-yl)acetate **INTB7** (2.4 g, 6.12 mmol) in MeCN (30 mL) was treated with 4-methylbenzenesulfonyl azide (30% wt. in toluene) (4.42 mL, 6.73 mmol) followed by dropwise addition of DBU (1.383 mL, 9.17 mmol). The reaction mixture was allowed to stir for 1 hr. The mixture was poured onto ice/water (50 mL) and extracted with EtOAc (3 x 50 mL). The organic phases were combined, dried ($Na_2SO_4$), filtered and concentrated on to silica (10 g). The crude product was purified by chromatography on silica gel (40 g column, 0-15% EtOAc/isohexane) to afford methyl 2-(2-((tert-butoxycarbonyl)(4-methoxybenzyl)amino)thiazol-4-yl)-2-diazoacetate (2.01 g, 4.71 mmol, 77 % yield) as a yellow gum; Rt 2.02 mins (UPLC acidic); m/z 441 $(M+Na)^+$ $(ES^+)$; $^1$H NMR (400 MHz, DMSO-d6) δ 7.36 - 7.16 (m, 3H), 6.99 - 6.76 (m, 2H), 5.10 (s, 2H), 3.81 (s, 3H), 3.72 (s, 3H), 1.51 (s, 9H).

Methyl 2-(2-((tert-butoxycarbonyl)(4-methoxybenzyl)amino)thiazol-4-yl)-2-methoxyacetate **INTB9**

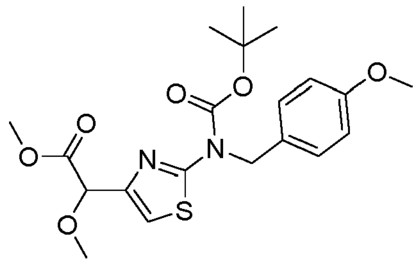

A solution of methyl 2-(2-((tert-butoxycarbonyl)(4-methoxybenzyl)amino)thiazol-4-yl)-2-diazoacetate **INTB8** (2 g, 4.78 mmol) in DCM (25 mL) was treated with MeOH (0.23 mL, 5.74 mmol), then rhodium(II) acetate dimer (0.042 g, 0.10 mmol) was added resulting in effervescence. After 5 mins the reaction mixture was diluted with DCM (25 mL) and

washed with water (50 mL) and brine (50 mL). The organic phase was dried ($Na_2SO_4$), filtered and concentrated onto silica (5 g) then the crude product was purified by chromatography on silica gel (24 g column, 0-15% EtOAc/isohexane) to afford methyl 2-(2-((tert-butoxycarbonyl)(4-methoxybenzyl)amino)thiazol-4-yl)-2-methoxyacetate (1.68 g, 3.78 mmol, 79 % yield) as a colourless oil; Rt 1.69 mins (UPLC acidic); m/z 445 $(M+Na)^+$ $(ES^+)$; $^1H$ NMR (400 MHz, DMSO-d6) δ 7.30 - 7.20 (m, 3H), 6.90 - 6.83 (m, 2H), 5.17 - 5.02 (m, 2H), 4.98 (s, 1 H), 3.71 (s, 3H), 3.64 (s, 3H), 3.31 (s, 3H), 1.49 (s, 9H).

Methyl 2-(2-aminothiazol-4-yl)-2-methoxyacetate **INTB10**

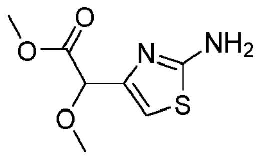

Methyl 2-(2-((tert-butoxycarbonyl)(4-methoxybenzyl)amino)thiazol-4-yl)-2-methoxyacetate **INTB9** (1.68 g, 3.78 mmol) was taken up in TFA (7 mL, 91 mmol) and heated to reflux for 5 hrs. The reaction mixture was concentrated in *vacuo,* then taken up in EtOAc (30 mL) and sat. $NaHCO_3$ (sat. aq, 30 mL), the phases were partitioned and the organic phase was washed with brine (20 mL). The organic phase was then dried ($MgSO_4$), filtered and concentrated on to silica (5 g). The crude product was purified by chromatography on silica gel (24 g column, 0-40% EtOAc/isohexane) to afford methyl 2-(2-aminothiazol-4-yl)-2-methoxyacetate (548 mg, 2.66 mmol, 56 % yield) as an off-white solid; Rt 0.31 mins (HPLC acidic); m/z not recorded; $^1H$ NMR (400 MHz, DMSO-d6) δ 7.00 (s, 2H), 6.55 (s, 1 H), 4.72 (s, 1 H), 3.32 (s, 3H), 3.27 (s, 3H).

Methyl 2-(2-bromothiazol-4-yl)-2-diazoacetate **INTB11**

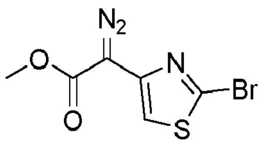

A solution of methyl 2-(2-bromothiazol-4-yl)acetate (2.5 g, 10.6 mmol) in MeCN (80 mL) was treated with 4-acetamidobenzenesulfonyl azide (2.80 g, 11.7 mmol) followed by dropwise addition of DBU (2.394 mL, 15.88 mmol). The reaction was then allowed to stir at RT for 5 mins. The reaction mixture was then poured onto ice/water (100 mL). The aqueous layer was extracted with EtOAc (3 x 50 mL), the organic phases were combined, dried ($MgSO_4$), filtered and concentrated in *vacuo.* The crude product was purified by chromatography on silica gel (24 g column, 0-10% EtOAc/isohexane) to afford methyl 2-(2-bromothiazol-4-yl)-2-diazoacetate (1.85 g, 6.92 mmol, 65 % yield) as a yellow solid; Rt 1.46 mins (HPLC acidic); m/z 262 $(^{79}Br\ M+H)^+$ $(ES^+)$; $^1H$ NMR (400 MHz, DMSO-d6) δ 7.65 (s, 1 H), 3.82 (s, 3H).

Methyl 2-(2-bromothiazol-4-yl)-2-isopropoxyacetate **INTB12**

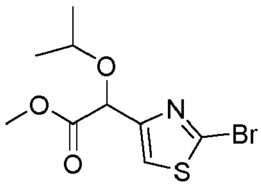

A solution of methyl 2-(2-bromothiazol-4-yl)-2-diazoacetate **INTB11** (475 mg, 1.81 mmol) in DCM (25 mL) was treated with IPA (0.140 mL, 1.81 mmol) then rhodium(II) acetate dimer (4 mg, 9.05 umol) was added resulting in effervescence. After 5 mins the reaction mixture was diluted with DCM (25 mL) and washed with water (50 mL) and brine (50 mL). The organic phase was dried ($Na_2SO_4$), filtered and concentrated on to silica (5 g) then the crude product was

purified by chromatography onto silica gel (24 g column, 0-15% EtOAc/isohexane) to afford methyl 2-(2-bromothiazol-4-yl)-2-isopropoxyacetate (297 mg, 0.99 mmol, 55 % yield) as a colourless oil; Rt 1.22 mins (UPLC acidic); m/z 316 ($^{79}$Br M+Na)$^+$ (ES$^+$); $^1$H NMR (400 MHz, DMSO-d6) δ 7.76 (d, J = 0.6 Hz, 1H), 5.26 (s, 1 H), 3.73 - 3.68 (m, 1 H), 3.66 (s, 3H), 1.14 (d, J = 6.1 Hz, 3H), 1.11 (d, J = 6.1 Hz, 3H).

2-(2-(cyclopropanesulfonamido)thiazol-4-yl)-2-isopropoxyacetic acid **INTB13**

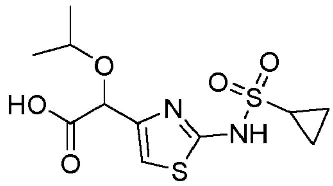

A suspension of methyl 2-(2-bromothiazol-4-yl)-2-isopropoxyacetate **INTB12** (295 mg, 1 mmol), cyclopropanesulfonamide (146 mg, 1.2 mmol) and cesium carbonate (653 mg, 2 mmol) in dioxane (20 mL) at 40 °C was degassed ($N_2$, 5 mins) then Pd 174 (36.2 mg, 0.05 mmol) was added. The mixture was further degassed ($N_2$, 5 mins) before being warmed to 90 °C. The reaction was left at 90 °C for 16 hrs. The reaction mixture was allowed to cool to RT then acidified carefully with 1 M HCl (aq, 5 mL). This was then extracted with EtOAc (3 x 40 mL), the organic phases were combined, dried ($Na_2SO_4$), filtered and concentrated on to silica (1 g). The crude product was purified by chromatography on silica gel (24 g column, 0-100% EtOAc (5% MeOH)/iso-hexane) to afford 2-(2-(cyclopropanesulfonamido)thiazol-4-yl)-2-isopropoxyacetic acid (59 mg, 0.19 mmol, 19 % yield) as a brown solid; Rt 1.39 mins (HPLC acidic); m/z 321 (M+H)$^+$ (ES$^+$).

Ethyl 2-(2-aminothiazol-4-yl)-2,2-difluoroacetate hydrochloride **INTB14**

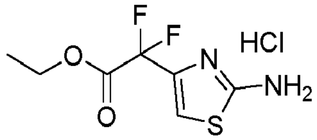

A solution of ethyl 2-(2-((tert-butoxycarbonyl)amino)thiazol-4-yl)-2,2-difluoroacetate (1.6 g, 4.96 mmol) in 4M HCl (dioxane, 12.4 mL, 49.6 mmol) was stirred at RT for 18 hrs. The reaction mixture was concentrated to afford ethyl 2-(2-aminothiazol-4-yl)-2,2-difluoroacetate, HCl (1.3 g, 4.77 mmol, 96 % yield) as a colourless solid; Rt 0.95 min (UPLC acidic); m/z 223 (M+H)$^+$ (ES$^+$); $^1$H NMR (400 MHz, DMSO-d6) δ 7.83 (v. br. s, 3H), 7.09 (t, J = 1.4 Hz, 1 H), 4.30 (q, J = 7.1 Hz, 2H), 1.24 (t, J = 7.1 Hz, 3H). $^{19}$F NMR (376 MHz, DMSO-d6) δ -101.55 (d, J = 1.5 Hz).

Methyl 2-(2-(methylsulfonamido)thiazol-4-yl)acetate **INTB15**

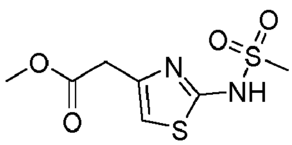

A solution of methyl 2-(2-aminothiazol-4-yl)acetate (3.56 g, 20.7 mmol) and DMAP (0.25 g, 2.07 mmol) in pyridine (16.74 mL, 207 mmol) was cooled to 0 °C whereupon methanesulfonyl chloride (1.61 mL, 20.70 mmol) was added dropwise. The yellow solution was allowed to warm to RT and stirred for 1 hr then added to 1 M HCl (aq, 228 mL) and stirred for 30 min. The resulting precipitate was then filtered and washed with $Et_2O$ (10 mL) to afford a yellow solid. This was slurried in $Et_2O$ (20 mL) and filtered to afford methyl 2-(2-(methylsulfonamido)thiazol-4-yl)acetate (3.56 g, 13.94 mmol, 67 % yield) as a colourless solid; Rt 0.83 min (HPLC acidic); m/z 251 (M+H)$^+$ (ES$^+$); 249 (M-H)$^-$ (ES$^-$); $^1$H NMR (400 MHz, DMSO-d6) δ 12.47 (s, 1 H), 6.60 (s, 1 H), 3.67 (d, J = 0.9 Hz, 2H), 3.66 (s, 3H), 2.90 (s, 3H).

Methyl 2-(2-(cyclopropanesulfonamido)thiazol-4-yl)acetate **INTB16**

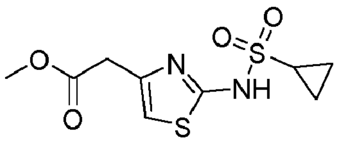

A solution of methyl 2-(2-aminothiazol-4-yl)acetate (6.7 g, 38.9 mmol) and cyclopropanesulfonyl chloride (4.33 mL, 42.8 mmol) in pyridine (19 mL) was warmed to 40 °C and stirred for 18 hrs. The reaction mixture was diluted with pyridine (10 mL) and DMSO (40 mL) and the crude product was purified by chromatography on C18-RP silica gel (330 g column, 10-20% MeCN/10 mM Ammonium bicarbonate) to afford the required product. This was then triturated with MeOH (20 mL) and filtered to afford methyl 2-(2-(cyclopropanesulfonamido)thiazol-4-yl)acetate (2.4 g, 8.51 mmol, 22 % yield) as a colourless solid; Rt 0.69 min (UPLC acidic); m/z 277 $(M+H)^+$ $(ES^+)$; $^1H$ NMR (400 MHz, DMSO-d6) δ 12.52 (s, 1 H), 6.57 (s, 1 H), 3.67 (s, 2H), 3.66 (s, 3H), 2.62 - 2.54 (m, 1 H), 0.91 - 0.88 (m, 4H).

Methyl 2-(2-(cyclopropanesulfonamido)thiazol-4-yl)-2-methylpropanoate **INTB17**

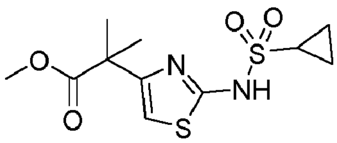

A solution of methyl 2-(2-aminothiazol-4-yl)-2-methylpropanoate **INTB2** (3.99 g, 19.9 mmol) in pyridine (9.67 mL) was heated to 40 °C. Cyclopropanesulfonyl chloride (2.22 mL, 21.9 mmol) was added dropwise. The reaction mixture was cooled to RT after 1 hr. The reaction mixture was poured onto 1 M HCl (aq, 120 mL) and diluted with DCM (50 mL), the phases were separated and the aqueous was extracted with DCM (2 x 50 mL). The combined organics were dried ($Na_2SO_4$) and concentrated in *vacuo.* The crude product was purified by chromatography on silica gel (80 g column, 20-80% EtOAc/iso-hexane). The resulting solid was recrystallised from MeOH (10 mL), filtered, rinsing with MeOH and dried in *vacuo* to afford methyl 2-(2-(cyclopropanesulfonamido)thiazol-4-yl)-2-methylpropanoate (1.41 g, 4.4 mmol, 22 %) as a pale brown cystalline solid; Rt 0.76 min (UPLC basic); m/z 305 $(M+H)^+$ $(ES^+)$; $^1H$ NMR (400 MHz, DMSO-d6) δ 12.62 (s, 1 H), 6.56 (s, 1 H), 3.65 (s, 3H), 2.71 - 2.57 (m, 1 H), 1.46 (s, 6H), 1.00 - 0.81 (m, 4H).

Ethyl 2-(2-(cyclopropanesulfonamido)thiazol-4-yl)butanoate **INTB18**

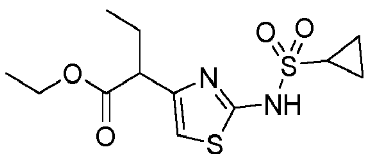

Ethyl 2-(2-aminothiazol-4-yl)butanoate **INTB3** (3.8 g, 17.7 mmol) in pyridine (7.17 mL) at 40 °C was treated with cyclopropanesulfonyl chloride (1.88 mL, 18.6 mmol) and stirred for 18 hrs. The mixture was cooled to RT then cyclopropanesulfonyl chloride (0.5 mL, 4.94 mmol) was added and the mixture stirred for another 3 hrs. The reaction mixture was then taken up in EtOAc (100 mL) and washed with 1 M HCl (aq, 106 mL) and brine (80 mL) before being dried ($Na_2SO_4$), filtered and concentrated on to silica. The crude product was purified by chromatography on silica gel (80 g column, 10-40% EtOAc/iso-hexane) to afford ethyl 2-(2-(cyclopropanesulfonamido)thiazol-4-yl)butanoate (2 g, 5.70 mmol, 32 % yield) as a red gum; Rt 1.04 min (UPLC acidic); m/z 319 $(M+H)^+$ $(ES^+)$; $^1H$ NMR (400 MHz, DMSO-d6) δ 12.59 (s, 1 H), 6.58 (s, 1 H), 4.18 - 4.07 (m, 2H), 3.61 - 3.53 (m, 1 H), 2.64 - 2.55 (m, 1H), 1.98 - 1.76 (m, 2H), 1.22 - 1.15 (m, 3H), 0.94 - 0.88 (m, 4H), 0.85 (t, J = 7.4 Hz, 3H).

Ethyl 2-(2-(cyclopropanesulfonamido)thiazol-4-yl)-3-methylbutanoate **INTB19**

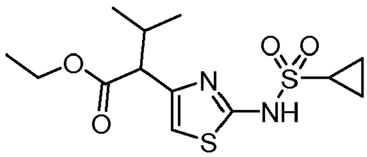

Cyclopropanesulfonyl chloride (1.55 mL, 15.3 mmol) was added to a solution of ethyl 2-(2-aminothiazol-4-yl)-3-methylbutanoate **INTB4** (3.50 g, 15.3 mmol) in pyridine (6.2 mL). The reaction was stirred at RT for 18 hrs. The reaction was poured onto 1 M HCl (aq, 92 mL) and the product was extracted with DCM (3 x 20 mL). The combined organic phases were passed through a phase separator and solvent removed to give a brown oil. The crude product was purified by chromatography on silica gel (220 g column, 0-100% EtOAc/iso-hexane) to afford ethyl 2-(2-(cyclopropanesulfonamido)thiazol-4-yl)-3-methylbutanoate (1.02 g, 2.91 mmol, 19 % yield) as an orange oil; Rt 1.81 min (HPLC acidic); m/z 333 $(M+H)^+$ $(ES^+)$; 331 $(M-H)^-$ (ES-); $^1H$ NMR (400 MHz, DMSO-d6) δ 12.59 (s, 1 H), 6.61 (s, 1 H), 4.24 - 4.05 (m, 2H), 3.33 (d, J = 9.4 Hz, 1 H, partially obscured by solvent), 2.64 - 2.54 (m, 1 H), 2.36 - 2.21 (m, 1 H), 1.20 (t, J = 7.1 Hz, 3H), 0.97 - 0.87 (m, 7H), 0.82 (d, J = 6.7 Hz, 3H).

**Method B: Formation of sulfonamides from sulfonyl chlorides**

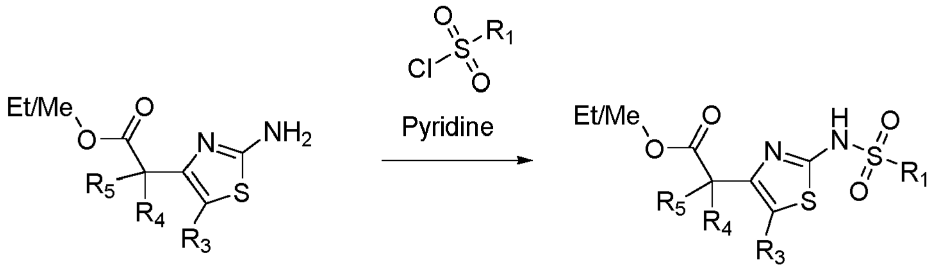


A solution of amine (1.0 eq) and appropriate sulfonyl chloride (1.1 eq) in pyridine (3M volumes) was warmed to 40 °C and stirred for 18 hrs. The reaction mixture was purified by normal or reverse phase chromatography or *via* trituration with an appropriate solvent.

**Table 3:** Additional intermediates were made according to Method B.

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB20 | ethyl 2-(2-(cyclopropanesulfonamido) thiazol-4-yl)propanoate 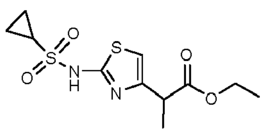 | Method B, using INTB1, [HPLC acidic], 305 , (1.50). | 12.60 (s, 1H), 6.56 (s, 1H), 4.18 - 4.00 (m, 2H), 3.83 - 3.69 (m, 1H), 2.64 - 2.54 (m, 1H), 1.41 (d, J = 7.3 Hz, 3H), 1.20 (t, J = 7.1 Hz, 3H), 0.96 - 0.79 (m, 4H). |

(continued)

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB21 | ethyl 2-(2-(cyclopropanesulfonamido) thiazol-4-yl)-2-ethylbutanoate 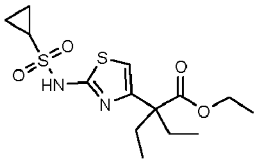 | Method B using INTB61, [UPLC acidic], 347, (1.24). | 12.48 (s, 1H), 6.63 (s, 1H), 4.14 (q, J = 7.1 Hz, 2H), 2.64 - 2.54 (m, 1H), 1.89 (q, J = 7.4 Hz, 4H), 1.18 (t, J = 7.1, 2.0 Hz, 3H), 0.94 - 0.87 (m, 4H), 0.70 (t, J = 7.4 Hz, 6H). |
| INTB22 | methyl 2-(2-(cyclopropanesulfonamido)-5-methylthiazol-4-yl)-2-methylpropanoate 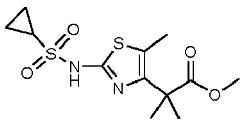 | Method B using INTB5, [UPLC acidic], 319, (0.92). | 12.07 (s, 1H), 3.68 (s, 3H), 2.63-2.55 (m, 1H), 2.03 (s, 3H), 1.49 (s, 6H), 0.97-0.85 (m, 4H). |
| INTB23 | methyl 2-(2-(cyclopropanesulfonamido) thiazol-4-yl)-2-methoxyacetate 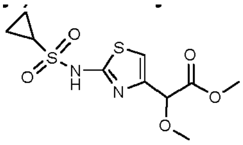 | Method B using INTB10, [UPLC acidic], 307, (0.66). | 12.77 (s, 1H), 6.82 (s, 1H), 4.93 (s, 1H), 3.72 (s, 3H), 3.34 (s, 3H), 2.68 - 2.56 (m, 1H), 1.00 - 0.78 (m, 4H). |
| INTB24 | ethyl 2-(2-(cyclopropanesulfonamido) thiazol-4-yl)-4-methoxybutanoate 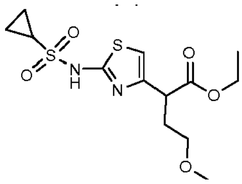 | Method B using INTB6, [HPLC acidic], 349, (1.53). | 12.61 (s, 1H), 6.60 (s, 1H), 4.10 (qt, J = 7.4, 3.8 Hz, 2H), 3.74 (t, J = 7.6 Hz, 1H), 3.36 - 3.19 (m, 2H, signal obscured by residual water), 3.19 (s, 3H), 2.64 - 2.54 (m, 1H), 2.25 - 1.92 (m, 2H), 1.18 (t, J = 7.1 Hz, 3H), 0.97 - 0.81 (m, 4H). |
| INTB25 | Ethyl 2-(2-(cyclopropanesulfonamido) thiazol-4-yl)-2,2-difluoroacetate 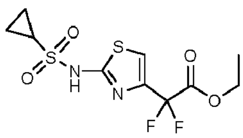 | Method B using INTB14, [UPLC acidic], 327, (1.16). | None recorded |

(continued)

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB62 | ethyl 2-(cyclopropanesulfonamido)-5,6-dihydro-4H-cyclopenta[d] thiazole-4-carboxylate 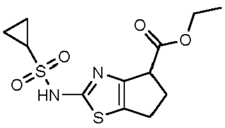 | Method B [UPLC acidic], 317, (0.99). | 12.59 (s, 1H), 4.21 - 4.04 (m, 2H), 3.97 - 3.88 (m, 1H), 2.81 - 2.64 (m, 2H), 2.63 - 2.58 (m, 1H), 2.49 - 2.38 (m, 2H), 1.22 (t, J = 7.1 Hz, 3H), 0.94 - 0.87 (m, 4H). |
| INTB63 | ethyl 2-(cyclopropanesulfonamido)-4,5,6,7-tetrahydrobenzo[d]thiazole-4-carboxylate 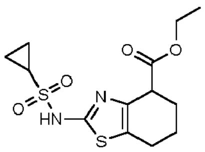 | Method B [HPLC acidic], 331, (1.71). | None recorded |
| INTB64 | ethyl 2-(2-(cyclopropanesulfonamido) thiazol-4-yl)-2-oxoacetate 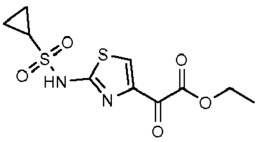 | Method B [UPLC acidic], 305, (0.88). | 13.11 (s, 1H), 8.32 (s, 1H), 4.35 (q, J = 7.1 Hz, 2H), 2.75 - 2.64 (m, 1H), 1.32 (t, J = 7.1 Hz, 3H), 1.01 - 0.87 (m, 4H). |

Methyl 2-(2-bromothiazol-4-yl)propanoate **INTB26**

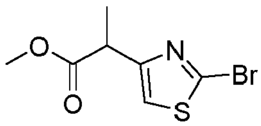

MeI (4.16 mL, 66.5 mmol) was added to a stirred mixture of methyl 2-(2-bromothiazol-4-yl)acetate (3.14 g, 13.3 mmol) and $K_2CO_3$ (9.19 g, 66.5 mmol) in acetone (75 mL). The reaction was heated to 60 °C and stirred for 48 hrs. The reaction mixture was cooled with an ice bath then was treated with 1M HCl (aq, 133 mL). This was then extracted with EtOAc (3 x 100 mL). The organic layers were combined, dried ($MgSO_4$), filtered and concentrated on to silica (20 g).The crude product was purified by chromatography on silica gel (40 g column, 0-20% EtOAc/iso-hexane) to afford methyl 2-(2-bromothiazol-4-yl)propanoate (2.45 g, 9.80 mmol, 74 % yield) as an oil; Rt 1.76 mins (HPLC acidic); m/z 250 ($^{79}$Br $M+H)^+$ ($ES^+$).

Methyl 2-(2-bromothiazol-4-yl)-2-methylpropanoate **INTB27**

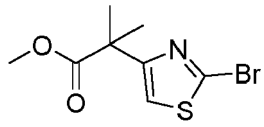

1M LiHMDS (THF, 19 mL, 19 mmol) was added dropwise to a solution of methyl 2-(2-bromothiazol-4-yl)propanoate (3.5 g, 14 mmol) in THF (10 mL) under nitrogen at -78 °C and the mixture was stirred for 20 mins. MeI (1.3 mL, 20.79 mmol) was added and the mixture was allowed to warm to RT and stirred for 2 hrs. The reaction mixture was cooled to 0 °C then quenched with $NH_4Cl$ (sat. aq, 50 mL), extracted with EtOAc (2 x 50 mL), then the organic phases were combined, washed with brine (50 mL), dried (MgSO4), filtered and concentrated on to silica (10 g). The crude product was purified by chromatography on silica gel (24 g column, 0-20% EtOAc/isohexane) to afford methyl 2-(2-bromothiazol-4-yl)-2-methylpropanoate (3.02 g, 11.2 mmol, 80 % yield) as a pale tan oil; Rt 1.28 mins (UPLC acidic); m/z 264 ($^{79}$Br M+H)$^+$ (ES$^+$); $^1$H NMR (500 MHz, DMSO-d6) $\delta$ 7.57 (s, 1 H), 3.60 (s, 3H), 1.50 (s, 6H).

Methyl 2-(2-(cyclobutanesulfonamido)thiazol-4-yl)acetate **INTB28**

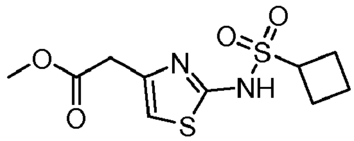

A suspension of (1*R*,2*R*)-*N*1,*N*2-dimethylcyclohexane-1,2-diamine (0.012 mL, 0.08 mmol), methyl 2-(2-bromothiazol-4-yl)acetate (87 mg, 0.37 mmol), cyclobutanesulfonamide (50 mg, 0.37 mmol) and $K_2CO_3$ (57 mg, 0.41 mmol) in dioxane (1.5 mL) at 40 °C was degassed ($N_2$, 5 mins) then copper(I) iodide (7 mg, 0.04 mmol) was added. The solution was again degassed ($N_2$, 5 mins) before being warmed to 80 °C for 2 hrs before being allowed to cool to RT. 1 M HCl (aq, 5 mL) was added and the aqueous phase was extracted with EtOAc (3 x 10 mL). The organic phases were combined, dried ($Na_2SO_4$), filtered and concentrated on to silica (500 mg). The crude product was purified by chromatography on silica gel (4 g column, 0-100% EtOAc/isohexane) to afford methyl 2-(2-(cyclobutanesulfonamido)thiazol-4-yl)acetate (31 mg, 0.11 mmol, 28 % yield) as a red gum; Rt 0.79 mins (UPLC acidic); m/z 291 (M+H)$^+$ (ES$^+$); $^1$H NMR (500 MHz, DMSO-d6) $\delta$ 12.49 (s, 1 H), 6.54 (s, 1 H), 3.64 (s, 3H), 3.62 (s, 2H), 2.47 - 2.44 (m, 1 H), 2.33 - 2.13 (m, 4H), 1.95 - 1.77 (m, 2H).

Methyl 1-(2-(cyclopropanesulfonamido)thiazol-4-yl)cyclopentane-1-carboxylate **INTB65**

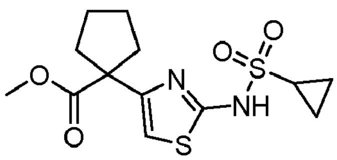

NaH (60 wt % in mineral oil, 0.13 g, 3.28 mmol) was added to a mixture of methyl 2-(2-bromothiazol-4-yl)acetate **INTB16** (0.31 g, 1.31 mmol) and 1,4-dibromobutane (0.17 mL, 1.44 mmol) in DMF (7.5 mL) at 0 °C. The mixture was allowed to warm to RT and stirred for 18 hrs. $NH_4Cl$ (aq) was added and the crude product extracted with EtOAc. The combined organic extracts were washed with brine, dried ($MgSO_4$) and concentrated onto silica. The crude product was purified by chromatography on silica gel (12 g column, 0-10% iso-hexane/MTBE) to afford methyl 1-(2-bromothiazol-4-yl)cyclopentanecarboxylate (0.22 g, 0.758 mmol, 57.7 % yield) as a pale yellow oil. No analysis was undertaken. This material was used as in **Method C** to afford title compound. Rt 1.76 mins (HPLC acidic); m/z 331 (M+H)$^+$ (ES$^+$).

**Method C: Formation of sulfonamides from heterocyclic halides**

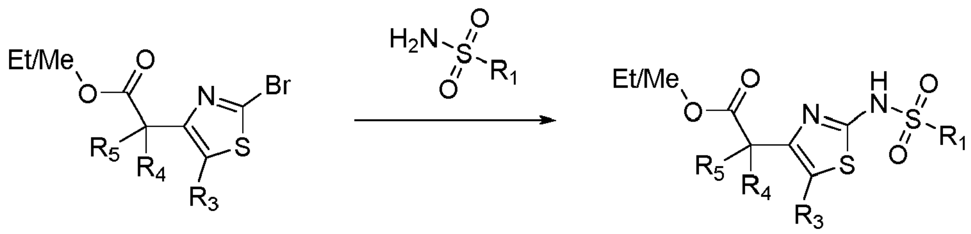

A suspension of (1*R*,2*R*)-*N*1,*N*2-dimethylcyclohexane-1,2-diamine (0.2 eq), bromothiazole intermediate (1 eq), alkylsulfonamide (1 eq), and $K_2CO_3$ (1.1 eq) in dioxane (10 volumes) at 40 °C was degassed ($N_2$, 5 mins) then copper(I) iodide (0.1 equiv.) was added. The solution was again degassed ($N_2$, 5 mins) before being warmed to 80 °C. The reaction was progressed for 2 hrs before being allowed to cool to RT. 1M HCl (aq) was added and the aqueous phase was extracted with EtOAc. The organic phases were combined, dried ($Na_2SO_4$), filtered and concentrated. The crude material was purified by normal phase chromatography.

**Table 3:** Additional intermediates made according to Method C.

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB29 | methyl 2-(2-(cyclobutanesulfonamido) thiazol-4-yl)-2-methylpropanoate 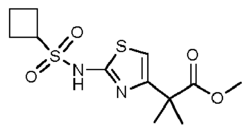 | Method C, using INTB27, [UPLC acidic], 319, (0.98). | 12.57 (s, 1H), 6.53 (s, 1H), 3.86 - 3.75 (m, 1H), 3.63 (s, 3H), 2.32 - 2.11 (m, 4H), 1.95 - 1.80 (m, 2H), 1.45 (s, 6H). |
| INTB30 | methyl 2-methyl-2-(2-((trifluoromethyl) sulfonamido)thiazol-4-yl)propanoate 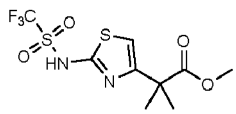 | Method C, using INTB27, [HPLC acidic], 333, (1.97). | 13.98 (s, 1H), 6.95 (s, 1H), 3.65 (s, 3H), 1.50 (s, 6H). |
| INTB31 | methyl 2-methyl-2-(2-((1-methylethyl) sulfonamido)thiazol-4-yl)propanoate 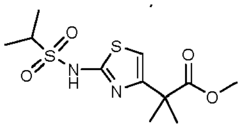 | Method C, using INTB27, [UPLC acidic], 307, (0.95). | None recorded |
| INTB32 | methyl 2-methyl-2-(2-((1-methylcyclopropane)-1-sulfonamido) thiazol-4-yl)propanoate 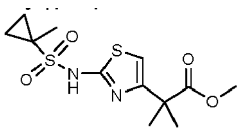 | Method C, using INTB27, [UPLC acidic], 319, (1.00). | None recorded |
| INTB33 | methyl 2-(2-((1,1-dimethylethyl) sulfonamido)thiazol-4-yl)-2-methylpropanoate 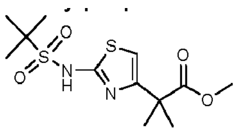 | Method C, using INTB27, [UPLC acidic], 321, (1.04). | None recorded |

(continued)

| INTB# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTB34 | methyl 2-methyl-2-(2-((2-methyl propyl) sulfonamido)thiazol-4-yl)propanoate 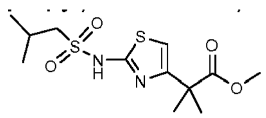 | Method C, using INTB27, [UPLC acidic], 321, (1.10). | 12.55 (s, 1H), 6.55 (s, 1H), 3.63 (d, J = 2.0 Hz, 3H), 2.89 (br. s, 2H), 2.16 - 2.02 (m, 1H), 1.46 (s, 6H), 1.06 - 0.96 (m, 6H). |

2-(2-(methylsulfonamido)thiazol-4-yl)acetic acid **INTB35**

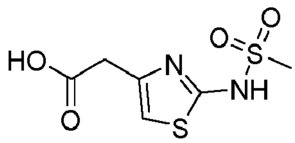

A solution of methyl 2-(2-(methylsulfonamido)thiazol-4-yl)acetate **INTB15** (3.61 g, 14.42 mmol) in THF (30 mL), MeOH (15 mL) and 1 M LiOH (aq, 28.8 mL, 28.8 mmol) was stirred at RT for 16 hrs. The reaction mixture was partially concentrated (30 mL) then washed with $Et_2O$ (10 mL) then acidified with 1 M HCl (aq, 30 mL). The resulting precipitate was filtered and washed with $Et_2O$ (10 mL) to afford 2-(2-(methylsulfonamido)thiazol-4-yl)acetic acid (2.78 g, 11.53 mmol, 80 % yield) as a colourless solid; Rt 0.28 min (UPLC acidic); m/z 237 $(M+H)^+$ $(ES^+)$; $^1H$ NMR (400 MHz, DMSO-d6) $\delta$ 12.97 - 12.26 (m, 2H), 6.57 (s, 1 H), 3.55 (d, J = 0.9 Hz, 2H), 2.89 (s, 3H).

2-(2-(cyclopropanesulfonamido)thiazol-4-yl)acetic acid **INTB36**

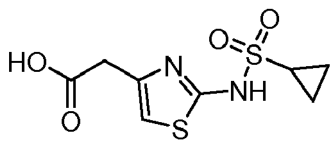

Methyl 2-(2-(cyclopropanesulfonamido)thiazol-4-yl)acetate **INTB16** (1.50 g, 5.43 mmol) in THF (13 mL) and MeOH (1 mL) was treated with 1 M LiOH (aq, 12.48 mL, 12.48 mmol) and stirred at RT for 1 hr. A further portion of 1 M LiOH (aq, 5.43 mL, 5.43 mmol) was added and the reaction mixture was stirred at RT for 18 hrs. The reaction mixture was part concentrated then acidified with 1 M HCl (20 mL). This was then extracted with EtOAc (3 x 50 mL). The organic phases were combined, dried ($Na_2SO_4$), filtered and concentrated in *vacuo* to afford 2-(2-(cyclopropanesulfonamido)thiazol-4-yl)acetic acid (1.45 g, 5.25 mmol, 97 % yield) as a colourless solid; Rt 0.59 min (UPLC acidic); m/z 263 $(M+H)^+$ $(ES^+)$; $^1H$ NMR (400 MHz, DMSO-d6) $\delta$ 12.75 (br. s, 1 H), 12.50 (br. s, 1H), 6.56 (s, 1 H), 3.55 (d, J = 0.9 Hz, 2H), 2.65 - 2.54 (m, 1 H), 1.03 - 0.75 (m, 4H).

2-(2-(cyclopropanesulfonamido)thiazol-4-yl)-2-methylpropanoic acid **INTB37**

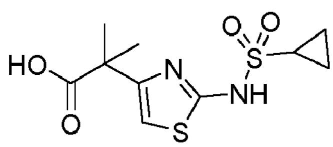

To a solution of methyl 2-(2-(cyclopropanesulfonamido)thiazol-4-yl)-2-methylpropanoate **INTB17** (1 g, 3.29 mmol) in THF (15 mL) and MeOH (7 mL) was added a solution of LiOH (0.16 g, 6.57 mmol) in water (6.57 mL). The reaction was stirred at RT for 48 hrs. The reaction was concentrated to 10 mL. The pale yellow solution was acidified with 1 M HCl (aq, 30 mL) and diluted with EtOAc (50 mL), the phases were separated and the aqueous extracted with EtOAc (3 x 50 mL). The combined organics were dried ($Na_2SO_4$), filtered and concentrated in *vacuo* to give 2-(2-(cyclopropanesulfonamido)thiazol-4-yl)-2-methylpropanoic acid (0.83 g, 2.79 mmol, 85 %) as a colourless solid; Rt 0.79 min (HPLC acidic); m/z 291 $(M+H)^+$ $(ES^+)$; $^1H$ NMR (400 MHz, DMSO-d6) δ 12.62 (brs, 2H), 6.51 (s, 1 H), 2.66 - 2.53 (m, 1 H), 1.45 (s, 6H), 0.95 - 0.89 (m, 4H).

2-(2-(cyclopropanesulfonamido)thiazol-4-yl)butanoic acid **INTB38**

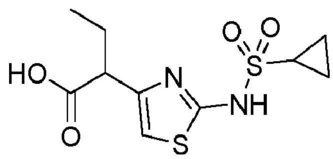

2M LiOH (aq, 3.11 mL, 6.22 mmol) was added to a solution of ethyl 2-(2-(cyclopropanesulfonamido)thiazol-4-yl)butanoate **INTB18** (1.8 g, 5.65 mmol) in THF (10 mL) and stirred at RT for 18 hrs. Additional 2M LiOH (aq, 3.11 mL, 6.22 mmol) solution was added and the reaction was heated to 40 °C for 2 hrs. The reaction was allowed to cool to RT and the solvent was removed in *vacuo.* It was then acidified with 1 M HCl (aq, 40 mL) and extracted with EtOAc (3 x 50 mL). The combined organic phases were passed through a phase separator and solvent removed in *vacuo* to give 2-(2-(cyclopropanesulfonamido)thiazol-4-yl)butanoic acid (1.54 g, 5.04 mmol, 89 % yield) as a yellow solid; Rt 1.29 min (HPLC acidic); m/z 291 $(M+H)^+$ $(ES^+)$; 289 $(M-H)^-$ $(ES^-)$; $^1H$ NMR (400 MHz, DMSO-d6) δ 12.58 (br. s, 2H), 6.55 (s, 1H), 3.47 (dd, J = 8.4, 6.7 Hz, 1 H), 2.64 - 2.56 (m, 1 H), 1.98 - 1.73 (m, 2H), 0.95 - 0.88 (m, 4H), 0.85 (t, J = 7.4 Hz, 3H).

**Method D: Formation of acids from esters**

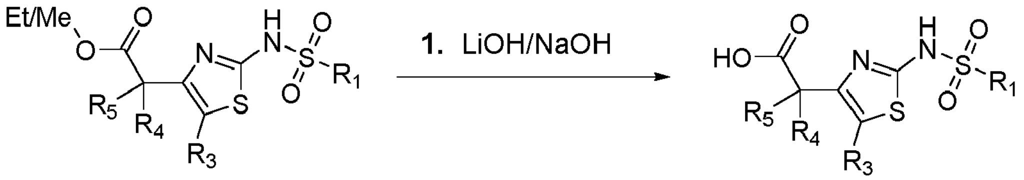


To a solution of ester (1.0 eq.) in THF/MeOH (2:1) was added a solution of appropriate base (2.0 eq. of LiOH or NaOH aq solutions at 1-2M). The reaction was stirred at RT or with heating up to 50 °C. The reaction was concentrated in *vacuo* to half volume and was acidified with 1 M HCl. The product was extracted using an appropriate organic solvent (EtOAc). The combined organics were dried ($Na_2SO_4$) and concentrated in *vacuo* to give the desired compound.

Table 4: Additional intermediates made according to Method D

| INTB# | Name/Structure (All examples containing chiral centres are racemates unless stated) | Synthesis Method, [LCMS Method], m/z (M+H)+, (Rt/min) | $^1H$ NMR Chemical Shift Data (DMSO-d6 unless stated) |
|---|---|---|---|
| INTB39 | 2-(2-(cyclopropanesulfonamido)thiazol-4-yl)propanoic acid 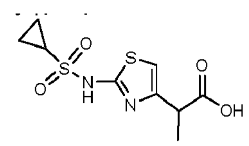 | Method D using INTB20, [HPLC acidic], 277, (1.09). | 12.92 - 12.27 (m, 2H), 6.53 (s, 1H), 3.71 - 3.59 (m, 1H), 2.63 - 2.55 (m, 1H), 1.40 (d, J = 7.3 Hz, 3H), 0.96 - 0.78 (m, 4H). |

(continued)

| INTB# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| INTB40 | 2-(2-(cyclopropanesulfonamido)thiazol-4-yl)-3-methylbutanoic acid 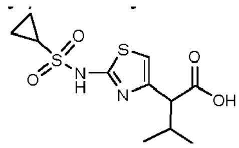 | Method D using INTB19, [HPLC acidic], 305, (1.41). | None recorded |
| INTB41 | 2-(2-(cyclopropanesulfonamido)thiazol-4-yl)-2-ethylbutanoic acid 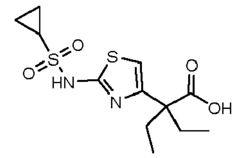 | Method D using INTB21, [HPLC acidic], 319, (1.60). | 12.78 (s, 1H), 12.44 (s, 1H), 6.62 (s, 1H), 2.64 - 2.56 (m, 1H), 1.94 - 1.82 (m, 4H), 0.94 - 0.87 (m, 4H), 0.71 (t, J = 7.4 Hz, 6H). |
| INTB42 | 2-(2-(cyclopropanesulfonamido)-5-methylthiazol-4-yl)-2-methylpropanoic acid 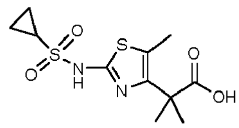 | Method D using INTB22, [PLC basic], 305, (0.48). | 12.84 (br, 1H), 12.02 (br, 1H), 2.60-2.54 (m, 1H), 2.09 (s, 3H), 1.46 (s, 6H), 0.96 - 0.86 (m, 4H). |
| INTB43 | 2-(2-(cyclopropanesulfonamido)thiazol-4-yl)-2-methoxyacetic acid 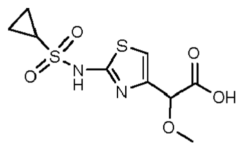 | Method D using INTB23, [UPLC acidic], 293, (0.63). | None recorded |
| INTB44 | 2-(2-(cyclopropanesulfonamido)thiazol-4-yl)-4-methoxybutanoic acid 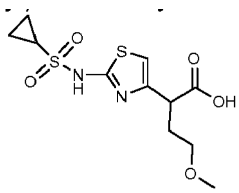 | Method D using INTB24, [UPLC acidic], 321, (0.73). | 13.01 - 12.26 (m, 2H), 6.57 (s, 1H), 3.66 (dd, J = 8.6, 6.5 Hz, 1H), 3.38 - 3.20 (m, 2H), 3.19 (s, 3H), 2.64 - 2.54 (m, 1H), 2.22 - 2.09 (m, 1H), 2.06 - 1.92 (m, 1H), 0.94 - 0.79 (m, 4H). |
| INTB45 | 2-(2-(cyclopropanesulfonamido)thiazol-4-yl)-2,2-difluoroacetic acid 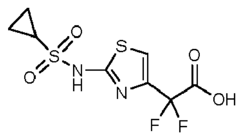 | Method D using INTB25, [UPLC acidic], 299, (0.79). | None recorded |

(continued)

| INTB# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| INTB46 | 2-(2-(cyclobutanesulfonamido)thiazol-4-yl)acetic acid 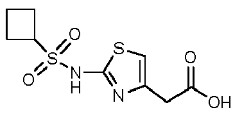 | Method D using INTB28, [HPLC acidic], 277, (1.13). | None recorded |
| INTB47 | 2-(2-(cyclobutanesulfonamido)thiazol-4-yl)-2-methylpropanoic acid 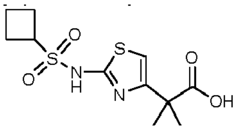 | Method D using INTB29, [HPLC acidic], 305, (1.49). | 12.77 (v, br. s, 1H), 12.55 (v, br. s, 1H), 6.48 (s, 1H), 3.85 - 3.69 (m, 1H), 2.33 - 2.09 (m, 4H), 1.97 - 1.78 (m, 2H), 1.43 (s, 6H). |
| INTB48 | 2-methyl-2-(2-((trifluoromethyl) sulfonamido) thiazol-4-yl)propanoic acid 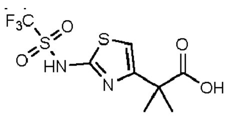 | Method D using INTB30, [HPLC acidic], 319, (1.10). | None recorded |
| INTB49 | 2-methyl-2-(2-((1-methylethyl) sulfonamido)thiazol-4-yl)propanoic acid 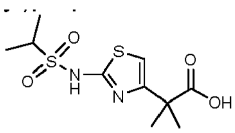 | Method D using INTB31, [HPLC acidic], 293, (1.36). | 12.75 (s, 1H), 12.57 (s, 1H), 6.48 (s, 1H), 3.15 - 3.03 (m, 1H), 1.44 (s, 6H), 1.22 (d, J = 6.8 Hz, 6H). |
| INTB50 | 2-methyl-2-(2-((1-methylcyclopropane)-1-sulfonamido)thiazol-4-yl)propanoic acid 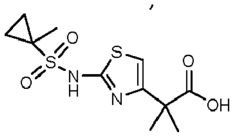 | Method D using INTB32, [HPLC acidic], 305, (1.42). | 12.86-12.48 (m, 2H), 6.47 (s, 1H), 1.44 (s, 6H), 1.39 (s, 3H), 1.25 - 1.08 (m, 2H), 0.82 - 0.71 (m, 2H). |
| INTB51 | 2-(2-((1,1-dimethylethyl)sulfonamido) thiazol-4-yl)-2- 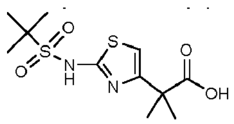 methylpropanoic acid | Method D using INTB33, [HPLC acidic], 307, (1.49). | 12.95 - 12.19 (m, 2H), 6.46 (s, 1H), 1.44 (s, 6H), 1.28 (s, 9H). |
| INTB52 | 2-methyl-2-(2-((2-methylpropyl) sulfonamido)thiazol-4-yl)propanoic acid 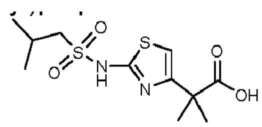 | Method D using INTB34, [UPLC acidic], 307, (0.96). | 12.78 (s, 1H), 12.53 (s, 1H), 6.50 (s, 1H), 2.96 - 2.82 (m, 2H), 2.15 - 2.03 (m, 1H), 1.44 (s, 6H), 1.01 (dd, J = 6.7, 2.2 Hz, 6H). |

(continued)

| INTB# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| INTB66 | 2-(cyclopropanesulfonamido)-5,6-dihydro-4H-cyclopenta[d]thiazole-4-carboxylic acid 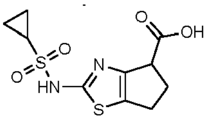 | Method D using INTB62, [UPLC acidic], 289, (0.75). | 12.59-12.52 (m, 2H), 3.85 - 3.77 (m, 1H), 2.78 - 2.64 (m, 2H), 2.60 (s, 1H), 2.50 - 2.38 (m, 2H), 0.94 - 0.87 (m, 4H). |
| INTB67 | 2-(cyclopropanesulfonamido)-4,5,6,7-tetrahydrobenzo[d]thiazole-4-carboxylic acid 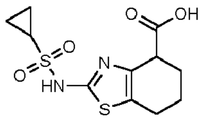 | Method D using INTB63, [UPLC acidic], 303, (0.83). | None recorded |
| INTB68 | 2-(2-(cyclopropanesulfonamido)thiazol-4-yl)-2-oxoacetic acid 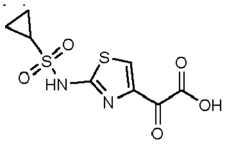 | Method D using INTB64, [UPLC acidic], 277, (0.85). | 13.49-12.80 (v. br s, 2H), 8.22 (s, 1H), 2.77 - 2.56 (m, 1H), 1.12 - 0.76 (m, 4H). |
| INTB69 | 1-(2-(cyclopropanesulfonamido)thiazol-4-yl)cyclopentane-1-carboxylic acid 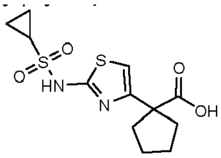 | Method D using INTB65, [HPLC acidic], 317, (1.60). | None recorded |

N-(4-bromophenyl)-2-(2-(cyclopropanesulfonamido)thiazol-4-yl)-2-methylpropanamide **INTB53**

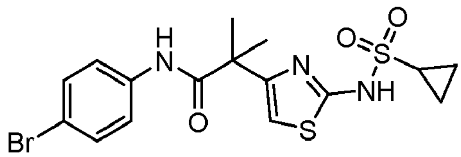

To a suspension of 2-(2-(cyclopropanesulfonamido)thiazol-4-yl)-2-methylpropanoic acid **INTB37** (0.75 g, 2.58 mmol) in DCM (10 mL)was added 1-chloro-N,N,2-trimethylprop-1-en-1-amine (0.63 mL, 5.17 mmol). The reaction mixture was stirred at RT for 2 hrs. The reaction mixture was concentrated to dryness. The orange residue was redissolved in pyridine (5 mL) before the addition of 4-bromoaniline (0.49 g, 2.84 mmol). The reaction mixture was stirred at RT for 16 hrs. The reaction mixture was poured onto 1 M HCl (aq, 100 mL) and the product was extracted using DCM (3 x 75 mL). The combined organics were passed through a phase separator and concentrated in *vacuo.* The orange residue was triturated with MeOH (2 x 10 mL) and the resultant solid was filtered, rinsing with MeOH (2 x 10 mL), and dried in *vacuo* to afford N-(4-bromophenyl)-2-(2-(cyclopropanesulfonamido)thiazol-4-yl)-2-methylpropanamide (956 mg, 2.09 mmol, 81 % yield) as a pale pink solid; Rt 1.26 min (UPLC acidic); m/z 446 $(M+H)^+$ $(ES^+)$; $^1$H NMR (400 MHz, DMSO-d6) δ 12.53 (s, 1 H), 9.40 (s, 1 H), 7.64 - 7.54 (m, 2H), 7.55 - 7.46 (m, 2H), 6.55 (s, 1 H), 2.63 - 2.55 (m, 1 H), 1.55 (s, 6H),

1.01 - 0.84 (m, 4H).

N-(4-bromo-2-methoxyphenyl)-2-(2-(cyclopropanesulfonamido)thiazol-4-yl)-2-methylpropanamide **INTB54**

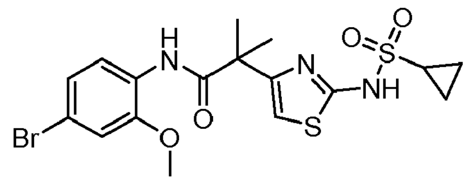

Prepared as **Method 1b in preparation of examples section** from **INTB37.** Rt 1.34 min (UPLC acidic); m/z 476 (79Br M+H)+ (ES+); 1 H NMR (400 MHz, DMSO-d6) δ 12.64 (s, 1 H), 8.37 (s, 1 H), 7.92 - 7.72 (m, 1H), 7.24 (s, 1 H), 7.14 (d, J = 8.5 Hz, 1 H), 6.69 (s, 1 H), 3.83 (s, 3H), 2.63 - 2.58 (m, 1 H), 1.53 (s, 6H), 0.94 - 0.89 (m, 4H).

N-(5-bromopyridin-2-yl)-2-(2-(cyclopropanesulfonamido)thiazol-4-yl)-2-methylpropanamide **INTB55**

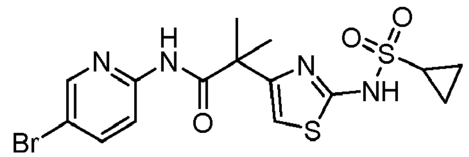

Prepared as **Method 1b in preparation of examples section** from **INTB37.** Rt 1.20 mins (UPLC acidic); m/z 445 ($^{79}$Br M+H)$^+$ (ES$^+$); $^1$H NMR (400 MHz, DMSO-d6) δ 12.55 (s, 1 H), 10.08 (s, 1 H), 8.50 - 8.45 (m, 1 H), 8.09 - 7.97 (m, 2H), 6.55 (d, J = 6.8 Hz, 1 H), 2.65 - 2.54 (m, 1 H), 1.58 (s, 6H), 0.94 - 0.81 (m, 4H).

2-(2-(cyclopropanesulfonamido)thiazol-4-yl)-2-methyl-N-(4-(4,4,5,5-tetramethyl-1,3,2-dioxaborolan-2-yl)phenyl)propanamide **INTB56**

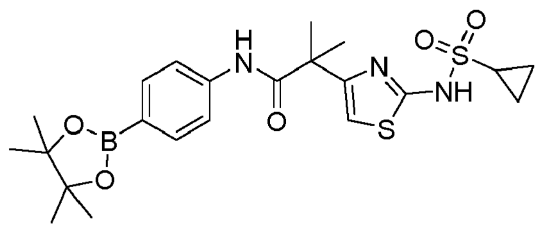

HATU (1.26 g, 3.31 mmol) was added to a solution 2-(2-(cyclopropanesulfonamido)thiazol-4-yl)-2-methylpropanoic acid **INTB37** (800 mg, 2.76 mmol), 4-(4,4,5,5-tetramethyl-1,3,2-dioxaborolan-2-yl)aniline (604 mg, 2.76 mmol) and DIPEA (1.44 mL, 8.27 mmol) in DCM (8 mL) at RT. The reaction was stirred at RT for 2 hrs. The solvent was removed to give a brown oil. The crude product was purified by chromatography on silica gel (40 g column, 0-100% EtOAc/ isohexane) to afford 2-(2-(cyclopropanesulfonamido)thiazol-4-yl)-2-methyl-N-(4-(4,4,5,5-tetramethyl-1,3,2-dioxaborolan-2-yl)phenyl)propanamide (1.36 g, 2.49 mmol, 90 % yield) as a colourless solid; Rt 2.22 min (HPLC acidic); m/z 492 (M+H)$^+$ (ES$^+$); 490 (M-H)$^-$ (ES$^-$); $^1$H NMR (400 MHz, DMSO-d6) δ 12.53 (br. s, 1H), 8.18 (br. s, 1H), 7.72 - 7.55 (m, 4H), 6.46 (s, 1H), 2.63 - 2.54 (m, 1H), 1.53 (s, 6H), 1.29 (s, 12H), 0.92 - 0.79 (m, 4H).

2-(2-(cyclopropanesulfonamido)thiazol-4-yl)-N-(2-fluoro-4-(4,4,5,5-tetramethyl-1,3,2-dioxaborolan-2-yl)phenyl)-2-methylpropanamide **INTB57**

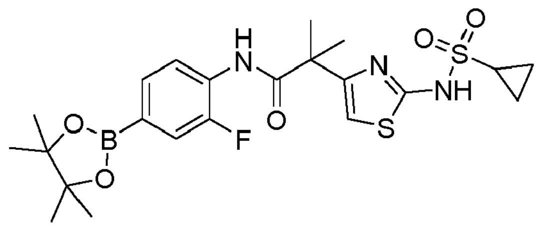

To a solution of 2-(2-(cyclopropanesulfonamido)thiazol-4-yl)-2-methylpropanoic acid **INTB37** (0.75 g, 2.58 mmol) in DCM (15 mL) was added 1-chloro-N,N,2-trimethylprop-1-en-1-amine (0.63 mL, 5.17 mmol). The reaction mixture was stirred at RT for 1 hr. The reaction mixture was concentrated to dryness and the resulting brown residue was redissolved in DCM (15 mL). 2-fluoro-4-(4,4,5,5-tetramethyl-1,3,2-dioxaborolan-2-yl)aniline (0.67 g, 2.84 mmol) and pyridine (1.67 mL) were added to the reaction mixture. The reaction mixture was stirred at RT for 18 hrs. The reaction mixture was diluted with $NH_4Cl$ (sat. aq, 10 mL) and further DCM (10 mL). The phases were separated and the aqueous was extracted with further DCM (2 x 20 mL). The combined organics were dried (phase separator) and concentrated in *vacuo.* The crude product was purified by chromatography on silica gel (40 g column, 0-100% EtOAc/iso-hexane) to afford a mixture of 2-(2-(cyclopropanesulfonamido)thiazol-4-yl)-N-(2-fluoro-4-(4,4,5,5-tetramethyl-1,3,2-dioxaborolan-2-yl)phenyl)-2-methylpropanamide (730 mg, 1.06 mmol, 41 % yield) as an orange gum Rt 2.30 min (HPLC acidic); m/z 511 $(M+H)^+$ $(ES^+)$., contaminated with boronic acid hydrolysis product; Rt 1.47 min (HPLC acidic); m/z 428 $(M+H)^+$ $(ES^+)$.

2-(2-(cyclopropanesulfonamido)thiazol-4-yl)-2-methylpropanamide **INTB58**

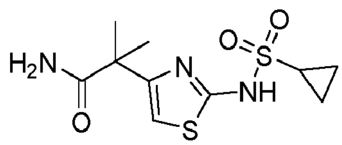

To a suspension of 2-(2-(cyclopropanesulfonamido)thiazol-4-yl)-2-methylpropanoic acid **INTB37** (0.4 g , 1.38 mmol) in DCM (15 mL) was added 1-chloro-N,N,2-trimethylprop-1-en-1-amine (0.34 mL, 2.76 mmol). The reaction mixture was stirred at RT for 1 hr. The reaction mixture was concentrated in *vacuo* and the resulting residue was redissolved in MeCN (10 mL). This solution was added dropwise to $NH_4OH$ (9.58 mL, 68.9 mmol) at 0 °C. The reaction mixture was warmed to RT and stirred for at this temperature for 18 hrs. The reaction mixture was concentrated in *vacuo* and the resulting residue was redissolved in $NH_4Cl$ (sat. aq, 30 mL) and EtOAc (30 mL). The phases were separated and the aqueous was further extracted with EtOAc (2 x 20 mL). The combined organics were dried ($MgSO_4$ and concentrated in *vacuo* to afford 2-(2-(cyclopropanesulfonamido)thiazol-4-yl)-2-methylpropanamide (0.37 g, 1.15 mmol, 83 % yield) as a pale brown residue; Rt 1.10 min (HPLC acidic); m/z 290 $(M+H)^+$ $(ES^+)$; $^1H$ NMR (400 MHz, DMSO-d6) $\delta$ 12.39 (s, 1H), 7.09 (s, 2H), 6.45 (s, 1H), 2.61 - 2.53 (m, 1H), 1.41 (s, 6H), 0.94 - 0.86 (m, 4H).

N-(4-bromo-3-methylphenyl)-2-(2-(cyclopropanesulfonamido)thiazol-4-yl)-2-methylpropanamide **INTB59**

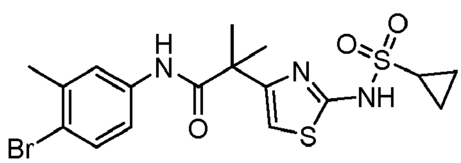

Prepared as **Method 1b** from **INTB37.** Rt 1.36 mins (UPLC acidic); m/z 458/460 $(M+H)^+$ $(ES^+)$; $^1H$ NMR (500 MHz, DMSO-d6) $\delta$ 12.55 (s, 1H), 9.33 (s, 1H), 7.61 (d, J = 2.6 Hz, 1H), 7.51 (d, J = 8.7 Hz, 1H), 7.42 (dd, J = 8.7, 2.6 Hz, 1H), 6.53 (s, 1H), 2.60 - 2.56 (m, 1H), 2.32 (s, 3H), 1.55 (s, 6H), 0.93 - 0.83 (m, 4H).

N-(4-bromo-2-methylphenyl)-2-(2-(cyclopropanesulfonamido)thiazol-4-yl)-2-methylpropanamide **INTB60**

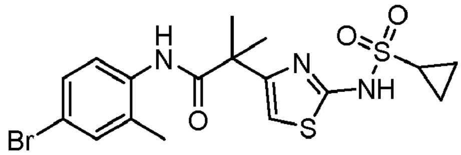

Prepared as **Method 1b** from INTB37. Rt 1.71 mins (HPLC acidic); m/z 459 ($^{79}$Br M+H)$^+$ (ES$^+$); $^1$H NMR (400 MHz, DMSO-d6) δ 12.54 (bs, 1H), 8.99 (bs, 1H), 7.44 (s, 1H), 7.42 - 7.33 (m, 1H), 7.32 (bs, 1H), 6.55 (bs, 1H), 2.59 - 2.54 (m, 1H), 2.16 (s, 3H), 1.54 (s, 6H), 0.93 - 0.80 (m, 4H).

N-(4-bromo-2,6-dimethylphenyl)-2-(2-(cyclopropanesulfonamido)thiazol-4-yl)-2-methylpropanamide **INTB70**

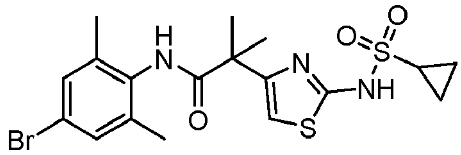

Prepared as **Method 1b** from **INTB37.** Rt 1.28 mins (UPLC acidic); m/z 472/474 (M+H)$^+$ (ES$^+$); $^1$H NMR (500 MHz, DMSO-d6) δ 12.52 (s, 1H), 8.88 (s, 1H), 7.28 (s, 2H), 6.59 (s, 1H), 2.61 - 2.51 (m, 1H), 2.09 (s, 6H), 1.58 (s, 6H), 0.93 - 0.83 (m, 4H).

N-(4-bromo-3-ethoxyphenyl)-2-(2-(cyclopropanesulfonamido)thiazol-4-yl)-2-methylpropanamide **INTB71**

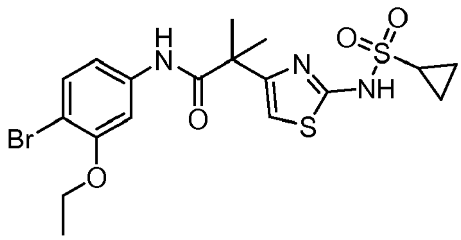

Prepared as **Method 1b** from **INTB37.** Rt 2.17 mins (HPLC acidic); m/z 488/490 (M+H)$^+$ (ES$^+$); $^1$H NMR (500 MHz, DMSO-d6) δ 12.55 (s, 1H), 9.38 (s, 1H), 7.52 - 7.45 (m, 2H), 7.21 (dd, J = 8.7, 2.3 Hz, 1H), 6.54 (s, 1H), 4.10 - 3.99 (m, 2H), 2.60 - 2.56 (m, 1H), 1.56 (s, 6H), 1.37 (t, J = 7.0 Hz, 3H), 0.93 - 0.87 (m, 4H).

N-(5-bromo-3-fluoropyridin-2-yl)-2-(2-(cyclopropanesulfonamido)thiazol-4-yl)-2-methylpropanamide **INTB72**

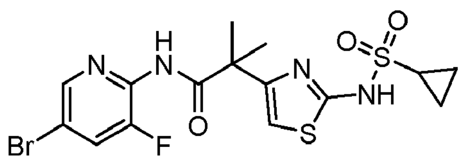

Prepared as **Method 1b** from **INTB37.** Rt 1.00 mins (UPLC acidic); m/z 463/465 (M+H)$^+$ (ES$^+$); $^1$H NMR (400 MHz, DMSO-d6) δ 12.60 (s, 1H), 9.98 (s, 1H), 8.45 (d, J = 2.0 Hz, 1H), 8.25 (dd, J = 9.3, 2.1 Hz, 1H), 6.57 (s, 1H), 2.61 - 2.56 (m, 1H), 1.56 (s, 6H), 0.94 - 0.86 (m, 4H).

N-(4-bromophenyl)-2-(2-(cyclopropanesulfonamido)thiazol-4-yl)acetamide **INTB73**

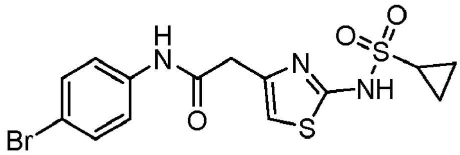

Prepared as **Method 1b** from **INTB37.** Rt 1.61 mins (UPLC acidic); m/z 416 ($^{79}$Br M+H)$^+$ (ES$^+$); $^1$H NMR (400 MHz, DMSO-d6) δ 12.54 (s, 1H), 10.33 (s, 1H), 7.59 - 7.54 (m, 2H), 7.54 - 7.48 (m, 2H), 6.55 (s, 1H), 3.64 (s, 2H), 2.63 - 2.54 (m, 1H), 0.93 - 0.80 (m, 4H).

## Aniline intermediate preparation

5-(pyridin-3-yl)pyrimidin-2-amine **INTA1**

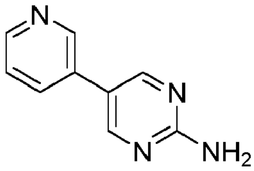

$PdCl_2$(dppf) (68.3 mg, 0.09 mmol) was added to a degassed ($N_2$, 5 mins) solution of pyridin-3-ylboronic acid (230 mg, 1.87 mmol), 5-bromopyrimidin-2-amine (325 mg, 1.87 mmol) and $K_2CO_3$ (774 mg, 5.60 mmol) in dioxane (7 mL) and water (1 mL). The solution was degassed ($N_2$, 5 mins) then heated to 100 °C for 4 hrs and then allowed to cool to RT and stirred for 16 hrs. The reaction mixture was concentrated onto silica. The crude product was purified by chromatography on silica gel (40 g column, 0-10% MeOH/DCM) to afford 5-(pyridin-3-yl)pyrimidin-2-amine (220 mg, 1.25 mmol, 67 % yield) as a pale brown solid; Rt 0.79 min (HPLC basic); m/z 173 (M+H)$^+$ (ES$^+$); $^1$H NMR (400 MHz, DMSO-d6) δ 8.86 (br. s, 1H), 8.64 (s, 2H), 8.57 - 8.47 (m, 1H), 8.10 - 8.00 (m, 1H), 7.45 (dd, J = 7.9, 4.7 Hz, 1H), 6.89 (s, 2H).

4-(6-methoxypyrazin-2-yl)aniline **INTA2**

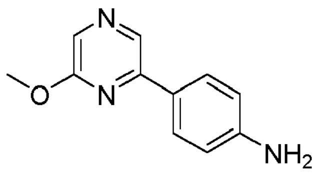

$Pd(PPh_3)_4$ (132 mg, 0.11 mmol) was added to a degassed ($N_2$, 5 mins) solution of 4-(4,4,5,5-tetramethyl-1,3,2-dioxaborolan-2-yl)aniline (500 mg, 2.28 mmol), 2-bromo-6-methoxypyrazine (431 mg, 2.28 mmol) and $NaHCO_3$ (575 mg, 6.85 mmol) in MeCN (4 mL) and water (1 mL). The solution was degassed further ($N_2$, 5 mins) and heated to 80 °C for 2 hrs then allowed to cool to RT. The solution was concentrated onto silica. The crude product was purified by chromatography on silica gel (40 g column, 0-10% MeOH/DCM) to afford 4-(6-methoxypyrazin-2-yl)aniline (423 mg, 2.00 mmol, 88 % yield) as a pale yellow solid; Rt 1.63 min (HPLC basic); m/z 202 (M+H)$^+$ (ES$^+$); $^1$H NMR (400 MHz, DMSO-d6) δ 8.61 (s, 1H), 8.04 (s, 1H), 7.94 - 7.75 (m, 2H), 6.75 - 6.54 (m, 2H), 5.59 (s, 2H), 3.98 (s, 3H).

5-(6-(trifluoromethyl)pyrazin-2-yl)pyridin-2-amine **INTA3**

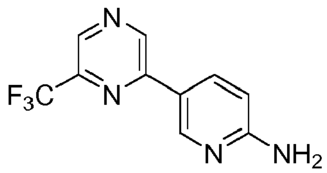

A suspension of 2-chloro-6-(trifluoromethyl)pyrazine (250 mg, 1.37 mmol) and 5-(4,4,5,5-tetramethyl-1,3,2-dioxaborolan-2-yl)pyridin-2-amine (301 mg, 1.37 mmol) in a mixture of 2M $NaHCO_3$ (2.05 mL, 4.11 mmol), EtOH (2 mL) and toluene (10 mL) was degassed ($N_2$, 10 mins) then $Pd(PPh_3)_4$ (79 mg, 0.07 mmol) was added and the degassing was continued ($N_2$, 5 mins) before being heated to 85 °C for 18 hrs. The reaction mixture was concentrated in *vacuo* then taken up in 1 M HCl (7 mL) and washed with DCM (20 mL). The aqueous phase was then brought to pH 12 by careful addition of 2M NaOH (aq). The aqueous layer was extracted with EtOAc (3 x 15 mL), the organic phases were combined, and concentrated on to silica. The crude product was purified by chromatography on silica gel (12 g column, 20-100% EtOAc/iso-hexane) to afford 5-(6-(trifluoromethyl)pyrazin-2-yl)pyridin-2-amine (227 mg, 0.89 mmol, 66 % yield) as a colourless solid; Rt 0.52 min (UPLC acidic); m/z 241 $(M+H)^+$ $(ES^+)$; $^1H$ NMR (400 MHz, DMSO-d6) $\delta$ 9.52 - 9.41 (m, 1H), 8.95 (t, J = 0.6 Hz, 1H), 8.81 (dd, J = 2.5, 0.8 Hz, 1H), 8.16 (dd, J = 8.8, 2.5 Hz, 1 H), 6.66 (s, 2H), 6.59 (dd, J = 8.8, 0.8 Hz, 1 H).

5-(6-propoxypyrazin-2-yl)pyridin-2-amine **INTA4**

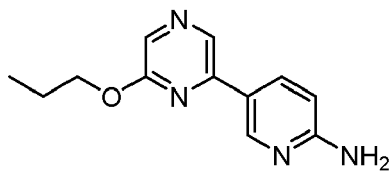

5-(4,4,5,5-tetramethyl-1,3,2-dioxaborolan-2-yl)pyridin-2-amine (319 mg, 1.45 mmol) and 2-chloro-6-propoxypyrazine (250 mg, 1.45 mmol) were dissolved in dioxane (10 mL), 2M $NaHCO_3$ (2.17 mL, 4.35 mmol) was added and the mixture was degassed ($N_2$, 15 mins). $Pd(PPh_3)_4$ (84 mg, 0.07 mmol) was then added and the mixture was heated to 80 °C for 18 hrs. The mixture was diluted with $H_2O$ (10 mL), the product was extracted with EtOAc (3 x 10 mL), the combined organic extract was passed through a phase separator and the solvent was removed in *vacuo*. The crude product was purified by chromatography on silica gel (40 g column, 0-100% EtOAc/iso-hexane) to afford 5-(6-propoxypyrazin-2-yl)pyridin-2-amine (261 mg, 1.00 mmol, 69 % yield) as a beige solid; Rt 1.12 min (UPLC basic); m/z 231 $(M+H)^+$ $(ES^+)$; $^1H$ NMR (400 MHz, DMSO-d6) $\delta$ 8.71 (dd, J = 2.5, 0.8 Hz, 1H), 8.65 (s, 1 H), 8.19 - 8.00 (m, 2H), 6.55 (dd, J = 8.7, 0.8 Hz, 1 H), 6.42 (s, 2H), 4.35 (t, J = 6.6 Hz, 2H), 1.89 - 1.70 (m, 2H), 1.00 (t, J = 7.4 Hz, 3H).

**Method E: Suzuki coupling of halo anilines with heteroaromatic boronates**

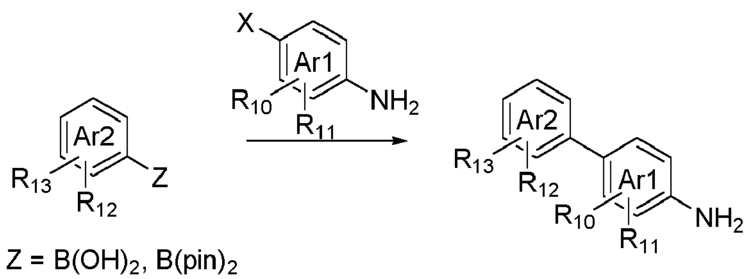

Z = $B(OH)_2$, $B(pin)_2$
X = Br, Cl

A solution of Ar1-X (1 eq) and Ar2-Z (1 eq) in solvent (3 volumes) and base (2.5 eq) was degassed ($N_2$, 5 min) and heated to 40 °C whereupon Pd catalyst (3 mol %) was added and the reaction mixture further degassed ($N_2$, 5 min) before being heated to 90 °C for 90 mins. The reaction mixture was allowed to cool to RT. In general, the desired

compound is purified by column chromatography.

**Method F: Suzuki coupling of heteroaromatic halides with aniline boronates**

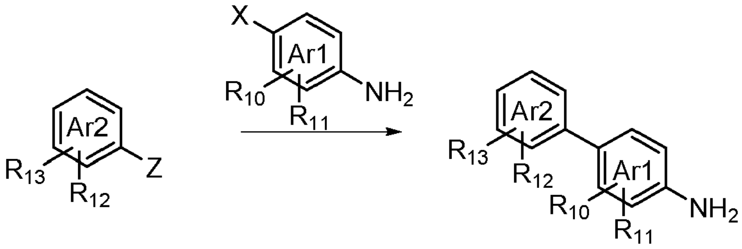

Z = Br, Cl

X = $B(OH)_2$, $B(pin)_2$

Pd catalyst (5 mol %) was added to a degassed ($N_2$, 5 mins) solution of Ar1-X (1 eq), Ar2-Z (1 eq) and base (3 eq, 6.85 mmol) in solvent (3 volumes). The solution was then degassed further ($N_2$, 5 mins) and then heated to 90 °C for 2 hrs then allowed to cool to RT. In general, the desired compound is purified by column chromatography.

**Table 5:** Additional intermediates were made according to Methods E or F.

| INTA# | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| INTA5 | 5-amino-2-(pyrazin-2-yl) benzonitrile 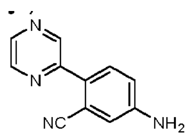 | Method F, [UPLC basic], 197, (0.71). | 8.99 (d, J = 1.6 Hz, 1H), 8.70 (dd, J = 2.5, 1.6 Hz, 1H), 8.59 (d, J = 2.5 Hz, 1H), 7.69 (d, J = 8.6 Hz, 1H), 7.04 (d, J = 2.4 Hz, 1H), 6.96 (dd, J = 8.6, 2.4 Hz, 1H), 6.04 (s, 2H). | $PdCl_2$ (dppf), $K_3PO_4$, dioxane |
| INTA6 | 2,3-difluoro-4-(pyridin-3-yl) aniline 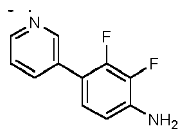 | Method E | 8.76 - 8.65 (m, 1H), 8.52 (dd, J = 4.8, 1.6 Hz, 1H), 7.94 - 7.83 (m, 1H), 7.46 (ddd, J = 8.0, 4.8, 0.9 Hz, 1H), 7.10 (td, J = 8.4, 2.0 Hz, 1H), 6.69 (td, J = 8.5, 1.8 Hz, 1H), 5.75 (s, 2H). | Pd $(PPh_3)_4$, $K_2CO_3$, dioxane |
| INTA7 | 3-fluoro-4-(pyrazin-2-yl)aniline 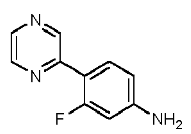 | Method F, [UPLC basic], 190, (0.79). | 8.88 (dd, J = 2.5, 1.6 Hz, 1H), (dd, J = 2.5, 1.6 Hz, 1H), 8.45 (d, J = 2.5 Hz, 1H), 7.72 (t, J = 8.9 Hz, 1H), 6.53 (dd, J = 8.6, 2.2 Hz, 1H), 6.43 (dd, J = 14.6, 2.1 Hz, 1H), 5.95 (s, 2H). | $PdCl_2$ (dppf), $K_3PO_4$, dioxane |
| INTA8 | 2-chloro-4-(pyridin-3-yl)aniline 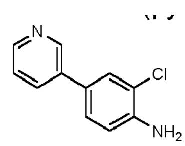 | Method E, [UPLC basic], 206, (1.04). | 8.81 (d, J = 2.5 Hz, 1H), 8.45 (dd, J = 4.7, 1.6 Hz, 1H), 7.97 (ddd, J = 8.0, 2.4, 1.6 Hz, 1H), 7.60 (d, J = 2.1 Hz, 1H), 7.48 - 7.28 (m, 2H), 6.89 (d, J = 8.4 Hz, 1H), 5.60 (s, 2H). | Pd $(PPh_3)_4$, $K_2CO_3$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA9 | 3-methoxy-4-(pyrazin-2-yl) aniline 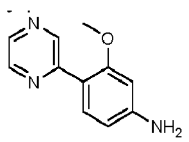 | Method F, [UPLC basic], 202, (0.75). | None recorded | $PdCl_2$ (dppf), $K_3PO_4$, dioxane |
| INTA10 | 3-(4-aminophenyl)-5-(difluoro-l3-methoxy)pyridine 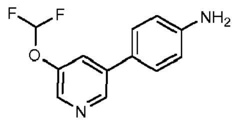 | Method E, [UPLC basic], 237, (1.05). | 8.74 - 8.66 (m, 1H), 8.36 - 8.24 (m, 1H), 7.81 - 7.74 (m, 1H), 7.53 - 7.43 (m, 2H), 7.61 - 7.15 (m, 1H), 6.72 - 6.58 (m, 2H), 5.44 (s, 2H). | $PdCl_2$ (dppf), $K_2CO_3$, dioxane |
| INTA11 | 4-(5-ethoxypyridin-3-yl)aniline 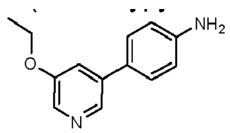 | Method F, [UPLC basic], 215, (1.05). | 8.36 (d, J = 1.9 Hz, 1H), 8.11 (d, J = 2.7 Hz, 1H), 7.48 - 7.39 (m, 3H), 6.70 - 6.62 (m, 2H), 5.34 (s, 2H), 4.17 (q, J = 6.9 Hz, 2H), 1.36 (t, J = 7.0 Hz, 3H). | $PdCl_2$ (dppf), $K_2CO_3$, dioxane |
| INTA12 | 5-(4-aminophenyl) nicotinonitrile 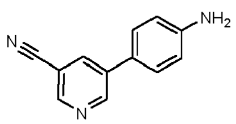 | Method E, [UPLC basic], 196, (0.89). | 9.08 (d, J = 2.4 Hz, 1H), 8.91 - 8.79 (m, 1H), 8.49 - 8.43 (m, 1H), 7.58 - 7.48 (m, 2H), 6.73 - 6.57 (m, 2H), 5.49 (d, J = 7.0 Hz, 2H). | $PdCl_2$ (dppf), $K_2CO_3$, dioxane |
| INTA13 | 4-(5-fluoropyridin-3-yl)aniline 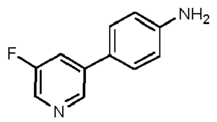 | Method E, [HPLC basic], 189, (1.53). | 8.69 (t, J = 2.0 Hz, 1H), 8.39 (d, J = 2.7 Hz, 1H), 7.87 (ddd, J = 10.9, 2.7, 1.9 Hz, 1H), 7.53 - 7.45 (m, 2H), 6.71 - 6.63 (m, 2H), 5.44 (s, 2H). | $PdCl_2$ (dppf), $K_2CO_3$, dioxane |
| INTA14 | 4-(5-(trifluoromethyl)pyridin-3-yl)aniline 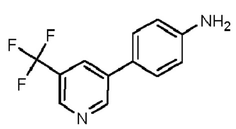 | Method E, [UPLC basic], 239, (1.21). | 9.15 - 9.06 (m, 1H), 8.82 - 8.72 (m, 1H), 8.30 - 8.23 (m, 1H), 7.60 - 7.51 (m, 2H), 6.74 - 6.64 (m, 2H), 5.50 (s, 2H). | $PdCl_2$ (dppf), $K_2CO_3$, dioxane |
| INTA15 | 5-amino-2-(pyridin-3-yl) benzonitrile 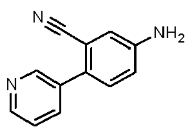 | Method E, [HPLC basic], 196, (1.12). | 8.68 (dd, J = 2.5, 0.9 Hz, 1H), 8.58 (dd, J = 4.8, 1.6 Hz, 1H), 7.91 (ddd, J = 7.9, 2.4, 1.6 Hz, 1H), 7.49 (ddd, J = 7.9, 4.8, 0.9 Hz, 1H), 7.34 (d, J = 8.4 Hz, 1H), 7.02 (d, J = 2.4 Hz, 1H), 6.96 (dd, J = 8.5, 2.5 Hz, 1 H), 5.84 (s, 2H). | $PdCl_2$ (dppf), $K_2CO_3$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA16 | 3-methoxy-4-(pyridin-3- 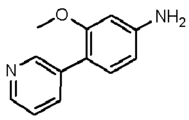 yl)aniline | Method E, [HPLC basic], 201, (1.39). | 8.60 (dd, J = 2.4, 0.9 Hz, 1H), 8.38 (dd, J = 4.7, 1.7 Hz, 1H), 7.78 (ddd, J = 7.9, 2.3, 1.7 Hz, 1H), 7.34 (ddd, J = 7.9, 4.8, 0.9 Hz, 1H), 7.02 (d, J = 8.1 Hz, 1H), 6.34 (d, J = 2.1 Hz, 1H), 6.26 (dd, J = 8.1, 2.0 Hz, 1H), 5.35 (s, 2H), 3.71 (s, 3H). | $PdCl_2$ (dppf), $K_3PO_4$, dioxane |
| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
| INTA17 | 3-chloro-4-(pyridin-3-yl)aniline 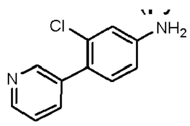 | Method E, [UPLC basic], 205, (0.98). | 8.56 (dd, J = 2.4, 0.9 Hz, 1H), 8.54 - 8.47 (m, 1H), 7.78 (ddd, J = 7.9, 2.3, 1.6 Hz, 1H), 7.42 (ddd, J = 7.9, 4.8, 0.9 Hz, 1H), 7.11 (d, J = 8.3 Hz, 1H), 6.74 (d, J = 2.2 Hz, 1H), 6.62 (dd, J = 8.3, 2.3 Hz, 1H), 5.62 (s, 2H). | $PdCl_2$ (dppf), $K_3PO_4$, dioxane |
| INTA18 | 4-(5-chloropyridin-3-yl)aniline 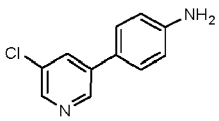 | Method E, [UPLC basic], 205, (1.11). | 8.79 - 8.74 (m, 1H), 8.49 - 8.39 (m, 1H), 8.10 - 7.98 (m, 1H), 7.54 - 7.42 (m, 2H), 6.72 - 6.61 (m, 2H), 5.45 (s, 2H). | $PdCl_2$ (dppf), $K_2CO_3$, dioxane |
| INTA19 | 4-(2-methylpyridin-3-yl)aniline 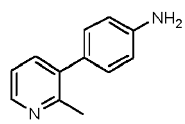 | Method E, [HPLC basic], 185, (1.37). | 8.36 (dd, J = 4.8, 1.8 Hz, 1H), 7.51 (dd, J = 7.7, 1.8 Hz, 1H), 7.22 (dd, J = 7.6, 4.8 Hz, 1H), 7.10 - 7.00 (m, 2H), 6.70 - 6.41 (m, 2H), 5.22 (s, 2H), 2.44 (s, 3H). | Pd 170, $K_3PO_4$, dioxane |
| INTA20 | 4-(6-methylpyridin-3-yl)aniline 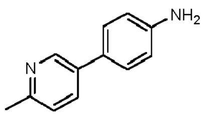 | Method E, [HPLC basic], 185, (1.43). | 8.63 (dd, J = 2.5, 0.8 Hz, 1H), 7.80 (dd, J = 8.1, 2.5 Hz, 1H), 7.40 - 7.32 (m, 2H), 7.23 (dt, J = 8.1, 0.7 Hz, 1H), 6.70 - 6.58 (m, 2H), 5.27 (s, 2H), 2.46 (s, 3H). | Pd 170, $K_3PO_4$, dioxane |
| INTA21 | 4-(5-methylpyridin-3-yl)aniline 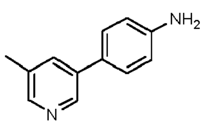 | Method E, [HPLC basic], 185, (1.47). | 8.57 (d, J = 2.3 Hz, 1H), 8.25 (dd, J = 2.0, 0.8 Hz, 1H), 7.74 (td, J = 2.2, 0.9 Hz, 1H), 7.43 - 7.30 (m, 2H), 6.72 - 6.52 (m, 2H), 5.31 (s, 2H), 2.33 (d, J = 0.8 Hz, 3H). | Pd 170, $K_3PO_4$, dioxane |
| INTA22 | 4-(5-isopropoxypyridin-3-yl) aniline 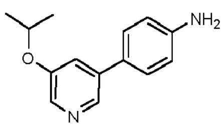 | Method F, [UPLC basic], 229, (1.15). | 8.41 - 8.30 (m, 1H), 8.13 - 8.05 (m, 1H), 7.48 - 7.35 (m, 3H), 6.72 - 6.59 (m, 2H), 5.34 (s, 2H), 4.86 - 4.69 (m, 1H), 1.37 - 1.23 (m, 6H). | $PdCl_2$ (dppf), $K_2CO_3$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA23 | 2-fluoro-4-(5-isopropoxypyridin-3-yl)aniline 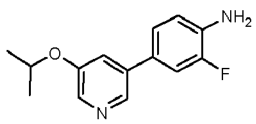 | Method F, [UPLC basic], 247, (1.25). | 8.42 - 8.34 (m, 1H), 8.17 - 8.06 (m, 1H), 7.53 - 7.42 (m, 2H), 7.35 - 7.28 (m, 1H), 6.89 - 6.80 (m, 1H), 5.39 (s, 2H), 4.90 - 4.75 (m, 1H), 1.35 - 1.26 (m, 6H). | $PdCl_2$ (dppf), $K_2CO_3$, dioxane |
| INTA24 | 4-(5-(difluoromethoxy)pyridin-3-yl)-2-fluoroaniline 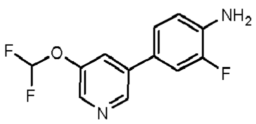 | Method F, [UPLC basic], 255, (1.15). | 8.74 (d, J = 1.9 Hz, 1H), 8.35 (d, J = 2.6 Hz, 1H), 7.85 (t, J = 2.3 Hz, 1H), 7.57 - 7.25 (m, 3H), 6.87 (dd, J = 9.5, 8.3 Hz, 1H), 5.50 (s, 2H). | $PdCl_2$ (dppf), $K_2CO_3$, dioxane |
| INTA25 | 4-(5-(difluoromethoxy)pyridin-3-yl)aniline 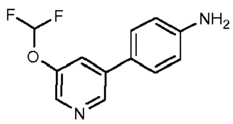 | Method F, [UPLC basic], 237, (1.10). | (Methanol-d4) δ 8.60 (d, J = 2.0 Hz, 1H), 8.27 (d, J = 2.3 Hz, 1H), 7.78 - 7.72 (m, 1H), 7.47 - 7.40 (m, 2H), 7.18 - 6.78 (m, 3H), (Exchangable protons not observed). | $PdCl_2$ (dppf), $K_2CO_3$, dioxane |
| INTA26 | 5-(4-amino-3-fluorophenyl) nicotinonitrile 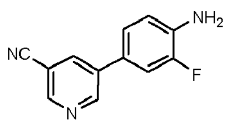 | Method E, [HPLC basic], 214, (1.6). | 9.13 (d, J = 2.3 Hz, 1H), 8.86 (d, J = 1.9 Hz, 1H), 8.54 (t, J = 2.1 Hz, 1 H), 7.60 (dd, J = 13.1, 2.1 Hz, 1H), 7.43 (dd, J = 8.3, 2.1 Hz, 1H), 6.87 (dd, J = 9.5, 8.3 Hz, 1H), 5.56 (s, 2H), . | $PdCl_2$ (dppf), $K_2CO_3$, dioxane |
| INTA27 | 4-(5-chloropyridin-3-yl)-2-fluoroaniline 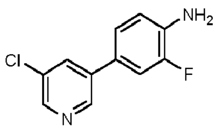 | Method E, [HPLC basic], 223, (1.9). | 8.80 (d, J = 2.0 Hz, 1H), 8.48 (d, J = 2.2 Hz, 1H), 8.14 (t, J = 2.2 Hz, 1H), 7.54 (dd, J = 13.0, 2.1 Hz, 1H), 7.38 (dd, J = 8.3, 2.1 Hz, 1H), 6.86 (dd, J = 9.5, 8.3 Hz, 1H), 5.51 (s, 2H). | $PdCl_2$ (dppf), $K_2CO_3$, dioxane |
| INTA28 | 4-(5-ethoxypyridin-3-yl)-2-fluoroaniline 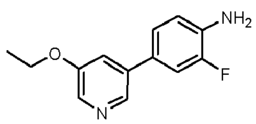 | Method F, [UPLC basic], 233, (1.16). | 8.40 (d, J = 1.9 Hz, 1H), 8.15 (d, J = 2.7 Hz, 1H), 7.51 (dd, J = 2.7, 1.9 Hz, 1H), 7.47 (dd, J = 13.0, 2.1 Hz, 1H), 7.33 (dd, J = 8.3, 2.1 Hz, 1H), 6.85 (dd, J = 9.5, 8.3 Hz, 1H), 5.40 (s, 2H), 4.18 (q, J = 7.0 Hz, 2H), 1.37 (t, J = 7.0 Hz, 3H). | $PdCl_2$ (dppf), $K_3PO_4$, dioxane |
| INTA29 | 2-fluoro-4-(5-fluoropyridin-3-yl)aniline 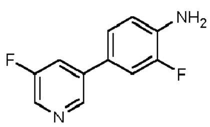 | Method F, [UPLC basic], 207, (1.07). | 8.74 (t, J = 1.9 Hz, 1H), 8.43 (d, J = 2.7 Hz, 1H), 8.00 - 7.89 (m, 1 H), 7.54 (dd, J = 13.0, 2.1 Hz, 1H), 7.39 (dd, J = 8.3, 2.1 Hz, 1H), 6.86 (dd, J = 9.5, 8.3 Hz, 1H), 5.50 (s, 2H). | $PdCl_2$ (dppf), $K_3PO_4$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA30 | 2-fluoro-4-(5-(2,2,2-trifluoro ethoxy)pyridin-3-yl)aniline 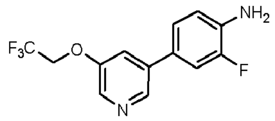 | Method F, [UPLC basic], 287, (1.26). | 8.61 - 8.50 (m, 1H), 8.31 - 8.22 (m, 1H), 7.77 - 7.64 (m, 1H), 7.59 - 7.47 (m, 1H), 7.42 - 7.33 (m, 1H), 6.93 - 6.75 (m, 1H), 5.44 (s, 2H), 5.01 - 4.88 (m, 2H). | $PdCl_2$ (dppf), $K_3PO_4$, dioxane |
| INTA31 | 4-(5-(2,2,2-trifluoro ethoxy) pyridin-3-yl)aniline 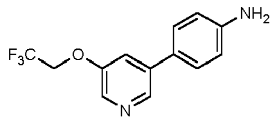 | Method F, [UPLC basic], 269, (1.18). | 8.55 - 8.41 (m, 1H), 8.29 - 8.13 (m, 1H), 7.69 - 7.61 (m, 1H), 7.53 - 7.43 (m, 2H), 6.73 - 6.61 (m, 2H), 5.38 (s, 2H), 5.02 - 4.82 (m, 2H). | $PdCl_2$ (dppf), $K_3PO_4$, dioxane |
| INTA32 | 5'-ethoxy-[3,3'-bipyridin]-6- 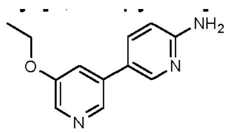 amine | Method F, [UPLC basic], 216, (0.88). | 8.39 (d, J = 1.9 Hz, 1H), 8.32 (d, J = 2.5 Hz, 1H), 8.17 (d, J = 2.7 Hz, 1H), 7.78 (dd, J = 8.6, 2.6 Hz, 1H), 7.51 (dd, J = 2.7, 1.9 Hz, 1H), 6.54 (dd, J = 8.6, 0.8 Hz, 1H), 6.18 (s, 2H), 4.18 (q, J = 7.0 Hz, 2H), 1.37 (t, J = 7.0 Hz, 3H). | $PdCl_2$ (dppf), $K_2CO_3$, dioxane |
| INTA33 | 6'-amino-[3,3'-bipyridine]-5-carbonitrile 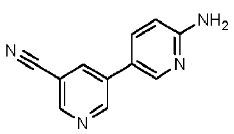 | Method E, [UPLC basic], 197, (0.72). | 9.12 (d, J = 2.3 Hz, 1H), 8.88 (d, J = 1.9 Hz, 1H), 8.55 (t, J = 2.1 Hz, 1H), 8.41 (dd, J = 2.6, 0.7 Hz, 1H), 7.86 (dd, J = 8.7, 2.6 Hz, 1H), 6.56 (dd, J = 8.7, 0.8 Hz, 1H), 6.33 (s, 2H). | $PdCl_2$ (dppf), $K_2CO_3$, dioxane |
| INTA34 | 5'-methyl-[3,3'-bipyridin]-6-amine 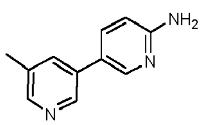 | Method F, [HPLC basic], 186, (1.22). | 8.60 (d, J = 2.2 Hz, 1H), 8.39 - 8.23 (m, 2H), 7.86 - 7.70 (m, 2H), 6.54 (dd, J = 8.7, 0.9 Hz, 1H), 6.15 (s, 2H), 2.34 (s, 3H). | Pd $(PPh_3)_4$, $NaHCO_3$, MeCN |
| INTA35 | 5'-(difluoromethoxy)-[3,3'-bipyridin]-6-amine 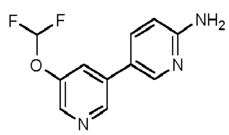 | Method F, [UPLC basic], 238, (0.88). | 8.73 (d, J = 1.9 Hz, 1H), 8.37 (d, J = 2.6 Hz, 2H), 7.88 - 7.79 (m, 2H), 7.41 (t, J = 73.6 Hz, 1H), 6.55 (dd, J = 8.6, 0.8 Hz, 1H), 6.28 (s, 2H). | $PdCl_2$ (dppf), $K_2CO_3$, dioxane |
| INTA36 | 5'-(2,2,2-trifluoroethoxy)-[3,3'-bipyridin]-6-amine 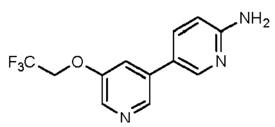 | Method F, [UPLC basic], 270, (1.00). | 8.57 - 8.46 (m, 1H), 8.41 - 8.34 (m, 1H), 8.32 - 8.23 (m, 1H), 7.88 - 7.78 (m, 1H), 7.76 - 7.68 (m, 1H), 6.60 - 6.50 (m, 1H), 6.22 (s, 2H), 5.03 - 4.87 (m, 2H). | $PdCl_2$ (dppf), $K_3PO_4$, dioxane |

(continued)

| INTA# | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| INTA37 | 5'-fluoro-[3,3'-bipyridin]-6- 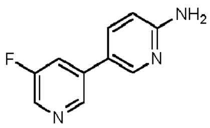 amine | Method F, [UPLC basic], 190, (0.76). | 8.72 (t, J = 1.9 Hz, 1H), 8.45 (d, J = 2.7 Hz, 1H), 8.38 (dd, J = 2.6, 0.8 Hz, 1H), 7.97 (ddd, J = 10.7, 2.7, 1.8 Hz, 1H), 7.83 (dd, J = 8.6, 2.6 Hz, 1H), 6.55 (dd, J = 8.6, 0.8 Hz, 1H), 6.28 (s, 2H). | $PdCl_2$ (dppf), $C_{S2}CO_3$, dioxane |
| INTA38 | 4-(6-ethoxypyrazin-2-yl) aniline 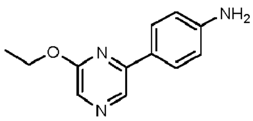 | Method F, [HPLC basic], 216, (1.78). | 8.59 (s, 1H), 8.00 (s, 1H), 7.86 - 7.75 (m, 2H), 6.69 - 6.59 (m, 2H), 5.59 (s, 2H), 4.43 (q, J = 7.0 Hz, 2H), 1.38 (t, J = 7.0 Hz, 3H). | Pd $(PPh_3)_4$, $NaHCO_3$, MeCN |
| INTA39 | 4-(6-(trifluoromethyl)pyrazin-2-yl)aniline 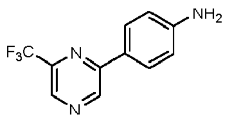 | Method F, [HPLC acidic], 240, (1.92). | 9.40 (s, 1H), 8.86 (s, 1H), 7.99 - 7.89 (m, 2H), 6.74 - 6.66 (m, 2H), 5.83 (s, 2H). | Pd $(PPh_3)_4$, $NaHCO_3$, MeCN |
| INTA40 | 4-(6-isopropoxypyrazin-2-yl) aniline 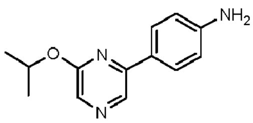 | Method F, [UPLC basic], 230, (1.31). | 8.57 (s, 1H), 7.95 (s, 1H), 7.87 - 7.73 (m, 2H), 6.73 - 6.56 (m, 2H), 5.59 (s, 2H), 5.45 - 5.27 (m, 1H), 1.47 - 1.25 (m, 6H). | $PdCl_2$ (dppf), $K_3PO_4$, dioxane |
| INTA41 | 4-(6-cyclopropoxypyrazin-2-yl)aniline 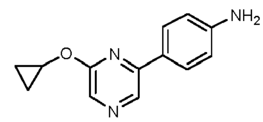 | Method F, [HPLC basic], 228, (1.83). | 8.19 - 8.02 (m, 1H), 7.58 - 7.41 (m, 1H), 7.40 - 7.22 (m, 2H), 6.21 - 5.99 (m, 2H), 5.09-4.99 (m, 2H), 3.86-3.73 (m, 1H), 0.37 - 0.09 (m, 4H). | $PdCl_2$ (dppf), $K_3PO_4$, dioxane |
| INTA42 | 4-(6-propoxypyrazin-2-yl) aniline 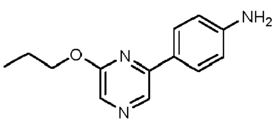 | Method F, [HPLC basic], 230, (2.07). | 8.59 (s, 1H), 8.01 (s, 1H), 7.90 - 7.76 (m, 2H), 6.72 - 6.59 (m, 2H), 5.59 (s, 2H), 4.34 (t, J = 6.6 Hz, 2H), 1.86 - 1.72 (m, 2H), 1.00 (t, J = 7.4 Hz, 3H). | $PdCl_2$ (dppf), $K_3PO_4$, dioxane |
| INTA43 | 4-(6-chloropyrazin-2-yl)aniline 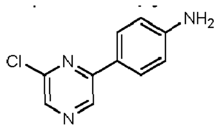 | Method F, [HPLC basic], 206, (1.75). | 9.06 (d, J = 0.6 Hz, 1H), 8.47 (d, J 0.6 Hz, 1H), 7.89 - 7.81 (m, 2H), 6.73 - 6.62 (m, 2H), 5.79 (s, 2H). | $PdCl_2$ (dppf), $K_3PO_4$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA44 | 6-(4-aminophenyl)pyrazine-2-carbonitrile 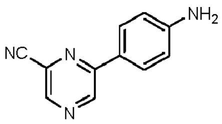 | Method F, [HPLC basic], 197, (1.54). | 9.36 (d, J = 0.5 Hz, 1H), 8.90 (d, J 0.5 Hz, 1H), 7.95 - 7.86 (m, 2H), 6.74 - 6.64 (m, 2H), 5.86 (s, 2H). | $PdCl_2$ (dppf), $K_3PO_4$, dioxane |
| INTA45 | 2-fluoro-4-(pyrazin-2-yl)aniline 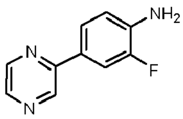 | Method F, [UPLC basic], 190, (0.84). | 9.12 (d, J = 1.6 Hz, 1H), 8.58 (dd, J = 2.6, 1.5 Hz, 1H), 8.44 (d, J = 2.5 Hz, 1H), 7.86 - 7.71 (m, 2H), 6.87 (dd, J = 9.3, 8.4 Hz, 1H), 5.67 (s, 2H). | $PdCl_2$ (dppf), $K_2CO_3$, dioxane |
| INTA47 | 4-(6-ethoxypyrazin-2-yl)-2-fluoroaniline 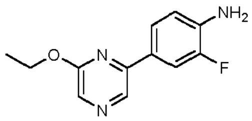 | Method F, [UPLC basic], 234, (1.31). | 8.66 (s, 1H), 8.06 (s, 1H), 7.84 - 7.63 (m, 2H), 6.93 - 6.75 (m, 1H), 5.65 (s, 2H), 4.54 - 4.34 (m, 2H), 1.47 - 1.29 (m, 3H), | $PdCl_2$ (dppf), $K_2CO_3$, dioxane |
| INTA48 | 2-fluoro-4-(6-(trifluoromethyl) pyrazin-2-yl)aniline 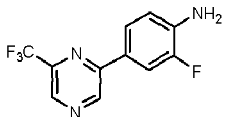 | Method F, [HPLC basic], 258, (2.13). | 9.46 (s, 1H), 8.92 (s, 1H), 7.88 (dd, J = 13.1,2.1 Hz, 1H), 7.83 (dd, J = 8.4, 2.1 Hz, 1H), 6.90 (t, J = 8.8 Hz, 1H), 5.90 (s, 2H). | $PdCl_2$ (dppf), $K_3PO_4$, dioxane |
| INTA49 | 4-(6-chloropyrazin-2-yl)-2-methylaniline 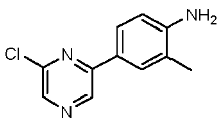 | Method F, [UPLC basic], 220, (1.24). | 9.06 (s, 1H), 8.46 (s, 1H), 7.84 - 7.65 (m, 2H), 6.78 - 6.63 (m, 1H), 5.55 (s, 2H), 2.14 (s, 3H). | $PdCl_2$ (dppf), $K_3PO_4$, dioxane |
| INTA50 | 6-(4-amino-3-methylphenyl) pyrazine-2-carbonitrile 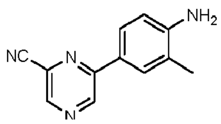 | Method F, [HPLC basic], 211, (1.67). | 9.36 (s, 1H), 8.89 (s, 1H), 7.88 - 7.72 (m, 2H), 6.78 - 6.67 (m, 1H), 5.62 (s, 2H), 2.14 (s, 3H). | $PdCl_2$ (dppf), $K_3PO_4$, dioxane |
| INTA51 | 4-(6-ethoxypyrazin-2-yl)-2-methylaniline 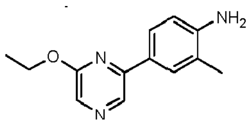 | Method F, [UPLC basic], 230, (1.27). | 8.60 (s, 1H), 8.00 (s, 1H), 7.80 - 7.63 (m, 2H), 6.75 - 6.61 (m, 1H), 5.35 (s, 2H), 4.51 - 4.33 (m, 2H), 2.13 (s, 3H), 1.45 - 1.31 (m, 3H). | $PdCl_2$ (dppf), $K_3PO_4$, dioxane |

(continued)

| INTA# | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| INTA52 | 2-fluoro-4-(6-(2,2,2-trifluoroethoxy)pyrazin-2-yl) aniline 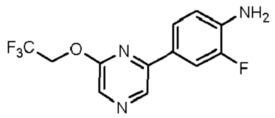 | Method F, [UPLC basic], 288, (1.38). | 8.82 (s, 1H), 8.24 (s, 1H), 7.94 - 7.85 (m, 1H), 7.81 - 7.74 (m, 1H), 6.92 - 6.82 (m, 1H), 5.72 (s, 2H), 5.23 - 5.09 (m, 2H). | $PdCl_2$ (dppf), $K_3PO_4$, dioxane |
| INTA53 | 4-(6-(2,2,2-trifluoroethoxy) pyrazin-2-yl)aniline 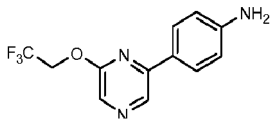 | Method F, [UPLC basic], 270, (1.31). | 8.76 (s, 1H), 8.18 (s, 1H), 7.95 - 7.84 (m, 2H), 6.73 - 6.58 (m, 2H), 5.67 (s, 2H), 5.22 - 5.04 (m, 2H). | $PdCl_2$ (dppf), $K_3PO_4$, dioxane |
| INTA55 | 5-(6-cyclobutoxypyrazin-2-yl) pyridin-2-amine 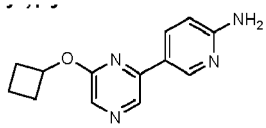 | Method F, [UPLC basic], 243, (1.16). | 8.70 (dd, J = 2.5, 0.8 Hz, 1H), 8.66 (s, 1H), 8.12 - 8.03 (m, 2H), 6.55 (dd, J = 8.8, 0.8 Hz, 1H), 6.42 (s, 2H), 5.31 - 5.14 (m, 1H), 2.49 - 2.43 (m, 2H), 2.21 - 2.04 (m, 2H), 1.90 - 1.64 (m, 2H). | Pd $(PPh_3)_4$, $Na_2CO^3$, Toluene, EtOH |
| INTA56 | 5-(6-cyclopropoxypyrazin-2-yl)pyridin-2-amine 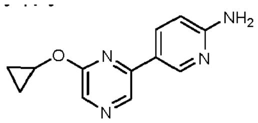 | Method F, [UPLC basic], 229, (0.98). | 8.81 - 8.64 (m, 2H), 8.18 - 8.00 (m, 2H), 6.55 (dd, J = 8.8, 0.8 Hz, 1H), 6.44 (s, 2H), 4.37 (tt, J = 6.2, 3.0 Hz, 1H), 0.94 - 0.69 (m, 4H). | Pd $(PPh_3)_4$, $Na_2CO_3$, Toluene, EtOH |
| INTA57 | 5-(6-ethoxypyrazin-2-yl)-3-fluoropyridin-2-amine 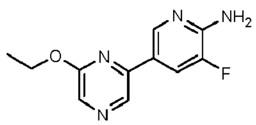 | Method F, [HPLC basic], 235, (1.73). | 8.72 (s, 1H), 8.62 - 8.57 (m, 1H), 8.12 (s, 1H), 8.04 (dd, J = 12.6, 1.9 Hz, 1H), 6.73 (s, 2H), 4.46 (q, J = 7.0 Hz, 2H), 1.39 (t, J = 7.0 Hz, 3H). | $PdCl_2$ (dppf), $K_2CO_3$, dioxane |
| INTA58 | 3-fluoro-5-(6-(trifluoromethyl) pyrazin-2-yl)pyridin-2-amine 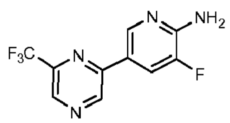 | Method F, [HPLC basic], 259, (1.82). | 9.53 (s, 1H), 9.00 (s, 1H), 8.72 - 8.66 (m, 1H), 8.09 (dd, J = 12.4, 2.0 Hz, 1H), 6.98 (s, 2H). | $PdCl_2$ (dppf), $K_2CO_3$, dioxane |
| INTA59 | 6-(6-amino-5-fluoropyridin-3-yl)pyrazine-2-carbonitrile 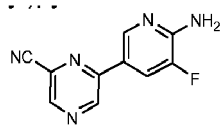 | Method F, [UPLC acidic], 216, (0.87). | 9.48 (s, 1H), 9.03 (s, 1H), 8.70 - 8.64 (m, 1H), 8.09 (dd, J = 12.5, 2.0 Hz, 1H), 6.99 (s, 2H). | $PdCl_2$ (dppf), $K_2CO_3$, dioxane |

(continued)

| INTA# | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| INTA60 | 3-fluoro-5-(pyrazin-2-yl) pyridin-2-amine 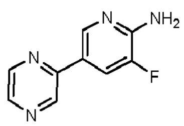 | Method F, [UPLC basic], 191, (0.7). | 9.18 (d, J = 1.5 Hz, 1H), 8.65 - 8.59 (m, 2H), 8.50 (d, J = 2.5 Hz, 1H), 8.06 (dd, J = 12.6, 1.9 Hz, 1H), 6.75 (s, 2H). | $PdCl_2$ (dppf), $K_2CO_3$, dioxane |
| INTA61 | 5-(6-methoxypyrazin-2-yl) pyridin-2-amine 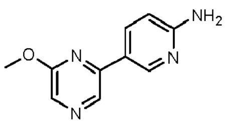 | Method F, [UPLC basic], 203, (0.85). | 8.76 - 8.71 (m, 1H), 8.67 (s, 1H), 8.14 - 8.08 (m, 2H), 6.56 (dd, J = 8.7, 0.8 Hz, 1H), 6.43 (s, 2H), 3.98 (s, 3H). | $PdCl_2$ (dppf), $K_3PO_4$, dioxane |
| INTA62 | 5-(6-(2,2,2-trifluoroethoxy) pyrazin-2-yl)pyridin-2-amine 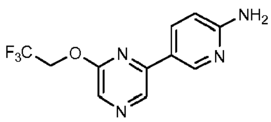 | Method F, [UPLC basic], 271, (1.11). | 8.84 - 8.80 (m, 1H), 8.79 - 8.75 (m, 1H), 8.30 - 8.23 (m, 1H), 8.19 - 8.12 (m, 1H), 6.62 - 6.52 (m, 1H), 6.49 (s, 2H), 5.23 - 5.06 (m, 2H). | $PdCl_2$ (dppf), $K_3PO_4$, dioxane |
| INTA63 | 6-(6-aminopyridin-3-yl) pyrazine-2-carbonitrile 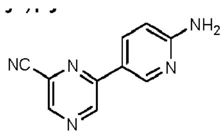 | Method F, [UPLC basic], 198, (0.76). | 9.44 (s, 1H), 8.98 (s, 1H), 8.79 (dd, J = 2.6, 0.7 Hz, 1H), 8.14 (dd, J = 8.8, 2.6 Hz, 1H), 6.68 (s, 2H), 6.58 (dd, J = 8.8, 0.7 Hz, 1H). | $PdCl_2$ (dppf), $K_3PO_4$, dioxane |
| INTA64 | 5-(6-chloropyrazin-2-yl) pyridin-2-amine 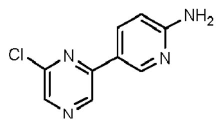 | Method F, [HPLC basic], 207, (0.41). | 9.12 (s, 1H), 8.73 (dd, J = 2.6, 0.7 Hz, 1H), 8.55 (s, 1H), 8.08 (dd, J = 8.8, 2.6 Hz, 1H), 6.60 (s, 2H), 6.55 (dd, J = 8.8, 0.8 Hz, 1H). | $PdCl_2$ (dppf), $C_{S2}CO_3$, dioxane |
| INTA65 | N-methyl-4-(6-(trifluoromethyl) pyrazin-2-yl)aniline 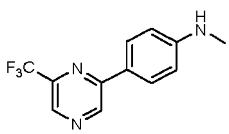 | Method F, [UPLC basic], 254, (1.47). | 9.42 (s, 1H), 8.86 (s, 1H), 8.03 - 7.97 (m, 2H), 6.72 - 6.65 (m, 2H), 6.41 (q, J = 5.0 Hz, 1H), 2.76 (d, J = 5.0 Hz, 3H). | $PdCl_2$ (dppf), $K_2CO_3$, dioxane |
| INTA66 | 4-(5-chloropyridin-3-yl)-N-methylaniline 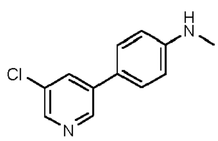 | Method E, [UPLC basic], 219, (1.34). | 8.77 (d, J = 2.0 Hz, 1H), 8.44 (d, J 2.3 Hz, 1H), 8.10 - 8.05 (m, 1H), 7.60 - 7.53 (m, 2H), 6.68 - 6.61 (m, 2H), 6.06 - 6.00 (m, 1H), 2.73 (d, J = 5.0 Hz, 3H). | $PdCl_2$ (dppf), $K_2CO_3$, dioxane |

(continued)

| INTA# | Name/Structure | Synthesis Method, [LCMS Method], m/z (M+H)$^+$, (Rt/min) | $^1$H NMR Chemical Shift Data (DMSO-d6 unless stated) | Catalyst, Base, solvent |
|---|---|---|---|---|
| INTA67 | 5-(4-(methylamino) phenyl) nicotinonitrile 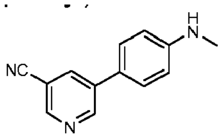 | Method E, [UPLC basic], 210, (1.14). | 9.10 (d, J = 2.4 Hz, 1H), 8.82 (d, J 1.9 Hz, 1H), 8.51 - 8.44 (m, 1H), 7.65 - 7.56 (m, 2H), 6.71 - 6.62 (m, 2H), 6.12 - 6.05 (m, 1H), 2.73 (d, J = 5.0 Hz, 3H). | $PdCl_2$ (dppf), $K_2CO_3$, dioxane |
| INTA68 | N-methyl-4-(pyridin-3-yl) aniline 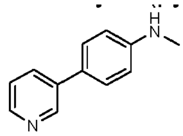 | Method E, [UPLC basic], 185, (1.04). | 8.80 (dd, J = 2.4, 0.9 Hz, 1H), 8.42 (dd, J = 4.7, 1.6 Hz, 1H), 7.97 - 7.90 (m, 1H), 7.55 - 7.46 (m, 2H), 7.42 - 7.35 (m, 1H), 6.70 - 6.61 (m, 2H), 5.96 - 5.89 (m, 1H), 2.72 (d, J = 5.0 Hz, 3H). | $PdCl_2$ (dppf), $K_2CO_3$, dioxane |
| INTA69 | 3-methoxy-4-(pyrazin-2-yl) aniline 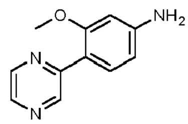 | Method F, [UPLC basic], 202, (0.75). | None recorded | $PdCl_2$ (dppf), $K_3PO_4$, dioxane |
| INTA70 | 2-fluoro-4-(5-(trifluoromethyl) pyridin-2-yl)aniline 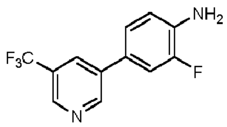 | Method F, [HPLC basic], 257, (2.06). | 9.14 (d, *J* = 2.2 Hz, 1H), 8.82 (dd, *J* = 2.1, 1.0 Hz, 1H), 8.35 (td, *J* = 2.3, 0.8 Hz, 1H), 7.63 (dd, *J* = 13.1, 2.1 Hz, 1H), 7.45 (dd, *J* = 8.3, 2.1 Hz, 1H), 6.88 (dd, *J* = 9.5, 8.3 Hz, 1H), 5.54 (s, 2H). | $PdCl_2$ (dppf), $K_2CO_3$, dioxane |
| INTA71 | 5-(6-isopropoxypyrazin-2-yl) pyridin-2-amine 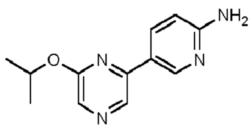 | Method F, [UPLC basic], 232, (1.09). | 8.69 (d, J = 2.4 Hz, 1H), 8.62 (s, 1H), 8.07 (dd, J = 8.7, 2.5 Hz, 1H), 8.02 (s, 1H), 6.55 (dd, J = 8.7, 0.8 Hz, 1H), 6.42 (s, 2H), 5.44 - 5.28 (m, 1H), 1.37 (d, J = 6.1 Hz, 6H). | Pd $(PPh_3)_4$, $Na_2CO_3$, dioxane |
| INTA72 | 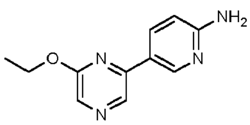 5-(6-ethoxypyrazin-2-yl)pyridin-2-amine | Method F, [UPLC,basic], 217, (0.98). | 8.70 (dd, J = 2.5, 0.8 Hz, 1H), 8.64 (s, 1H), 8.10 - 8.06 (m, 2H), 6.54 (dd, J = 8.7, 0.8 Hz, 1H), 6.41 (s, 2H), 4.43 (q, J = 7.0 Hz, 2H), 1.38 (t, J = 7.0 Hz, 3H). | $PdCl_2$ (dppf), $C_{S2}CO_3$, dioxane |

tert-butyl 5-(6-ethoxypyrazin-2-yl)indoline-1-carboxylate **INTA73**

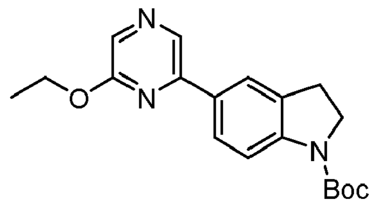

*Tert*-butyl 5-(4,4,5,5-tetramethyl-1,3,2-dioxaborolan-2-yl)indoline-1-carboxylate (1 g, 2.9 mmol), 2-chloro-6-ethoxypyrazine (0.418 g, 2.63 mmol) and $K_2CO_3$ (1.09 g, 7.9 mmol) were suspended in dioxane (10 mL) and water (3 mL). The mixture was degassed ($N_2$, mins), $PdCl_2$(dppf) (0.215 g, 0.26 mmol) was added and the resulting mixture was heated under $N_2$ at 90 °C for 18 hrs. The mixture was allowed to cool to RT, diluted with water (50 mL) and extracted with DCM (3 x 40 mL). The organics were combined, dried (phase separator) and concentrated in *vacuo.* The crude product was purified by chromatography on silica gel (40 g column, 0-100% EtOAc/isohexane) to afford tert-butyl 5-(6-ethoxypyrazin-2-yl)indoline-1-carboxylate (0.65 g, 1.71 mmol, 65 % yield) as a white solid; Rt 1.90 mins (UPLC acidic); m/z 342 $(M+H)^+$ $(ES^+)$; $^1H$ NMR (400 MHz, DMSO-d6) δ 8.82 - 8.66 (m, 1 H), 8.15 (s, 1 H), 8.03 - 7.87 (m, 2H), 7.87 - 7.30 (m, 1 H), 4.53 - 4.39 (m, 2H), 4.03 - 3.90 (m, 2H), 3.22 - 3.03 (m, 2H), 1.53 (s, 9H), 1.44 - 1.35 (m, 3H).

5-(6-ethoxypyrazin-2-yl)indoline **INTA74**

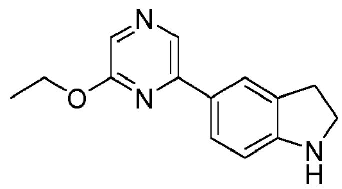

4M HCl (dioxane, 2.4 mL, 9.52 mmol) was added to *tert*-butyl 5-(6-ethoxypyrazin-2-yl)indoline-1-carboxylate (0.65 g, 1.9 mmol) in MeOH (20 mL). The resulting mixture was stirred under $N_2$ at 60 °C for 18 hrs. The mixture was allowed to cool to RT and concentrated in *vacuo.* The crude material was diluted in $NaHCO_3$ (sat. aq, 50 mL) and extracted with DCM (3 x 40 mL). The organics were combined, dried (phase separator) and concentrated in *vacuo.* The crude product was purified by chromatography on silica gel (24 g column, 0-100% EtOAc/isohexane) to afford 5-(6-ethoxypyrazin-2-yl)indoline (0.41 g, 1.36 mmol, 71 % yield) as a dark red solid; 1.35 mins (UPLC basic); m/z 242 $(M+H)^+$ $(ES^+)$; $^1H$ NMR (400 MHz, DMSO-d6) δ 8.62 (s, 1 H), 8.03 (s, 1 H), 7.87 - 7.82 (m, 1 H), 7.81 - 7.73 (m, 1 H), 6.69 - 6.62 (m, 1 H), 4.49 - 4.37 (m, 2H), 3.59 - 3.49 (m, 2H), 3.07 - 2.97 (m, 2H), 1.42 - 1.31 (m, 3H), 1 exchangeable proton not observed.

4-(6-((trimethylsilyl)ethynyl)pyrazin-2-yl)aniline **INTA46**

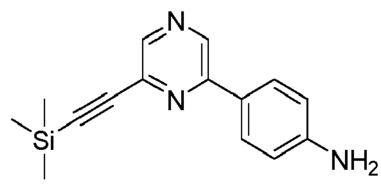

A solution of 4-(6-chloropyrazin-2-yl)aniline (150 mg, 0.73 mmol), ethynyltrimethylsilane (0.15 mL, 1.09 mmol) and DIPEA (0.19 mL, 1.09 mmol) in DMF (1 mL) was degassed ($N_2$, 5 mins). To this, Cu(I)I (28 mg, 0.15 mmol) and $Pd(PPh_3)_2Cl_2$ (51 mg, 0.07 mmol) were added and the mixture degassed ($N_2$, 5 mins) with sonication. The mixture was stirred at RT for 18 hrs. Solvent was evaporated and the mixture partitioned between EtOAc (20 mL) and water (20 mL). The phases were separated and the organics loaded onto silica. The crude product was purified by chromatography on silica gel (12 g column, 0-100% EtOAc/iso-hexane) to afford 4-(6-((trimethylsilyl)ethynyl)pyrazin-2-yl)aniline (200 mg, 0.73 mmol, 99 % yield) as a pale orange solid; Rt 2.44 mins (HPLC acidic); 268 $(M+H)^+$ $(ES^+)$; $^1H$ NMR (400 MHz, DMSO-$d_6$) δ 9.01 (s, 1 H), 8.46 (s, 1 H), 7.97 - 7.69 (m, 2H), 6.74 - 6.56 (m, 2H), 5.68 (s, 2H), 0.28 (s, 9H).

4-(6-ethynylpyrazin-2-yl)aniline **INTA75**

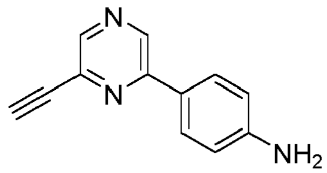

To a solution of 4-(6-((trimethylsilyl)ethynyl)pyrazin-2-yl)aniline (190 mg, 0.71 mmol) in MeOH (10 mL), $Na_2CO_3$ (565 mg, 5.33 mmol) was added and the mixture stirred at RT for 1 hr. Solvent was evaporated and the mixture was partitioned between water (20 mL) and EtOAc (20 mL). The organics were concentrated onto silica. The crude product was purified by chromatography on silica gel (12 g column, 0-100% EtOAc/iso-hexane) to afford 4-(6-ethynylpyrazin-2-yl)aniline (107 mg, 0.54 mmol, 77 % yield) as a yellow solid; Rt 1.46 mins (HPLC acidic); $^1H$ NMR (400 MHz, DMSO-d6) $\delta$ 9.05 (s, 1 H), 8.51 (s, 1 H), 8.01 - 7.75 (m, 2H), 6.86 - 6.50 (m, 2H), 5.70 (s, 2H), 4.60 (s, 1H).

**Preparation of Examples**

**Amide couplings**

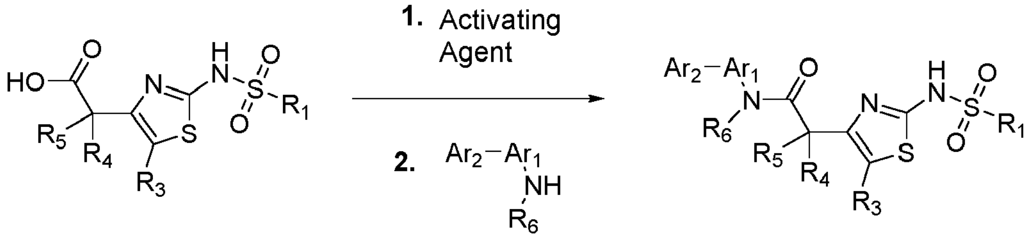


**Method 1:** HATU (1.2 eq) was added to a solution of appropriate acid (1 eq), amine (1 eq) and DIPEA (3 eq) in DCM (10 volumes) at RT. The reaction was stirred at RT for 18 hrs. The solvent was removed and the crude product was purified by normal phase chromatography, reverse phase chromatography or trituration from an appropriate solvent.

**Method 1b:** 1-chloro-N,N,2-trimethylprop-1-en-1-amine (2 eq) was added to a solution of 2-(2-(cyclopropanesulfonamido)thiazol-4-yl)-2-methylpropanoic acid (1 eq) in DCM (20 volumes). The reaction mixture was stirred at RT for 2 hrs. The reaction mixture was concentrated in *vacuo* and the residue redissolved in pyridine (2 mL) before addition of the appropriate amine (1.1 eq). The reaction mixture was stirred at RT for 2 hrs. An aqueous work up was performed and the crude product was purified by normal phase chromatography, reverse phase chromatography or trituration from an appropriate solvent.

**Method 1 c:** T3P (50% in EtOAc, 2.5 eq) was added to a solution of appropriate acid (1 eq), amine (1 eq) and pyridine (3 eq) in a mixture of EtOAc (20 volumes) and DMF (10 volumes). The reaction was stirred for 1 hr at RT. An aqueous work up was performed and the crude product was purified by normal phase chromatography, reverse phase chromatography or trituration from an appropriate solvent.

**Method 1e:** Thionyl chloride (2 eq) was added to a solution of an appropriate acid (1 eq) in toluene (20 volumes) at 70 °C. The reaction mixture was stirred at 70 °C for 1 hr. The reaction mixture was cooled to RT and concentrated to dryness. The resulting intermediate was redissolved in EtOAc (10 volumes) and a solution of amine (1.1 eq) in EtOAc (20 volumes) was added followed by triethylamine (2 eq). The reaction mixture was stirred at 40 °C for 16 hrs. An aqueous work up was performed and the crude product was purified by normal phase chromatography, reverse phase chromatography or trituration from an appropriate solvent.

**Suzuki couplings**

**Method 2: Suzuki coupling of Ar1-Bromide with heteroaromatic boronates**

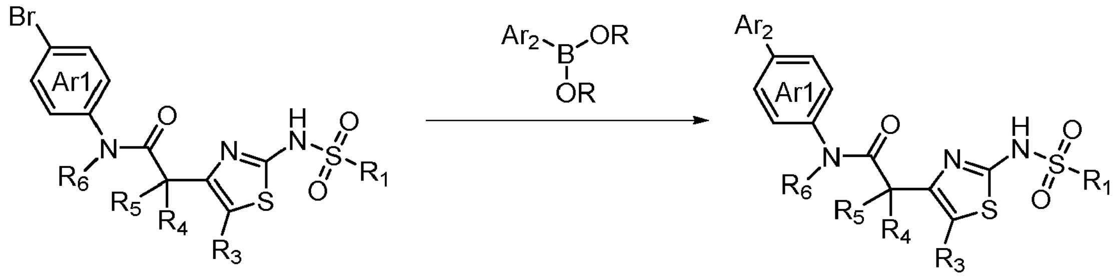


To a suspension of Ar1-Br (1 eq) in dioxane (10 volumes) was added arylboronic acid or ester (1 eq) and 2M $K_2PO_4$ (2 eq). The resulting suspension was heated to 60 °C and degassed ($N_2$, 5 mins). Pd 170 or other appropriate catalyst (5 mol %) was added and the reaction mixtures were stirred at 60 °C for 16 hrs. The reaction mixture was then cooled to RT. An aqueous work up was performed and the crude product was purified by normal phase chromatography, reverse phase chromatography or trituration from an appropriate solvent.

**Method 2b: Suzuki coupling of Ar1-B(OR)$_2$ with heteroaromatic halides**

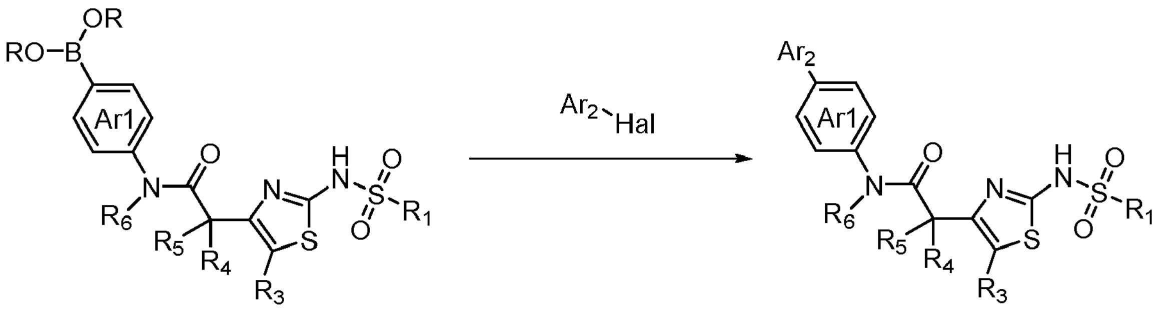


PdCl$_2$(dppf)-CH$_2$Cl$_2$ (10 mol %) or other appropriate catalyst was added to a degassed ($N_2$, 5 mins) solution of Ar1-B(OR)$_2$ (1 eq), Ar2-halide (1.2 eq) and $K_2CO_3$ (5 eq) in dioxane (10 volumes) and water (15 volumes). The solution was then degassed further ($N_2$, 5 mins) and then heated to 90 °C for 1-2 hrs. The reaction mixture was allowed to cool to RT. An aqueous work up was performed and the crude product was purified by normal phase chromatography, reverse phase chromatography or trituration from an appropriate solvent.

**Method 3: Coupling of primary amides with 2-chloropyrimidines**

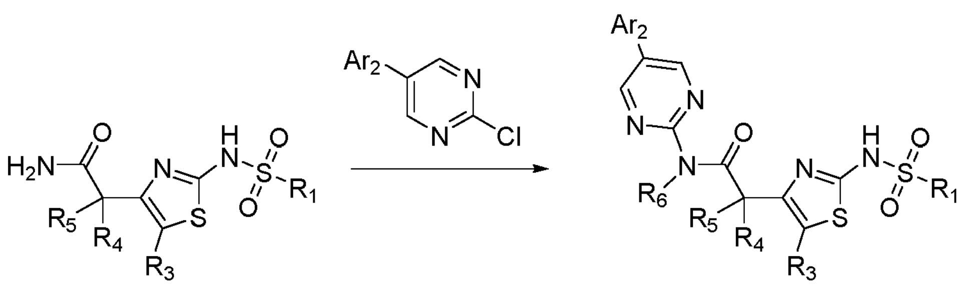

To a solution of amide (1 eq) and 2-chloropyrimidine (1 eq) in dioxane (30 volumes) was added $C_{S2}CO_3$ (1.5 eq). The reaction mixture was heated to 60 °C and degassed ($N_2$, 5 mins). Pd 177 (10 mol %) was added to the reaction mixture and the temperature was increased to 90 °C. After 2 hrs, the reaction was stirred for 16 hrs at 60 °C. The reaction mixture was cooled to RT and an aqueous work up was performed. The crude product was purified by normal phase chromatography, reverse phase chromatography or trituration from an appropriate solvent.

N-([1,1'-biphenyl]-4-yl)-2-(2-(methylsulfonamido)thiazol-4-yl)acetamide **T1**

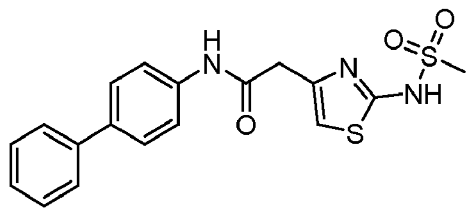

HATU (133 mg, 0.35 mmol) was added to a solution of 2-(2-(cyclopropanesulfonamido)thiazol-4-yl)acetic acid **INTB35** (75 mg, 0.32 mmol), [1,1'-biphenyl]-4-amine (53 mg, 0.32 mmol) and DIPEA (166 uL, 0.95 mmol) in DMF (1 mL) at RT. The reaction was stirred at RT for 18 hrs. The reaction mixture was acidified with addition of formic acid (100 μL), shaken for 5 min then filtered. The crude product was purified by preparative HPLC (Waters, Acidic (0.1% Formic acid), Acidic, Waters X-Select Prep-C18, 5 um, 19x50 mm column, 25-55% MeCN in Water) to afford N-([1,1'-biphenyl]-4-yl)-2-(2-(methylsulfonamido)thiazol-4-yl)acetamide; Rt 1.26 min (UPLC acidic); m/z 388 $(M+H)^+$ $(ES^+)$; $^1H$ NMR (400 MHz, DMSO-d6) δ 12.51 (s, 1H), 10.27 (s, 1H), 7.77 - 7.58 (m, 5H), 7.53 - 7.39 (m, 2H), 7.39 - 7.26 (m, 2H), 6.59 (s, 1 H), 3.67 (s, 2H), 2.90 (s, 3H).

N-([1,1'-biphenyl]-4-yl)-2-(2-(cyclopropanesulfonamido)thiazol-4-yl)acetamide **T2**

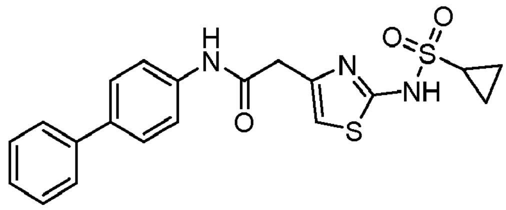

HATU (80 mg, 0.21 mmol) was added to a solution of 2-(2-(cyclopropanesulfonamido)thiazol-4-yl)acetic acid **INTB36** (50 mg, 0.19 mmol), [1,1'-biphenyl]-4-amine (35.5 mg, 0.21 mmol) and DIPEA (1.0 mL, 0.57 mmol) in DMF (0.5 mL) at RT. The reaction was stirred at RT for 18 hrs. The reaction mixture was acidified with addition of formic acid (50 μL), shaken for 5 mins then filtered. The crude product was purified by preparative HPLC (Waters, Acidic (0.1% Formic acid), Acidic, Waters X-Select Prep-C18, 5 um, 19x50 mm column, 35-65% MeCN in Water) to afford N-([1,1'-biphenyl]-4-yl)-2-(2-(cyclopropanesulfonamido)thiazol-4-yl)acetamide (32 mg, 0.077 mmol, 40 % yield) as an off-white solid; Rt 2.01 min (HPLC acidic); m/z 414 $(M+H)^+$ $(ES^+)$; 412 $(M-H)^-$ (ES-); $^1H$ NMR (400 MHz, DMSO-d6) δ 12.54 (s, 1H), 10.27 (s, 1H), 7.72 - 7.61 (m, 6H), 7.45 (dd, J = 8.4, 7.0 Hz, 2H), 7.38 - 7.29 (m, 1 H), 6.57 (s, 1 H), 3.71 - 3.65 (m, 2H), 2.62-2.56 (m, 6.0, 4.6 Hz, 1 H), 0.93 - 0.88 (m, 4H).

2-(2-(cyclopropanesulfonamido)thiazol-4-yl)-2-ethyl-N-(5-(pyrazin-2-yl)pyridin-2-yl)butanamide **T3**

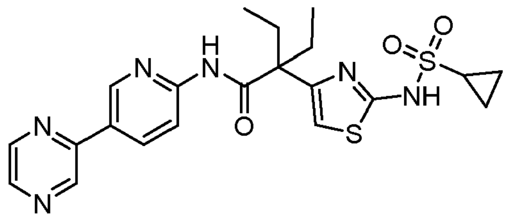

Prepared as **Method 1 b** from **INTB41** and 5-(pyrazin-2-yl)pyridin-2-amine (Cheng et al., 2016). Rt 1.17 min (UPLC acidic); m/z 473 $(M+H)^+$ $(ES^+)$; $^1H$ NMR (400 MHz, DMSO-d6) δ 12.49 (s, 1 H), 10.02 (s, 1 H), 9.32 (s, 1 H), 9.11 (s, 1 H), 8.74 (t, J = 2.0 Hz, 1 H), 8.64 (d, J = 2.5 Hz, 1 H), 8.60 - 8.52 (m, 1H), 8.23 (d, J = 8.7 Hz, 1H), 6.66 (s, 1H),

2.57 (s, 1 H), 2.17 - 1.97 (m, 4H), 0.94 - 0.87 (m, 4H), 0.74 (t, J = 7.3 Hz, 6H).

2-(2-(cyclopropanesulfonamido)thiazol-4-yl)-2-methyl-N-(4-(pyrimidin-2-yl)phenyl)propanamide **T4**

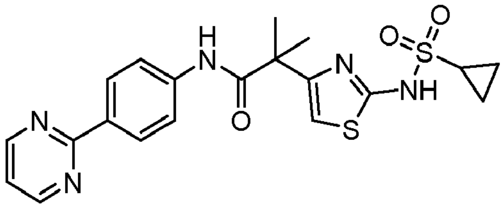

HATU (118 mg, 0.31 mmol) was added to a solution 2-(2-(cyclopropanesulfonamido)thiazol-4-yl)-2-methylpropanoic acid **INTB37** (75 mg, 0.26 mmol), 4-(pyrimidin-2-yl)aniline (44 mg, 0.26 mmol) and DIPEA (135 uL, 0.78 mmol) in DCM (1 mL) at RT. The reaction was stirred at RT for 18 hrs. The solvent was removed to give a yellow oil. The crude product was purified by chromatography on silica gel (24 g column, 0-100% EtOAc/iso-hexane) to afford 2-(2-(cyclopropanesulfonamido)thiazol-4-yl)-2-methyl-N-(4-(pyrimidin-2-yl)phenyl)propanamide (102 mg, 0.22 mmol, 85 % yield) as a white solid; Rt 1.72 min (HPLC acidic); m/z 444 $(M+H)^+$ $(ES^+)$; $^1H$ NMR (400 MHz, DMSO-d6) $\delta$ 12.60 (s, 1 H), 9.51 (s, 1 H), 8.92 (d, J = 4.8 Hz, 2H), 8.68 (t, J = 1.9 Hz, 1H), 8.13 (dt, J = 7.8, 1.4 Hz, 1H), 7.82 (ddd, J = 8.1, 2.3, 1.1 Hz, 1H), 7.52 - 7.41 (m, 2H), 6.56 (s, 1 H), 2.65 - 2.53 (m, 1 H), 1.59 (s, 6H), 1.00 - 0.85 (m, 4H).

2-(2-(cyclopropanesulfonamido)thiazol-4-yl)-N-(4-(pyridin-3-yl)phenyl)butanamide **T5**

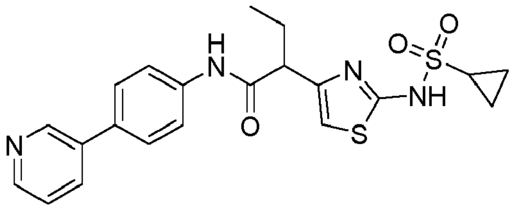

HATU (314 mg, 0.83 mmol) was added to a solution of 2-(2-(cyclopropanesulfonamido)thiazol-4-yl)butanoic acid **INTB38** (200 mg, 0.69 mmol), 4-(pyridin-3-yl)aniline (Xing-Li et al., 2009) (117 mg, 0.69 mmol) and DIPEA (3.6 mL, 2.07 mmol) in DCM (2 mL) at RT. The reaction was stirred at RT for 2 hrs. Water (10 mL) was added and the phases were separated. The solvent was removed to give a brown oil. The crude product was purified by chromatography on silica gel (24 g column, 0-10% MeOH/DCM) to afford 2-(2-(cyclopropanesulfonamido)thiazol-4-yl)-N-(4-(pyridin-3-yl)phenyl)butanamide (207 mg, 0.458 mmol, 66.5 % yield) as a pale orange solid; Rt 1.29 min (HPLC acidic); m/z 443 $(M+H)^+$ $(ES^+)$; $^1H$ NMR (400 MHz, DMSO-d6) $\delta$ 12.59 (s, 1 H), 10.29 (s, 1 H), 8.89 (dd, J = 2.4, 0.9 Hz, 1 H), 8.54 (dd, J = 4.7, 1.6 Hz, 1 H), 8.06 (ddd, J = 8.0, 2.5, 1.6 Hz, 1 H), 7.78 - 7.65 (m, 4H), 7.47 (ddd, J = 7.9, 4.7, 0.8 Hz, 1 H), 6.59 - 6.52 (m, 1 H), 3.60 (t, J = 7.4 Hz, 1 H), 2.65 - 2.55 (m, 1 H), 2.03 - 1.83 (m, 2H), 0.97 - 0.83 (m, 7H).

The racemate **T5** (180 mg) was separated by chiral preparative HPLC (Gilson, iso-hexane + 0.2% TFA: DCM, 4:1 with EtOH 30%). A salt exchange (TFA to HCl) was undertaken by adding 1.25M HCl (EtOH, 2 mL x 5) and removing solvent to afford:

**Peak A: Stereochemistry of product was not assigned**

2-(2-(cyclopropanesulfonamido)thiazol-4-yl)-N-(4-(pyridin-3-yl)phenyl)butanamide.HCl **T6** (98 mg, 0.20 mmol, 29 % yield); Rt 1.28 mins (HPLC acidic); m/z 443 $(M+H)^+$ $(ES^+)$; 441 $(M-H)^-$ $(ES^-)$; $^1H$ NMR (400 MHz, DMSO-d6) $\delta$ 12.63 (s, 1 H), 10.53 (s, 1 H), 9.13 (s, 1 H), 8.82 - 8.71 (m, 1 H), 8.62 (d, J = 8.3 Hz, 1 H), 7.91 (dd, J = 8.2, 5.3 Hz, 1 H), 7.86 - 7.77 (m, 4H), 6.57 (s, 1 H), 3.68 (t, J = 7.5 Hz, 1 H), 2.63 - 2.57 (m, 1 H), 1.94 (dtt, J = 27.6, 13.7, 7.3 Hz, 2H), 0.97 - 0.85 (m, 7H).

The product was analysed by Chiral IA method HPLC; Rt = 4.58 mins, 99% ee at 254 nm.

**Peak B: Stereochemistry of product was not assigned**

2-(2-(cyclopropanesulfonamido)thiazol-4-yl)-N-(4-(pyridin-3-yl)phenyl)butanamide.HCl **T7** (92 mg, 0.19 mmol, 28 % yield); Rt 1.28 min (HPLC acidic); m/z 443 $(M+H)^+$ $(ES^+)$; 441 $(M-H)^-$ (ES-); $^1$H NMR (400 MHz, DMSO-d6) δ 12.64 (s, 1H), 10.55 (s, 1H), 9.14 (d, J = 2.2 Hz, 1 H), 8.84 - 8.73 (m, 1 H), 8.64 (d, J = 8.3 Hz, 1 H), 7.93 (dd, J = 8.2, 5.4 Hz, 1 H), 7.88 - 7.76 (m, 4H), 6.57 (s, 1 H), 3.68 (t, J = 7.5 Hz, 1 H), 2.65 - 2.56 (m, 1 H), 1.94 (dtt, J = 27.7, 13.6, 7.3 Hz, 2H), 0.97 - 0.85 (m, 7H).

The product was analysed by Chiral IA method HPLC Rt = 11.04 mins, >99% ee at 254 nm.

N-(4-(5-chloropyridin-3-yl)phenyl)-2-(2-(cyclopropanesulfonamido)thiazol-4-yl)butanamide **T8**

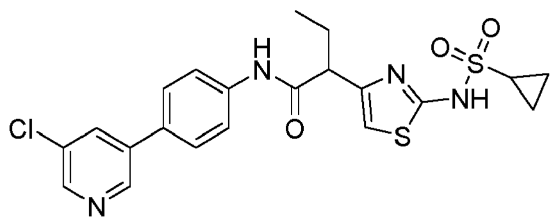

Prepared as **Method 1** from **INTB38.** Rt 1.11 mins (UPLC basic); m/z 477 $(M+H)^+$ $(ES^+)$; $^1$H NMR (400 MHz, DMSO-d6) δ 12.63 (s, 1 H), 10.34 (s, 1 H), 8.87 (d, J = 2.0 Hz, 1 H), 8.59 (d, J = 2.3 Hz, 1 H), 8.23 (t, J = 2.2 Hz, 1 H), 7.84 - 7.70 (m, 4H), 6.55 (s, 1 H), 3.60 (t, J = 7.4 Hz, 1 H), 2.60 (d, J = 5.4 Hz, 1 H), 1.92 (dtt, J = 20.6, 13.9, 7.2 Hz, 2H), 0.96 - 0.80 (m, 7H).

The racemate **T8** was separated by chiral preparative HPLC (30% EtOH vs 4:1 isoehexanes +0.2%TFA:DCM IA column). A salt exchange (TFA to HCl) was undertaken by adding 1.25M HCl (EtOH, 2 mL x 5) and removing solvent to afford:

**Peak A: Stereochemistry of product was not assigned**

N-(4-(5-chloropyridin-3-yl)phenyl)-2-(2-(cyclopropanesulfonamido)thiazol-4-yl)butanamide.HCl **T9;** Rt 1.1 mins (UPLC basic); m/z 477 $(M+H)^+$ $(ES^+)$; $^1$H NMR (400 MHz, DMSO-d6) δ 12.57 (s, 1 H), 10.43 (s, 1 H), 8.81 (d, J = 2.0 Hz, 1 H), 8.53 (d, J = 2.3 Hz, 1 H), 8.18 (t, J = 2.1 Hz, 1 H), 7.75 - 7.62 (m, 4H), 6.49 (s, 1 H), 3.64 - 3.56 (m, 1 H), 2.57 - 2.48 (m, 1 H), 1.94 - 1.74 (m, 2H), 0.93 - 0.75 (m, 7H).

**Peak B: Stereochemistry of product was not assigned**

N-(4-(5-chloropyridin-3-yl)phenyl)-2-(2-(cyclopropanesulfonamido)thiazol-4-yl)butanamide.HCl **T10;** Rt 1.1 mins (UPLC basic); m/z 477 $(M+H)^+$ $(ES^+)$; $^1$H NMR (400 MHz, DMSO-d6) δ 12.64 (s, 1H), 10.47 (s, 1 H), 8.88 (d, J = 1.9 Hz, 1 H), 8.60 (d, J = 2.3 Hz, 1H), 8.25 (t, J = 2.1 Hz, 1H), 7.82 - 7.75 (m, 4H), 6.56 (s, 1 H), 3.76 - 3.61 (m, 1 H), 2.70 - 2.58 (m, 1 H), 2.04 - 1.84 (m, 2H), 0.96 - 0.81 (m, 7H).

2-(2-(cyclopropanesulfonamido)thiazol-4-yl)-3-methyl-N-(4-(pyrimidin-5-yl)phenyl)butanamide **T11**

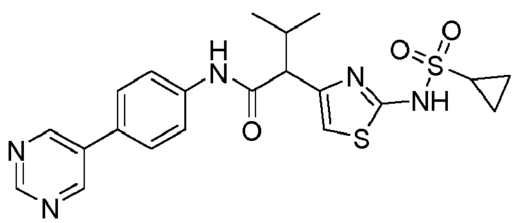

Prepared as **Method 1** from **INTB40.** Rt 1.71 min (HPLC acidic); m/z 458 $(M+H)^+$ $(ES^+)$; 456 $(M-H)^-$ (ES-); $^1$H NMR (400 MHz, DMSO-d6) δ 12.59 (s, 1H), 10.37 (s, 1H), 9.15 (s, 1H), 9.12 (s, 2H), 7.83 - 7.73 (m, 4H), 6.60 (s, 1 H), 3.37 (d, J = 9.9 Hz, 1 H), 2.64 - 2.54 (m, 1 H), 2.46 - 2.37 (m, 1 H), 0.98 (d, J = 6.6 Hz, 3H), 0.94 - 0.83 (m, 7H).

2-(2-(cyclopropanesulfonamido)thiazol-4-yl)-3-methyl-N-(4-(pyridin-3-yl)phenyl)butanamide **T12**

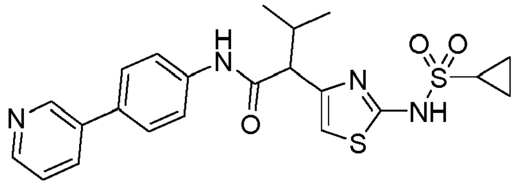

Prepared as **Method 1** from **INTB16** and 4-(pyridin-3-yl)aniline (Xing-Li et al, 2009) Rt 1.39 mins (HPLC acidic); m/z 457 $(M+H)^+$ $(ES^+)$; 455 $(M-H)^-$ $(ES^-)$; $^1H$ NMR (400 MHz, DMSO-d6) δ 12.56 (s, 1 H), 10.32 (s, 1 H), 8.88 (dd, J = 2.5, 0.9 Hz, 1 H), 8.54 (dd, J = 4.8, 1.6 Hz, 1 H), 8.05 (ddd, J = 8.0, 2.5, 1.6 Hz, 1 H), 7.83 - 7.67 (m, 4H), 7.47 (ddd, J = 8.0, 4.8, 0.9 Hz, 1 H), 6.60 (s, 1 H), 3.36 (d, J = 9.9 Hz, 1 H), 2.64 - 2.57 (m, 1 H), 2.47 - 2.33 (m, 1 H), 0.98 (d, J = 6.5 Hz, 3H), 0.93 - 0.83 (m,7H).

The racemate **T12** was separated by chiral preparative HPLC (Gilson, iso-hexane + 0.2% TFA: DCM, 4:1 with EtOH 30%). A salt exchange (TFA to HCl) was undertaken by adding 1.25M HCl (EtOH, 2 mL x 5) and removing solvent to afford:

**Peak A: Stereochemistry of product was not assigned**

2-(2-(cyclopropanesulfonamido)thiazol-4-yl)-3-methyl-N-(4-(pyridin-3-yl)phenyl)butanamide.HCl **T13** (97 mg, 0.20 mmol, 30 % yield); Rt 1.36 min (HPLC acidic); m/z 457 $(M+H)^+$ $(ES^+)$; 455 $(M-H)^-$ $(ES^-)$; $^1H$ NMR (400 MHz, DMSO-d6) δ 12.59 (s, 1H), 10.59 (s, 1H), 9.22 - 9.11 (m, 1H), 8.77 (d, J = 5.4 Hz, 1 H), 8.66 (d, J = 8.3 Hz, 1 H), 7.95 (dd, J = 8.3, 5.4 Hz, 1 H), 7.89 - 7.77 (m, 4H), 6.62 (s, 1H), 3.46 (d, J = 10.0 Hz, 1 H), 2.64 - 2.56 (m, 1 H), 2.46 - 2.39 (m, 1H), 0.98 (d, J = 6.5 Hz, 3H), 0.94 - 0.80 (m, 7H).

The product was analysed by Chiral IA method HPLC; Rt = 4.66 min, >99% ee at 254 nm.

**Peak B: Stereochemistry of product was not assigned**

2-(2-(cyclopropanesulfonamido)thiazol-4-yl)-3-methyl-N-(4-(pyridin-3-yl)phenyl)butanamide.HCl **T14** (95 mg, 0.19 mmol, 29 % yield); Rt 1.36 mins (HPLC acidic); m/z 457 $(M+H)^+$ $(ES^+)$; 455 $(M-H)^-$ $(ES^-)$; $^1H$ NMR (400 MHz, DMSO-d6) δ 12.60 (s, 1 H), 10.62 (s, 1 H), 9.24 - 9.12 (m, 1H),, 8.78 (d, J = 5.4 Hz, 1 H), 8.68 (d, J = 8.2, 1.6 Hz, 1 H), 7.97 (dd, J = 8.2, 5.4 Hz, 1 H), 7.92 - 7.77 (m, 4H), 6.63 (s, 1 H), 3.47 (d, J = 10.0 Hz, 1 H), 2.64 - 2.57 (m, 1 H), 2.47 - 2.39 (m, 1H), 0.98 (d, J = 6.5 Hz, 3H), 0.94 - 0.83 (m, 7H).

The product was analysed by Chiral IA method HPLC; Rt = 10.84 min, >99% ee at 254 nm.

2-(2-(cyclopropanesulfonamido)thiazol-4-yl)-N-(4-(5-methoxypyridin-3-yl)phenyl)-2-methylpropanamide **T15**

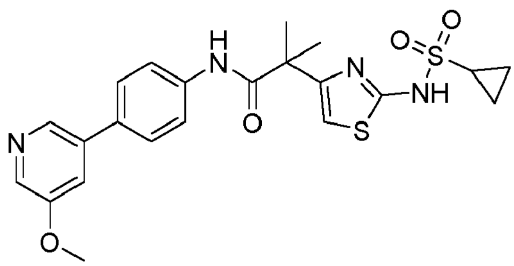

To a solution of 2-(2-(cyclopropanesulfonamido)thiazol-4-yl)-2-methylpropanoic acid **INTB37** (50 mg, 0.17 mmol) in DCM (2 mL) was added 1-chloro-N,N,2-trimethylprop-1-en-1-amine (0.04 mL, 0.34 mmol). The reaction mixture was stirred at RT for 2 hrs. The reaction mixture was concentrated in *vacuo* and the residue redissolved in pyridine (2 mL) before addition of 4-(5-methoxypyridin-3-yl)aniline (38 mg, 0.19 mmol). The reaction mixture was poured into $NH_4Cl$ (sat. aq, 10 mL) and the products were extracted using EtOAc (3 x 10 mL). The combined extracts were dried ($MgSO_4$), filtered and concentrated in *vacuo.* The crude product was purified by chromatography on silica gel (12 g column, 0-100%

EtOAc/iso-hexane) to afford 2-(2-(cyclopropanesulfonamido)thiazol-4-yl)-N-(4-(5-methoxypyridin-3-yl)phenyl)-2-methylpropanamide (6 mg, 0.01 mmol, 7 % yield) as a colourless solid; Rt 0.90 min (UPLC acidic); m/z 473 $(M+H)^+$ $(ES^+)$; $^1H$ NMR (400 MHz, DMSO-d6) δ 12.56 (s, 1 H), 9.43 (s, 1 H), 8.49 (d, J = 1.8 Hz, 1H), 8.25 (d, J = 2.7 Hz, 1H), 7.75 (s, 4H), 7.61 (t, J = 2.3 Hz, 1H), 6.55 (s, 1H), 3.91 (s, 3H), 2.63 - 2.58 (m, 1H), 1.58 (s, 6H), 0.93 - 0.88 (m, 4H).

N-(2-chloro-4-(pyridin-3-yl)phenyl)-2-(2-(cyclopropanesulfonamido)thiazol-4-yl)acetamide **T16**

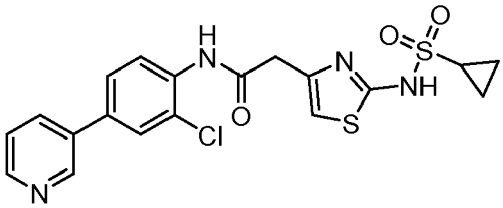

2-(2-(cyclopropanesulfonamido)thiazol-4-yl)acetic acid **INTB36** (75 mg, 0.29 mmol), 2-chloro-4-(pyridin-3-yl)aniline (58.5 mg, 0.29 mmol) and T3P (50% in EtOAc, 0.4 mL, 0.72 mmol) were dissolved in a mixture of EtOAc (2 mL) and DMF (1 mL). Triethylamine (1.2 mL, 0.86 mmol) was added and the mixture was stirred for 1 hr at RT. The mixture was partitioned between EtOAc (10 mL) and $H_2O$ (10 mL), the two phases were separated, the aqueous phase was re-extracted with EtOAc (2 x 10 mL), the combined organic extracts were passed through a phase separator and the solvent was removed in *vacuo.* The crude product was purified by chromatography on silica gel (24 g column, 0-10% MeOH/DCM) and the sample was dried under vacuum at 50 °C to give N-(2-chloro-4-(pyridin-3-yl)phenyl)-2-(2-(cyclopropanesulfonamido)thiazol-4-yl)acetamide (10 mg, 0.02 mmol, 7 % yield) as a pale brown solid; Rt 1.47 min (HPLC basic); m/z 449 $(M+H)^+$ $(ES^+)$; $^1H$ NMR (400 MHz, DMSO-d6) δ 12.53 (s, 1H), 9.91 (s, 1H), 8.98 - 8.88 (m, 1H), 8.64 - 8.52 (m, 1H), 8.12 (ddd, J = 8.0, 2.5, 1.6 Hz, 1H), 7.96 (d, J = 8.6 Hz, 1H), 7.91 (d, J = 2.1 Hz, 1H), 7.73 (dd, J = 8.5, 2.2 Hz, 1H), 7.52 - 7.46 (m, 1H), 6.56 (s, 1H), 3.74 (s, 2H), 2.61 - 2.51 (m, 1H), 0.93 - 0.82 (m, 4H).

2-(2-(cyclopropanesulfonamido)-5-methylthiazol-4-yl)-2-methyl-N-(4-(6-(trifluoromethyl)pyrazin-2-yl)phenyl)propanamide **T17**

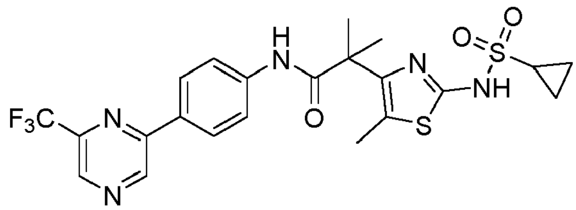

To a solution of 2-(2-(cyclopropanesulfonamido)-5-methylthiazol-4-yl)-2-methylpropanoic acid **INTB42** (100 mg, 0.33 mmol) in toluene (20 mL) at 70 °C was added thionyl chloride (0.8 mL, 10.91 mmol). The reaction mixture was stirred at 70 °C for 1 hr. The reaction mixture was cooled to RT and concentrated in *vacuo.* The resulting oil was redissolved in EtOAc (10 mL) and a solution of 4-(6-(trifluoromethyl)pyrazin-2-yl)aniline (86 mg, 0.36 mmol) in EtOAc (20 mL) was added followed by triethylamine (0.8 mL, 5.74 mmol). The reaction mixture was stirred at 40 °C for 16 hrs. The reaction mixture was diluted with $NH_4Cl$ (sat. aq, 30 mL) and EtOAc (30 mL). The phases were separated and the aqueous was extracted with further EtOAc (2 x 40 mL). The combined organic phases were dried ($MgSO_4$ and concentrated onto silica. The crude product was purified by chromatography on silica gel (40 g cartridge, 0-100% EtOAc/iso-hexane) to afford 2-(2-(cyclopropanesulfonamido)-5-methylthiazol-4-yl)-2-methyl-N-(4-(6-(trifluoromethyl)pyrazin-2-yl)phenyl)propanamide as a pale yellow solid; Rt 2.30 mins (HPLC acidic); m/z 526 $(M+H)^+$ $(ES^+)$; $^1H$ NMR (500 MHz, DMSO-d6) δ 12.05 (s, 1H), 9.99 (s, 1H), 9.58 (s, 1H), 9.08 (s, 1H), 8.23 - 8.13 (m, 2H), 7.92 - 7.81 (m, 2H), 2.62 - 1.54 (m, 1H), 1.95 (s, 3H), 1.53 (s, 6H), 0.97 - 0.86 (m, 4H).

2-(2-(cyclopropanesulfonamido)thiazol-4-yl)-2-methyl-N-(4-(pyrimidin-5-yl)phenyl)propanamide **T18**

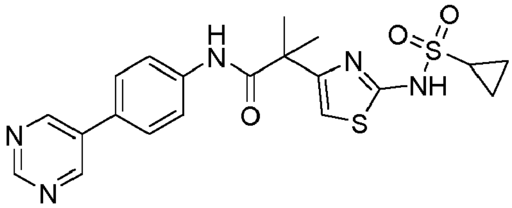

To a suspension of N-(4-bromophenyl)-2-(2-(cyclopropanesulfonamido)thiazol-4-yl)-2-methylpropanamide **INTB37** (58 mg, 0.13 mmol) in dioxane (3 mL) was added pyrimidin-5-ylboronic acid (16 mg, 0.13 mmol) and 2M $K_2PO_4$ (aq, 131 uL, 0.26 mmol). The resulting suspension was heated to 60 °C and degassed ($N_2$, 5 mins). Pd 170 (4.40 mg, 6.53 umol) was added and the reaction mixtures were stirred at 60 °C for 16 hrs. The reaction mixture was cooled to RT, diluted with $NH_4Cl$ (aq, 20 mL) and EtOAc (20 mL). The phases were separated, and the aqueous phase extracted with further EtOAc (3 x 20 mL). The combined organics were washed with brine, dried ($Na_2SO_4$) and concentrated to dryness. The crude product was purified by chromatography on silica gel (4 g column, 0-80% EtOAc/isohexane) to afford 2-(2-(cyclopropanesulfonamido)thiazol-4-yl)-2-methyl-N-(4-(pyrimidin-5-yl)phenyl)propanamide (15 mg, 0.03 mmol, 25 % yield) as a pale yellow solid; Rt 0.98 min (UPLC acidic); m/z 444 $(M+H)^+$ $(ES^+)$; $^1H$ NMR (400 MHz, DMSO-d6) $\delta$ 12.58 (s, 1H), 9.47 (s, 1H), 9.18 - 9.12 (m, 3H), 7.81 (s, 4H), 6.55 (s, 1H), 2.62 - 2.57 (m, 1H), 1.58 (s, 6H), 0.94 - 0.87 (m, 4H).

6-(4-(2-(2-(cyclopropanesulfonamido)thiazol-4-yl)-2-methylpropanamido)phenyl)-N,N-dimethylpyrazine-2-carboxamide **T19**

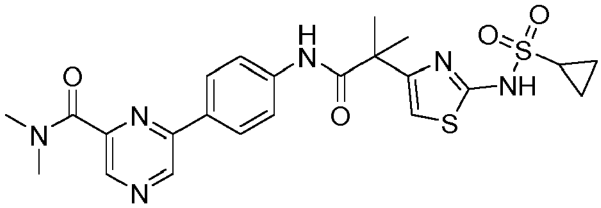

$Pd(PPh_3)_4$ (8.8 mg, 0.008 mmol) was added to a degassed ($N_2$, 5 mins) solution of 2-(2-(cyclopropanesulfonamido)thiazol-4-yl)-2-methyl-N-(4-(4,4,5,5-tetramethyl-1,3,2-dioxaborolan-2-yl)phenyl)propanamide **INTB56** (75 mg, 0.15 mmol), 6-chloro-N,N-dimethylpyrazine-2-carboxamide (34.0 mg, 0.18 mmol) and $NaHCO_3$ (38.5 mg, 0.46 mmol) in MeCN (4 mL) and water (1 mL). The solution was then degassed ($N_2$, 5 mins) and then heated to 90 °C for 1 hr. The reaction mixture was allowed to cool to RT for 18 hrs. The solution was concentrated onto silica. The crude product was purified by chromatography on silica gel (24 g column, 0-10% MeOH/DCM) to afford 6-(4-(2-(2-(cyclopropanesulfonamido)thiazol-4-yl)-2-methylpropanamido)phenyl)-N,N-dimethylpyrazine-2-carboxamide (35 mg, 0.07 mmol, 44 % yield) as a colourless solid; Rt 1.57 min (HPLC acidic); m/z 515 $(M+H)^+$ $(ES^+)$; 513 $(M-H)^-$ (ES-); $^1H$ NMR (400 MHz, DMSO-d6) $\delta$ 12.61 (s, 1H), 9.55 (s, 1H), 9.31 (s, 1H), 8.72 (s, 1H), 8.24 - 8.06 (m, 2H), 7.91 - 7.75 (m, 2H), 6.56 (s, 1H), 3.07 (s, 3H), 3.05 (s, 3H), 2.64 - 2.54 (m, 1H), 1.59 (s, 6H), 0.98 - 0.84 (m, 4H).

N-(5-(5-cyanopyridin-3-yl)pyrimidin-2-yl)-2-(2-(cyclopropanesulfonamido)thiazol-4-yl)-2-methylpropanamide **T20**

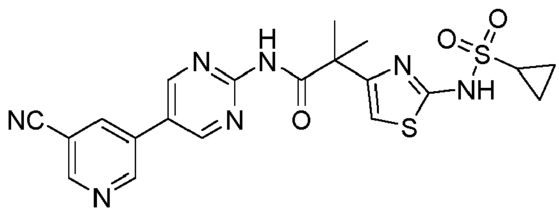

To a solution of 2-(2-(cyclopropanesulfonamido)thiazol-4-yl)-2-methylpropanamide **INTB58** (82 mg, 0.28 mmol) and 5-(2-chloropyrimidin-5-yl)nicotinonitrile (56 mg, 0.26 mmol) in dioxane (3 mL) was added $C_{S2}CO_3$ (118 mg, 0.36 mmol). The reaction mixture was heated to 60 °C and degassed ($N_2$, 5 mins). Pd 177 (20 mg, 0.03 mmol) was added

to the reaction mixture and the temperature was increased to 90 °C. After 2 hrs, the reaction was stirred for 16 hrs at 60 °C. The reaction mixture was cooled to RT and diluted with $NH_4Cl$ (sat. aq, 10 mL) and EtOAc (10 mL). The phases were separated and the aqueous was extracted with EtOAc (2 x 10 mL) and DCM (1 x 10 mL). The combined organics were dried ($MgSO_4$ and concentrated onto silica. The crude product was purified by chromatography on silica gel (12 g column, 0-100% EtOAc/iso-hexane, then 5% MeOH/EtOAc) and then by chromatography on C18-RP silica gel (12 g column, 5-40% MeCN/Water 0.1% Formic Acid) to afford N-(5-(5-cyanopyridin-3-yl)pyrimidin-2-yl)-2-(2-(cyclopropanesulfonamido)thiazol-4-yl)-2-methylpropanamide (5 mg, 0.01 mmol, 4 % yield) as a yellow solid; Rt 0.90 min (UPLC acidic); m/z 470 $(M+H)^+$ $(ES^+)$; $^1H$ NMR (400 MHz, DMSO-d6) δ 12.64 (s, 1H), 10.39 (s, 1H), 9.32 (s, 1H), 9.18 (s, 2H), 9.08 (s, 1H), 8.83 (s, 1H), 6.55 (s, 1H), 2.70 - 2.38 (m, 1H), 1.59 (s, 6H), 0.98 - 0.78 (m, 4H).

N-([1,1'-biphenyl]-4-yl)-2-(5-chloro-2-(cyclopropanesulfonamido)thiazol-4-yl)acetamide **T21**

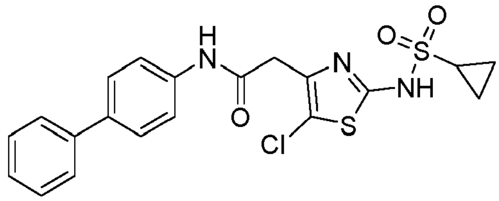

A solution of N-([1,1'-biphenyl]-4-yl)-2-(2-(cyclopropanesulfonamido)thiazol-4-yl)acetamide **T2** (27 mg, 0.07 mmol) in MeCN (0.5 mL) was treated with NCS (9.2 mg, 0.07 mmol) and stirred for 2 hrs. The reaction mixture was diluted with water (3 mL) and DCM (5 mL) and partitioned with a phase separator. The organic phase was concentrated and the crude product was purified by preparative HPLC (Gilson, Acidic (0.1% Formic acid), Acidic, Waters X-Select Prep-C18, 5 um, 19x50 mm column, 20-60% MeCN in Water) to afford N-([1,1'-biphenyl]-4-yl)-2-(5-chloro-2-(cyclopropanesulfonamido)thiazol-4-yl)acetamide (3 mg, 6.03 umol, 9 % yield) as a yellow solid; Rt 2.26 min (HPLC acidic); m/z 448 ($^{35}Cl$ $M+H)^+$ $(ES^+)$; $^1H$ NMR (400 MHz, DMSO-d6) δ 12.82 (s, 1 H), 10.35 (s, 1 H), 7.74 - 7.61 (m, 6H), 7.44 (dd, J = 8.4, 7.0 Hz, 2H), 7.37 - 7.28 (m, 1 H), 3.69 (s, 2H), 2.67 - 2.58 (m, 1 H), 0.96 - 0.81 (m, 4H).

2-(2-(cyclopropanesulfonamido)thiazol-4-yl)-N-(4-(6-ethynylpyrazin-2-yl)phenyl)butanamide **T22**

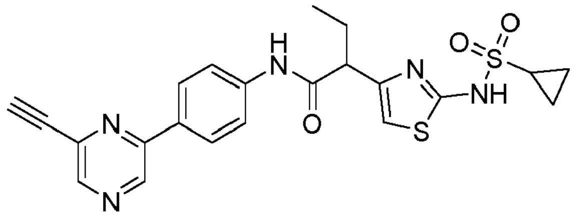

A solution of N-(4-(6-chloropyrazin-2-yl)phenyl)-2-(2-(cyclopropanesulfonamido)thiazol-4-yl)butanamide **T162** (94 mg, 0.2 mmol), ethynyltrimethylsilane (0.04 mL, 0.3 mmol) and DIPEA (0.05 mL, 0.3 mmol) in DMF (1 mL) was degassed ($N_2$, 5 mins). To this Cu(I)I (7.5 mg, 0.04 mmol) and $Pd(PPh_3)2Cl_2$ (13.8 mg, 0.02 mmol) were added and the mixture degassed ($N_2$, 5 mins) with sonication. The mixture was stirred at RT for 16 hrs. The solvent was removed in *vacuo* and the mixture partitioned between EtOAc (20 mL) and water (20 mL). The phases were separated and the organic phase concentrated onto silica. The crude product was purified by chromatography on silica gel (24 g cartridge, 0-100% EtOAc/iso-hexane) to afford 2-(2-(cyclopropanesulfonamido)thiazol-4-yl)-N-(4-(6-((trimethylsilyl)ethynyl)pyrazin-2-yl)phenyl)butanamide. The material was taken up in MeOH (2 mL) and $Na_2CO_3$ (aq, 208 mg, 1.97 mmol) was added. The mixture was stirred at RT for 1 hr. The solvent was evaporated and water (10 mL) was added. The pH was neutralised to pH 7 and extracted with EtOAc (2 x 20 mL). The organic phases were combined, dried ($MgSO_4$), filtered and solvent removed. The crude product was purified by chromatography on silica gel (24 g cartridge, 0-100% EtOAc/iso-hexane) and then on C18-RP silica gel (12 g cartridge, 0-80% MeCN/Water 0.1% Formic Acid) to afford 2-(2-(cyclopropanesulfonamido)thiazol-4-yl)-N-(4-(6-ethynylpyrazin-2-yl)phenyl)butanamide (21 mg, 0.044 mmol, 23 % yield) as a white solid; 1.89 mins (HPLC acidic); m/z 468 $(M+H)^+$ $(ES^+)$; $^1H$ NMR (500 MHz, DMSO-$d_6$) δ 12.62 (s, 1H), 10.42 (s, 1H), 9.23 (s, 1H), 8.70 (s, 1H), 8.19 - 8.07 (m, 2H), 7.85 - 7.74 (m, 2H), 6.54 (s, 1H), 4.69 (s, 1H), 3.61 (t, J =

7.4 Hz, 1H), 2.63 - 2.56 (m, 1H), 2.03 - 1.83 (m, 2H), 1.00 - 0.78 (m, 7H).

**Table 6:** Preparation methods and characterisation of Examples **T23-T322.**

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T23** | 2-(2-(cyclopropanesulfonamido)thiazol-4-yl)-N-(6-(pyrimidin-5-yl)pyridin-3-yl)acetamide 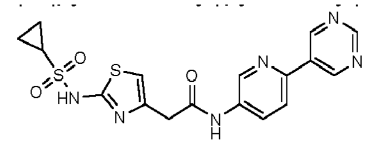 | Method 1 using INTB36, [HPLC acidic], 417 , (1.24) | 12.53 (s, 1H), 10.61 (s, 1H), 9.41 (s, 2H), 9.21 (s, 1H), 8.89 (d, J = 2.5 Hz, 1H), 8.21 (dd, J = 8.7, 2.5 Hz, 1H), 8.14 (d, J = 8.5 Hz, 1H), 6.59 (s, 1H), 3.72 (s, 2H), 2.61 - 2.53 (m, 1H), 0.94 - 0.81 (m, 4H). |
| **T24** | 2-(2-(cyclopropanesulfonamido)thiazol-4-yl)-N-(5-phenylpyridin-2-yl)acetamide 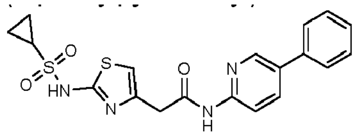 | Method 1 using INTB36, [UPLC acidic], 415, (1.18) | 12.63 - 12.38 (m, 1H), 10.85 (s, 1H), 8.68 (t, J = 1.7 Hz, 1H), 8.14 (br. s, J = 1.9 Hz, 2H), 7.76 - 7.70 (m, 2H), 7.49 (dd, J = 8.4, 6.9 Hz, 2H), 7.42 - 7.36 (m, 1H), 6.58 (s, 1H), 3.75 (s, 2H), 2.63 - 2.55 (m, 1H), 0.95 - 0.85 (m, 4H). |
| **T25** | 2-(2-(cyclopropanesulfonamido)thiazol-4-yl)-N-(4'-fluoro-[1,1'-biphenyl]-4-yl)acetamide 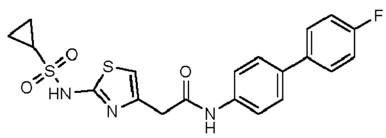 | Method 1 using INTB36, [HPLC acidic], 432, (2.04) | 12.56 (s, 1H), 10.29 (s, 1H), 7.77-7.58 (m, 6H), 7.36 - 7.19 (m, 2H), 6.57 (s, 1H), 3.67 (s, 2H), 2.63 - 2.55 (m, 1H), 0.94 - 0.81 (m, 4H). |
| **T26** | 2-(2-(cyclopropanesulfonamido)thiazol-4-yl)-N-methyl-N-(4-(pyridin-3-yl)phenyl)acetamide 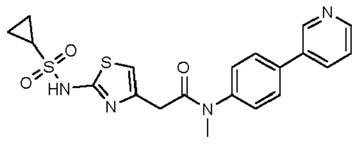 | Method 1 using INTB36 and INTA68, [UPLC acidic], 429, (0.69) | 12.33 (s, 1H), 8.93 (d, J = 2.3 Hz, 1H), 8.59 (dd, J = 4.8, 1.6 Hz, 1H), 8.11 (dt, J = 8.1, 1.8 Hz, 1H), 7.84 (d, J = 8.0 Hz, 2H), 7.54 - 7.46 (m, 3H), 6.42 (s, 1H), 3.38 (s, 2H), 3.23 (s, 3H), 2.60 - 2.50 (m, 1H), 0.90 - 0.80 (m, 4H). |
| **T27** | 2-([2,3'-bipyridin]-5-yl)-2-(2-(cyclopropanesulfonamido)thiazol-4-yl) propanamide 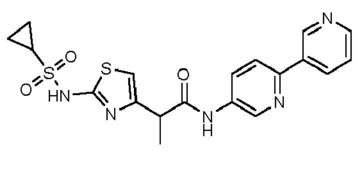 | Method 1 using INTB39, [HPLC acidic], 430, (1.07) | (Methanol-d4) 9.16 (dd, J = 2.3, 0.9 Hz, 1H), 8.88 (dd, J = 2.6, 0.7 Hz, 1H), 8.58 (dd, J = 4.9, 1.6 Hz, 1H), 8.42 (ddd, J = 8.0, 2.3, 1.6 Hz, 1H), 8.28 (dd, J = 8.7, 2.6 Hz, 1H), 7.95 (dd, J = 8.6, 0.8 Hz, 1H), 7.56 (ddd, J = 8.0, 4.9, 0.9 Hz, 1H), 6.55 (d, J = 0.9 Hz, 1H), 3.92 - 3.77 (m, 1H), 2.67 - 2.56 (m, 1H), 1.61 (d, J = 7.1 Hz, 3H), 1.20-1.05 (m, 2H), 1.07 - 0.87 (m, 2H), 2 exchangeable protons observed. |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T28** | 2-(2-(cyclopropanesulfonamido)thiazol-4-yl)-N-(3'-methoxy-[1,1'-biphenyl]-4-yl)-2-methylpropanamide 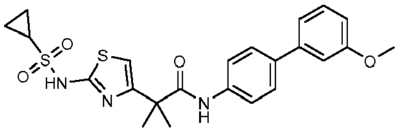 | Method 1 using INTB37, [HPLC basic], 472, (1.94) | 12.57 (s, 1H), 9.38 (s, 1H), 7.74 - 7.67 (m, 2H), 7.67 - 7.61 (m, 2H), 7.35 (t, J = 7.9 Hz, 1H), 7.22 (ddd, J = 7.7, 1.8, 1.0 Hz, 1H), 7.17 (dd, J = 2.6, 1.7 Hz, 1H), 6.90 (ddd, J = 8.2, 2.6, 0.9 Hz, 1H), 6.54 (s, 1H), 3.82 (s, 3H), 2.64 - 2.54 (m, 1H), 1.57 (s, 6H), 0.96 - 0.84 (m, 4H). |
| **T29** | 2-(2-(cyclopropanesulfonamido)thiazol-4-yl)-2-methyl-N-(4-(pyridin-3-yl)phenyl)propanamide 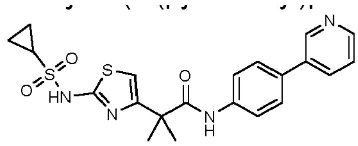 | Method 1 using INTB37, [UPLC acidic], 443, (0.73) | 12.58 (s, 1H), 9.47 (s, 1H), 8.95 (d, J = 2.7 Hz, 1H), 8.62 - 8.56 (m, 1H), 8.21 (s, 1H), 7.82 - 7.71 (m, 4H), 7.57 (s, 1H), 6.57 (s, 1H), 2.60 (dd, J = 7.4, 4.7 Hz, 1H), 1.58 (s, 6H), 0.94 - 0.85 (m, 4H). |
| **T30** | 2-(2-(cyclopropanesulfonamido)thiazol-4-yl)-2-methyl-N-(4-(5-methylpyridin-3-yl)phenyl)propanamide 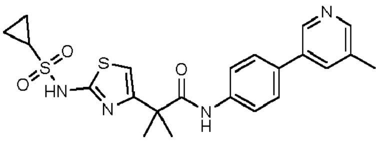 | Method 1 using INTB37 and INTA21, [HPLC acidic], 457, (1.25) | 12.55 (s, 1H), 9.42 (s, 1H), 8.71 (d, J = 2.2 Hz, 1H), 8.40 (d, J = 2.0 Hz, 1H), 7.93 (s, 1H), 7.80 - 7.67 (m, 4H), 6.57 (s, 1H), 2.66 - 2.52 (m, 1H), 2.38 (s, 3H), 1.58 (s, 6H), 0.98 - 0.85 (m, 4H). |
| T31 | 2-(2-(cyclopropanesulfonamido)thiazol-4-yl)-2-methyl-N-(4-(pyridazin-4-yl)phenyl)propanamide 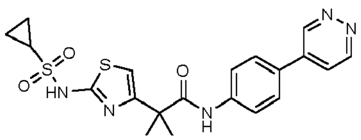 | Method 1 using INTB37, [HPLC acidic], 444, (1.50) | 12.56 (s, 1H), 9.71 - 9.49 (m, 2H), 9.23 (dd, J = 5.5, 1.2 Hz, 1H), 8.00 (dd, J = 5.5, 2.6 Hz, 1H), 7.97 - 7.92 (m, 2H), 7.87 - 7.81 (m, 2H), 6.57 (s, 1H), 2.66 - 2.56 (m, 1H), 1.59 (s, 6H), 0.96 - 0.87 (m, 4H). |
| **T32** | 2-(2-(cyclopropanesulfonamido)thiazol-4-yl)-2-methyl-N-(4-(pyrazin-2-yl)phenyl)propanamide 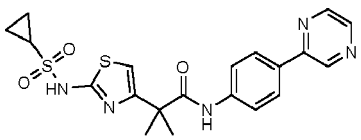 | Method 1 using INTB37, [HPLC acidic], 444, (1.66) | 12.59 (s, 1H), 9.50 (s, 1H), 9.24 (d, J = 1.5 Hz, 1H), 8.68 (dd, J = 2.5, 1.5 Hz, 1H), 8.57 (d, J = 2.5 Hz, 1H), 8.18 - 8.09 (m, 2H), 7.85-7.77 (m, 2H), 6.56 (s, 1H), 2.64 - 2.54 (m, 1H), 1.59 (s, 6H), 0.98 - 0.83 (m, 4H). |
| **T33** | 2-(2-(cyclopropanesulfonamido)thiazol-4-yl)-N-(4-(6-methoxypyrazin-2-yl)phenyl)butanamide 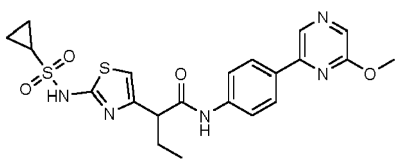 | Method 1 using INTB38 and INTA2, [HPLC acidic], 474, (1956) | 12.62 (s, 1H), 10.37 (s, 1H), 8.79 (s, 1H), 8.22 (s, 1H), 8.19 - 8.09 (m, 2H), 7.83 - 7.71 (m, 2H), 6.55 (s, 1H), 4.02 (s, 3H), 3.62 (t, J = 7.6 Hz, 1H), 2.63 - 2.55 (m, 1H), 2.02 - 1.84 (m, 2H), 0.96 - 0.87 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T34** | N-(3-cyano-4-(pyrazin-2-yl)phenyl)-2-(2-(cyclopropanesulfonamido)thiazol-4-yl)-2-methylpropanamide 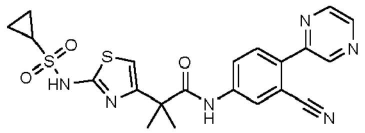 | Method 1b using INTB37 and INTA5, [UPLC basic],469, (0.95) | 12.62 (s, 1H), 9.80 (s, 1H), 9.13 (d, J = 1.5 Hz, 1H), 8.81 (dd, J = 2.5, 1.5 Hz, 1H), 8.74 (d, J = 2.5 Hz, 1H), 8.29 (d, J = 2.2 Hz, 1H), 8.08 (dd, J = 8.7, 2.2 Hz, 1H), 8.02 (d, J = 8.6 Hz, 1H), 6.61 (s, 1H), 2.65 - 2.55 (m, 1H), 1.59 (s, 6H), 0.97 - 0.84 (m, 4H). |
| T35 | 2-(2-(cyclopropanesulfonamido)thiazol-4-yl)-2-methyl-N-(5-(6-(trifluoromethyl)pyrazin-2-yl)pyridin-2-yl) propanamide 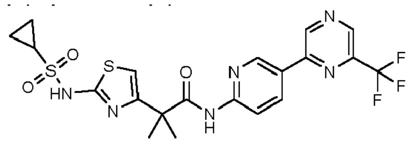 | Method 1b using INTB37 and INTA3, [UPLC acidic], 513, (1.35) | 12.58 (s, 1H), 10.26 (s, 1H), 9.68 (s, 1H), 9.16 (br. s, 2H), 8.60 (d, J = 8.6 Hz, 1H), 8.26 (dd, J = 8.8, 0.8 Hz, 1H), 6.58 (s, 1H), 2.65 - 2.54 (m, 1H), 1.61 (s, 6H), 0.91 (s, 4H). |
| **T36** | 2-(2-(cyclopropanesulfonamido)thiazol-4-yl)-N-(2,3-difluoro-4-(pyridin-3-yl)phenyl)-2-methylpropanamide 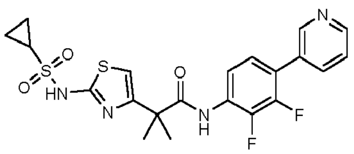 | Method 1b using INTB37 and INTA6, [HPLC basic], 479, (1.63) | 12.59 (s, 1H), 9.48 (s, 1H), 8.87 - 8.72 (m, 1H), 8.64 (dd, J = 4.8, 1.6 Hz, 1H), 8.08 - 7.96 (m, 1H), 7.55 (ddd, J = 8.0, 4.8, 0.9 Hz, 1H), 7.44 (d, J = 4.9 Hz, 2H), 6.62 (s, 1H), 2.63 - 2.53 (m, 1H), 1.59 (s, 6H), 0.99 - 0.86 (m, 4H). |
| **T37** | 2-(2-(cyclopropanesulfonamido)thiazol-4-yl)-2-methyl-N-(5-(pyridin-3-yl)pyrimidin-2-yl)propanamide 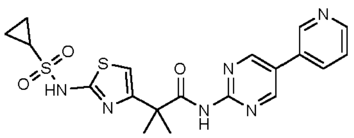 | Method 1b using INTB37 and INTA1, [HPLC acidic], 445, (1.12) | 12.60 (s, 1H), 10.26 (s, 1H), 9.11 (s, 2H), 9.05 - 8.99 (m, 1H), 8.65 (dd, J = 4.8, 1.6 Hz, 1H), 8.23 (ddd, J = 8.0, 2.4, 1.6 Hz, 1H), 7.60 - 7.51 (m, 1H), 6.58 (s, 1H), 2.64 - 2.55 (m, 1H), 1.60 (s, 6H), 0.98 - 0.83 (m, 4H). |
| **T38** | 2-(2-(cyclopropanesulfonamido)thiazol-4-yl)-2-methyl-N-(5-(6-propoxypyrazin-2-yl)pyridin-2-yl)propanamide 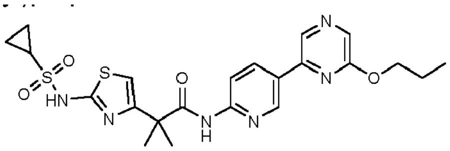 | Method 1b using INTB37 and INTA4, [HPLC basic], 503, (1.90) | 12.58 (s, 1H), 10.11 (s, 1H), 9.09 (d, J = 2.4 Hz, 1H), 8.87 (s, 1H), 8.53 (dd, J = 8.8, 2.5 Hz, 1H), 8.27 (s, 1H), 8.19 (dd, J = 8.8, 0.8 Hz, 1H), 6.59 (s, 1H), 4.40 (t, J = 6.6 Hz, 2H), 2.65 - 2.56 (m, 1H), 1.88 - 1.76 (m, 2H), 1.62 (s, 6H), 1.02 (t, J = 7.4 Hz, 3H), 0.95 - 0.87 (m, 4H). |
| **T39** | 2-(2-(cyclopropanesulfonamido)thiazol-4-yl)-2-methyl-N-(4-(pyridin-3-yl)-2-(trifluoromethoxy)phenyl) propanamide 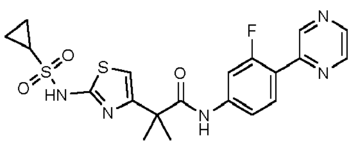 | Method 1b using INTB37 and INTA7, [UPLC basic], 463, (0.96) | 12.61 (s, 1H), 9.72 (s, 1H), 9.03 (dd, J = 2.5, 1.6 Hz, 1H), 8.78 (dd, J = 2.5, 1.6 Hz, 1H), 8.63 (d, J = 2.5 Hz, 1H), 7.97 (t, J = 8.8 Hz, 1H), 7.82 (dd, J = 14.2, 2.0 Hz, 1H), 7.62 (dd, J = 8.6, 2.1 Hz, 1H), 6.58 (s, 1H), 2.66 - 2.54 (m, 1H), 1.58 (s, 6H), 0.95 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T40** | 2-(2-(cyclopropanesulfonamido)thiazol-4-yl)-2-methyl-N-(4-(pyridin-3-yl)-2-(trifluoromethoxy)phenyl) propanamide 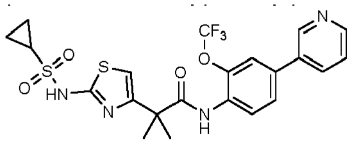 | Method 1b using INTB37, [UPLC basic], 527, (1.07) | 12.57 (s, 1H), 9.18 (s, 1H), 8.95 (d, J = 2.4 Hz, 1H), 8.61 (dd, J = 4.8, 1.7 Hz, 1H), 8.18 - 8.10 (m, 1H), 7.85 - 7.77 (m, 3H), 7.55 - 7.47 (m, 1H), 6.66 (s, 1H), 2.64 - 2.54 (m, 1H), 1.57 (s, 6H), 0.96 - 0.86 (m, 4H). |
| **T41** | N-(2-chloro-4-(pyridin-3-yl)phenyl)-2-(2-(cyclopropanesulfonamido)thiazol-4-yl)-2-methylpropanamide 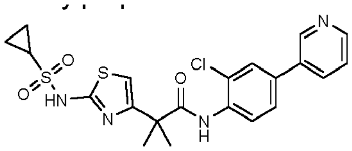 | Method 1b using INTB37 and INTA8, [HPLC basic], 477, (1.67) | 12.59 (s, 1H), 9.01 (s, 1H), 8.94 (dd, J = 2.4, 0.8 Hz, 1H), 8.59 (dd, J = 4.8, 1.6 Hz, 1H), 8.13 (ddd, J = 8.0, 2.5, 1.6 Hz, 1H), 7.95 - 7.89 (m, 1H), 7.80 - 7.73 (m, 2H), 7.49 (ddd, J = 7.9, 4.8, 0.9 Hz, 1H), 6.70 (s, 1H), 2.63 - 2.55 (m, 1H), 1.59 (s, 6H), 0.99-0.84 (m, 4H). |
| **T42** | 2-(2-(cyclopropanesulfonamido)thiazol-4-yl)-N-(2-fluoro-4-(pyridin-3-yl)phenyl)-2-methylpropanamide 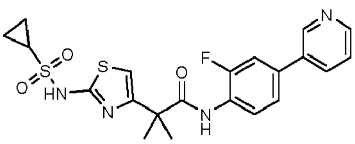 | Method 1b using INTB37, [HPLC basic], 461, (1.60) | 12.58 (s, 1H), 9.24 (s, 1H), 8.95 (d, J = 2.1 Hz, 1H), 8.59 (dd, J = 4.8, 1.6 Hz, 1H), 8.14 (dt, J = 8.2, 1.9 Hz, 1H), 7.76 - 7.63 (m, 2H), 7.63 - 7.58 (m, 1H), 7.50 (dd, J = 8.0, 4.8 Hz, 1H), 6.61 (s, 1H), 2.64 - 2.55 (m, 1H), 1.58 (s, 6H), 0.94 - 0.87 (m, 4H). |
| **T43** | 2-(2-(cyclopropanesulfonamido)thiazol-4-yl)-N-(3-methoxy-4-(pyrazin-2-yl)phenyl)-2-methylpropanamide 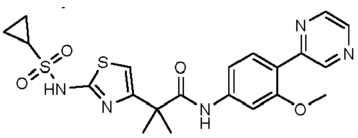 | Method 1b using INTB37 and INTA69, [UPLC basic], 474, (0.94) | 12.59 (s, 1H), 9.65 (s, 1H), 9.11 (d, J = 1.6 Hz, 1H), 8.70 (dd, J = 2.5, 1.6 Hz, 1H), 8.51 (d, J = 2.5 Hz, 1H), 7.79 (d, J = 8.5 Hz, 1H), 7.63 (d, J = 1.9 Hz, 1H), 7.46 (dd, J = 8.5, 1.9 Hz, 1H), 6.53 (s, 1H), 3.88 (s, 3H), 2.68 - 2.57 (m, 1H), 1.58 (s, 6H), 0.99 - 0.82 (m, 4H). |
| **T44** | 2-(2-(cyclopropanesulfonamido)thiazol-4-yl)-N-(4-(2-methoxypyridin-3-yl)phenyl)-2-methylpropanamide 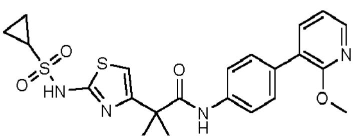 | Method 2 using INTB53, [UPLC acidic], 487, (1.30) | 12.56 (s, 1H), 9.33 (s, 1H), 8.00 (d, J = 5.2 Hz, 1H), 7.69 - 7.61 (m, 2H), 7.15 (d, J = 8.5 Hz, 2H), 6.96 - 6.90 (m, 1H), 6.52 (d, J = 12.8 Hz, 1H), 3.73 (s, 3H), 2.61 - 2.56 (m, 1H), 2.05 (s, 3H), 1.56 (s, 6H), 0.93 - 0.74 (m, 4H). |

(continued)

| T# | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **T45** | 2-(2-(cyclopropanesulfonamido)thiazol-4-yl)-N-(4-(5-(hydroxymethyl)pyridin-3-yl)phenyl) acetamide 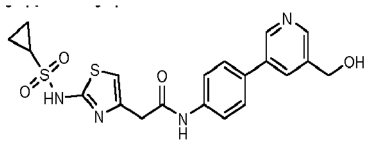 | Method 2 using INTB53, [HPLC acidic], 445, (0.97) | 12.53 (s, 1H), 10.32 (s, 1H), 8.76 (d, J = 2.3 Hz, 1H), 8.49 (d, J = 2.0 Hz, 1H), 7.97 (t, J = 2.2 Hz, 1H), 7.72 (d, J = 2.1 Hz, 4H), 6.56 (s, 1H), 5.36 (t, J = 5.7 Hz, 1H), 4.61 (d, J = 5.7 Hz, 2H), 3.67 (s, 2H), 2.64 - 2.55 (m, 1H), 0.95 - 0.83 (m, 4H). |
| **T46** | 2-(2-(cyclopropanesulfonamido)thiazol-4-yl)-N-(4-(6-methoxypyridin-3-yl)phenyl)-2-methylpropanamide 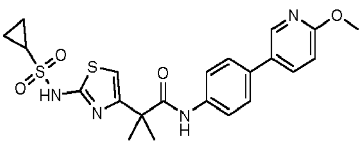 | Method 2 using INTB53, [UPLC acidic], 473, (1.24) | 12.56 (s, 1H), 9.36 (s, 1H), 8.46 (dd, J = 2.6, 0.8 Hz, 1H), 7.99 (dd, J = 8.7, 2.6 Hz, 1H), 7.74 - 7.65 (m, 2H), 7.66 - 7.58 (m, 2H), 6.88 (dd, J = 8.7, 0.7 Hz, 1H), 6.52 (s, 1H), 3.88 (s, 3H), 2.60 - 2.55 (m, 1H), 1.56 (s, 6H), 0.94-0.87 (m, 4H). |
| **T47** | N-(4-(5-cyanopyridin-3-yl)phenyl)-2-(2-(cyclopropanesulfonamido)thiazol-4-yl)-2-methylpropanamide 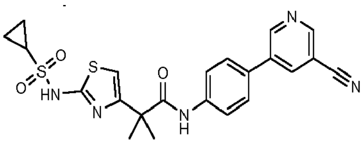 | Method 2 using INTB53, [UPLC acidic], 468, (1.20) | 12.58 (s, 1H), 9.47 (s, 1H), 9.20 (d, J = 2.3 Hz, 1H), 8.97 (d, J = 1.9 Hz, 1H), 8.65 (t, J = 2.1 Hz, 1H), 7.81 (q, J = 8.7 Hz, 4H), 6.55 (s, 1H), 2.65 - 2.51 (m, 1H), 1.58 (s, 6H), 0.92 (s, 4H). |
| **T48** | 2-(2-(cyclopropanesulfonamido)thiazol-4-yl)-2-methyl-N-(4-(5-(trifluoromethyl)pyridin-3-yl)phenyl) propanamide 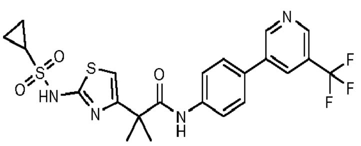 | Method 2 using INTB53, [UPLC acidic], 511, (1.36) | 12.58 (s, 1H), 9.47 (s, 1H), 9.22 (d, J = 2.3 Hz, 1H), 8.93 (d, J = 2.0 Hz, 1H), 8.45 (d, J = 2.2 Hz, 1H), 7.90 - 7.76 (m, 4H), 6.55 (s, 1H), 2.63 - 2.56 (m, 1H), 1.58 (s, 6H), 0.94 - 0.87 (m, 4H). |
| **T49** | 2-(2-(cyclopropanesulfonamido)thiazol-4-yl)-2-methyl-N-(4-(5-(methylsulfonyl)pyridin-3-yl)phenyl) propanamide 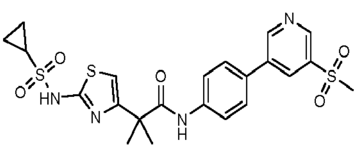 | Method 2 using INTB53, [UPLC acidic], 521, (1.06) | 12.58 (s, 1H), 9.48 (s, 1H), 9.24 (d, J = 2.2 Hz, 1H), 9.00 (d, J = 2.1 Hz, 1H), 8.56-8.50 (m, 1H), 7.90 - 7.77 (m, 4H), 6.55 (s, 1H), 3.40 (s, 3H), 2.62 - 2.56 (m, 1H), 1.58 (s, 6H), 1.04 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T50** | 2-(2-(cyclopropanesulfonamido)thiazol-4-yl)-N-(2-methoxy-4-(5-methoxypyridin-3-yl)phenyl)-2-methylpropanamide 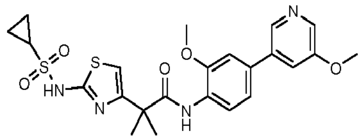 | Method 2 using INTB54, [UPLC acidic], 503, (1.02) | 12.65 (s, 1H), 8.54 (d, J = 1.9 Hz, 1H), 8.41 (s, 1H), 8.28 (d, J = 2.7 Hz, 1 H), 8.03 (d, J = 8.3 Hz, 1H), 7.65 (t, J = 2.3 Hz, 1H), 7.43 - 7.32 (m, 2H), 6.77 (s, 1H), 3.92 (s, 6H), 2.66-2.58 (m, 1H), 1.57 (s, 6H), 0.96 - 0.88 (m, 4H). |
| **T51** | 2-(2-(cyclopropanesulfonamido)thiazol-4-yl)-2-methyl-N-(6-(trifluoromethyl)-[2,3'-bipyridin]-6'-yl) propanamide 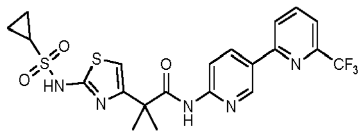 | Method 2 using INTB55, [HPLC acidic], 512, (1.93) | 12.51 (s, 1H), 10.00 (s, 1H), 9.28 (d, J = 2.1 Hz, 1H), 9.01 - 8.95 (m, 1H), 8.87 (d, J = 2.5 Hz, 1H), 8.56 (s, 1H), 8.35 (dd, J = 8.8, 2.6 Hz, 1H), 8.22 - 8.14 (m, 1H), 6.61 (s, 1H), 2.61 (s, 1H), 1.62 (s, 6H), 0.97 - 0.86 (m, 4H). |
| **T52** | 2-(2-(cyclopropanesulfonamido)thiazol-4-yl)-N-(4-(6-ethoxypyrazin-2-yl)phenyl)-2-methylpropanamide 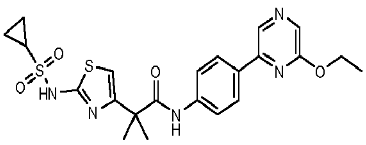 | Method 2b using INTB56, [HPLC acidic], 488, (2.10) | 12.59 (s, 1H), 9.50 (s, 1H), 8.78 (s, 1H), 8.19 (s, 1H), 8.15 - 8.05 (m, 2H), 7.85 - 7.74 (m, 2H), 6.55 (s, 1H), 4.48 (q, J = 7.1 Hz, 2H), 2.63 2.56 (m, 1H), 1.59 (s, 6H), 1.41 (t, J = 7.0 Hz, 3H), 0.99 - 0.84 (m, 4H). |
| **T53** | 2-(2-(cyclopropanesulfonamido)thiazol-4-yl)-2-methyl-N-(4-(6-morpholinopyrazin-2-yl)phenyl)propanamide 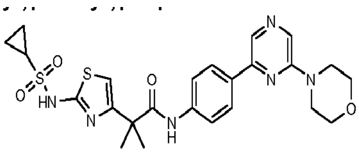 | Method 2b using INTB56, [HPLC acidic], 529, (1.96) | 12.59 (s, 1H), 9.47 (s, 1H), 8.49 (s, 1H), 8.24 (s, 1H), 8.07 (d, J = 8.5 Hz, 2H), 7.83 - 7.68 (m, 2H), 6.55 (s, 1H), 3.79-3.72 (m, 4H), 3.66-3.58 (m, 4H), 2.63 - 2.55 (m, 1H), 1.58 (s, 6H), 0.97 - 0.82 (m, 4H). |
| **T54** | N-(4-(6-cyclobutoxypyrazin-2-yl)phenyl)-2-(2-(cyclopropanesulfonamido)thiazol-4-yl)-2-methylpropanamide 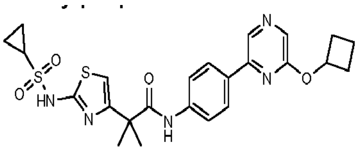 | Method 2b using INTB56, [HPLC basic], 514, (2.00) | 12.58 (s, 1H), 9.50 (s, 1H), 8.79 (s, 1H), 8.17 (s, 1H), 8.14 - 8.04 (m, 2H), 7.86 - 7.74 (m, 2H), 6.55 (s, 1H), 5.34 - 5.18 (m, 1H), 2.65 - 2.56 (m, 1H), 2.57 - 2.50 (m, 1H), 2.51 - 2.43 (m, 1H), 2.22 - 2.09 (m, 2H), 1.90 - 1.67 (m, 2H), 1.59 (s, 6H), 0.95 - 0.86 (m, 4H). |
| **T55** | 2-(2-(cyclopropanesulfonamido)thiazol-4-yl)-2-methyl-N-(4-(6-propoxypyrazin-2-yl)phenyl)propanamide 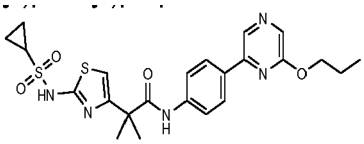 | Method 2b using INTB56, [HPLC basic], 502, (2.00) | 12.59 (s, 1H), 9.50 (s, 1H), 8.78 (s, 1H), 8.20 (s, 1H), 8.17 - 8.06 (m, 2H), 7.83 - 7.77 (m, 2H), 6.55 (s, 1H), 4.39 (t, *J* = 6.6 Hz, 2H), 2.65 - 2.54 (m, 1H), 1.87 - 1.76 (m, 2H), 1.59 (s, 6H), 1.02 (t, *J* = 7.4 Hz, 3H), 0.94 - 0.85 (m, 4H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T56** | 2-(2-(cyclopropanesulfonamido)thiazol-4-yl)-N-(2-fluoro-4-(5-methoxypyridin-3-yl)phenyl)-2-methylpropanamide 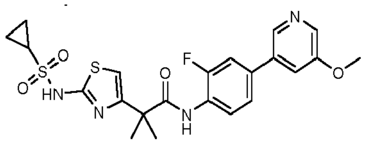 | Method 2b using INTB56, [UPLC acidic], 491, (0.99) | 12.57 (s, 1H), 9.21 (s, 1H), 8.52 (d, J = 1.9 Hz, 1H), 8.28 (d, J = 2.8 Hz, 1 H), 7.80-7.72 (m, 1H), 7.70 - 7.60 (m, 3H), 6.60 (s, 1H), 3.91 (s, 3H), 2.61 - 2.56 (m, 1H), 1.57 (s, 6H), 0.93 - 0.88 (m, 4H). |
| **T57** | 2-(2-(cyclopropanesulfonamido)thiazol-4-yl)-2-methoxy-N-(4-(6-methoxypyrazin-2-yl)phenyl) acetamide 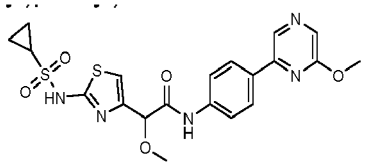 | Method 1 using INTB43 and INTA2, [HPLC acidic], 476, (1.53) | 12.77 (v. br. s, 1H), 10.12 (s, 1H), 8.80 (s, 1H), 8.22 (s, 1H), 8.14 (d, J = 8.8 Hz, 2H), 7.87 (d, J = 8.8 Hz, 2H), 6.74 (v. br. s, 1H), 4.81 (s, 1H), 4.02 (s, 3H), 3.38 (s, 3H), 2.54 - 2.50 (m, 1H), 0.91 - 0.76 (m, 4H). |
| **T58** | N-(4-(5-chloropyridin-3-yl)phenyl)-2-(2-(cyclopropanesulfonamido)thiazol-4-yl)-2-isopropoxyacetamide 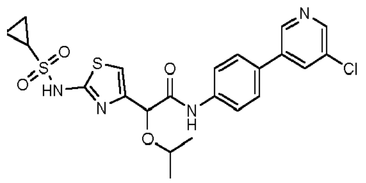 | Method 1c using INTB13 and INTA18, [UPLC acidic], 507, (1.78) | 12.78 (s, 1H), 9.89 (s, 1H), 8.88 (d, J = 2.0 Hz, 1H), 8.59 (d, J = 2.3 Hz, 1H), 8.25 (t, J = 2.2 Hz, 1H), 7.86 - 7.67 (m, 4H), 6.79 (s, 1H), 5.02 (s, 1H), 3.83 - 3.67 (m, 1H), 2.60-2.50 (m, 1H), 1.24 - 1.18 (m, 6H), 0.93 - 0.84 (m, 4H). |
| **T59** | 2-(2-(cyclopropanesulfonamido)thiazol-4-yl)-4-methoxy-N-(5-(6-(trifluoromethyl)pyrazin-2-yl)pyridin-2-yl)butanamide 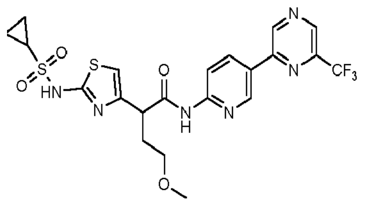 | Method 1c using INTB44 and INTA3, [UPLC acidic], 543, (1.30) | 12.60 (s, 1H), 11.01 (s, 1H), 9.66 (s, 1H), 9.16 (d, J = 4.7 Hz, 2H), 8.59 (dd, J = 8.8, 2.5 Hz, 1H), 8.27 (d, J = 8.8 Hz, 1H), 6.57 (s, 1H), 4.09 - 3.97 (m, 1H), 3.38 - 3.26 (m, 2H obscured by residual water), 3.20 (s, 3H), 2.64 - 2.55 (m, 1H), 2.30 - 2.18 (m, 1H), 2.14 - 1.99 (m, 1H), 0.95 - 0.72 (m, 4H). |
| **T60** | N-([1,1'-biphenyl]-4-yl)-2-(2-(cyclopropanesulfonamido)thiazol-4-yl)-2,2-difluoroacetamide 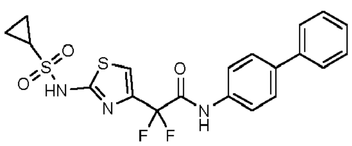 | Method 1 using INTB45, [UPLC acidic], 450, (1.54) | 10.90 (s, 1H), 7.86-7.80 (m, 2H), 7.74 - 7.66 (m, 4H), 7.65 - 7.54 (m, 1H), 7.47 (dd, J = 8.3, 6.9 Hz, 2H), 7.42 - 7.32 (m, 1H), 2.87 - 2.74 (m, 1 H), 1.10 - 0.88 (m, 4H), N-H not observed. |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T61** | 2-(2-(cyclobutanesulfonamido)thiazol-4-yl)-N-(4-(6-methoxypyrazin-2-yl)phenyl)acetamide 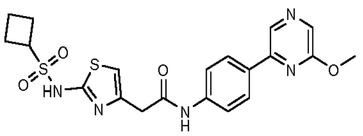 | Method 1 using INTB46 and INTA2, [UPLC acidic], 460, (1.89) | (Methanol-d4) δ 8.66 (s, 1H), 8.18 - 8.02 (m, 3H), 7.81 - 7.72 (m, 2H), 6.54 (s, 1H), 4.09 (s, 3H), 3.96 - 3.86 (m, 1H), 3.74 (d, J = 1.0 Hz, 2H), 2.56 - 2.41 (m, 2H), 2.32 2.41 - 2.23 (m, 2H), 2.15 - 1.94 (m, 2H), 2 x exchnagable protons not observed. |
| **T62** | N-([3,3'-bipyridin]-6-yl)-2-(2-(cyclopropanesulfonamido)thiazol-4-yl) butanamide 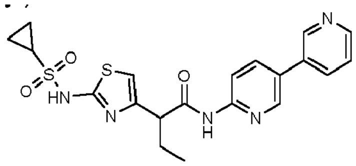 | Method 1c using INTB38, [HPLC acidic] 444, (1.23) | 12.57 (s, 1H), 10.86 (s, 1H), 8.95 (dd, J = 2.4, 0.9 Hz, 1H), 8.75 (t, J = 1.7 Hz, 1H), 8.63 - 8.55 (m, 1H), 8.21 (d, J = 1.7 Hz, 2H), 8.19-8.10 (m, 1H), 7.51 (ddd, J = 8.0, 4.8, 0.9 Hz, 1H), 6.57 (s, 1H), 3.80 (t, J = 7.4 Hz, 1H), 2.64 - 2.55 (m, 1H), 2.02 - 1.84 (m, 2H), 0.96 - 0.83 (m, 7H). |
| **T63** | 2-(2-(cyclopropanesulfonamido)thiazol-4-yl)-2-methyl-N-(5-phenylpyridin-2-yl)propanamide 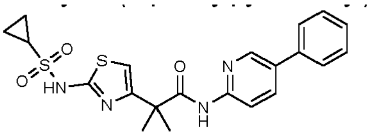 | Method 1 using INTB37, [UPLC acidic], 443, (1.34) | 12.60 (s, 1H), 9.98 (s, 1H), 8.67 (s, 1H), 8.18 - 8.07 (m, 2H), 7.76 - 7.68 (m, 2H), 7.53 - 7.44 (m, 2H), 7.44 - 7.34 (m, 1H), 6.57 (s, 1H), 2.62 - 2.56 (m, 1H), 1.60 (s, 6H), 0.93 - 0.86 (m, 4H). |
| **T64** | 2-(2-(cyclopropanesulfonamido)thiazol-4-yl)-N-(5-(pyrimidin-5-yl)pyridin-2-yl)acetamide 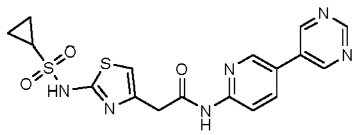 | Method 1 using INTB36, [HPLC acidic], 417, (1.25) | 12.52 (s, 1H), 10.94 (s, 1H), 9.22 (s, 2H), 9.21 (s, 1H), 8.83 (dd, J = 2.5, 0.9 Hz, 1H), 8.30 (dd, J = 8.6, 2.5 Hz, 1H), 8.19 (d, J = 8.6 Hz, 1H), 6.58 (s, 1H), 3.77 (s, 2H), 2.64 - 2.56 (m, 1H), 0.97 - 0.79 (m, 4H). |
| **T65** | N-([3,3'-bipyridin]-6-yl)-2-(2-(cyclopropanesulfonamido)thiazol-4- 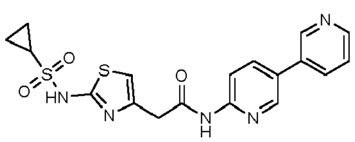 yl)acetamide | Method 1 using INTB36, [HPLC acidic], 416, (1.04) | 12.51 (s, 1H), 10.89 (s, 1H), 9.01 (d, J = 2.4 Hz, 1H), 8.77 (dd, J = 2.4, 0.9 Hz, 1H), 8.63 (dd, J = 4.9, 1.5 Hz, 1H), 8.29 - 8.19 (m, 2H), 8.17 (d, J = 8.8 Hz, 1H), 7.60 (dd, J = 8.0, 5.0 Hz, 1H), 6.58 (s, 1H), 3.57 (s, 2H), 2.63 - 2.56 (m, 1H), 0.93 - 0.85 (m, 4H). |
| **T66** | 2-(2-(cyclopropanesulfonamido)thiazol-4-yl)-N-(6-phenylpyridin-3-yl)acetamide 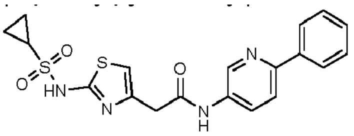 | Method 1 using INTB36, [UPLC acidic], 415, (1.03) | 12.56 (s, 1H), 10.52 (s, 1H), 8.88 - 8.78 (m, 1H), 8.14 (dd, J = 8.7, 2.6 Hz, 1H), 8.08 - 8.03 (m, 2H), 7.98 (d, J = 8.7 Hz, 1H), 7.51 - 7.45 (m, 2H), 7.44 - 7.36 (m, 1H), 6.60 (s, 1H), 3.75 - 3.66 (m, 2H), 2.65 - 2.56 (m, 1H), 0.96 - 0.85 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T67** | N-([2,3'-bipyridin]-5-yl)-2-(2-(cyclopropanesulfonamido)thiazol-4- 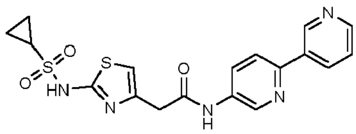 yl)acetamide | Method 1 using INTB36, [HPLC acidic], 416, (1.05) | 12.53 (s, 1H), 10.56 (s, 1H), 9.25 (dd, J = 2.3, 0.9 Hz, 1H), 8.87 (d, J = 2.4 Hz, 1H), 8.62 (dd, J = 4.8, 1.6 Hz, 1H), 8.44 (dt, J = 8.1, 2.0 Hz, 1H), 8.19 (dd, J = 8.7, 2.6 Hz, 1H), 8.07 (d, J = 8.7 Hz, 1H), 7.54 (dd, J = 8.0, 4.8 Hz, 1H), 6.60 (s, 1H), 3.72 (s, 2H), 2.64 - 2.56 (m, 1H), 0.94 - 0.84 (m, 4H). |
| **T68** | 2-(2-(cyclopropanesulfonamido)thiazol-4-yl)-2-methyl-N-(4-(pyridazin-3-yl)phenyl)propanamide 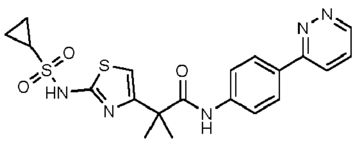 | Method 1 using INTB37, [HPLC basic], 444, (1.44) | 12.58 (s, 1H), 9.52 (s, 1H), 9.17 (dd, J = 4.9, 1.6 Hz, 1H), 8.21 (dd, J = 8.7, 1.6 Hz, 1H), 8.16 (d, J = 8.8 Hz, 2H), 7.83 (d, J = 8.8 Hz, 2H), 7.76 (dd, J = 8.7, 4.9 Hz, 1H), 6.57 (s, 1H), 2.65 - 2.56 (m, 1H), 1.59 (s, 6H), 0.96 - 0.84 (m, 4H). |
| **T69** | 2-(2-(cyclopropanesulfonamido)thiazol-4-yl)-N-(4-(pyridazin-4-yl)phenyl)butanamide 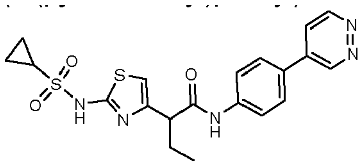 | Method 1 using INTB38, [HPLC acidic], 444, (1.91) | 12.61 (s, 1H), 10.41 (s, 1H), 9.64 (dd, J = 2.6, 1.2 Hz, 1H), 9.24 (dd, J = 5.5, 1.2 Hz, 1H), 8.05 - 7.87 (m, 3H), 7.85 - 7.73 (m, 2H), 6.56 (s, 1H), 3.61 (t, J = 7.4 Hz, 1H), 2.65 - 2.56 (m, 1H), 1.98 - 1.87 (m, 2H), 0.98 - 0.82 (m, 7H). |
| **T70** | 2-(2-(cyclopropanesulfonamido)thiazol-4-yl)-N-(4-(pyrazin-2-yl)phenyl)butanamide 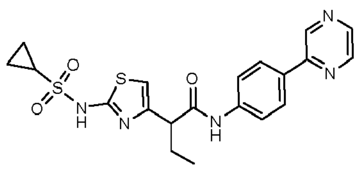 | Method 1 using INTB38, [HPLC acidic], 444, (1.71) | 12.62 (s, 1H), 10.38 (s, 1H), 9.23 (d, J = 1.5 Hz, 1H), 8.69 (dd, J = 2.5, 1.5 Hz, 1H), 8.57 (d, J = 2.5 Hz, 1H), 8.21 - 8.09 (m, 2H), 7.85 - 7.72 (m, 2H), 6.56 (s, 1H), 3.62 (t, J = 7.4 Hz, 1H), 2.65 - 2.56 (m, 1H), 2.02 - 1.84 (m, 2H), 0.97 - 0.83 (m, 7H). |
| **T71** | N-(4-(5-chloropyridin-3-yl)phenyl)-2-(2-(cyclopropanesulfonamido)thiazol-4-yl)-4-methoxybutanamide 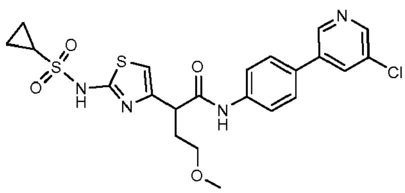 | Method 1 using INTB44 and INTA18, [HPLC acidic], 507, (1.92) | 12.63 (1H, s), 10.34 (1H, s), 8.87 (1H, d), 8.59 (1H, d), 8.23 (1H, t), 7.82 - 7.71 (4H, m), 6.52 (1H, s), 3.86 - 3.77 (1H, m), 3.23 (3H, s), 2.64 - 2.56 (1H, m), 2.27 - 2.01 (2H, m), 0.95 - 0.84 (4H, m) CH2 obscured at 3.3 ppm |

(continued)

| T# | Name/Structure (All examples containing chiral centres are racemates unless stated) | Synthesis Method, [LCMS Method], m/z $(M+H)^{+}$, (Rt/min) | $^{1}$H NMR Chemical Shift Data (DMSO-d6 unless stated) |
|---|---|---|---|
| **T72** | **Single enantiomer - stereochemistry unassigned** 2-(2-(cyclopropanesulfonamido)thiazol-4-yl)-N-(4-(5-fluoropyridin-3-yl)phenyl)butanamide 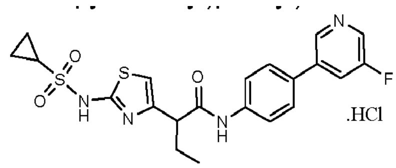 | Method 1 using INTB38 and INTA13, Chiral IA [UPLC basic], 461, (1) | 12.65 (s, 1H), 10.46 (s, 1H), 8.82 (t, J = 1.8 Hz, 1H), 8.57 (d, J = 2.7 Hz, 1H), 8.09 (ddd, J = 10.5, 2.7, 1.8 Hz, 1H), 7.86 - 7.59 (m, 4H), 6.56 (s, 1H), 3.74 - 3.61 (m, 1H), 2.66 - 2.56 (m, 1H), 2.09 - 1.80 (m, 2H), 1.02 - 0.84 (m, 7H). |
| **T73** | 2-(2-(cyclopropanesulfonamido)thiazol-4-yl)-N-(4-(6-(trifluoromethyl)pyrazin-2-yl)phenyl) butanamide 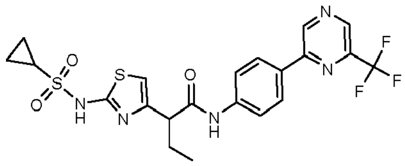 | Method 1 using INTB38 and INTA39, [HPLC acidic], 512, (2.16) | 12.62 (s, 1H), 10.46 (s, 1H), 9.59 (s, 1H), 9.09 (s, 1H), 8.26 - 8.15 (m, 2H), 7.90 - 7.76 (m, 2H), 6.56 (s, 1H), 3.63 (t, J = 7.1 Hz, 1H), 2.63 - 2.55 (m, 1H), 2.04 - 1.85 (m, 2H), 0.97 - 0.83 (m, 7H). |
| **T74** | 2-(2-(cyclopropanesulfonamido)thiazol-4-yl)-3-methyl-N-(4-(pyrazin-2-yl)phenyl)butanamide 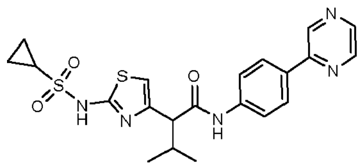 | Method 1 using INTB40, [HPLC basic], 458, (1.6) | 12.59 (s, 1H), 10.40 (s, 1H), 9.23 (d, J = 1.6 Hz, 1H), 8.68 (dd, J = 2.5, 1.6 Hz, 1H), 8.57 (d, J = 2.5 Hz, 1H), 8.18 - 8.09 (m, 2H), 7.82 7.73 (m, 2H), 6.60 (s, 1H), 3.38 (d, J = 9.8 Hz, 1H), 2.64 - 2.55 (m, 1H), 2.47 - 2.32 (m, 1H), 0.98 (d, J = 6.6 Hz, 3H), 0.94 - 0.84 (m, 7H). |
| **T75** | 2-(2-(cyclopropanesulfonamido)thiazol-4-yl)-N-(4-(6-propoxypyrazin-2-yl)phenyl)butanamide 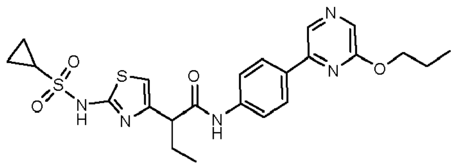 | Method 1 using INTB38 and INTA42, [HPLC acidic], 502, (2.29) | 12.45 (s, 1H), 10.36 (s, 1H), 8.76 (s, 1H), 8.19 (s, 1H), 8.15 - 8.06 (m, 2H), 7.82 - 7.71 (m, 2H), 6.55 (s, 1H), 4.38 (t, J = 6.6 Hz, 2H), 3.61 (t, J = 7.5 Hz, 1H), 2.65-2.53 (m, 1H), 2.04 - 1.83 (m, 2H), 1.86 - 1.73 (m, 2H), 1.01 (t, J = 7.4 Hz, 3H), 0.95 - 0.83 (m, 7H). |
| **T76** | 2-(2-(cyclopropanesulfonamido)thiazol-4-yl)-N-(4-(6-isopropoxypyrazin-2-yl)phenyl)butanamide 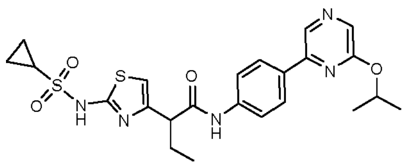 | Method 1 using INTB38 and INTA40, [HPLC acidic], 502, (2.26) | 12.60 (s, 1H), 10.36 (s, 1H), 8.74 (s, 1H), 8.12 (s, 1H), 8.11 - 8.06 (m, 2H), 7.87 - 7.63 (m, 2H), 6.54 (s, 1H), 5.47 - 5.34 (m, 1H), 3.61 (t, J = 7.5 Hz, 1H), 2.65 - 2.53 (m, 1H), 2.03 - 1.82 (m, 2H), 1.38 (d, J = 6.2 Hz, 6H), 0.96 - 0.84 (m, 7H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T77** | 2-(2-(cyclopropanesulfonamido)thiazol-4-yl)-N-(4-(6-cyclopropoxypyrazin-2-yl)phenyl)butanamide 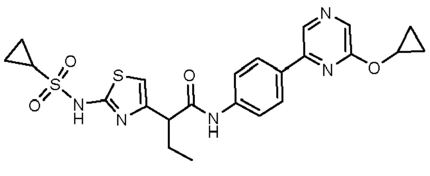 | Method 1 using INTB38 and INTA41, [HPLC basic], 500, (1.8) | 12.62 (s, 1H), 10.37 (s, 1H), 8.84 (s, 1H), 8.24 (s, 1H), 8.18 - 8.10 (m, 2H), 7.77 (d, J = 8.8 Hz, 2H), 6.56 (s, 1H), 4.42 (tt, J = 6.2, 3.1 Hz, 1H), 3.62 (t, J = 7.5 Hz, 1H), 2.65 - 2.55 (m, 1H), 2.04 - 1.83 (m, 2H), 0.97 - 0.80 (m, 11H). |
| **T78** | N-(4-(6-chloropyrazin-2-yl)phenyl)-2-(2-(cyclopropanesulfonamido)thiazol-4-yl) butanamide 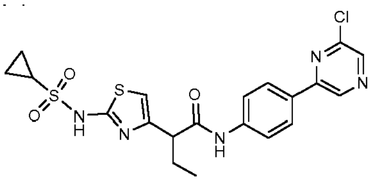 | Method 1 using INTB38 and INTA43, [HPLC basic], 478, (1.8) | 12.62 (s, 1H), 10.43 (s, 1H), 9.25 (s, 1H), 8.70 (s, 1H), 8.17 - 8.10 (m, 2H), 7.85 - 7.75 (m, 2H), 6.56 (s, 1H), 3.62 (t, J = 7.4 Hz, 1H), 2.65-2.55 (m, 1H), 2.04 - 1.85 (m, 2H), 0.96 - 0.88 (m, 7H). |
| **T79** | N-(4-(6-cyanopyrazin-2-yl)phenyl)-2-(2-(cyclopropanesulfonamido)thiazol-4-yl) butanamide 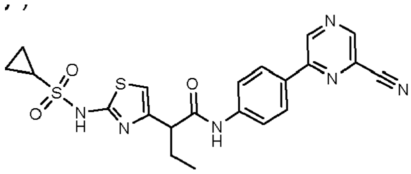 | Method 1 using INTB38 and INTA44, [HPLC basic], 469, (1.7) | 12.62 (s, 1H), 10.45 (s, 1H), 9.54 (s, 1H), 9.12 (s, 1H), 8.27 - 8.13 (m, 2H), 7.87 - 7.75 (m, 2H), 6.56 (s, 1H), 3.63 (t, J = 7.4 Hz, 1H), 2.64 - 2.55 (m, 1H), 2.04 - 1.82 (m, 2H), 0.95 - 0.80 (m, 7H). |
| **T80** | **Single enantiomer - stereochemistry unassigned** 2-(2-(cyclopropanesulfonamido)thiazol-4-yl)-N-(4-(6-(trifluoromethyl)pyrazin-2-yl)phenyl) butanamide 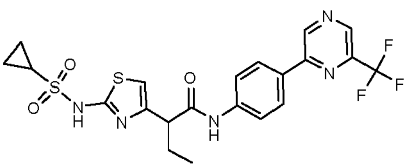 | Method 1 using INTB38 and INTA39, Chiral IA (5.8), [HPLC acidic], 512, (2.2) | 12.63 (s, 1H), 10.50 (s, 1H), 9.59 (s, 1H), 9.09 (s, 1H), 8.29 - 8.13 (m, 2H), 7.93 - 7.78 (m, 2H), 6.54 (s, 1H), 3.61 (t, J = 7.4 Hz, 1H), 2.65 - 2.56 (m, 1H), 2.03 - 1.81 (m, 2H), 1.00 - 0.72 (m, 7H). |
| **T81** | 2-(2-(cyclopropanesulfonamido)thiazol-4-yl)-N-(4-(6-methoxypyrazin-2-yl)phenyl)acetamide 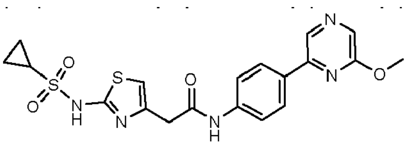 | Method 1 using INTB36 and INTA2, [HPLC acidic], 446, (1.78) | 12.53 (s, 1H), 10.39 (s, 1H), 8.78 (s, 1H), 8.21 (s, 1H), 8.19 - 8.08 (m, 2H), 7.75 (d, J = 8.7 Hz, 2H), 6.57 (s, 1H), 4.01 (s, 3H), 3.68 (s, 2H), 2.63 - 2.54 (m, 1H), 0.97 - 0.82 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T82** | 2-(2-(cyclopropanesulfonamido)thiazol-4-yl)-N-(4-(pyrazin-2-yl)phenyl)acetamide 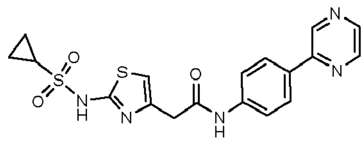 | Method 1 using INTB36, [HPLC acidic], 416, (1.44) | 12.54 (s, 1H), 10.40 (s, 1H), 9.23 (d, J = 1.6 Hz, 1H), 8.69 (dd, J = 2.5, 1.5 Hz, 1H), 8.57 (d, J = 2.5 Hz, 1H), 8.24 - 8.01 (m, 2H), 7.86 - 7.65 (m, 2H), 6.58 (s, 1H), 3.74 - 3.63 (m, 2H), 2.64 - 2.55 (m, 1H), 0.96 - 0.85 (m, 4H). |
| **T83** | N-([1,1'-biphenyl]-4-yl)-2-(cyclopropanesulfonamido)-4,5,6,7-tetrahydrobenzo[d]thiazole-4-carboxamide 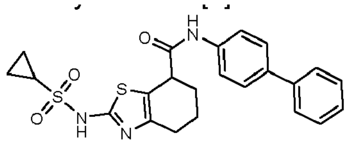 | Method 1 using INTB67, [HPLC acidic], 454, (2.23) | 12.35 (s, 1H), 10.32 (s, 1H), 7.72 (d, J = 8.7 Hz, 2H), 7.69 - 7.61 (m, 4H), 7.45 (t, J = 7.7 Hz, 2H), 7.36 - 7.30 (m, 1H), 3.71 - 3.62 (m, 1H), 3.30 (s, 2H), 2.62 - 2.53 (m, 1H), 2.19 - 1.66 (m, 4H), 0.98 - 0.74 (m, 4H). |
| **T84** | 2-(cyclopropanesulfonamido)-N-(4-(pyridin-3-yl) phenyl)-4,5,6,7-tetrahydrobenzo[d]thiazole-4-carboxamide 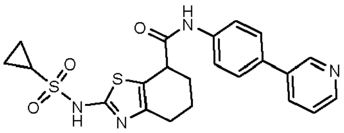 | Method 1 using INTB67, [HPLC acidic], 455, (1.27) | 12.32 (s, 1H), 10.28 (s, 1H), 8.89 (dd, J = 2.4, 0.8 Hz, 1H), 8.53 (dd, J = 4.7, 1.6 Hz, 1H), 8.06 (ddd, J = 8.0, 2.5, 1.6 Hz, 1H), 7.78 - 7.68 (m, 4H), 7.46 (ddd, J = 8.0, 4.7, 0.9 Hz, 1H), 3.66 (t, J = 6.0 Hz, 1H), 2.62-2.51 (m, 1H), 2.14-2.07 (m, 1H), 2.00 - 1.95 (m, 1H), 1.89 - 1.84 (m, 1H), 1.78 - 1.73 (m, 1H), 1.30 - 1.22 (m, 2H), 0.93 - 0.85 (m, 4H). |
| **T85** | N-([1,1'-biphenyl]-4-yl)-2-(2-(cyclopropanesulfonamido)thiazol-4- 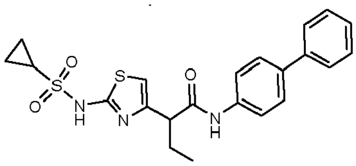 yl)butanamide | Method 1 using INTB38, [HPLC basic], 442, (2) | 12.59 (s, 1H), 10.24 (s, 1H), 7.75 - 7.66 (m, 2H), 7.68 - 7.60 (m, 4H), 7.44 (dd, J = 8.4, 7.0 Hz, 2H), 7.37 - 7.28 (m, 1H), 6.53 (s, 1H), 3.58 (t, J = 7.4 Hz, 1H), 2.62 - 2.54 (m, 1H), 2.02 - 1.81 (m, 2H), 0.95 - 0.78 (m, 7H). |
| **T86** | N-([1,1'-biphenyl]-4-yl)-2-(2-(cyclopropanesulfonamido)thiazol-4-yl)-3-methylbutanamide 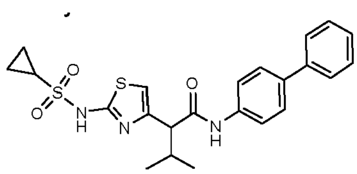 | Method 1 using INTB40, [HPLC acidic], 456, (2.35) | 12.58 (s, 1H), 10.27 (s, 1H), 7.76-7.60 (m, 6H), 7.45 (dd, J = 8.4, 7.0 Hz, 2H), 7.39 - 7.28 (m, 1H), 6.57 (s, 1H), 3.36 (d, J = 9.9 Hz, 1H), 2.66 - 2.56 (m, 1H), 2.47 - 2.35 (m, 1H), 1.04 - 0.81 (m, 10H). |

(continued)

| T# | Name/Structure (All examples containing chiral centres are racemates unless stated) | Synthesis Method, [LCMS Method], m/z (M+H)+, (Rt/min) | 1H NMR Chemical Shift Data (DMSO-d6 unless stated) |
|---|---|---|---|
| **T87** | N-(3'-chloro-[1,1'-biphenyl]-4-yl)-2-(2-(cyclopropanesulfonamido)thiazol-4-yl)-2-methylpropanamide 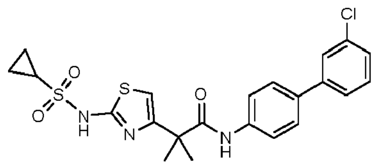 | Method 1 using INTB37, [HPLC acidic], 477, (2.36) | 12.58 (s, 1H), 9.41 (s, 1H), 7.77 - 7.67 (m, 5H), 7.67 - 7.63 (m, 1H), 7.48 (t, J = 7.9 Hz, 1H), 7.42 - 7.37 (m, 1H), 6.55 (s, 1H), 2.64 - 2.55 (m, 1H), 1.58 (s, 6H), 0.96 - 0.86 (m, 4H). |
| **T88** | N-(3'-cyano-[1,1'-biphenyl]-4-yl)-2-(2-(cyclopropanesulfonamido)thiazol-4-yl)-2-methylpropanamide 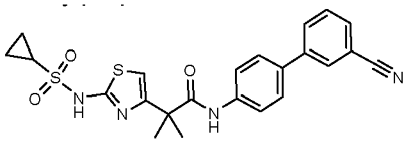 | Method 1 using INTB37, [HPLC acidic], 467, (2.11) | 12.57 (s, 1H), 9.42 (s, 1H), 8.17 - 8.13 (m, 1H), 8.05 - 8.00 (m, 1H), 7.81 - 7.76 (m, 1H), 7.75 (s, 4H), 7.64 (t, J = 7.8 Hz, 1H), 6.54 (s, 1H), 2.62 - 2.54 (m, 1H), 1.57 (s, 6H), 0.96 - 0.83 (m, 4H). |
| **T89** | 2-(2-(cyclopropanesulfonamido)thiazol-4-yl)-2,2-difluoro-N-(4-(pyridin-3-yl)phenyl)acetamide 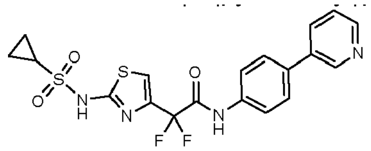 | Method 1 using INTB45, [UPLC acidic], 451, (0.84) | 10.95 (s, 1H), 8.92 (d, J = 2.5 Hz, 1H), 8.57 (dd, J = 4.8, 1.6 Hz, 1H), 8.10 (dt, J = 7.9, 1.9 Hz, 1H), 7.91 - 7.82 (m, 2H), 7.82 - 7.76 (m, 2H), 7.63-7.57 (m, 1H), 7.49 (dd, J = 8.1, 4.9 Hz, 1H), 2.86 - 2.76 (m, 1H), 1.01 (s, 4H), N-H not observed. |
| **T90** | N-(4-(5-fluoropyridin -3-yl)phenyl)-2-(2-(cyclopropanesulfonamido)thiazol-4-yl) butanamide 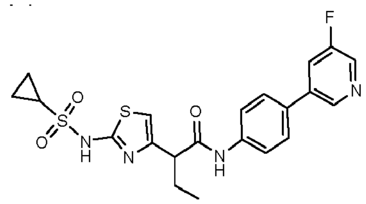 | Method 1 using INTB38 and INTA18, [UPLC basic], 477, (1.11) | 12.62 (s, 1H), 10.34 (s, 1H), 8.80 (t, J = 1.9 Hz, 1H), 8.54 (d, J = 2.7 Hz, 1H), 8.05 (ddd, J = 10.5, 2.7, 1.8 Hz, 1H), 7.83 - 7.67 (m, 4H), 6.55 (s, 1H), 3.60 (t, J = 7.4 Hz, 1H), 2.55-2.66 (m, 1H), 2.01 - 1.84 (m, 2H), 0.97 - 0.84 (m, 7H). |
| **T91** | **Single enantiomer - stereochemistry unassigned** N-(4-(5-fluoropyridin-3-yl)phenyl)-2-(2-(cyclopropanesulfonamido)thiazol-4-yl) butanamide.HCl 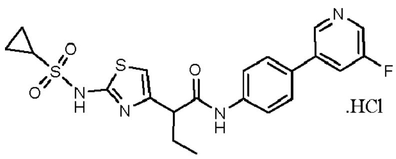 | Method 1 using INTB38 and INTA18, Chiral IA [UPLC basic], 475, (1.1) | 12.57 (s, 1H), 10.43 (s, 1H), 8.81 (d, J = 2.0 Hz, 1H), 8.53 (d, J = 2.3 Hz, 1H), 8.18 (dd, J = 2.1 Hz, 1H), 7.75 - 7.62 (m, 4H), 6.49 (s, 1H), 3.60 (dd, J = 7.5 Hz, 1H), 2.57 - 2.48 (m, 1H), 1.94 - 1.74 (m, 2H), 0.93 - 0.75 (m, 7H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T92** | 2-(2-(cyclopropanesulfonamido)thiazol-4-yl)-2-ethyl-N-(4-(pyridin-3-yl)phenyl)butanamide 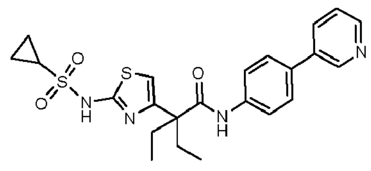 | Method 1 using INTB41, [UPLC acidic], 471.1, (0.88) | 12.44 (s, 1H), 9.33 (s, 1H), 8.87 (d, J = 2.4 Hz, 1H), 8.52 (dd, J = 4.7, 1.6 Hz, 1H), 8.04 (ddd, J = 8.0, 2.5, 1.6 Hz, 1H), 7.78 - 7.65 (m, 4H), 7.47 (ddd, J = 7.9, 4.8, 0.9 Hz, 1H), 6.63 (s, 1H), 2.56-2.63 (m, 1H), 2.08 - 1.90 (m, 4H), 0.93 - 0.82 (m, 4H), 0.73 (t, J = 7.3 Hz, 6H). |
| **T93** | N-(4-(5-cyanopyridin-3-yl)phenyl)-2-(2-(cyclopropanesulfonamido)thiazol-4-yl)-2-ethylbutanamide 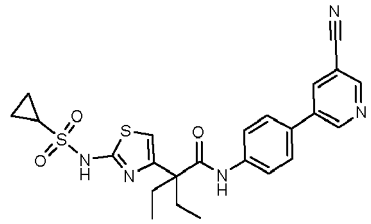 | Method 1 using INTB41 and INTA12, [UPLC acidic], 496.4, (1.3) | 12.48 (s, 1H), 9.38 (s, 1H), 9.18 (d, J = 2.3 Hz, 1H), 8.96 (d, J = 1.9 Hz, 1H), 8.63 (dd, J = 2.1 Hz, 1H), 7.84 - 7.73 (m, 4H), 6.61 (s, 1H), 2.54-2.62 (m, 1H), 2.08 - 1.92 (m, 4H), 0.92 - 0.85 (m, 4H), 0.72 (t, J = 7.3 Hz, 6H). |
| **T94** | 2-(2-(cyclopropanesulfonamido)thiazol-4-yl)-N-(4-(5-ethoxypyridin-3-yl)phenyl)propanamide 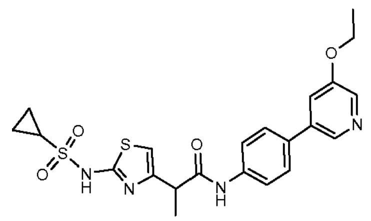 | Method 1 using INTB39 and INTA11, [UPLC acidic], 473, (0.94) | (Methanol-d4) 8.38 (s, 1H), 8.19 (d, J = 2.7 Hz, 1H), 7.82 - 7.72 (m, 2H), 7.72 - 7.63 (m, 2H), 7.60 (dd, J = 2.7, 1.8 Hz, 1H), 6.54 (d, J = 0.9 Hz, 1H), 4.21 (q, J = 7.0 Hz, 2H), 3.84 (q, J = 7.2 Hz, 1H), 2.66-2.58 (m, 1H), 1.60 (d, J = 7.1 Hz, 3H), 1.47 (t, J = 7.0 Hz, 3H), 1.16 - 1.05 (m, 2H), 1.00 - 0.89 (m, 2H), 2 exchangeable N-H not observed. |
| **T95** | N-(4-(5-chloropyridin-3-yl)phenyl)-2-(2-(cyclopropanesulfonamido)thiazol-4-yl) propanamide 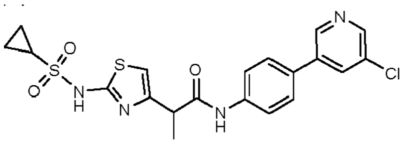 | Method 1 using INTB39 and INTA18, [UPLC acidic], 464, (1.23) | 12.63 (s, 1H), 10.31 (s, 1H), 8.86 (d, J = 2.0 Hz, 1H), 8.58 (d, J = 2.3 Hz, 1H), 8.22 (dd, J = 2.2 Hz, 1H), 7.82 - 7.65 (m, 4H), 6.49 (s, 1H), 3.76 (q, J = 7.1 Hz, 1H), 2.62 - 2.53 (m, 1H), 1.46 (d, J = 7.1 Hz, 3H), 0.96 - 0.80 (m, 4H). |
| **T96** | 2-(2-(cyclopropanesulfonamido)thiazol-4-yl)-N-(4-(5-ethoxypyridin-3-yl)phenyl)butanamide 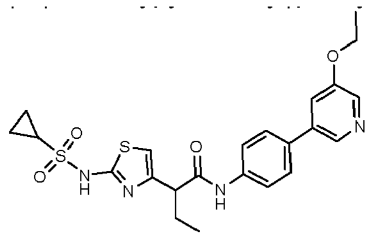 | Method 1 using INTB38 and INTA11, [HPLC basic], 487, (1.7) | 12.59 (s, 1H), 10.29 (s, 1H), 8.47 (d, J = 1.9 Hz, 1H), 8.24 (d, J = 2.7 Hz, 1H), 7.74 (s, 4H), 7.66 - 7.52 (m, 1H), 6.56 (s, 1H), 4.20 (q, J = 7.0 Hz, 2H), 3.60 (t, J = 7.4 Hz, 1H), 2.64 - 2.55 (m, 1H), 2.02 - 1.83 (m, 2H), 1.38 (t, J = 7.0 Hz, 3H), 0.96 - 0.86 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T97** | N-([1,1'-biphenyl]-4-yl)-2-(2-(cyclopropanesulfonamido)thiazol-4-yl)-2-methylpropanamide 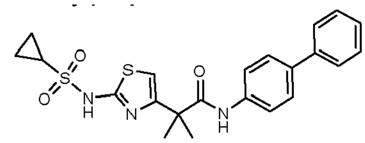 | Method 1 using INTB37, [HPLC acidic], 442, (1.43) | 12.60 (s, 1H), 9.40 (s, 1H), 7.76 - 7.68 (m, 2H), 7.70 - 7.60 (m, 4H), 7.49 - 7.41 (m, 2H), 7.38 - 7.29 (m, 1H), 6.55 (s, 1H), 2.63 - 2.56 (m, 1H), 1.58 (s, 6H), 0.94 - 0.87 (m, 4H). |
| **T98** | 2-(2-(cyclopropanesulfonamido)thiazol-4-yl)-N-(4-(4-methylpyridin-3-yl)phenyl)acetamide 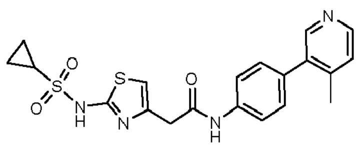 | Method 1 using INTB36 and INTA, [HPLC acidic], 429, (0.99) | 12.54 (s, 1H), 10.37 (s, 1H), 8.63 (d, J = 5.7 Hz, 2H), 7.86 - 7.65 (m, 3H), 7.54 - 7.40 (m, 2H), 6.58 (s, 1H), 3.68 (d, J = 1.0 Hz, 2H), 2.63 - 2.55 (m, 1H), 2.44 (s, 3H), 0.94 - 0.86 (m, 4H). |
| **T99** | 2-(2-(cyclopropanesulfonamido)thiazol-4-yl)-N-(4-(5-methylpyridin-3-yl)phenyl)acetamide 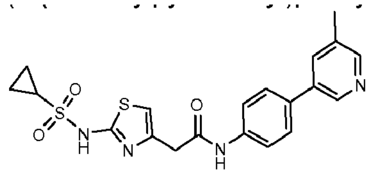 | Method 1 using INTB36 and INTA21, [HPLC acidic], 429, (1.07) | 12.53 (s, 1H), 10.31 (s, 1H), 8.70 (d, J = 2.2 Hz, 1H), 8.39 (dd, J = 2.0, 0.8 Hz, 1H), 7.91 (d, J = 2.3 Hz, 1H), 7.83 - 7.58 (m, 4H), 6.58 (s, 1H), 3.76 - 3.56 (m, 2H), 2.62 - 2.56 (m, 1H), 2.37 (s, 3H), 0.95 - 0.86 (m, 4H). |
| **T100** | 2-(2-(cyclopropanesulfonamido)thiazol-4-yl)-N-(4-(2-methylpyridin-3-yl)phenyl)acetamide 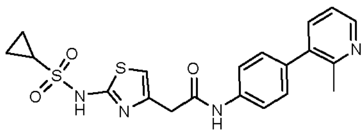 | Method 1 using INTB36 and INTA19, [HPLC acidic], 429, (0.94) | 12.54 (s, 1H), 10.35 (s, 1H), 8.61 (s, 1H), 7.99 (s, 1H), 7.78 - 7.72 (m, 2H), 7.61 (s, 1H), 7.52 - 7.31 (m, 2H), 6.58 (s, 1H), 3.68 (s, 2H), 2.62 - 2.56 (m, 1H), 0.98 - 0.76 (m, 4H), 1 proton obscured by solvent |
| **T101** | 2-(2-(cyclopropanesulfonamido)thiazol-4-yl)-N-(4-(6-methylpyridin-3-yl)phenyl)acetamide 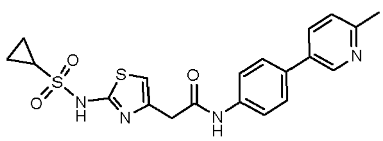 | Method 1 using INTB36 and INTA20, [HPLC acidic], 429, (1) | 12.53 (s, 1H), 10.30 (s, 1H), 8.78 (d, J = 2.4 Hz, 1H), 8.03 (d, J = 8.2 Hz, 1H), 7.77 - 7.63 (m, 4H), 7.38 (d, J = 8.2 Hz, 1H), 6.58 (s, 1H), 3.72 - 3.62 (m, 2H), 2.63 - 2.56 (m, 1H), 0.96 - 0.84 (m, 4H), 1 proton obscured by solvent |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T102** | 2-(2-(cyclopropanesulfonamido)thiazol-4-yl)-N-(4-(pyridin-3-yl)phenyl)propanamide 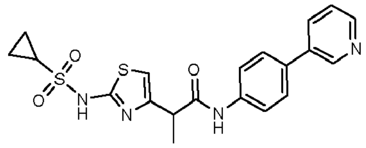 | Method 1 using INTB39, [HPLC acidic], 429, (2.11) | 12.60 (s, 1H), 10.27 (s, 1H), 8.88 (dd, J = 2.5, 0.9 Hz, 1H), 8.53 (dd, J = 4.8, 1.6 Hz, 1H), 8.05 (ddd, J = 8.0, 2.5, 1.6 Hz, 1H), 7.78 - 7.67 (m, 4H), 7.46 (ddd, J = 8.0, 4.8, 0.9 Hz, 1H), 6.50 (s, 1H), 3.77 (q, J = 7.1 Hz, 1H), 2.60- 2.50 (m, 1H), 1.46 (d, J = 7.1 Hz, 3H), 0.92-0.84 (m, 4H). |
| **T103** | 2-(2-(cyclopropanesulfonamido)thiazol-4-yl)-2-methyl-N-(4-(2-methylpyridin-3-yl)phenyl)propanamide 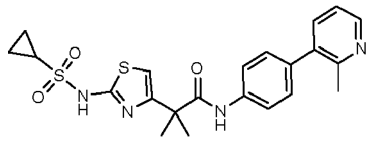 | Method 1 using INTB37 and INTA19, [HPLC acidic], 457, (1.1) | 12.63 (s, 2H), 9.45 (s, 1H), 8.44 (dd, J = 4.8, 1.8 Hz, 1H), 8.13 (s, 1H), 7.76 - 7.67 (m, 2H), 7.58 (dd, J = 7.7, 1.8 Hz, 1H), 7.39 - 7.30 (m, 2H), 7.28 (dd, J = 7.7, 4.9 Hz, 1H), 6.55 (s, 1H), 2.63 - 2.51 (m, 1H), 2.43 (s, 3H), 1.57 (s, 6H), 0.95 - 0.83 (m, 4H). |
| **T104** | 2-(2-(cyclopropanesulfonamido)thiazol-4-yl)-2-oxo-N-(4-(pyridin-3-yl)phenyl)acetamide 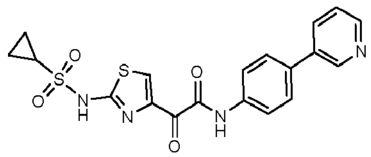 | Method 1 using INTB68, [UPLC acidic], 429, (0.8) | 13.19 (s, 1H), 11.01 (s, 1H), 8.93 (dd, J = 2.4, 0.9 Hz, 1H), 8.57 (dd, J = 4.7, 1.6 Hz, 1H), 8.50 (s, 1H), 8.16 - 8.07 (m, 1H), 7.98-7.85 (m, 2H), 7.81 - 7.74 (m, 2H), 7.50 (ddd, J = 8.0, 4.8, 0.9 Hz, 1H), 2.76 - 2.56 (m, 1H), 1.00 - 0.87 (m, 4H). |
| **T105** | 2-(2-(cyclopropanesulfonamido)thiazol-4-yl)-2-methyl-N-(4-(6-methylpyridin-3-yl)phenyl)propanamide 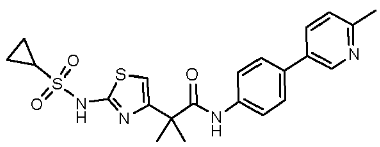 | Method 1 using INTB37 and INTA20, [UPLC acidic], 457, (0.7) | 12.55 (s, 1H), 9.41 (s, 1H), 8.78 - 8.72 (m, 1H), 7.95 (dd, J = 8.0, 2.5 Hz, 1H), 7.78 - 7.64 (m, 4H), 7.32 (d, J = 8.1 Hz, 1H), 6.56 (s, 1H), 3.32 (s, 3H), 2.64 - 2.54 (m, 1H), 1.58 (s, 6H), 0.95 - 0.85 (m, 4H). |
| **T106** | **Single enantiomer - stereochemistry unassigned** 2-(2-(cyclopropanesulfonamido)thiazol-4-yl)-N-(4-(6-(trifluoromethyl)pyrazin-2-yl)phenyl) butanamide 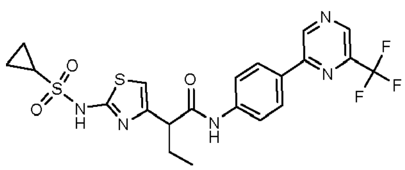 | Method 1 using INTB38 and INTA39, Chiral IA (7.8), [HPLC acidic], 512, (2.19) | 12.64 (s, 1H), 10.50 (s, 1H), 9.59 (s, 1H), 9.09 (s, 1H), 8.21 (d, J = 8.8 Hz, 2H), 7.84 (d, J = 8.9 Hz, 2H), 6.52 (s, 1H), 3.60 (t, J = 7.5 Hz, 1H), 2.66 - 2.55 (m, 1H), 2.02-1.81 (m, 2H), 0.973-0.785 (m, 7H). 1968-02B |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T107** | **Single enantiomer - stereochemistry unassigned** 2-(2-(cyclopropanesulfonamido)thiazol-4-yl)-2-methoxy-N-(4-(6-methoxypyrazin-2-yl)phenyl) acetamide 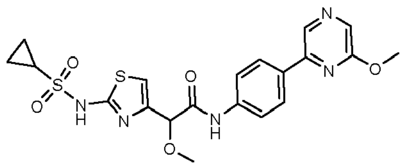 | Method 1 using INTB43 and INTA2 Chiral IC (10.2), [UPLC acidic], 476, (1.31) | 12.80 (s, 1H), 10.09 (s, 1H), 8.80 (s, 1H), 8.23 (s, 1H), 8.17 - 8.11 (m, 2H), 7.88 - 7.83 (m, 3H), 6.84 (s, 1H), 4.90 (s, 1H), 4.02 (s, 4H), 3.40 (s, 4H), 2.55 - 2.51 (m, 1H), 0.90 (d, J = 7.5 Hz, 6H). |
| **T108** | **Single enantiomer - stereochemistry unassigned** 2-(2-(cyclopropanesulfonamido)thiazol-4-yl)-2-methoxy-N-(4-(6-methoxypyrazin-2-yl)phenyl) acetamide 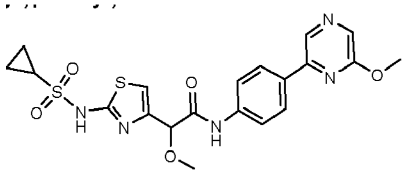 | Method 1 using INTB43 and INTA2 Chiral IC (25.2), [UPLC acidic], 476, (1.31) | 12.80 (s, 1H), 10.09 (s, 1H), 8.80 (s, 1H), 8.23 (s, 1H), 8.17 - 8.11 (m, 2H), 7.88 - 7.83 (m, 3H), 6.84 (s, 1H), 4.90 (s, 1H), 4.02 (s, 4H), 3.40 (s, 4H), 2.55 - 2.51 (m, 1H), 0.90 (d, J = 7.5 Hz, 6H). |
| **T109** | 2-(2-(cyclopropanesulfonamido)thiazol-4-yl)-2-methyl-N-(5-(6-(2,2,2-trifluoroethoxy)pyrazin-2-yl)pyridin-2-yl) propanamide 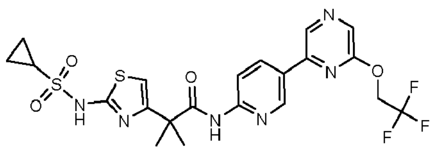 | Method 1b using INTB37 and INTA53, [HPLC basic], 543, (1.91) | 12.59 (s, 1H), 10.15 (s, 1H), 9.16 (d, J = 2.4 Hz, 1H), 9.04 (s, 1H), 8.62 (dd, J = 8.8, 2.5 Hz, 1H), 8.46 (s, 1H), 8.20 (d, J = 8.8 Hz, 1H), 6.58 (s, 1H), 5.22 (q, J = 9.0 Hz, 2H), 2.60 (s, 1H), 1.62 (s, 6H), 1.01 - 0.82 (m, 4H). |
| **T110** | 2-(2-(cyclopropanesulfonamido)thiazol-4-yl)-N-(3-fluoro-5-(pyrazin-2-yl)pyridin-2-yl)-2-methylpropanamide 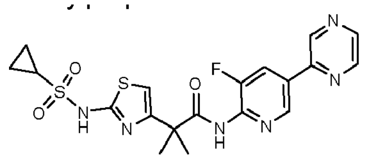 | Method 1b using INTB37 and INTA60, [UPLC acidic], 463, (0.93) | 12.64 (s, 1H), 10.10 (s, 1H), 9.39 (d, J = 1.5 Hz, 1H), 9.07 - 9.03 (m, 1H), 8.79 - 8.75 (m, 1H), 8.70 (d, J = 2.5 Hz, 1H), 8.51 - 8.45 (m, 1H), 6.57 (s, 1H), 2.60 - 2.56 (m, 1H), 1.59 (s, 6H), 0.93 - 0.87 (m, 4H). |
| **T111** | 2-(2-(cyclopropanesulfonamido)thiazol-4-yl)-N-(5-(6-ethoxypyrazin-2-yl)-3-fluoropyridin-2-yl)-2-methylpropanamide 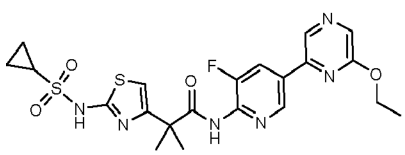 | Method 1b using INTB37 and INTA57, [UPLC acidic], 507, (1.22) | 12.64 (s, 1H), 10.08 (s, 1H), 9.05 - 9.00 (m, 1H), 8.93 (s, 1H), 8.44 (dd, J = 11.0, 1.9 Hz, 1H), 8.32 (s, 1H), 6.56 (s, 1H), 4.49 (q, J = 7.1 Hz, 2H), 2.60 - 2.56 (m, 1H), 1.59 (s, 6H), 1.39 (t, J = 7.0 Hz, 3H), 0.90 (s, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T112** | 2-(2-(cyclopropanesulfonamido)thiazol-4-yl)-N-(3-fluoro-5-(6-(trifluoromethyl)pyrazin-2-yl)pyridin-2-yl)-2-methylpropanamide 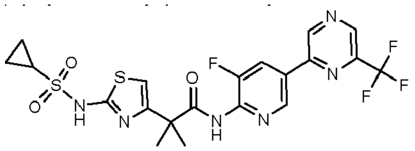 | Method 1b using INTB37 and INTA58, [UPLC acidic], 531, (1.25) | 12.66 (s, 1H), 10.20 (s, 1H), 9.73 (s, 1H), 9.24 (s, 1H), 9.11 - 9.07 (m, 1H), 8.57 - 8.50 (m, 1H), 6.59 (s, 1H), 2.61 - 2.57 (m, 1H), 1.61 (s, 6H), 0.94 - 0.90 (m, 4H). |
| **T113** | N-(5-(6-cyanopyrazin-2-yl)-3-fluoropyridin-2-yl)-2-(2-(cyclopropanesulfonamido)thiazol-4-yl)-2-methylpropanamide 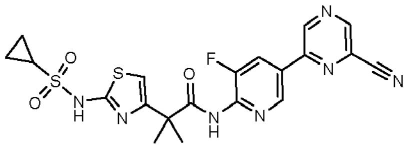 | Method 1b using INTB37 and INTA59, [UPLC acidic], 488, (1.07) | 12.66 (s, 1H), 10.19 (s, 1H), 9.68 (s, 1H), 9.26 (s, 1H), 9.08 (s, 1H), 8.58 - 8.51 (m, 1H), 6.59 (s, 1H), 2.61 - 2.57 (m, 1H), 1.61 (s, 6H), 0.94 - 0.90 (m, 4H). |
| **T114** | 2-(2-(cyclopropanesulfonamido)thiazol-4-yl)-2-methyl-N-(5'-(2,2,2-trifluoroethoxy)-[3,3'-bipyridin]-6-yl) propanamide 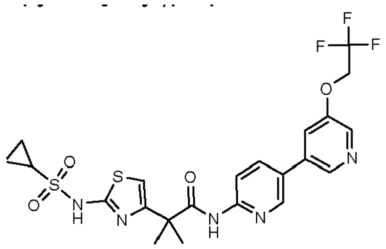 | Method 1b using INTB37 and INTA62, [HPLC acidic], 542, (1.94) | 12.56 (s, 1H), 10.05 (s, 1H), 8.81 (dd, J = 2.5, 0.8 Hz, 1H), 8.68 (d, J = 1.8 Hz, 1H), 8.42 (d, J = 2.8 Hz, 1 H), 8.27 (dd, J = 8.8, 2.5 Hz, 1H), 8.16 (dd, J = 8.8, 0.8 Hz, 1H), 7.92 (dd, J = 2.8, 1.8 Hz, 1H), 6.60 (s, 1H), 4.99 (q, J = 8.9 Hz, 2H), 2.66 - 2.54 (m, 1H), 1.62 (s, 6H), 0.98 - 0.81 (m, 4H). |
| **T115** | 2-(2-(cyclopropanesulfonamido)thiazol-4-yl)-N-(5'-(difluoromethoxy)-[3,3'-bipyridin]-6-yl)-2-methylpropanamide 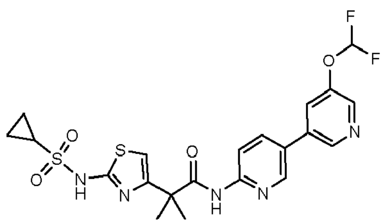 | Method 1b using INTB37 and INTA35, [UPLC acidic], 510, (1.2) | 12.57 (s, 1H), 10.08 (s, 1H), 8.87 (d, J = 1.9 Hz, 1H), 8.80 (d, J = 2.5 Hz, 1H), 8.50 (d, J = 2.6 Hz, 1H), 8.27 (dd, J = 8.7, 2.6 Hz, 1H), 8.15 (dd, J = 8.8, 0.8 Hz, 1H), 8.07 - 8.03 (m, 1H), 7.42 (t, J = 73.4 Hz, 1H), 6.57 (s, 1H), 2.60 - 2.56 (m, 1H), 1.60 (s, 6H), 0.92 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T116** | N-([2,3'-bipyridin]-5-yl)-2-(2-(cyclopropanesulfonamido)thiazol-4-yl)-2-methylpropanamide 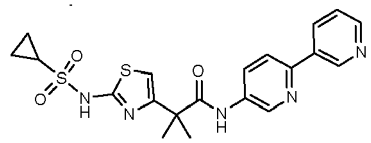 | Method 1b using INTB37, [UPLC acidic], 444, (0.7) | 12.57 (s, 1H), 9.67 (s, 1H), 9.24 (dd, J = 2.5, 0.9 Hz, 1H), 8.92 (d, J = 2.5 Hz, 1H), 8.60 (dd, J = 4.8, 1.6 Hz, 1H), 8.41 (dt, J = 8.0, 1.9 Hz, 1H), 8.18 (dd, J = 8.7, 2.5 Hz, 1H), 8.06 (d, J = 8.7 Hz, 1H), 7.50 (ddd, J = 8.0, 4.8, 0.9 Hz, 1H), 6.60 (s, 1H), 2.66 - 2.55 (m, 1H), 1.59 (s, 6H), 0.96 - 0.85 (m, 4H). |
| **T117** | 2-(2-(cyclopropanesulfonamido)thiazol-4-yl)-2-methyl-N-(6-(pyrimidin-5-yl)pyridin-3-yl)propanamide 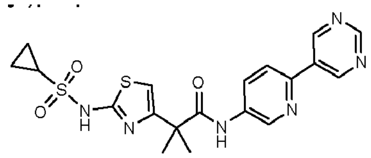 | Method 1b using INTB37, [UPLC acidic], 445, (0.89) | 12.58 (s, 1H), 9.71 (s, 1H), 9.40 (s, 2H), 9.20 (s, 1H), 8.95 (d, J = 2.5 Hz, 1H), 8.20 (dd, J = 8.6, 2.5 Hz, 1H), 8.13 (d, J = 8.6 Hz, 1H), 6.58 (s, 1H), 2.59 (s, 1H), 1.58 (s, 6H), 0.93 - 0.85 (m, 4H). |
| **T118** | 2-(2-(cyclopropanesulfonamido)thiazol-4-yl)-N-(4-(5-(difluoromethoxy)pyridin-3-yl)phenyl)-2-methylpropanamide 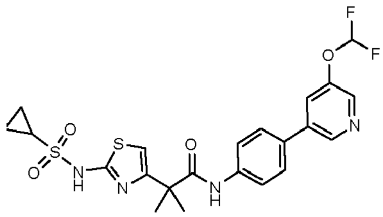 | Method 1b using INTB37 and INTA25, [UPLC acidic], 509.3, (1.24) | 12.58 (s, 1H), 9.45 (s, 1H), 8.81 (d, J = 1.9 Hz, 1H), 8.45 (d, J = 2.6 Hz, 1H), 7.94 (dd, J = 2.3 Hz, 1H), 7.78 (s, 4H), 7.43 (t, J = 73.5 Hz, 1H), 6.55 (s, 1H), 2.62 - 2.57 (m, 1H), 1.58 (s, 6H), 0.94 - 0.87 (m, 4H). |
| **T119** | 2-(2-(cyclopropanesulfonamido)thiazol-4-yl)-2-ethyl-N-(4-(6-methoxypyrazin-2-yl)phenyl)butanamide 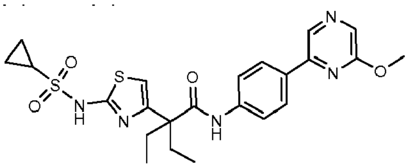 | Method 1b using INTB41 and INTA2, [UPLC acidic], 502, (1.4) | 12.50 (s, 1H), 9.42 (s, 1H), 8.79 (s, 1H), 8.22 (s, 1H), 8.16 - 8.08 (m, 2H), 7.84 - 7.75 (m, 2H), 6.63 (s, 1H), 4.02 (s, 3H), 2.61 - 2.56 (m, 1H), 2.11 - 1.92 (m, 4H), 0.94 - 0.86 (m, 4H), 0.74 (t, J = 7.3 Hz, 6H). |
| **T120** | N-(4-(5-chloropyridin-3-yl)phenyl)-2-(2-(cyclopropanesulfonamido)thiazol-4-yl)-2-ethylbutanamide 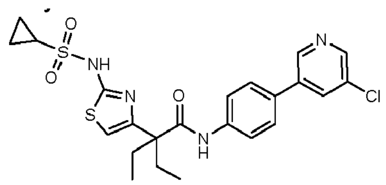 | Method 1b using INTB41 and INTA18, [HPLC basic], 506, (1.9) | 12.49 (s, 1H), 9.38 (s, 1H), 8.87 (d, J = 2.0 Hz, 1H), 8.59 (d, J = 2.3 Hz, 1H), 8.24 (dd, J = 2.2 Hz, 1H), 7.78 (s, 4H), 6.63 (s, 1H), 2.63-2.54 (m, 1H), 2.11-1.91(m, 4H), 0.97 - 0.86 (m, 4H), 0.74 (t, J = 7.4 Hz, 6H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T121** | 2-(2-(cyclopropanesulfonamido)thiazol-4-yl)-2-ethyl-N-(2-fluoro-4-(pyridin-3-yl)phenyl)butanamide 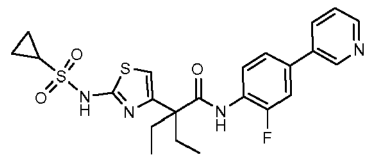 | Method 1b using INTB41, [HPLC basic], 489, (1.7) | 12.46 (s, 1H), 9.21 (s (br), 1H), 8.95 (d, J = 2.4 Hz, 1H), 8.64 - 8.56 (m, 1H), 8.13 (d, J = 8.1 Hz, 1H), 7.71 (d, J = 11.7 Hz, 1H), 7.66-7.56 (m, 2H), 7.50 (dd, J = 8.0, 4.8 Hz, 1H), 6.68 (s, 1H), 2.64 - 2.54 (m, 1H), 2.07 - 1.94 (m, 4H), 0.96 - 0.84 (m, 4H), 0.77 (t, J = 7.3 Hz, 6H). |
| **T122** | 2-(2-(cyclopropanesulfonamido)thiazol-4-yl)-2-ethyl-N-(4-(pyrazin-2-yl)phenyl)butanamide 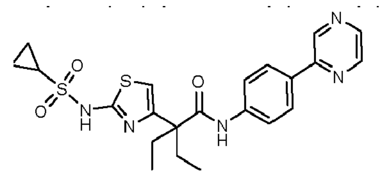 | Method 1b using INTB41, [HPLC acidic], 472, (1.88) | 12.50 (s, 1H), 9.42 (s, 1H), 9.23 (d, J = 1.5 Hz, 1H), 8.68 (dd, J = 2.5, 1.5 Hz, 1H), 8.57 (d, J = 2.5 Hz, 1H), 8.18 - 8.08 (m, 2H), 7.86-7.76 (m, 2H), 6.64 (s, 1H), 2.64 - 2.54 (m, 1H), 2.11 - 1.93 (m, 4H), 0.96 - 0.85 (m, 4H), 0.74 (t, J = 7.3 Hz, 6H). |
| **T123** | 2-(2-(cyclopropanesulfonamido)thiazol-4-yl)-2-ethyl-N-(4-(6-propoxypyrazin-2-yl)phenyl)butanamide 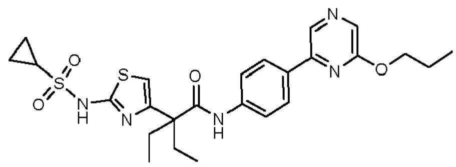 | Method 1b using INTB41 and INTA42, [HPLC acidic], 530, (2.44) | 12.49 (s, 1H), 9.41 (s, 1H), 8.77 (s, 1H), 8.19 (s, 1H), 8.17 - 8.02 (m, 2H), 7.84 - 7.70 (m, 2H), 6.62 (s, 1H), 4.38 (t, J = 6.6 Hz, 2H), 2.62 - 2.54 (m, 1H), 2.01 (hept, J = 7.2 Hz, 4H), 1.80 (h, J = 7.1 Hz, 2H), 1.01 (t, J = 7.4 Hz, 3H), 0.89 (d, J = 8.2 Hz, 4H), 0.73 (t, J = 7.4 Hz, 6H). |
| **T124** | 2-(2-(cyclopropanesulfonamido)thiazol-4-yl)-N-(2-fluoro-4-(6-(trifluoromethyl)pyrazin-2-yl)phenyl)-2-methylpropanamide 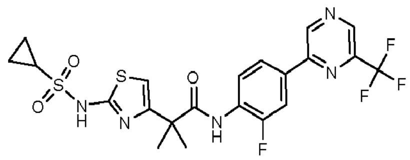 | Method 1b using INTB37 and INTA48, [HPLC basic], 530, (1.93) | 12.60 (s, 1H), 9.66 (s, 1H), 9.39 (s, 1H), 9.16 (s, 1H), 8.13 (dd, J = 11.7, 2.0 Hz, 1H), 8.08 (dd, J = 8.4, 2.0 Hz, 1H), 7.86 (s, 1H), 6.60 (s, 1H), 2.64 - 2.54 (m, 1H), 1.59 (s, 6H), 0.99 - 0.77 (m, 4H). |
| **T125** | 2-(2-(cyclopropanesulfonamido)thiazol-4-yl)-N-(4-(5-ethoxypyridin-3-yl)-2-fluorophenyl)-2-methylpropanamide 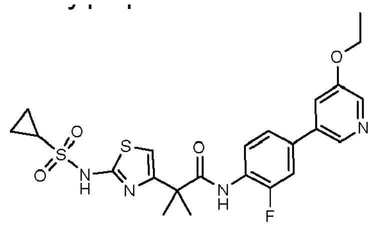 | Method 1b using INTB37 and INTA28, [HPLC basic], 505, (1.75) | 12.57 (s, 1H), 9.29 (s, 1H), 8.52 (d, J = 1.9 Hz, 1H), 8.28 (d, J = 2.7 Hz, 1H), 7.82 - 7.51 (m, 4H), 6.57 (s, 1H), 4.21 (q, J = 7.0 Hz, 2H), 2.61 - 2.53 (m, 1H), 1.57 (s, 6H), 1.38 (t, J = 7.0 Hz, 3H), 0.95 - 0.86 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T126 | 2-(2-(cyclopropanesulfonamido)thiazol-4-yl)-N-(2-fluoro-4-(5-fluoropyridin-3-yl)phenyl)-2-methylpropanamide 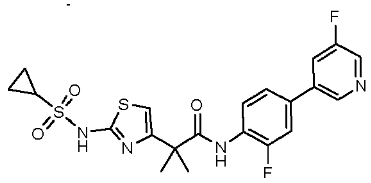 | Method 1b using INTB37 and INTA29, [HPLC basic], 479, (1.65) | 12.59 (s, 1H), 9.27 (s, 1H), 8.87 (t, J = 1.9 Hz, 1H), 8.59 (d, J = 2.7 Hz, 1H), 8.18 - 8.12 (m, 1H), 7.85 - 7.78 (m, 1H), 7.68 (s, 2H), 6.61 (s, 1H), 2.66 - 2.55 (m, 1H), 1.58 (s, 6H), 0.94 - 0.88 (m, 4H). |
| T127 | 2-(2-(cyclopropanesulfonamido)thiazol-4-yl)-2-methyl-N-(4-(6-(2,2,2-trifluoroethoxy)pyrazin-2-yl)phenyl) propanamide 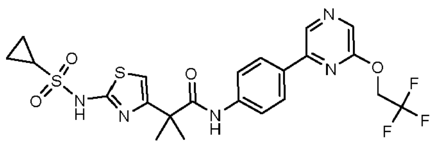 | Method 1b using INTB37 and INTA53, [HPLC basic], 542, (1.93) | 12.59 (s, 1H), 9.52 (s, 1H), 8.95 (s, 1H), 8.39 (s, 1H), 8.25 - 8.11 (m, 2H), 7.88 - 7.74 (m, 2H), 6.55 (s, 1H), 5.20 (q, J = 9.0 Hz, 2H), 2.64 - 2.55 (m, 1H), 1.59 (s, 6H), 0.94 - 0.83 (m, 4H). |
| T128 | N-(4-(5-chloropyridin-3-yl)-2-fluorophenyl)-2-(2-(cyclopropanesulfonamido)thiazol-4-yl)-2-methylpropanamide 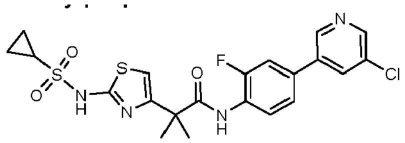 | Method 1b using INTB37 and INTA27, [HPLC acidic], 495, (2.03) | 12.60 (s, 1H), 9.25 (s, 1H), 8.93 (d, J = 2.0 Hz, 1H), 8.64 (d, J = 2.3 Hz, 1H), 8.33 (t, J = 2.2 Hz, 1H), 7.89 - 7.76 (m, 1H), 7.68 (s, 2H), 6.60 (s, 1H), 2.63 - 2.56 (m, 1H), 1.58 (s, 6H), 1.01 - 0.81 (m, 4H). |
| T129 | N-(4-(5-cyanopyridin-3-yl)-2-fluorophenyl)-2-(2-(cyclopropanesulfonamido)thiazol-4-yl)-2-methylpropanamide 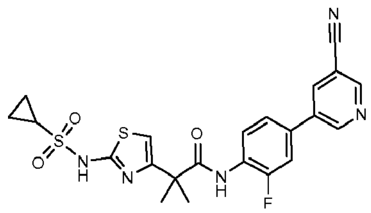 | Method 1b using INTB37 and INTA26, [HPLC acidic], 486, (1.85) | 12.60 (s, 1H), 9.28 (s, 1H), 9.26 (d, J = 2.3 Hz, 1H), 9.02 (d, J = 1.9 Hz, 1H), 8.73 (t, J = 2.1 Hz, 1H), 7.91 - 7.82 (m, 1H), 7.71 (s, 2H), 6.60 (s, 1H), 2.65 - 2.57 (m, 1H), 1.58 (s, 6H), 1.06 - 0.86 (m, 4H). |
| T130 | 1-(2-(cyclopropanesulfonamido)thiazol-4-yl)-N-(4-(6-methoxypyrazin-2-yl)phenyl)cyclopentane-1-carboxamide 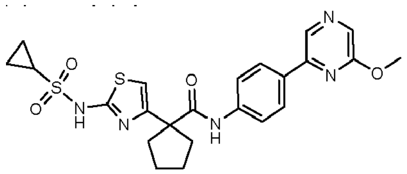 | Method 1b using INTB69 and INTA2, [HPLC acidic], 500, (2.32) | 12.57 (s, 1H), 9.51 (s, 1H), 8.79 (s, 1H), 8.22 (s, 1H), 8.16 - 8.09 (m, 2H), 7.82 - 7.75 (m, 2H), 6.64 (s, 1H), 4.02 (s, 3H), 2.64 - 2.54 (m, 1H), 2.45 - 2.35 (m, 2H), 2.13 - 2.04 (m, 2H), 1.75 - 1.61 (m, 4H), 0.96 - 0.84 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T131 | 2-(2-(cyclopropanesulfonamido)thiazol-4-yl)-N-(2-fluoro-4-(6-(2,2,2-trifluoroethoxy)pyrazin-2-yl)phenyl)-2-methylpropanamide 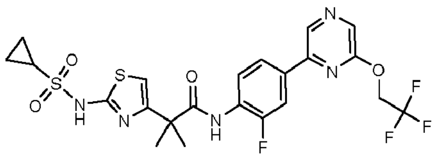 | Method 1b using INTB37 and INTA52, [UPLC basic], 560, (1.27) | 12.60 (s, 1H), 9.30 (s, 1H), 9.02 (s, 1H), 8.45 (s, 1H), 8.15 (dd, J = 12.0, 1.9 Hz, 1H), 8.06 (dd, J = 8.5, 1.9 Hz, 1H), 7.75 (s, 1H), 6.60 (s, 1H), 5.23 (q, J = 9.0 Hz, 2H), 2.64 - 2.54 (m, 1H), 1.59 (s, 6H), 0.96 - 0.83 (m, 4H). |
| T132 | 2-(2-(cyclopropanesulfonamido)thiazol-4-yl)-N-(2-fluoro-4-(5-(2,2,2-trifluoroethoxy)pyridin-3-yl)phenyl)-2-methylpropanamide 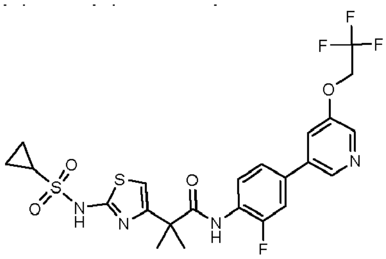 | Method 1b using INTB37 and INTA30, [HPLC acidic], 559, (2.03) | 12.58 (s, 1H), 9.24 (s, 1H), 8.66 (d, J = 1.8 Hz, 1H), 8.40 (d, J = 2.8 Hz, 1H), 7.88 (dd, J = 2.8, 1.9 Hz, 1H), 7.83 - 7.76 (m, 1H), 7.66 (s, 2H), 6.60 (s, 1H), 4.98 (q, J = 8.9 Hz, 2H), 2.65 - 2.54 (m, 1H), 1.58 (s, 6H), 0.95 - 0.84 (m, 4H). |
| T133 | 2-(2-(cyclopropanesulfonamido)thiazol-4-yl)-N-(2-fluoro-4-(5-(trifluoromethyl)pyridin-3-yl)phenyl)-2-methylpropanamide 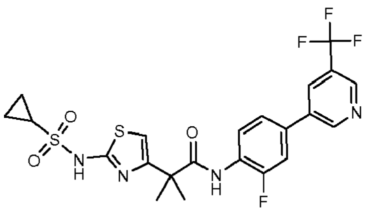 | Method 1b using INTB37 and INTA70, [HPLC acidic], 529, (2.08) | 12.61 (s, 1H), 9.27 (d, J = 2.2 Hz, 2H), 9.05 - 8.90 (m, 1H), 8.59 - 8.49 (m, 1H), 7.91 (dd, J = 12.0, 1.8 Hz, 1H), 7.75 (dd, J = 8.4, 2.0 Hz, 1H), 7.73 - 7.64 (m, 1H), 6.61 (s, 1H), 2.65 - 2.55 (m, 1H), 1.59 (s, 6H), 0.96 - 0.82 (m, 4H). |
| T134 | 2-(2-(cyclopropanesulfonamido)thiazol-4-yl)-N-(4-(6-ethoxypyrazin-2-yl)-2-fluorophenyl)-2-methylpropanamide 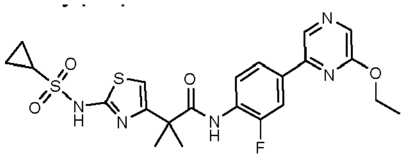 | Method 1b using INTB37 and INTA47, [HPLC acidic], 506, (2.11) | 12.61 (s, 1H), 9.28 (s, 1H), 8.86 (s, 1 H), 8.26 (s, 1H), 8.12 - 7.94 (m, 2H), 7.72 (s, 1H), 6.61 (s, 1H), 4.49 (q, J = 7.1 Hz, 2H), 2.59 (s, 1H), 1.59 (s, 6H), 1.41 (t, J = 7.0 Hz, 3H), 0.99 - 0.84 (m, 4H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T135** | 2-(2-(cyclopropanesulfonamido)thiazol-4-yl)-2-methyl-N-(4-(5-(2,2,2-trifluoroethoxy)pyridin-3-yl)phenyl) propanamide 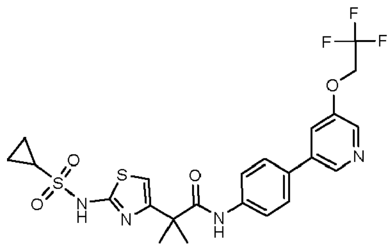 | Method 1b using INTB37 and INTA31, [HPLC acidic], 541, (1.97) | 12.56 (s, 1H), 9.45 (s, 1H), 8.61 (d, J = 1.8 Hz, 1H), 8.36 (d, J = 2.7 Hz, 1H), 7.81 (dd, J = 2.8, 1.9 Hz, 1H), 7.78 (d, J = 1.0 Hz, 4H), 6.57 (s, 1H), 4.98 (q, J = 8.9 Hz, 2H), 2.66 - 2.55 (m, 1H), 1.59 (s, 6H), 0.96 - 0.82 (m, 4H). |
| **T136** | 2-(2-(cyclopropanesulfonamido)thiazol-4-yl)-N-(4-(6-ethynylpyrazin-2-yl)phenyl)-2-methylpropanamide 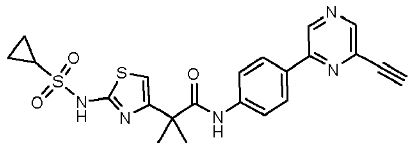 | Method 1b using INTB37 and INTA75, [UPLC acidic], 468, (1.85) | 12.59 (s, 1H), 9.53 (s, 1H), 9.24 (s, 1H), 8.70 (s, 1H), 8.19 - 8.06 (m, 2H), 7.90 - 7.75 (m, 2H), 6.56 (s, 1H), 4.69 (s, 1H), 2.59 (s, 1H), 1.59 (s, 6H), 1.01 - 0.81 (m, 4H). |
| **T137** | 2-(2-(cyclopropanesulfonamido)thiazol-4-yl)-N-(4-(6-ethoxypyrazin-2-yl)-2-methylphenyl)-2-methylpropanamide 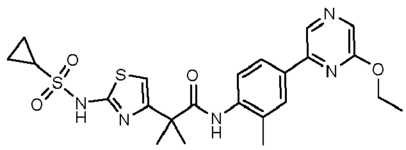 | Method 1b using INTB37 and INTA51, [HPLC acidic], 502, (1.8) | 12.62 (s, 1H), 8.96 (s, 1H), 8.80 (s, 1H), 8.21 (s, 1H), 8.01 (d, J = 2.1 Hz, 1H), 7.96 (dd, J = 8.3, 2.1 Hz, 1H), 7.44 (d, J = 8.3 Hz, 1H), 6.62 (s, 1H), 4.49 (q, J = 7.0 Hz, 2H), 2.63 - 2.54 (m, 1H), 2.25 (s, 3H), 1.60 (s, 6H), 1.41 (t, J = 7.0 Hz, 3H), 0.96 - 0.87 (m, 4H). |
| **T138** | N-(4-(6-chloropyrazin-2-yl)-2-methylphenyl)-2-(2-(cyclopropanesulfonamido)thiazol-4-yl)-2-methylpropanamide 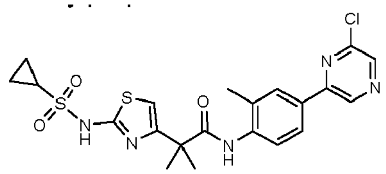 | Method 1b using INTB37 and INTA49, [HPLC acidic], 493, (1.99) | 12.61 (s, 1H), 9.28 (s, 1H), 8.99 (s, 1H), 8.73 (s, 1H), 8.05 - 8.01 (m, 1H), 7.98 (dd, J = 8.4, 2.2 Hz, 1H), 7.51 (s, 1H), 6.62 (s, 1H), 2.63 - 2.54 (m, 1H), 2.26 (s, 3H), 1.59 (s, 6H), 0.96 - 0.88 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T139 | 2-(2-(cyclopropanesulfonamido)thiazol-4-yl)-N-(4-(5-(difluoromethoxy)pyridin-3-yl)-2-fluorophenyl)-2-methylpropanamide 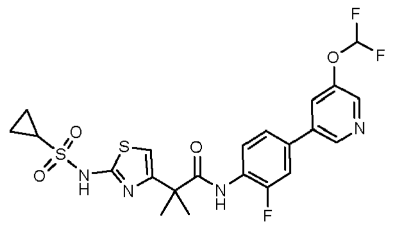 | Method 1b using INTB37 and INTA24, [UPLC acidic], 527, (1.27) | 12.60 (s, 1H), 9.24 (s, 1H), 8.86 (d, J = 1.9 Hz, 1H), 8.49 (d, J = 2.6 Hz, 1H), 8.04 - 7.99 (m, 1H), 7.83 - 7.76 (m, 1H), 7.68 - 7.64 (m, 2H), 7.43 (t, J = 73.4 Hz, 1H), 6.59 (s, 1H), 2.60 - 2.56 (m, 1H), 1.57 (s, 6H), 0.93 - 0.87 (m, 4H). |
| T140 | 2-(2-(cyclopropanesulfonamido)thiazol-4-yl)-2-methyl-N-(5-(pyrazin-2-yl)pyridin-2-yl)propanamide 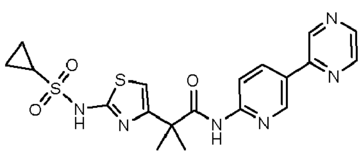 | Method 1b using INTB37, [UPLC acidic], 445, (1) | 12.58 (s, 1H), 10.14 (s, 1H), 9.33 (d, J = 1.6 Hz, 1H), 9.12 (d, J = 2.3 Hz, 1H), 8.74 (dd, J = 2.5, 1.5 Hz, 1H), 8.64 (d, J = 2.5 Hz, 1H), 8.56 (dd, J = 8.8, 2.5 Hz, 1H), 8.21 (dd, J = 8.8, 0.8 Hz, 1H), 6.59 (s, 1H), 2.66 - 2.56 (m, 1H), 1.62 (s, 6H), 1.05 - 0.79 (m, 4H). |
| T141 | N-(5-(6-cyclobutoxypyrazin-2-yl)pyridin-2-yl)-2-(2-(cyclopropanesulfonamido)thiazol-4-yl)-2-methylpropanamide 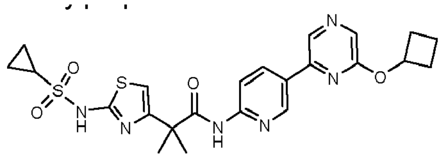 | Method 1b using INTB37 and INTA55, [HPLC basic], 515, (2) | 12.59 (s, 1H), 10.09 (s, 1H), 9.07 (d, J = 2.4 Hz, 1H), 8.87 (s, 1H), 8.51 (dd, J = 8.8, 2.4 Hz, 1H), 8.24 (s, 1H), 8.19 (dd, J = 8.8, 0.8 Hz, 1H), 6.59 (s, 1H), 5.32 - 5.22 (m, 1H), 2.66 - 2.50 (m, 1H), 2.23 - 2.10 (m, 2H), 1.92 - 1.66 (m, 2H), 1.62 (s, 6H), 0.95 - 0.80 (m, 6H). |
| T142 | 2-(2-(cyclopropanesulfonamido)thiazol-4-yl)-N-(5-(6-cyclopropoxypyrazin-2-yl)pyridin-2-yl)-2-methylpropanamide 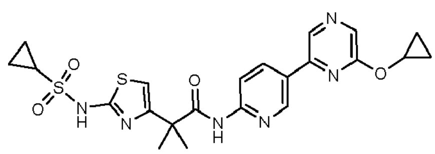 | Method 1b using INTB37 and INTA56, [HPLC basic], 501, (1.8) | 12.59 (s, 1H), 10.11 (s, 1H), 9.12 (d, J = 2.4 Hz, 1H), 8.94 (s, 1H), 8.55 (dd, J = 8.8, 2.5 Hz, 1H), 8.32 (s, 1H), 8.19 (dd, J = 8.8, 0.8 Hz, 1H), 6.59 (s, 1H), 4.43 (tt, J = 6.3, 3.0 Hz, 1H), 2.65 - 2.54 (m, 1H), 1.62 (s, 6H), 0.95 - 0.76 (m, 9H). |
| T143 | 2-(2-(cyclopropanesulfonamido)thiazol-4-yl)-N-(5-(6-isopropoxypyrazin-2-yl)pyridin-2-yl)-2-methylpropanamide 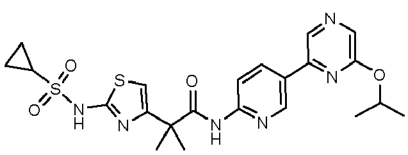 | Method 1b using INTB37 and INTA71, [HPLC basic], 503, (1.9) | 12.59 (s, 1H), 10.10 (s, 1H), 9.07 (t, J = 1.5 Hz, 1H), 8.84 (s, 1H), 8.51 (dd, J = 8.8, 2.5 Hz, 1H), 8.21 (d, J = 0.5 Hz, 1H), 8.19 (dd, J = 8.8, 0.8 Hz, 1H), 6.59 (s, 1H), 5.41 (hept, J = 6.2 Hz, 1H), 2.60 (s, 1H), 1.62 (s, 6H), 1.39 (d, J = 6.1 Hz, 6H), 0.96 - 0.85 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T144** | N-([3,3'-bipyridin]-6-yl)-2-(2-(cyclopropanesulfonamido)thiazol-4-yl)-2-ethylbutanamide 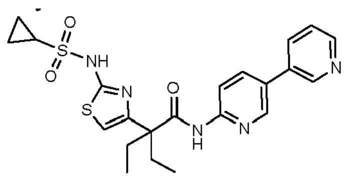 | Method 1b using INTB41, [HPLC basic], 472, (1.69) | 12.45 (s, 1H), 9.88 (s, 1H), 8.95 (dd, J = 2.4, 0.9 Hz, 1H), 8.73 (dd, J = 2.4, 0.9 Hz, 1H), 8.60 (dd, J = 4.8, 1.6 Hz, 1H), 8.29 - 8.06 (m, 3H), 7.51 (ddd, J = 8.0, 4.8, 0.9 Hz, 1H), 6.67 (s, 1H), 2.65 - 2.56 (m, 1H), 2.18 - 1.95 (m, 4H), 0.94 - 0.87 (m, 4H), 0.74 (t, J = 7.4 Hz, 6H). |
| **T145** | 2-(2-(cyclopropanesulfonamido)thiazol-4-yl)-N-(5'-ethoxy-[3,3'-bipyridin]-6-yl)-2- 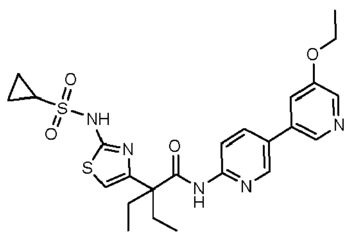 ethylbutanamide | Method 1b using INTB41 and INTA32, [HPLC basic], 516, (1.85) | 12.47 (s, 1H), 9.93 (s, 1H), 8.75 (dd, J = 2.5, 0.8 Hz, 1H), 8.55 (d, J = 1.9 Hz, 1H), 8.32 (d, J = 2.7 Hz, 1H), 8.24 (dd, J = 8.7, 2.5 Hz, 1H), 8.16 (dd, J = 8.7, 0.8 Hz, 1H), 7.73 (t, J = 2.3 Hz, 1H), 6.67 (s, 1H), 4.22 (q, J = 7.0 Hz, 2H), 2.65 - 2.56 (m, 1H), 2.17 - 1.95 (m, 4H), 1.38 (t, J = 7.0 Hz, 3H), 0.95 - 0.85 (m, 4H), 0.73 (t, J = 7.4 Hz, 6H). |
| **T146** | 2-(2-(cyclopropanesulfonamido)thiazol-4-yl)-2-methyl-N-(5'-propoxy-[3,3'-bipyridin]-6-yl)propanamide 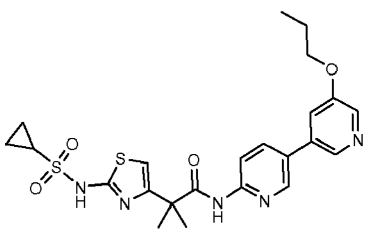 | Method 1b using INTB41 and INTA4, [HPLC basic], 502, (1.81) | 12.57 (s, 1H), 10.06 (s, 1H), 8.77 (dd, J = 2.5, 0.8 Hz, 1H), 8.54 (d, J = 1.8 Hz, 1H), 8.30 (d, J = 2.7 Hz, 1H), 8.24 (d, J = 2.6 Hz, 1H), 8.14 (dd, J = 8.7, 0.8 Hz, 1H), 7.71 (dd, J = 2.8, 1.9 Hz, 1H), 6.60 (s, 1H), 4.12 (t, J = 6.5 Hz, 2H), 2.67 - 2.55 (m, 1H), 1.86 - 1.71 (m, 2H), 1.61 (s, 6H), 1.01 (t, J = 7.4 Hz, 3H), 0.96 - 0.89 (m, 4H). |
| **T147** | 2-(2-(cyclopropanesulfonamido)thiazol-4-yl)-2-ethyl-N-(5-(6-(trifluoromethyl)pyrazin-2-yl)pyridin-2-yl) butanamide 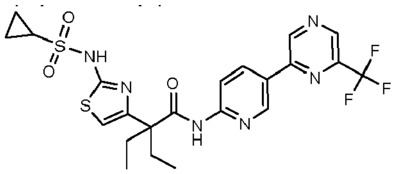 | Method 1b using INTB41 and INTA3, [HPLC basic], 541, (2.05) | 12.50 (s, 1H), 10.14 (s, 1H), 9.68 (s, 1H), 9.21 - 9.11 (m, 2H), 8.60 (dd, J = 8.9, 2.5 Hz, 1H), 8.30 - 8.24 (m, 1H), 6.66 (s, 1H), 2.63 - 2.55 (m, 1H), 2.19 - 1.92 (m, 4H), 0.95 - 0.87 (m, 4H), 0.74 (t, J = 7.4 Hz, 6H). |
| **T148** | N-([3,3'-bipyridin]-6-yl)-2-(2-(cyclopropanesulfonamido)thiazol-4-yl)-2-methylpropanamide 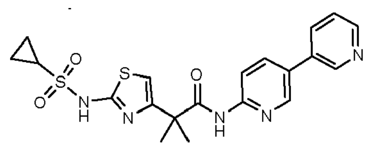 | Method 1b using INTB37, [UPLC acidic], 444, (0.76) | 12.55 (s, 1H), 10.00 (s, 1H), 8.98-8.92 (m, 1H), 8.77 - 8.71 (m, 1H), 8.59 (dd, J = 4.8, 1.6 Hz, 1H), 8.22 (dd, J = 8.8, 2.5 Hz, 1H), 8.19 - 8.11 (m, 2H), 7.51 (ddd, J = 8.0, 4.8, 0.9 Hz, 1H), 6.60 (s, 1H), 2.66 - 2.54 (m, 1H), 1.61 (s, 6H), 0.95 - 0.84 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T149 | 2-(2-(cyclopropanesulfonamido)thiazol-4-yl)-N-(2-methoxy-4-(pyridin-3-yl)phenyl)-2-methylpropanamide 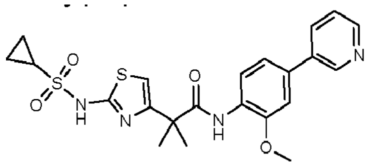 | Method 1b using INTB37 and INTA, [HPLC basic], 473, (1.6) | 12.64 (s, 1H), 8.94 (dd, J = 2.4, 0.9 Hz, 1H), 8.56 (dd, J = 4.8, 1.6 Hz, 1H), 8.42 (s, 1H), 8.11 (ddd, J = 8.0, 2.5, 1.6 Hz, 1H), 8.05 (d, J = 8.3 Hz, 1H), 7.48 (ddd, J = 8.0, 4.7, 0.9 Hz, 1H), 7.40 (d, J = 2.0 Hz, 1H), 7.33 (dd, J = 8.3, 2.0 Hz, 1H), 6.76 (s, 1H), 3.92 (s, 3H), 2.67 - 2.56 (m, 1H), 1.57 (s, 6H), 0.96 - 0.89 (m, 4H). |
| T150 | 2-(2-(cyclopropanesulfonamido)thiazol-4-yl)-N-(3-methoxy-4-(pyridin-3-yl)phenyl)-2-methylpropanamide 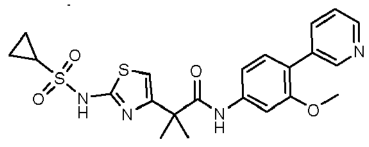 | Method 1b using INTB37 and INTA16, [HPLC basic], 473, (1.79) | 12.58 (s, 1H), 9.45 (s, 1H), 8.67 (d, J = 2.1 Hz, 1H), 8.49 (d, J = 6.7 Hz, 1H), 7.88 (ddd, J = 7.9, 1.9 Hz, 1H), 7.55 (d, J = 1.9 Hz, 1H), 7.45 - 7.37 (m, 2H), 7.32 (d, J = 8.3 Hz, 1H), 6.57 (s, 1H), 3.78 (s, 3H), 2.68 - 2.55 (m, 1H), 1.59 (s, 6H), 0.91 (m, 4H). |
| T151 | 2-(2-(cyclopropanesulfonamido)thiazol-4-yl)-N-(3-fluoro-4-(pyridin-3-yl)phenyl)-2-methylpropanamide 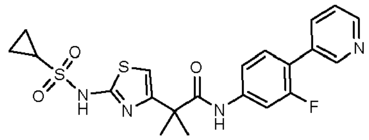 | Method 1b using INTB37, [HPLC basic], 461, (1.9) | 12.55 (s (br), 1H), 9.66 (s (br), 1H), 8.75 (m, 1H), 8.58 (dd, J = 4.8, 1.6 Hz, 1H), 7.96 (m, 1H), 7.80 - 7.74 (m, 1H), 7.61 - 7.55 (m, 2H), 7.50 (ddd, J = 8.0, 4.8, 0.9 Hz, 1H), 6.56 (s, 1H), 2.66 - 2.55 (m, 1H), 1.57 (s, 6H), 0.95 - 0.86 (m, 4H). |
| T152 | N-(3-cyano-4-(pyridin-3-yl)phenyl)-2-(2-(cyclopropanesulfonamido)thiazol-4-yl)-2-methylpropanamide 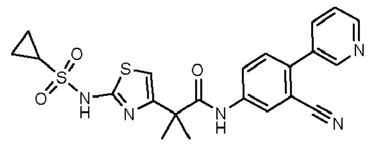 | Method 1b using INTB37 and INTA15, [HPLC basic], 468, (1.88) | 12.56 (s (br), 1H), 9.74 (s (br), 1H), 8.78 (dd, J = 2.4, 0.9 Hz, 1H), 8.68 (dd, J = 4.8, 1.6 Hz, 1H), 8.25 (d, J = 2.2 Hz, 1H), 8.07 - 7.99 (m, 2H), 7.69 (d, J = 8.6 Hz, 1H), 7.57 (ddd, J = 7.9, 4.8, 0.9 Hz, 1H), 6.61 (s, 1H), 2.66 - 2.55 (m, 1H), 1.58 (s, 6H), 0.96 - 0.86 (m, 4H). |
| T153 | N-(3-chloro-4-(pyridin-3-yl)phenyl)-2-(2-(cyclopropanesulfonamido)thiazol-4-yl)-2-methylpropanamide 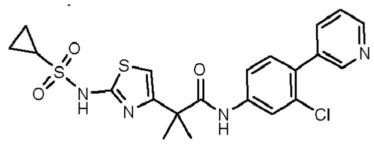 | Method 1b using INTB37 and INTA17, [UPLC basic], 477, (1.04) | 10.87 (s (br), 1H), 8.62 (dd, J = 2.4, 0.9 Hz, 1H), 8.59 (dd, J = 4.8, 1.6 Hz, 1H), 8.08 (m, 1H), 7.87 (ddd, J = 7.9, 2.4, 1.6 Hz, 1H), 7.77 (dd, J = 8.4, 2.1 Hz, 1H), 7.49 (ddd, J = 7.9, 4.8, 0.9 Hz, 1H), 7.41 (d, J = 8.4, 1H), 6.32 (s, 1H), 2.64 - 2.59 (m, 1H), 1.50 (s, 6H), 0.94 - 0.85 (m, 2H), 0.81 - 0.70 (m, 2H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T154** | N-(4-(6-cyanopyrazin-2-yl)phenyl)-2-(2-(cyclopropanesulfonamido)thiazol-4-yl)-2-methylpropanamide 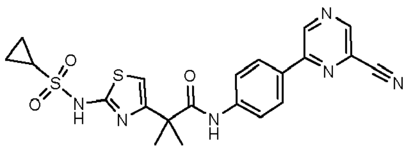 | Method 1b using INTB37 and INTA44, [HPLC basic], 469, (1.7) | 12.56 (s, 1H), 10.07 - 9.57 (bs, 1H), 9.55 (s, H), 9.11 (s, 1H), 8.28 - 8.10 (m, 2H), 8.01 - 7.77 (m, 2H), 6.52 (s, 1H), 2.65 - 2.57 (m, 1H), 1.56 (s, 6H), 0.98 - 0.65 (m, 4H). |
| **T155** | N-(4-(6-chloropyrazin-2-yl)phenyl)-2-(2-(cyclopropanesulfonamido)thiazol-4-yl)-2-methylpropanamide 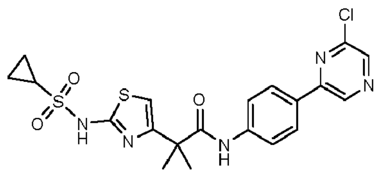 | Method 1b using INTB37 and INTA43, [HPLC basic], 478, (1.8) | 12.56 (s, 1H), 9.26 (s, 1H), 8.69 (s, 1H), 8.22 - 8.04 (m, 2H), 7.94 - 7.75 (m, 2H), 6.53 (s, 1H), 2.71 - 2.55 (m, 1H), 1.57 (s, 7H), 0.98 - 0.79 (m, 4H). |
| **T156** | 2-(2-(cyclopropanesulfonamido)thiazol-4-yl)-2-ethyl-N-(4-(5-fluoropyridin-3-yl)phenyl)butanamide 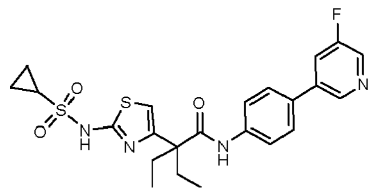 | Method 1b using INTB41 and INTA13, [HPLC basic], 489, (1.8) | 12.49 (s, 1H), 9.38 (s, 1H), 8.81 (dd, J = 1.9 Hz, 1H), 8.54 (d, J = 2.7 Hz, 1H), 8.05 (ddd, J = 10.5, 2.8, 1.8 Hz, 1H), 7.78 (s, 4H), 6.63 (s (br), 1H), 2.64-2.65 (m, 1H), 2.11-1.93 (m, 4H), 0.95-0.85 (m, 4H), 0.74 (t, J = 7.4 Hz, 6H) |
| **T157** | 2-(2-(cyclopropanesulfonamido)thiazol-4-yl)-2-methyl-N-(4-(5-propoxypyridin-3-yl)phenyl)propanamide 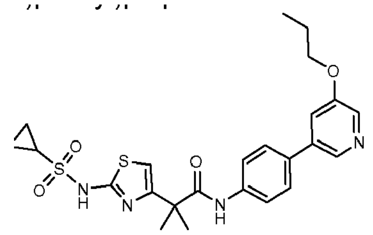 | Method 1b using INTB37, [HPLC basic], 501, (1.85) | 12.58 (s, 1H), 9.45 (s, 1H), 8.48 (d, J = 1.8 Hz, 1H), 8.24 (d, J = 2.7 Hz, 1H), 7.75 (s, 4H), 7.61 (dd, J = 2.7, 1.9 Hz, 1H), 6.56 (s, 1H), 4.10 (t, J = 6.5 Hz, 2H), 2.65 - 2.54 (m, 1H), 1.85 - 1.71 (m, 2H), 1.58 (s, 6H), 1.01 (t, J = 7.4 Hz, 3H), 0.95 - 0.86 (m, 4H). |
| **T158** | 2-(2-(cyclopropanesulfonamido)thiazol-4-yl)-N-(4-(5-isopropoxypyridin-3-yl)phenyl)-2-methylpropanamide 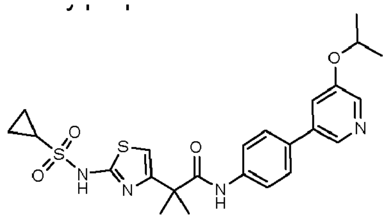 | Method 1b using INTB37 and INTA22, [HPLC basic], 501, (1.79) | 12.55 (s, 1H), 9.42 (s, 1H), 8.46 (d, J = 1.9 Hz, 1H), 8.21 (d, J = 2.7 Hz, 1H), 7.84 - 7.67 (m, 4H), 7.59 (dd, J = 2.3 Hz, 1H), 6.56 (s, 1H), 4.92 - 4.78 (m, 1H), 2.65 - 2.56 (m, 1H), 1.58 (s, 6H), 1.32 (d, J = 6.0 Hz, 6H), 0.96 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T159** | 2-(2-(cyclopropanesulfonamido)thiazol-4-yl)-N-(2-fluoro-4-(5-isopropoxypyridin-3-yl)phenyl)-2-methylpropanamide 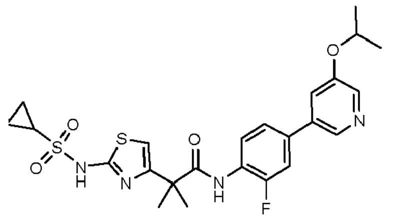 | Method 1b using INTB37 and INTA23, [HPLC basic], 519, (1.82) | 12.58 (s, 1H), 9.22 (s, 1H), 8.51 (d, J = 1.9 Hz, 1H), 8.26 (d, J = 2.7 Hz, 1H), 7.81 - 7.71 (m, 1H), 7.69 - 7.66 (m, 1H), 7.65 - 7.59 (m, 2H), 6.62 (s, 1H), 4.93 - 4.82 (m, 1H), 2.64 - 2.56 (m, 1H), 1.59 (s, 6H), 1.32 (d, J = 6.0 Hz, 6H), 0.96 - 0.87 (m, 4H). |
| **T160** | N-(4-(6-chloropyrazin-2-yl)phenyl)-2-(2-(cyclopropanesulfonamido)thiazol-4-yl)-2-ethylbutanamide 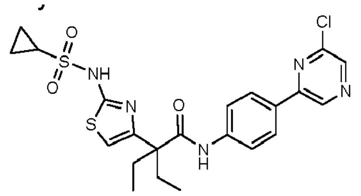 | Method 1b using INTB41 and INTA43, [HPLC basic], 507, (1.94) | 12.51 (s, 1H), 9.47 (s, 1H), 9.25 (s, 1H), 8.70 (s, 1H), 8.13 (d, J = 8.8 Hz, 2H), 7.88 - 7.76 (m, 2H), 6.64 (s, 1H), 2.64 - 2.54 (m, 1H), 2.15 - 1.90 (m, 4H), 0.95 - 0.84 (m, 4H), 0.74 (t, J = 7.4 Hz, 6H). |
| **T161** | N-(4-(6-cyanopyrazin-2-yl)phenyl)-2-(2-(cyclopropanesulfonamido)thiazol-4-yl)-2-ethylbutanamide 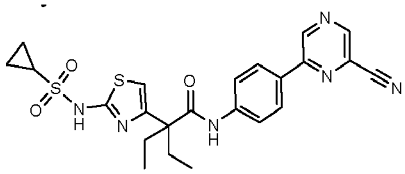 | Method 1b using INTB41 and INTA44, [HPLC basic], 497, (1.87) | 12.51 (s, 1H), 9.55 (s, 1H), 9.49 (s, 1H), 9.12 (s, 1H), 8.27-8.11 (m, 2H), 7.93 - 7.75 (m, 2H), 6.64 (s, 1H), 2.65 - 2.54 (m, 1H), 2.16 - 1.87 (m, 4H), 0.96 - 0.76 (m, 4H), 0.74 (t, J = 7.3 Hz, 6H). |
| **T162** | 4-(6-chloropyrazin-2-yl)-N-(1-(2-(cyclopropanesulfonamido)thiazol-4-yl) propyl)benzamide 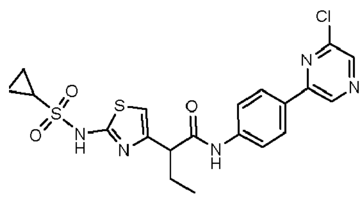 | Method 1b using INTB38 and INTA43, [HPLC acidic], 478, (1.91) | 12.60 (s, 1H), 9.38 (d, J = 0.6 Hz, 1H), 8.82 (s, 1H), 8.76 (d, J = 8.2 Hz, 1H), 8.31 - 8.20 (m, 2H), 8.07 (d, J = 8.4 Hz, 2H), 6.55 (s, 1H), 4.90 (q, J = 8.5, 7.9 Hz, 1H), 2.62 - 2.52 (m, 1H), 2.00 - 1.60 (m, 2H), 0.97 - 0.81 (m, 7H). |
| **T163** | 2-methyl-2-(2-(methylsulfonamido)thiazol-4-yl)-N-(4-(pyridin-3-yl)phenyl)propanamide 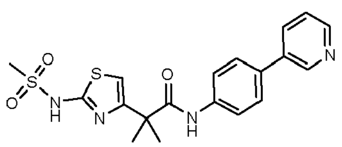 | Method 1b using INTB37, [HPLC acidic], 417, (1.06) | 12.52 (s, 1H), 9.43 (s, 1H), 8.89 (dd, J = 2.4, 0.9 Hz, 1H), 8.53 (dd, J = 4.8, 1.6 Hz, 1H), 8.06 (ddd, J = 8.0, 2.4, 1.6 Hz, 1H), 7.80 - 7.67 (m, 4H), 7.46 (ddd, J = 8.1, 4.8, 0.9 Hz, 1H), 6.58 (s, 1H), 2.91 (s, 3H), 1.57 (s, 6H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T164** | 2-(2-(cyclopropanesulfonamido)thiazol-4-yl)-N,2-dimethyl-N-(4-(pyridin-3-yl)phenyl)propanamide 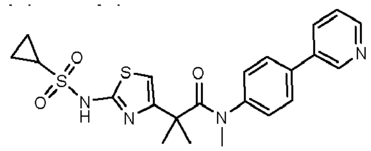 | Method 1b using INTB37 and INTA68, [UPLC acidic], 457, (0.74) | 12.53 (s, 1H), 8.85 (dd, J = 2.5, 0.8 Hz, 1H), 8.60 (dd, J = 4.8, 1.6 Hz, 1H), 8.03 (ddd, J = 8.0, 2.4, 1.6 Hz, 1H), 7.62 - 7.54 (m, 2H), 7.51 (ddd, J = 7.9, 4.8, 0.9 Hz, 1H), 7.23 - 7.15 (m, 2H), 5.77 (s, 1H), 3.15 (s, 3H), 2.66 - 2.55 (m, 1H), 1.42 (s, 6H), 0.92 (d, J = 6.3 Hz, 4H). |
| **T165** | **Single enantiomer - stereochemistry unassigned** N-(1-(2-(cyclopropanesulfonamido)thiazol-4-yl) propyl)-4-(5-(trifluoromethyl)pyridin-3-yl)benzamide 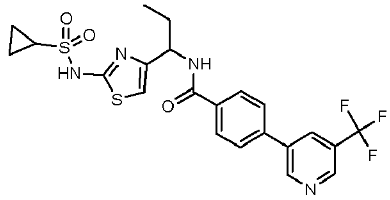 | Method 1b using INTB38 and INTA14, Chiral IC (16.8), [UPLC acidic], 511, (1.3) | 12.61 (s, 1H), 9.30 (s, 1H), 9.03 (s, 1H), 8.75 (d, J = 8.3 Hz, 1H), 8.56 (s, 1H), 8.09 - 8.04 (m, 2H), 8.03 - 7.98 (m, 2H), 6.57 (s, 1H), 4.94 - 4.87 (m, 1H), 2.54 - 2.51 (m, 1H), 1.97 - 1.74 (m, 2H), 0.96 - 0.85 (m, 7H). |
| **T166** | **Single enantiomer - stereochemistry unassigned** N-(1-(2-(cyclopropanesulfonamido)thiazol-4-yl) propyl)-4-(5-(trifluoromethyl)pyridin-3-yl)benzamide 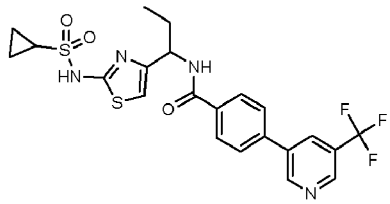 | Method 1b using INTB38 and INTA14, Chiral IC (16.8), [UPLC acidic], 511, (1.3) | 12.61 (s, 1H), 9.30 (d, J = 2.2 Hz, 1H), 9.03 (d, J = 2.1 Hz, 1H), 8.75 (d, J = 8.1 Hz, 1H), 8.56 (s, 1H), 8.08 - 8.04 (m, 2H), 8.03 - 7.99 (m, 2H), 6.57 (s, 1H), 4.95 - 4.85 (m, 1H), 2.63 - 2.55 (m, 1H), 1.97 - 1.73 (m, 2H), 0.97 - 0.82 (m, 7H). |
| **T167** | **Single enantiomer - stereochemistry unassigned** N-(1-(2-(cyclopropanesulfonamido)thiazol-4-yl) propyl)-4-(5-fluoropyridin-3-yl)benzamide 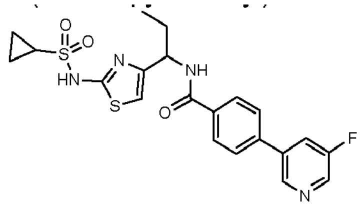 | Method 1b using INTB38 and INTA13, Chiral IC (21.4), [UPLC acidic], 461, (1.13) | 12.60 (s, 1H), 8.89 (t, J = 1.8 Hz, 1H), 8.72 (d, J = 8.3 Hz, 1H), 8.64 (d, J = 2.7 Hz, 1H), 8.21 - 8.16 (m, 1H), 8.08 - 8.02 (m, 2H), 7.99 - 7.92 (m, 2H), 6.56 (s, 1H), 4.90 (q, J = 8.2 Hz, 1H), 2.64 - 2.55 (m, 1H), 1.97 - 1.72 (m, 2H), 0.97-0.76 (m, 7H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T168 | **Single enantiomer - stereochemistry unassigned** N-(1-(2-(cyclopropanesulfonamido)thiazol-4-yl) propyl)-4-(5-fluoropyridin-3-yl)benzamide 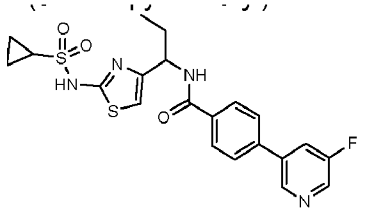 | Method 1b using INTB38 and INTA13, Chiral IC (27.5), [UPLC acidic], 461, (1.13) | 12.60 (s, 1H), 8.89 (t, J = 1.8 Hz, 1H), 8.72 (d, J = 8.3 Hz, 1H), 8.64 (d, J = 2.7 Hz, 1H), 8.21 - 8.16 (m, 1H), 8.08 - 8.02 (m, 2H), 7.99 - 7.92 (m, 2H), 6.56 (s, 1H), 4.90 (q, J = 8.2 Hz, 1H), 2.64 - 2.55 (m, 1H), 1.97 - 1.72 (m, 2H), 0.97 - 0.76 (m, 7H). |
| T169 | 2-(cyclopropanesulfonamido)-N-(5-(6-(trifluoromethyl) pyrazin-2-yl)pyridin-2-yl)-5,6-dihydro-4H-cyclopenta[d] thiazole-4- 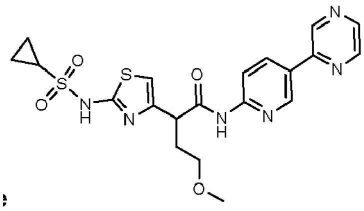 carboxamide | Method 1c using INTB44, [UPLC acidic], 511, (1.3) | 12.52 (s, 1H), 11.08 (s, 1H), 9.67 (s, 1H), 9.21 - 9.14 (m, 2H), 8.60 (dd, J = 8.8, 2.5 Hz, 1H), 8.28 (d, J = 8.8 Hz, 1H), 4.18 (s, 1H), 2.86 - 2.64 (m, 3H), 2.60 (s, 2H), 0.94 - 0.89 (m, 4H). |
| T170 | 2-(2-(cyclopropanesulfonamido)thiazol-4-yl)-4-methoxy-N-(5-(pyrazin-2-yl)pyridin-2- 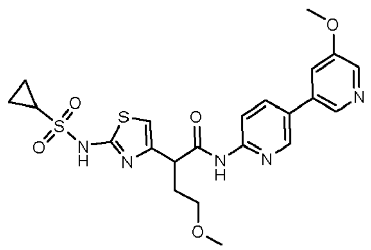 yl)butanamide | Method 1c using INTB44 and INTA, [HPLC acidic], 475, (1.55) | 12.63 (s, 1H), 10.95 (s, 1H), 9.32 (d, J = 1.6 Hz, 1H), 9.12 (d, J = 2.4 Hz, 1H), 8.74 (dd, J = 2.6, 1.5 Hz, 1H), 8.64 (d, J = 2.5 Hz, 1H), 8.55 (dd, J = 8.8, 2.5 Hz, 1H), 8.24 (d, J = 8.8 Hz, 1H), 6.58 (s, 1H), 4.05 - 3.97 (m, 1H), 3.21 (s, 3H), 2.64 - 2.56 (m, 1H), 2.32 - 2.19 (m, 1H), 2.14 - 2.01 (m, 1H), 0.95 - 0.79 (m, 4H), CH2 at 3.3 obscured by residual water |
| T171 | 2-(2-(cyclopropanesulfonamido)thiazol-4-yl)-4-methoxy-N-(5'-methoxy-[3,3'-bipyridin]-6-yl) butanamide 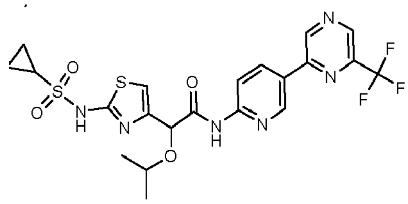 | Method 1c using INTB13 and INTA3, [HPLC acidic], 504, (1.45) | (Chloroform-d) 9.69 (s, 1H), 8.52 (d, J = 2.4 Hz, 1H), 8.45 (s, 1H), 8.39 - 8.33 (m, 1H), 8.26 (d, J = 8.7 Hz, 1H), 7.90 (dd, J = 8.7, 2.5 Hz, 1H), 7.41 - 7.35 (m, 1H), 6.42 (s, 1H), 4.21 (t, J = 7.5 Hz, 1H), 3.94 (s, 3H), 3.43 (tt, J = 6.4, 5.2, 2.4 Hz, 2H), 3.29 (s, 3H), 2.62 (tt, J = 8.1, 4.9 Hz, 1H), 2.44 - 2.31 (m, 1H), 2.09 (ddd, J = 14.4, 6.8, 5.3 Hz, 1H), 1.32 - 1.19 (m, 2H), 1.00-0.88 (m, 2H), N-H sulfonamide not observed. |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T172 | 2-(2-(cyclopropanesulfonamido)thiazol-4-yl)-2-isopropoxy-N-(5-(6-(trifluoromethyl)pyrazin-2-yl) pyridin-2-yl)acetamide 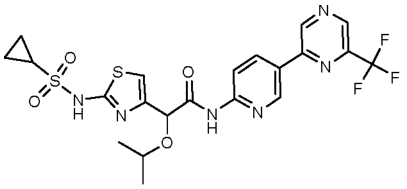 | Method 1c using INTB13 and INTA3, [UPLC acidic], 543, (1.43) | 9.29 (s, 1H), 9.25 (s, 1H), 9.04 (d, J = 2.3 Hz, 1H), 8.92 (s, 1H), 8.46 (dd, J = 8.8, 2.4 Hz, 1H), 8.36 (d, J = 8.7 Hz, 1H), 6.50 (d, J = 1.2 Hz, 1H), 5.13 (d, J = 1.3 Hz, 1H), 4.01 3.88 (m, 1H), 2.69 - 2.57 (m, 1H), 1.38 (d, J = 6.1 Hz, 3H), 1.35 (d, J = 6.1 Hz, 3H), 1.29 - 1.20 (m, 2H), 1.00 - 0.85 (m, 2H), N-H not observed. |
| T173 | 2-(2-(cyclopropanesulfonamido)thiazol-4-yl)-N-(5-(6-propoxypyrazin-2-yl)pyridin-2-yl)butanamide 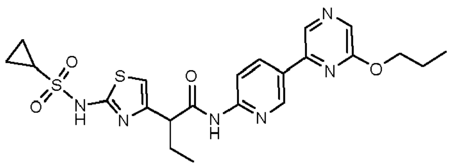 | Method 1c using INTB38 and INTA4, [HPLC basic], 503, (1.92) | 12.59 (s, 1H), 10.95 (s, 1H), 9.09 (dd, J = 2.4, 0.8 Hz, 1H), 8.86 (s, 1H), 8.52 (dd, J = 8.7, 2.4 Hz, 1H), 8.27 (s, 1H), 8.24 (d, J = 8.8 Hz, 1H), 6.56 (s, 1H), 4.40 (t, J = 6.6 Hz, 2H), 3.87 - 3.77 (m, 1H), 2.64 - 2.55 (m, 1H), 2.05 - 1.85 (m, 2H), 1.85 - 1.75 (m, 2H), 1.02 (t, J = 7.4 Hz, 3H), 0.94 - 0.86 (m, 7H). |
| T174 | 2-(2-(cyclopropanesulfonamido)thiazol-4-yl)-N-(5-(6-isopropoxypyrazin-2-yl)pyridin-2-yl)butanamide 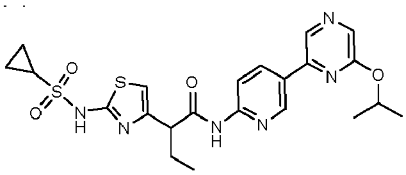 | Method 1c using INTB38 and INTA71, [UPLC acidic], 503, (1.41) | 12.59 (s, 1H), 10.93 (s, 1H), 9.07 (d, J = 2.4 Hz, 1H), 8.82 (s, 1H), 8.49 (dd, J = 8.8, 2.5 Hz, 1H), 8.23 (d, J = 8.9 Hz, 1H), 8.20 (s, 1H), 6.55 (s, 1H), 5.49 - 5.36 (m, 1H), 3.89 - 3.74 (m, 1H), 2.60 2.50 (m, 1H), 2.05 - 1.81 (m, 2H), 1.38 (d, J = 6.2 Hz, 6H), 0.95 - 0.80 (m, 7H). |
| T175 | 2-(2-(cyclopropanesulfonamido)thiazol-4-yl)-N-(5-(6-(trifluoromethyl)pyrazin-2-yl)pyridin-2-yl) butanamide 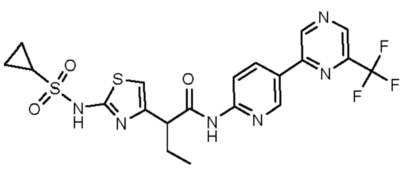 | Method 1c using INTB38 and INTA3, [UPLC acidic], 513, (1.35) | 12.59 (s, 1H), 11.06 (s, 1H), 9.66 (s, 1H), 9.29 - 9.10 (m, 2H), 8.59 (dd, J = 8.8, 2.5 Hz, 1H), 8.29 (d, J = 8.8 Hz, 1H), 6.56 (s, 1H), 3.82 (s, 1H), 2.62 - 2.55 (m, 1H), 2.05 - 1.82 (m, 2H), 0.96 - 0.76 (m, 7H). |
| T176 | 2-(2-(cyclopropanesulfonamido)thiazol-4-yl)-N-(5-(6-methoxypyrazin-2-yl)pyridin-2-yl)butanamide 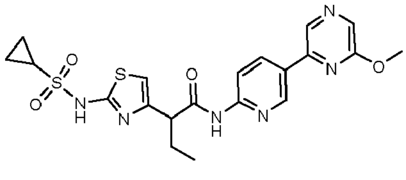 | Method 1c using INTB38 and INTA61, [UPLC acidic], 475, (1.21) | 12.60 (s, 1H), 10.95 (s, 1H), 9.12 (d, J = 2.4 Hz, 1H), 8.88 (s, 1H), 8.55 (dd, J = 8.8, 2.5 Hz, 1H), 8.29 (s, 1H), 8.24 (d, J = 8.8 Hz, 1H), 6.56 (s, 1H), 4.04 (s, 3H), 3.82 (s, 1H), 2.60 -2.52 (m, 1H), 2.05 - 1.80 (m, 2H), 0.98 - 0.82 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T177** | 2-(2-(cyclopropanesulfonamido)thiazol-4-yl)-N-(5-(6-ethoxypyrazin-2-yl)pyridin-2-yl)butanamide 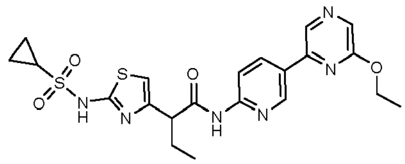 | Method 1c using INTB38 and INTA72, [UPLC acidic], 489, (1.32) | 12.59 (s, 1H), 10.94 (s, 1H), 9.13 - 9.04 (m, 1H), 8.85 (s, 1H), 8.51 (dd, J = 8.7, 2.4 Hz, 1H), 8.25 (s, 1H), 8.23 (d, J = 8.8 Hz, 1H), 6.55 (s, 1H), 4.49 (q, J = 7.0 Hz, 2H), 3.87 - 3.74 (m, 1H), 2.63 - 2.54 (m, 1H), 2.02 - 1.82 (m, 2H), 1.40 (t, J = 7.1 Hz, 3H), 0.96 - 0.75 (m, 7H). |
| **T178** | N-(5-(6-cyanopyrazin-2-yl)pyridin-2-yl)-2-(2-(cyclopropanesulfonamido)thiazol-4-yl) butanamide 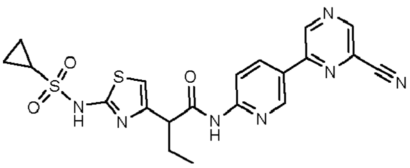 | Method 1c using INTB38 and INTA63, [UPLC acidic], 470, (1.17) | 12.59 (s, 1H), 11.06 (s, 1H), 9.62 (s, 1H), 9.18 (s, 1H), 9.15 (dd, J = 2.5, 0.8 Hz, 1H), 8.58 (dd, J = 8.8, 2.5 Hz, 1H), 8.27 (d, J = 8.8 Hz, 1H), 6.56 (s, 1H), 3.81 (t, J = 7.8 Hz, 1H), 2.62 - 2.54 (m, 1H), 2.04 - 1.79 (m, 2H), 0.94 - 0.78 (m, 7H). |
| **T179** | 2-(2-(cyclopropanesulfonamido)thiazol-4-yl)-N-(5'-fluoro-[3,3'-bipyridin]-6-yl)butanamide 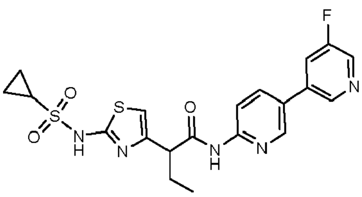 | Method 1c using INTB38 and INTA37, [HPLC acidic], 462, (1.78) | 12.58 (s, 1H), 10.90 (s, 1H), 8.87 (t, J = 1.9 Hz, 1H), 8.81 (dd, J = 2.5, 0.9 Hz, 1H), 8.59 (d, J = 2.7 Hz, 1H), 8.27 (dd, J = 8.7, 2.6 Hz, 1H), 8.23 - 8.12 (m, 2H), 6.55 (s, 1H), 3.80 (s, 1H), 2.64 - 2.54 (m, 1H), 2.04 - 1.82 (m, 2H), 0.96 - 0.79 (m, 7H). |
| **T180** | N-(5'-cyano-[3,3'-bipyridin]-6-yl)-2-(2-(cyclopropanesulfonamido)thiazol-4-yl) butanamide 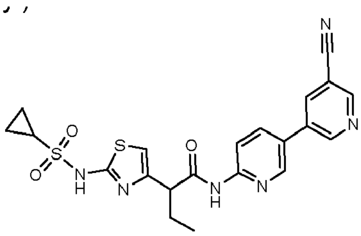 | Method 1c using INTB38 and INTA33, [HPLC acidic], 469, (1.75) | 12.60 (s, 1H), 10.94 (s, 1H), 9.26 (d, J = 2.3 Hz, 1H), 9.03 (d, J = 1.9 Hz, 1H), 8.85 (d, J = 2.5 Hz, 1H), 8.75 (t, J = 2.1 Hz, 1H), 8.31 (dd, J = 8.7, 2.6 Hz, 1H), 8.22 (d, J = 8.8 Hz, 1H), 6.55 (s, 1H), 3.80 (s, 1H), 2.64 - 2.55 (m, 1H), 2.06 - 1.85 (m, 2H), 0.96 - 0.71 (m, 7H). |
| **T181** | 2-(2-(cyclopropanesulfonamido)thiazol-4-yl)-N-(5-phenylpyridin-2-yl)butanamide 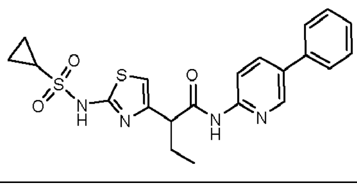 | Method 1c using INTB38, [UPLC acidic], 443, (1.36) | 12.58 (s, 1H), 10.80 (s, 1H), 8.67 (dd, J = 2.4, 0.9 Hz, 1H), 8.21 - 8.09 (m, 2H), 7.75 - 7.69 (m, 2H), 7.54 - 7.44 (m, 2H), 7.43 - 7.33 (m, 1H), 6.55 (s, 1H), 3.79 (t, J = 7.4 Hz, 1H), 2.63 - 2.53 (m, 1H), 2.05 - 1.79 (m, 2H), 0.95 - 0.79 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T182** | 2-(2-(cyclopropanesulfonamido)thiazol-4-yl)-N-(5-(6-(2,2,2-trifluoroethoxy)pyrazin-2-yl)pyridin-2-yl)butanamide 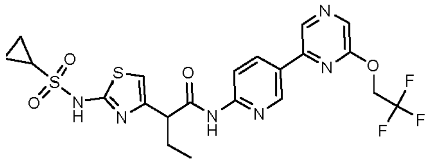 | Method 1c using INTB38 and INTA62, [UPLC acidic], 543, (1.39) | 12.60 (s, 1H), 10.98 (s, 1H), 9.17 (dd, J = 2.4, 0.9 Hz, 1H), 9.03 (d, 1H), 8.61 (dd, J = 8.8, 2.4 Hz, 1H), 8.46 (d, J = 0.5 Hz, 1H), 8.25 (d, J = 8.7 Hz, 1H), 6.57 (s, 1H), 5.23 (q, J = 9.0 Hz, 2H), 3.87 - 3.80 (m, 1H), 2.64 - 2.56 (m, 1H), 2.03 - 1.86 (m, 2H), 0.95 - 0.88 (m, 7H). |
| **T183** | 2-(2-(cyclopropanesulfonamido)thiazol-4-yl)-N-(5-(6-ethoxypyrazin-2-yl)-3-fluoropyridin-2-yl)butanamide 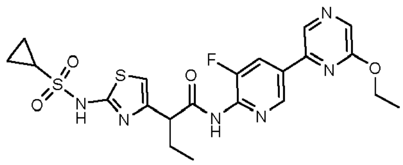 | Method 1c using INTB38 and INTA57, [UPLC acidic], 507, (1.24) | 12.63 (s, 1H), 10.72 (s, 1H), 9.04 - 9.00 (m, 1H), 8.93 (s, 1H), 8.46 (dd, J = 11.1, 2.0 Hz, 1H), 8.33 (s, 1H), 6.54 (s, 1H), 4.51 (q, J = 7.0 Hz, 2H), 3.79 - 3.72 (m, 1H), 2.63 - 2.56 (m, 1H), 2.03 - 1.83 (m, 2H), (t, J = 7.0 Hz, 3H), 0.98 - 0.87 (m, 7H). |
| **T184** | N-(5-(6-cyanopyrazin-2-yl)-3-fluoropyridin-2-yl)-2-(2-(cyclopropanesulfonamido)thiazol-4-yl) butanamide 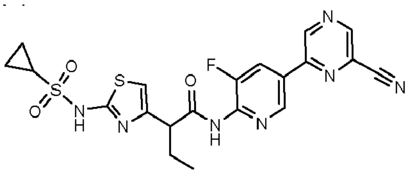 | Method 1c using INTB38 and INTA59, [UPLC acidic], 488, (1.09) | 12.63 (s, 1H), 10.82 (s, 1H), 9.67 (s, 1H), 9.25 (s, 1H), 9.08 - 9.04 (m, 1H), 8.54 (dd, J = 11.0, 2.0 Hz, 1H), 6.55 (s, 1H), 3.80 - 3.73 (m, 1H), 2.62 - 2.58 (m, 1H), 2.05 - 1.84 (m, 2H), 0.98 - 0.80 (m, 7H). |
| **T185** | 2-(2-(cyclopropanesulfonamido)thiazol-4-yl)-N-(5'-(2,2,2-trifluoroethoxy)-[3,3'-bipyridin]-6-yl) butanamide 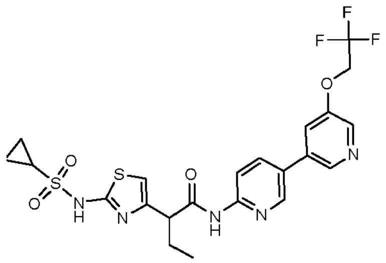 | Method 1c using INTB38 and INTA36, [UPLC acidic], 542, (1.27) | 12.59 (s, 1H), 10.90 (s, 1H), 8.81 (d, J = 2.4 Hz, 1H), 8.67 (d, J = 1.8 Hz, 1H), 8.42 (d, J = 2.8 Hz, 1H), 8.30 - 8.23 (m, 1H), 8.24 - 8.18 (m, 1H), 7.94 - 7.89 (m, 1H), 6.56 (s, 1H), 4.99 (q, J = 8.9 Hz, 2H), 3.83 3.79 (m, 1H), 2.61 - 2.57 (m, 1H), 2.03 - 1.84 (m, 2H), 0.94 - 0.83 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T186** | 2-(2-(cyclopropanesulfonamido)thiazol-4-yl)-N-(5'-(difluoromethoxy)-[3,3'-bipyridin]-6-yl) butanamide 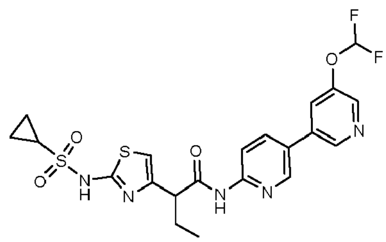 | Method 1c using INTB38 and INTA35, [UPLC acidic], 510, (1.2) | 12.59 (s, 1H), 10.92 (s, 1H), 8.87 (d, J = 1.9 Hz, 1H), 8.81 (d, J = 2.5 Hz, 1H), 8.51 (d, J = 2.6 Hz, 1H), 8.30 - 8.24 (m, 1H), 8.24 - 8.19 (m, 1H), 8.08 - 8.03 (m, 1H), 7.44 (t, J = 73.4 Hz, 1H), 6.56 (s, 1H), 3.84 - 3.77 (m, 1H), 2.61 - 2.57 (m, 1H), 2.03 - 1.84 (m, 2H), 0.94 - 0.87 (m, 7H). |
| **T187** | N-(5-(6-chloropyrazin-2-yl)pyridin-2-yl)-2-(2-(cyclopropanesulfonamido)thiazol-4-yl) butanamide 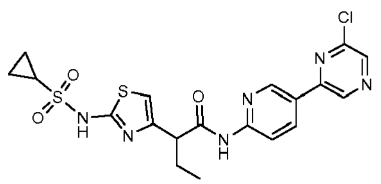 | Method 1c using INTB38 and INTA64, [UPLC acidic], 479, (1.27) | 12.59 (s, 1H), 11.03 (s, 1H), 9.33 (s, 1H), 9.10 (dd, J = 2.4, 0.8 Hz, 1H), 8.77 (s, 1H), 8.53 (dd, J = 8.8, 2.5 Hz, 1H), 8.26 (d, J = 8.8 Hz, 1H), 6.55 (s, 1H), 3.86 - 3.74 (m, 1H), 2.61 - 2.55 (m, 1H), 2.04 - 1.81 (m, 2H), 0.94 - 0.81 (m, 7H). |
| **T188** | 2-(2-(cyclopropanesulfonamido)thiazol-4-yl)-N-(2,3-difluoro-4-(pyridin-3-yl)phenyl)acetamide 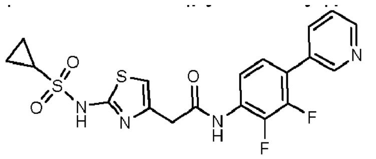 | Method 1c using INTB36 and INTA6, [HPLC basic], 451, (1.4) | 12.53 (s, 1H), 10.34 (s, 1H), 8.79 (q, J = 1.5, 0.9 Hz, 1H), 8.63 (dd, J = 4.8, 1.6 Hz, 1H), 8.02 (dq, J = 8.0, 1.7 Hz, 1H), 7.93 - 7.83 (m, 1H), 7.54 (ddd, J = 8.0, 4.8, 0.9 Hz, 1H), 7.43 (td, J = 8.5, 2.2 Hz, 1H), 6.58 (s, 1H), 3.77 (s, 2H), 2.64 - 2.54 (m, 1H), 0.98 - 0.86 (m, 4H). |
| **T189** | 2-(2-(cyclopropanesulfonamido)thiazol-4-yl)-N-(4-(6-ethoxypyrazin-2-yl)phenyl)butanamide 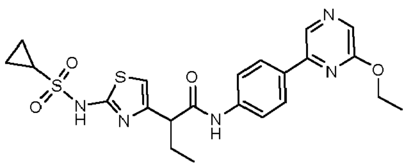 | Method 1c using INTB38 and INTA38, [HPLC basic], 488, (1.83) | 12.64 (s, 1H), 10.40 (s, 1H), 8.78 (s, 1H), 8.19 (s, 1H), 8.16 - 8.08 (m, 2H), 7.80 - 7.74 (m, 2H), 6.56 (s, 1H), 4.48 (q, J = 7.1 Hz, 2H), 3.61 (t, J = 7.4 Hz, 1H), 2.65 - 2.55 (m, 1H), 2.01 - 1.85 (m, 2H), 1.40 (t, J = 7.0 Hz, 3H), 0.95 - 0.84 (m, 7H). |
| **T190** | 2-(2-(cyclopropanesulfonamido)thiazol-4-yl)-4-methoxy-N-(4-(6-methoxypyrazin-2-yl)phenyl) butanamide 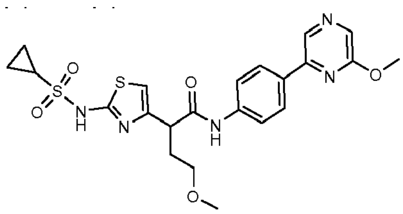 | Method 1c using INTB44 and INTA2, [HPLC acidic], 504, (1.92) | 12.65 (s, 1H), 10.40 (s, 1H), 8.79 (s, 1H), 8.22 (s, 1H), 8.13 (d, J = 8.8 Hz, 2H), 7.79 - 7.64 (m, 2H), 6.55 (s, 1H), 4.01 (s, 3H), 3.83 (s, 1H), 3.22 (s, 3H), 2.62 - 2.50 (m, 1H), 2.27 - 2.00 (m, 2H), 0.97 - 0.79 (m, 4H), CH2 at 3.3 obscured by residual water. |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T191 | 2-(2-(cyclopropanesulfonamido)thiazol-4-yl)-N-(4-(5-fluoropyridin-3-yl)phenyl)-4-methoxybutanamide 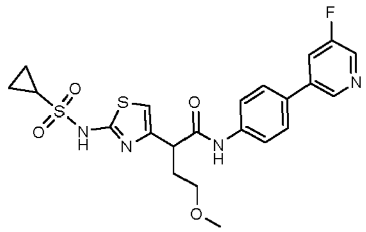 | Method 1c using INTB44 and INTA13, [HPLC acidic], 491, (1.82) | 12.65 (s, 1H), 10.36 (s, 1H), 8.80 (t, J = 1.8 Hz, 1H), 8.54 (d, J = 2.7 Hz, 1H), 8.10 - 8.00 (m, 1H), 7.86 - 7.66 (m, 4H), 6.55 (s, 1H), 3.82 (t, J = 7.6 Hz, 1H), 3.22 (s, 3H), 2.64 - 2.54 (m, 1H), 2.26 - 2.03 (m, 2H), 0.94 - 0.84 (m, 4H), CH2 at 3.3 obscured by residual water. |
| T192 | 2-(2-(cyclopropanesulfonamido)thiazol-4-yl)-N-(4-(6-ethoxypyrazin-2-yl)phenyl)propanamide 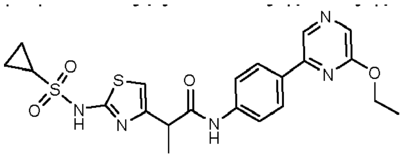 | Method 1c using INTB39 and INTA38, [HPLC acidic], 474, (1.73) | 12.62 (s, 1H), 10.34 (s, 1H), 8.77 (s, 1H), 8.18 (s, 1H), 8.14 - 8.05 (m, 2H), 7.82 - 7.71 (m, 2H), 6.52 (s, 1H), 4.47 (q, J = 7.0 Hz, 2H), 3.78 (q, J = 7.1 Hz, 1H), 2.62 - 2.54 (m, 1H), 1.46 (d, J = 7.1 Hz, 3H), 1.40 (t, J = 7.0 Hz, 3H), 0.95 - 0.81 (m, 4H). |
| T193 | 2-(2-(cyclopropanesulfonamido)thiazol-4-yl)-N-(4-(5-(trifluoromethyl)pyridin-3-yl)phenyl) butanamide 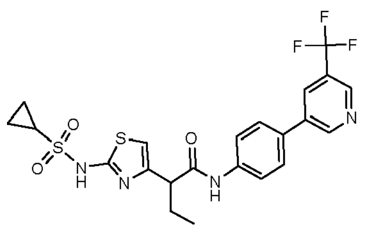 | Method 1c using INTB38 and INTA14, [UPLC acidic], 511, (1.38) | 12.62 (s, 1H), 10.35 (s, 1H), 9.21 (d, J = 2.2 Hz, 1H), 8.93 (dd, J = 2.1, 1.0 Hz, 1H), 8.44 (d, J = 2.4 Hz, 1H), 7.90 - 7.83 (m, 2H), 7.83 - 7.73 (m, 2H), 6.55 (s, 1H), 3.61 (t, J = 7.5 Hz, 1H),2.64 - 2.53 (m, 1H), 2.03 - 1.73 (m, 2H), 0.96 - 0.84 (m, 7H). |
| T194 | N-(4-(5-cyanopyridin-3-yl)phenyl)-2-(2-(cyclopropanesulfonamido)thiazol-4-yl) butanamide 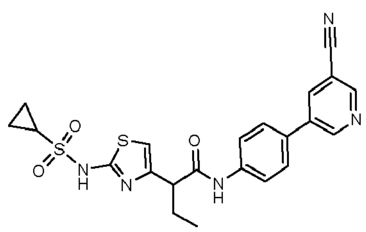 | Method 1c using INTB38 and INTA12, [UPLC acidic], 468, (1.19) | 12.62 (s, 1H), 10.36 (s, 1H), 9.19 (d, J = 2.3 Hz, 1H), 8.97 (d, J = 2.0 Hz, 1H), 8.64 (t, J = 2.1 Hz, 1H), 7.87 - 7.81 (m, 2H), 7.80 - 7.75 (m, 2H), 6.54 (s, 1H), 3.60 (t, J = 7.4 Hz, 1H), 2.63 - 2.55 (m, 1H), 2.04 - 1.79 (m, 2H), 0.97 - 0.81 (m, 7H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| T195 | 2-(2-(cyclopropanesulfonamido)thiazol-4-yl)-N-(4-(6-(2,2,2-trifluoroethoxy)pyrazin-2-yl)phenyl) butanamide 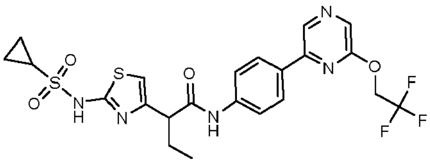 | Method 1c using INTB38 and INTA53, [HPLC acidic], 542, (2.25) | 12.60 (s, 1H), 10.95 (s, 1H), 9.12 (d, J = 2.4 Hz, 1H), 8.88 (s, 1H), 8.55 (dd, J = 8.8, 2.5 Hz, 1H), 8.29 (s, 1H), 8.24 (d, J = 8.8 Hz, 1H), 6.56 (s, 1H), 4.04 (s, 3H), 3.82 (s, 1H), 2.60 -2.52 (m, 1H), 2.05 - 1.80 (m, 2H), 0.98 - 0.82 (m, 7H). |
| T196 | 2-(2-(cyclopropanesulfonamido)thiazol-4-yl)-N-(2-fluoro-4-(pyrazin-2-yl)phenyl)butanamide 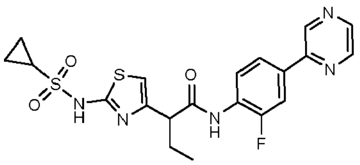 | Method 1c using INTB38 and INTA45, [HPLC acidic], 462, (1.75) | 12.56 (s, 1H), 10.17 (s, 1H), 9.30 (d, J = 1.5 Hz, 1H), 8.75 - 8.69 (m, 1H), 8.62 (d, J = 2.5 Hz, 1H), 8.19 - 8.10 (m, 1H), 8.11 - 7.98 (m, 2H), 6.55 (s, 1H), 3.82 (t, J = 7.4 Hz, 1H), 2.65 - 2.50 (m, 1H), 1.98 1.83 (m, 2H), 0.97 - 0.79 (m, 7H). |
| T197 | 2-(2-(cyclopropanesulfonamido)thiazol-4-yl)-N-(2-fluoro-4-(6-(trifluoromethyl)pyrazin-2-yl)phenyl) butanamide 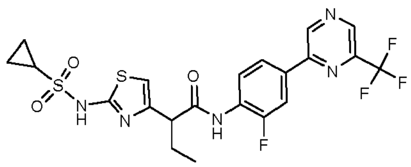 | Method 1c using INTB38 and INTA48, [HPLC acidic], 530, (2.23) | 12.59 (s, 1H), 10.22 (s, 1H), 9.63 (s, 1H), 9.13 (s, 1H), 8.21 (t, J = 8.3 Hz, 1H), 8.16 - 8.08 (m, 1H), 8.10 - 8.02 (m, 1H), 6.54 (s, 1H), 3.83 (t, J = 7.4 Hz, 1H), 2.63 - 2.54 (m, 1H), 2.02 - 1.82 (m, 2H), 0.95 - 0.85 (m, 7H). |
| T198 | 2-(2-(cyclopropanesulfonamido)thiazol-4-yl)-N-(4-(6-ethoxypyrazin-2-yl)-2-fluorophenyl)butanamide 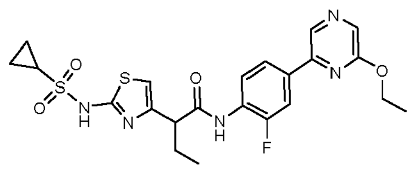 | Method 1c using INTB38 and INTA47, [HPLC acidic], 506, (2.19) | 12.59 (s, 1H), 10.14 (s, 1H), 8.83 (s, 1H), 8.23 (s, 1H), 8.11 (t, J = 8.3 Hz, 1H), 8.07 - 7.93 (m, 2H), 6.53 (s, 1H), 4.47 (q, J = 7.0 Hz, 2H), 3.81 (t, J = 7.4 Hz, 1H), 2.62 - 2.54 (m, 1H), 2.02 - 1.82 (m, 2H), 1.39 (t, J = 7.0 Hz, 3H), 0.95 - 0.85 (m, 7H). |
| T199 | 2-(2-(cyclopropanesulfonamido)thiazol-4-yl)-N-(4-(5-ethoxypyridin-3-yl)-2-fluorophenyl)butanamide 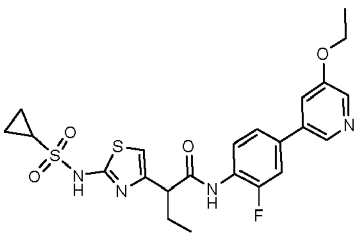 | Method 1c using INTB38 and INTA28, [HPLC acidic], 505, (1.77) | 12.56 (s, 1H), 10.06 (s, 1H), 8.50 (d, J = 1.9 Hz, 1H), 8.26 (d, J = 2.7 Hz, 1H), 8.01 (t, J = 8.4 Hz, 1H), 7.79 - 7.71 (m, 1H), 7.67 - 7.56 (m, 2H), 6.54 (s, 1H), 4.19 (q, J = 7.0 Hz, 2H), 3.79 (t, J = 7.4 Hz, 1H), 2.64 - 2.54 (m, 1H), 2.00 - 1.81 (m, 2H), 1.36 (t, J = 7.0 Hz, 3H), 0.95 - 0.83 (m, 7H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T200** | 2-(2-(cyclopropanesulfonamido)thiazol-4-yl)-N-(2-fluoro-4-(5-fluoropyridin-3-yl)phenyl)butanamide 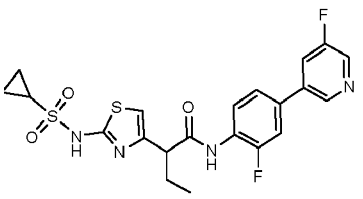 | Method 1c using INTB38 and INTA29, [HPLC acidic], 479, (1.93) | 12.60 (s, 1H), 10.12 (s, 1H), 8.89 - 8.83 (m, 1H), 8.59 (d, J = 2.7 Hz, 1H), 8.18 - 8.09 (m, 1H), 8.12-8.03 (m, 1H), 7.87 - 7.79 (m, 1H), 7.71 - 7.63 (m, 1H), 6.55 (s, 1H), 3.81 (t, J = 7.5 Hz, 1H), 2.66 - 2.56 (m, 1H), 2.02 - 1.83 (m, 2H), 0.97 - 0.87 (m, 7H). |
| **T201** | 2-(2-(cyclopropanesulfonamido)thiazol-4-yl)-N-(2-fluoro-4-(pyridin-3-yl)phenyl)butanamide 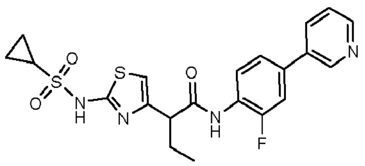 | Method 1c using INTB38, [HPLC acidic], 461, (1.37) | 12.58 (s, 1H), 10.08 (s, 1H), 8.94 (d, J = 2.4 Hz, 1H), 8.61 - 8.54 (m, 1H), 8.16 - 8.08 (m, 1H), 8.04 (t, J = 8.4 Hz, 1H), 7.78 - 7.70 (m, 1H), 7.63 - 7.56 (m, 1H), 7.53 - 7.45 (m, 1H), 6.56 (s, 1H), 3.85 - 3.76 (m, 1H), 2.65 - 2.54 (m, 1H), 2.04 - 1.81 (m, 2H), 0.97 - 0.84 (m, 7H). |
| **T202** | N-(4-(5-cyanopyridin-3-yl)-2-fluorophenyl)-2-(2-(cyclopropanesulfonamido)thiazol-4- 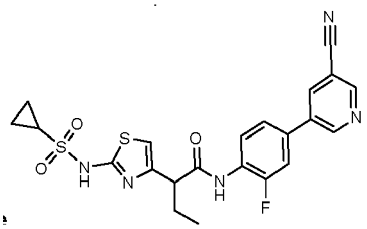 yl)butanamide | Method 1c using INTB38 and INTA26, [UPLC acidic], 486.1, (1.2) | 12.59 (s, 1H), 10.13 (s, 1H), 9.23 (d, J = 2.3 Hz, 1H), 9.00 (d, J = 1.9 Hz, 1H), 8.73 - 8.67 (m, 1H), 8.13 - 8.03 (m, 1H), 7.91 - 7.82 (m, 1H), 7.73 - 7.66 (m, 1H), 6.54 (s, 1H), 3.80 (t, J = 7.5 Hz, 1H), 2.63 - 2.53 (m, 1H), 2.01 - 1.82 (m, 2H), 0.96 - 0.85 (m, 7H). |
| **T203** | N-(4-(5-chloropyridin-3-yl)-2-fluorophenyl)-2-(2-(cyclopropanesulfonamido)thiazol-4- 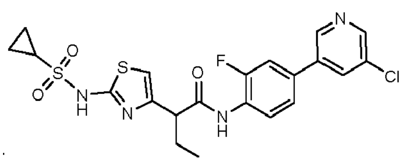 yl)butanamide | Method 1c using INTB38 and INTA27, [UPLC acidic], 495.3, (1.34) | 12.60 (s, 1H), 10.12 (s, 1H), 8.92 (d, J = 2.0 Hz, 1H), 8.63 (d, J = 2.3 Hz, 1H), 8.35 - 8.28 (m, 1H), 8.11 - 8.02 (m, 1H), 7.88 - 7.79 (m, 1H), 7.71 - 7.63 (m, 1H), 6.55 (s, 1H), 3.81 (t, J = 7.4 Hz, 1H), 2.64 - 2.52 (m, 1H), 2.02 - 1.83 (m, 2H), 0.97 - 0.79 (m, 7H). |
| **T204** | **Single enantiomer - stereochemistry unassigned** 2-(2-(cyclopropanesulfonamido)thiazol-4-yl)-N-(4-(6-ethoxypyrazin-2-yl)phenyl)butanamide 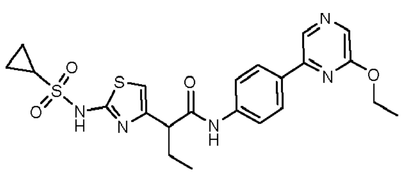 | Method 1c using INTB38 and INTA38, Chiral IA (28.0), [HPLC basic], 488, (1.85) | 12.63 (s, 1H), 10.41 (s, 1H), 8.77 (s, 1H), 8.19 (s, 1H), 8.16 - 8.07 (m, 2H), 7.82 - 7.73 (m, 2H), 6.56 (s, 1H), 4.48 (q, J = 7.0 Hz, 2H), 3.66-3.59 (m, 1H), 2.60 (d, J = 6.1 Hz, 1H), 2.01- 1.85 (m, 2H), 1.40 (t, J = 7.0 Hz, 3H), 0.95 - 0.84 (m, 7H). |

(continued)

| T# | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **T205** | **Single enantiomer - stereochemistry unassigned** 2-(2-(cyclopropanesulfonamido)thiazol-4-yl)-N-(4-(6-ethoxypyrazin-2-yl)phenyl)butanamide 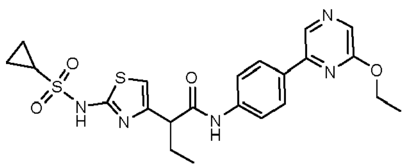 | Method 1c using INTB38 and INTA38, Chiral IA (30.6), [HPLC basic], 488, (1.85) | 12.63 (s, 1H), 10.41 (s, 1H), 8.77 (s, 1H), 8.19 (s, 1H), 8.16 - 8.07 (m, 2H), 7.84 - 7.71 (m, 2H), 6.56 (s, 1H), 4.48 (q, J = 7.0 Hz, 2H), 3.63 (t, J = 7.4 Hz, 1H), 2.59 (d, J = 6.5 Hz, 1H), 2.03 - 1.81 (m, 2H), 1.40 (t, J = 7.0 Hz, 3H), 0.97 - 0.81 (m, 7H). |
| **T206** | N-(4-(1-(5-(6-ethoxypyrazin-2-yl)indolin-1-yl)-1-oxobutan-2-yl)thiazol-2-yl)cyclopropanesulfonamide 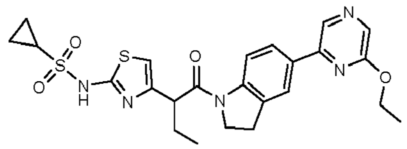 | Method 1c using INTB38 and INTA74, [UPLC acidic], 514.4, (1.47) | 12.60 (s, 1H), 8.77 (s, 1H), 8.32 - 8.11 (m, 2H), 8.09 - 7.89 (m, 2H), 6.58 (s, 1H), 4.55 - 4.41 (m, 2H), 4.35 - 4.18 (m, 2H), 3.98 - 3.82 (m, 1H), 3.30 - 3.23 (m, 2H), 2.64 - 2.54 (m, 1H), 2.08 - 1.81 (m, 2H), 1.49 - 1.34 (m, 3H), 1.00 - 0.79 (m, 7H). |
| **T207** | 2-(2-(cyclopropanesulfonamido)thiazol-4-yl)-N-(2-fluoro-4-(6-(2,2,2-trifluoroethoxy)pyrazin-2-yl)phenyl) butanamide 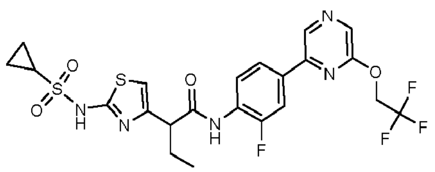 | Method 1c using INTB38 and INTA52, [UPLC acidic], 560, (1.48) | 12.61 (s, 1H), 10.18 (s, 1H), 9.01 (s, 1H), 8.44 (s, 1H), 8.20 - 8.12 (m, 2H), 8.05 (dd, J = 8.6, 2.0 Hz, 1H), 6.56 (s, 1H), 5.22 (q, J = 9.0 Hz, 2H), 3.88 - 3.81 (m, 1H), 2.61 - 2.52 (m, 1H), 2.01 - 1.85 (m, 2H), 0.96 - 0.87 (m, 7H). |
| **T208** | 2-(2-(cyclopropanesulfonamido)thiazol-4-yl)-N-(2-fluoro-4-(6-methoxypyrazin-2-yl)phenyl)butanamide 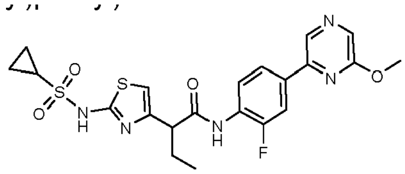 | Method 1c using INTB38 and INTA46, [UPLC acidic], 492, (1.32) | 12.61 (s, 1H), 10.16 (s, 1H), 8.86 (s, 1H), 8.28 (s, 1H), 8.17 - 8.10 (m, 1H), 8.07 (dd, J = 12.3, 2.0 Hz, 1H), 8.01 (dd, J = 8.5, 2.0 Hz, 1H), 6.56 (s, 1H), 4.03 (s, 3H), 3.87 - 3.80 (m, 1H), 2.63 - 2.55 (m, 1H), 2.03 - 1.83 (m, 2H), 0.96 - 0.87 (m, 7H). |
| **T209** | 2-(2-(cyclopropanesulfonamido)thiazol-4-yl)-N-(4-(5-(difluoromethoxy)pyridin-3-yl)-2-fluorophenyl)butanamide 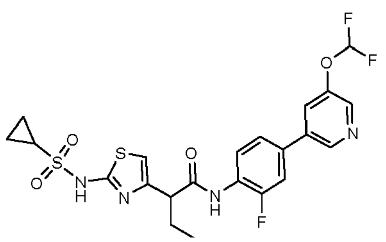 | Method 1c using INTB38 and INTA24, [UPLC acidic], 527, (1.29) | 12.61 (s, 1H), 10.12 (s, 1H), 8.86 (d, J = 1.9 Hz, 1H), 8.49 (d, J = 2.6 Hz, 1H), 8.11 - 8.04 (m, 1H), 8.04 - 7.99 (m, 1H), 7.82 (dd, J = 12.2, 2.1 Hz, 1H), 7.66 (dd, J = 8.5, 2.0 Hz, 1H), 7.44 (t, J = 73.4 Hz, 1H), 6.55 (s, 1H), 3.85 - 3.75 (m, 1H), 2.61 - 2.57 (m, 1H), 2.03 - 1.84 (m, 2H), 0.96 - 0.87 (m, 7H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T210** | 2-(2-(cyclopropanesulfonamido)thiazol-4-yl)-N-(4-(5-(difluoromethoxy)pyridin-3-yl)phenyl) butanamide 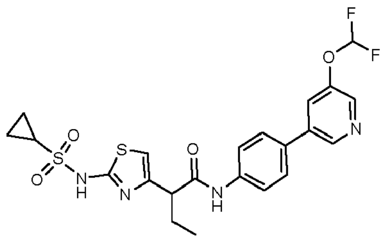 | Method 1c using INTB38 and INTA25, [UPLC acidic], 509, (1.26) | 12.61 (s, 1H), 10.33 (s, 1H), 8.80 (d, J = 1.9 Hz, 1H), 8.45 (d, J = 2.6 Hz, 1H), 7.97 - 7.90 (m, 1H), 7.81 - 7.72 (m, 4H), 7.43 (t, J = 73.5 Hz, 1H), 6.55 (s, 1H), 3.64 - 3.57 (m, 1H), 2.62 - 2.56 (m, 1H), 2.03 1.83 (m, 2H), 0.95 - 0.86 (m, 7H). |
| **T211** | 2-(2-(cyclopropanesulfonamido)thiazol-4-yl)-N-(4-(5-(2,2,2-trifluoroethoxy)pyridin-3-yl)phenyl) butanamide 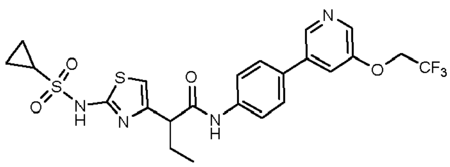 | Method 1c using INTB38 and INTA31, [UPLC acidic], 541, (1.3) | 12.59 (s, 1H), 10.31 (s, 1H), 8.59 (d, J = 1.8 Hz, 1H), 8.36 (d, J = 2.8 Hz, 1H), 7.82 - 7.71 (m, 5H), 6.56 (s, 1H), 4.98 (q, J = 8.9 Hz, 2H), 3.60 (s, 1H), 2.64 - 2.55 (m, 1H), 2.03 - 1.83 (m, 2H), 0.95 - 0.85 (m, 7H). |
| **T212** | 2-(2-(cyclopropanesulfonamido)thiazol-4-yl)-N-(2-fluoro-4-(5-(2,2,2-trifluoroethoxy)pyridin-3-yl)phenyl) butanamide 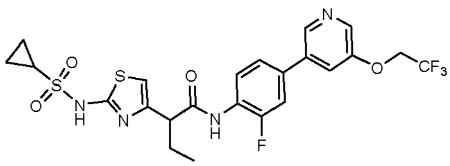 | Method 1c using INTB38 and INTA30, [UPLC acidic], 559, (1.34) | 12.58 (s, 1H), 10.09 (s, 1H), 8.64 (d, J = 1.8 Hz, 1H), 8.38 (d, J = 2.8 Hz, 1H), 8.08 - 8.00 (m, 1H), 7.86 (dd, J = 2.8, 1.9 Hz, 1H), 7.84 - 7.77 (m, 1H), 7.65 (dd, J = 8.5, 2.1 Hz, 1H), 6.54 (s, 1H), 4.97 (q, J = 8.8 Hz, 2H), 3.83 - 3.76 (m, 1H), 2.60 - 2.56 (m, 1H), 2.00 - 1.83 (m, 2H), 0.95 - 0.86 (m, 7H). |
| **T213** | 2-(cyclopropanesulfonamido)-N-(4-(pyridin-3-yl) phenyl)-5,6-dihydro-4H-cyclopenta[d]thiazole-4-carboxamide 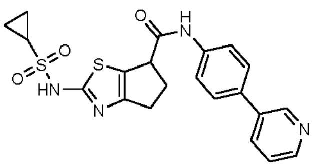 | Method 1c using INTB66, [HPLC acidic], 441, (1.15) | 12.56 (s, 1H), 10.31 (s, 1H), 8.90 (dd, J = 2.4, 0.9 Hz, 1H), 8.54 (dd, J = 4.8, 1.6 Hz, 1H), 8.07 (ddd, J = 8.0, 2.4, 1.6 Hz, 1H), 7.80 - 7.71 (m, 4H), 7.47 (ddd, J = 8.0, 4.7, 0.9 Hz, 1H), 4.06 - 3.97 (m, 1H), 2.85 - 2.71 (m, 3H), 2.67 - 2.56 (m, 1H), 2.50 - 2.44 (m, 1H, partially obscured by solvent), 0.97 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T214** | 2-(2-(cyclopropanesulfonamido)thiazol-4-yl)-2-methoxy-N-(5-(6-(trifluoromethyl)pyrazin-2-yl)pyridin-2-yl)acetamide 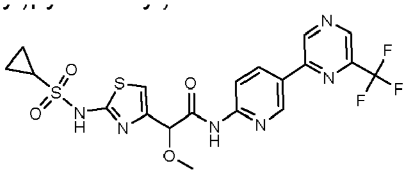 | Method 1c using INTB43 and INTA3, [UPLC acidic], 515, (1.27) | 12.85 (s, 1H), 10.44 (s, 1H), 9.68 (s, 1H), 9.20 (d, J = 2.4 Hz, 1H), 9.17 (s, 1H), 8.63 (dd, J = 8.8, 2.5 Hz, 1H), 8.27 (d, J = 8.8 Hz, 1H), 6.89 (s, 1H), 5.02 (s, 1H), 3.40 (s, 3H), 2.65 - 2.53 (m, 1H), 0.94 - 0.82 (m, 4H). |
| **T215** | 2-(2-(cyclopropanesulfonamido)thiazol-4-yl)-N-(2-methoxy-4-(pyridin-3-yl)phenyl)acetamide 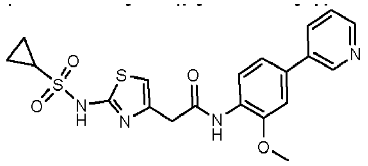 | Method 1c using INTB36 and INTA, [HPLC basic], 445, (1.4) | 12.52 (s, 1H), 9.51 (s, 1H), 8.95 (dd, J = 2.4, 0.9 Hz, 1H), 8.56 (dd, J = 4.8, 1.6 Hz, 1H), 8.18 - 8.07 (m, 2H), 7.48 (ddd, J = 8.0, 4.8, 0.9 Hz, 1H), 7.41 (d, J = 2.0 Hz, 1H), 7.31 (dd, J = 8.4, 2.0 Hz, 1H), 6.56 (s, 1H), 3.98 (s, 3H), 3.75 (s, 2H), 2.64 - 2.53 (m, 1H), 0.90 (dt, J = 8.1, 2.5 Hz, 4H). |
| **T216** | 2-(2-(cyclopropanesulfonamido)thiazol-4-yl)-N-(2-fluoro-4-(pyridin-3-yl)phenyl)acetamide 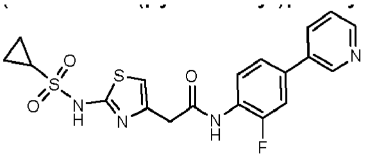 | Method 1c using INTB36, [HPLC basic], 433, (1.36) | 12.52 (s, 1H), 10.11 (s, 1H), 8.94 (d, J = 2.4 Hz, 1H), 8.57 (dd, J = 4.8, 1.6 Hz, 1H), 8.15 - 8.04 (m, 2H), 7.74 (dd, J = 12.3, 2.1 Hz, 1H), 7.59 (dd, J = 8.4, 1.9 Hz, 1H), 7.51 - 7.46 (m, 1H), 6.57 (s, 1H), 3.75 (s, 2H), 2.63 - 2.52 (m, 1H), 0.93 - 0.86 (m, 4H). |
| **T217** | N-(4-(5-cyanopyridin-3-yl)phenyl)-2-(cyclopropanesulfonamido)-5,6-dihydro-4H-cyclopenta[d]thiazole-4-carboxamide 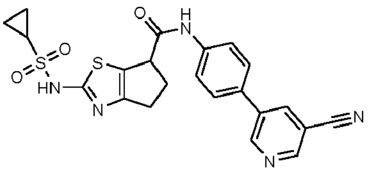 | Method 1c using INTB66 and INTA12, [UPLC acidic], 466, (1.14) | 12.59 (s (br), 1H), 10.38 (s, 1H), 9.19 (d, J = 2.3 Hz, 1H), 8.97 (d, J = 1.9 Hz, 1H), 8.63 (dd, J = 2.1 Hz, 1H), 7.84 (d, J = 8.9 Hz, 1H), 7.77 (d, J = 8.9 Hz, 1H), 4.06 - 3.99 (m, 1H), 2.86 - 2.68 (m, 3H), 2.60 (s, 1H), 0.90 (dd, J = 6.9, 4.5 Hz, 4H). 1 proton under DMSO peak |
| **T218** | 2-(cyclopropanesulfonamido)-N-(4-(5-fluoropyridin-3-yl)phenyl)-5,6-dihydro-4H-cyclopenta[d]thiazole-4-carboxamide 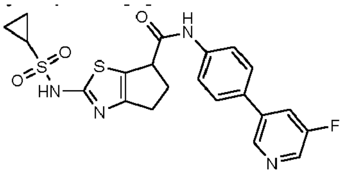 | Method 1c using INTB66 and INTA13, [UPLC acidic], 459.3, (1.14) | 12.59 (s, 1H), 10.34 (s, 1H), 8.81 (dd, J = 1.9 Hz, 1H), 8.54 (d, J = 2.7 Hz, 1H), 8.06 (ddd, J = 10.6, 2.8, 1.9 Hz, 1H), 7.84 - 7.73 (m, 4H), 4.04 - 3.97 (m, 1H), 2.84 - 2.70 (m, 3H), 2.63 - 2.58 (m, 1H), 0.94 - 0.87 (m, 5H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T219** | 2-(2-(cyclopropanesulfonamido)thiazol-4-yl)-2-methoxy-N-(4-(pyridin-3-yl)phenyl)acetamide 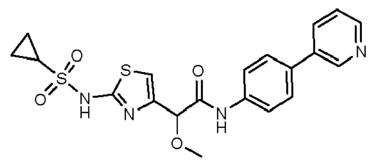 | Method 1c using INTB43, [HPLC basic], 445, (1.35) | 12.76 (s, 1H), 10.05 (s, 1H), 8.97 - 8.84 (m, 1H), 8.55 (dd, J = 4.8, 1.6 Hz, 1H), 8.12 - 8.04 (m, 1H), 7.84 (d, J = 8.7 Hz, 2H), 7.74 (d, J = 8.7 Hz, 2H), 7.53 - 7.44 (m, 1H), 6.82 (s, 1H), 4.86 (s, 1H), 3.40 (s, 3H), 2.65 - 2.54 (m, 1H), 0.88 0.97 - 0.74 (m, 4H). |
| **T220** | 2-(2-(cyclopropanesulfonamido)thiazol-4-yl)-N-(2-fluoro-4-(pyridin-3-yl)phenyl)-2-methoxyacetamide 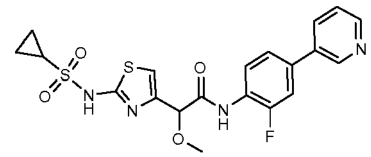 | Method 1c using INTB43, [HPLC basic], 463, (1.41) | 9.71 (s, 1H), 8.99 - 8.92 (m, 1H), 8.57 (dd, J = 4.8, 1.6 Hz, 1H), 8.16 - 8.04 (m, 2H), 7.76 (dd, J = 12.2, 2.1 Hz, 1H), 7.61 (dd, J = 8.4, 2.0 Hz, 1H), 7.48 (dd, J = 8.0, 4.9 Hz, 1H), 6.66 (s, 1H), 4.81 (s, 1H), 3.37 (s, 3H), 2.56 - 2.52 (m, 1H), 0.91 - 0.62 (m, 4H) 1 x N-H not observed. |
| **T221** | N-(2-chloro-4-(pyridin-3-yl)phenyl)-2-(2-(cyclopropanesulfonamido)thiazol-4-yl) butanamide 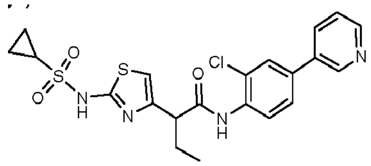 | Method 1c using INTB38 and INTA8, [HPLC basic], 477, (1.59) | 12.62 (s, 1H), 9.87 (s, 1H), 8.94 (d, J = 2.3 Hz, 1H), 8.60 (dd, J = 4.8, 1.6 Hz, 1H), 8.17 - 8.11 (m, 1H), 7.93 (d, J = 2.1 Hz, 1H), 7.85 (d, J = 8.4 Hz, 1H), 7.74 (dd, J = 8.4, 2.2 Hz, 1H), 7.50 (dd, J = 8.0, 4.7 Hz, 1H), 6.59 (s, 1H), 3.80 (t, J = 7.4 Hz, 1H), 2.66 - 2.55 (m, 1H), 2.04 - 1.82 (m, 2H), 0.99 - 0.85 (m, 7H). |
| **T222** | **Single enantiomer - stereochemistry unassigned** 2-(2-(cyclopropanesulfonamido)thiazol-4-yl)-N-(4-(6-(2,2,2-trifluoroethoxy)pyrazin-2-yl)phenyl) butanamide 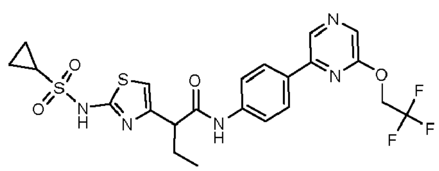 | Method 1c using INTB38 and INTA53 Chiral IC (20.6), [UPLC acidic], 542, (1.44) | 12.62 (s, 1H), 10.39 (s, 1H), 8.94 (s, 1H), 8.39 (s, 1H), 8.23 - 8.13 (m, 2H), 7.80 - 7.73 (m, 2H), 6.56 (s, 1H), 5.20 (q, J = 9.0 Hz, 2H), 2.63 - 2.56 (m, 1H), 2.03 - 1.83 (m, 2H), 0.96 - 0.79 (m, 7H), 1 proton obscured by solvent |
| **T223** | **Single enantiomer - stereochemistry unassigned** 2-(2-(cyclopropanesulfonamido)thiazol-4-yl)-N-(4-(6-(2,2,2-trifluoroethoxy)pyrazin-2-yl)phenyl) butanamide 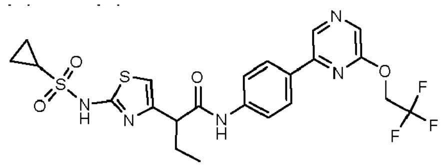 | Method 1c using INTB38 and INTA53 Chiral IC (26.1), [UPLC acidic], 542, (1.44) | 12.62 (s, 1H), 10.39 (s, 1H), 8.93 (s, 1H), 8.38 (s, 1H), 8.22 - 8.16 (m, 2H), 7.81 - 7.74 (m, 2H), 6.55 (s, 1H), 5.19 (q, J = 9.0 Hz, 2H), 3.60 (d, J = 7.5 Hz, 1H), 2.61 - 2.53 (m, 1H), 2.04 - 1.82 (m, 2H), 0.96 - 0.86 (m, 7H). |

(continued)

| T# | Name/Structure (All examples containing chiral centres are racemates unless stated) | Synthesis Method, [LCMS Method], m/z (M+H)+, (Rt/min) | $^{1}$H NMR Chemical Shift Data (DMSO-d6 unless stated) |
|---|---|---|---|
| **T224** | 2-(2-(cyclopropanesulfonamido)thiazol-4-yl)-N-(5'-methoxy-[3,3'-bipyridin]-6-yl)-2-methylpropanamide 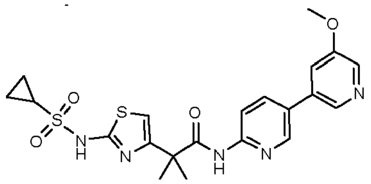 | Method 2 using INTB55, [UPLC acidic], 474, (0.94) | 12.54 (s, 1H), 10.01 (s, 1H), 8.76 (d, J = 2.4 Hz, 1H), 8.54 (d, J = 1.9 Hz, 1H), 8.30 (d, J = 2.8 Hz, 1H), 8.23 (dd, J = 8.7, 2.6 Hz, 1H), 8.13 (d, J = 8.7 Hz, 1H), 7.70 (t, J = 2.3 Hz, 1H), 6.58 (s, 1H), 3.91 (s, 3H), 2.61 - 2.56 (m, 1H), 1.60 (s, 6H), 0.93 - 0.86 (m, 4H). |
| **T225** | N-(5'-chloro-[3,3'-bipyridin]-6-yl)-2-(2-(cyclopropanesulfonamido)thiazol-4-yl)-2-methylpropanamide 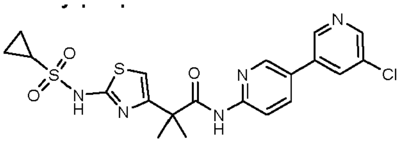 | Method 2 using INTB55, [UPLC basic], 478, (1.07) | 12.58 (s, 1H), 10.09 (s, 1H), 8.95 (d, J = 2.0 Hz, 1H), 8.82 (d, J = 2.5 Hz, 1H), 8.65 (d, J = 2.3 Hz, 1H), 8.37 (t, J = 2.2 Hz, 1H), 8.29 (dd, J = 8.7, 2.6 Hz, 1H), 8.19 - 8.10 (m, 1H), 6.58 (s, 1H), 2.65 - 2.55 (m, 1H), 1.61 (s, 6H), 1.01 - 0.88 (m, 4H). |
| **T226** | N-(5'-cyano-[3,3'-bipyridin]-6-yl)-2-(2-(cyclopropanesulfonamido)thiazol-4-yl)-2-methylpropanamide 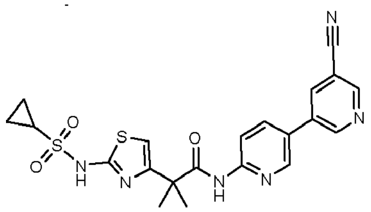 | Method 2 using INTB55, [UPLC acidic], 469, (1.1) | 12.57 (s, 1H), 10.11 (s, 1H), 9.26 (d, J = 2.3 Hz, 1H), 9.02 (d, J = 1.9 Hz, 1H), 8.85 (s, 1H), 8.75 (t, J = 2.1 Hz, 1H), 8.31 (dd, J = 8.7, 2.6 Hz, 1H), 8.16 (d, J = 8.8 Hz, 1H), 6.57 (s, 1H), 2.63 - 2.53 (m, 1H), 1.60 (s, 6H), 0.93 - 0.78 (m, 4H). |
| **T227** | 2-(2-(cyclopropanesulfonamido)thiazol-4-yl)-N-(5-fluoro-[3,3'-bipyridin]-6-yl)-2-methylpropanamide 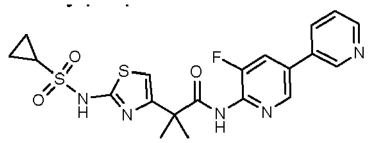 | Method 2 using INTB72, [UPLC acidic], 462, (0.76) | 12.60 (s, 1H), 9.98 (s, 1H), 9.01 (d, J = 2.3 Hz, 1H), 8.72 - 8.67 (m, 1H), 8.66 - 8.60 (m, 1H), 8.29 - 8.18 (m, 2H), 7.57 - 7.49 (m, 1H), 6.58 (s, 1H), 2.63 - 2.55 (m, 1H), 1.58 (s, 6H), 0.94 - 0.84 (m, 4H). |
| **T228** | N-(5'-cyano-5-fluoro-[3,3'-bipyridin]-6-yl)-2-(2-(cyclopropanesulfonamido)thiazol-4-yl)-2-methylpropanamide 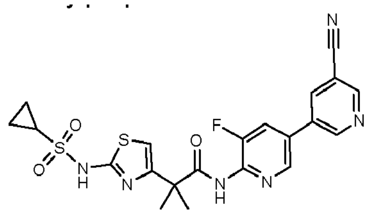 | Method 2, using INTB72 [UPLC acidic], 487.3, (1.01) | 12.64 (s, 1H), 10.06 (s, 1H), 9.33 (d, J = 2.3 Hz, 1H), 9.08 (d, J = 1.9 Hz, 1H), 8.86 - 8.77 (m, 2H), 8.40 - 8.32 (m, 1H), 6.57 (s, 1H), 2.61 - 2.56 (m, 1H), 1.59 (s, 6H), 0.94 - 0.87 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T229 | N-(5'-chloro-5-fluoro-[3,3'-bipyridin]-6-yl)-2-(2-(cyclopropanesulfonamido)thiazol-4-yl)-2-methylpropanamide 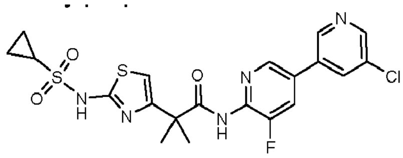 | Method 2, using INTB72 [UPLC acidic], 496.3, (1.14) | 12.63 (s, 1H), 10.03 (s, 1H), 9.00 (d, J = 2.0 Hz, 1H), 8.75 (s, 1H), 8.68 (d, J = 2.3 Hz, 1H), 8.47 - 8.41 (m, 1H), 8.36 - 8.27 (m, 1H), 6.56 (s, 1H), 2.60 - 2.55 (m, 1H), 1.58 (s, 6H), 0.93 - 0.86 (m, 4H). |
| T230 | 2-(2-(cyclopropanesulfonamido)thiazol-4-yl)-N-(5,5'-difluoro-[3,3'-bipyridin]-6-yl)-2-methylpropanamide 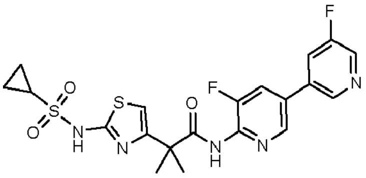 | Method 2, using INTB72 [UPLC acidic], 480.1, (1.02) | 12.65 (s, 1H), 10.05 (s, 1H), 8.98 - 8.92 (m, 1H), 8.80 - 8.75 (m, 1H), 8.66 (d, J = 2.7 Hz, 1H), 8.37 - 8.24 (m, 2H), 6.58 (s, 1H), 2.62 - 2.57 (m, 1H), 1.60 (s, 6H), 0.95 - 0.90 (m, 4H). |
| T231 | N-(5-(3-chloro-5-methylphenyl)pyridin-2-yl)-2-(2-(cyclopropanesulfonamido)thiazol-4-yl)-2-methylpropanamide 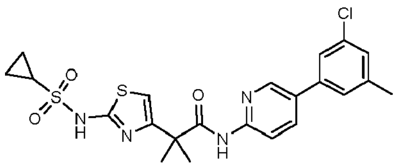 | Method 2 using INTB55, [UPLC acidic], 492, (1.6) | 12.57 (s, 1H), 10.00 (s, 1H), 8.70 (d, J = 2.1 Hz, 1H), 8.20 - 8.06 (m, 2H), 7.63 - 7.57 (m, 1H), 7.56 - 7.50 (m, 1H), 7.29 (s, 1H), 6.57 (s, 1H), 2.64 - 2.53 (m, 1H), 2.38 (s, 3H), 1.60 (s, 6H), 0.93 - 0.87 (m, 4H). |
| T232 | 2-(2-(cyclopropanesulfonamido)thiazol-4-yl)-N-(5-(3-methoxyphenyl)pyridin-2-yl)-2-methylpropanamide 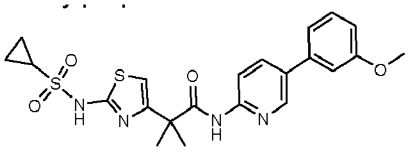 | Method 2 using INTB55, [UPLC acidic], 473, (1.36) | 12.58 (s, 1H), 9.94 (s, 1H), 8.67 (s, 1H), 8.20 - 8.00 (m, 2H), 7.39 (t, J = 7.9 Hz, 1H), 7.33 - 7.19 (m, 2H), 6.99 - 6.90 (m, 1H), 6.57 (s, 1H), 3.83 (s, 3H), 2.65 - 2.53 (m, 1H), 1.60 (s, 6H), 0.96 - 0.80 (m, 4H). |
| T233 | 2-(2-(cyclopropanesulfonamido)thiazol-4-yl)-N-(5-(3-fluoro-5-methoxyphenyl)pyridin-2-yl)-2-methylpropanamide 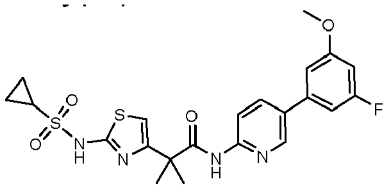 | Method 2 using INTB55, [UPLC acidic], 491, (1.43) | 12.57 (s, 1H), 10.02 (s, 1H), 8.70 (s, 1H), 8.21 - 8.09 (m, 2H), 7.22 - 7.11 (m, 2H), 6.85 (d, J = 10.8 Hz, 1H), 6.56 (s, 1H), 3.85 (s, 3H), 2.63 - 2.51 (m, 1H), 1.56 (s, 6H), 0.97 - 0.69 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T234 | 2-(2-(cyclopropanesulfonamido)thiazol-4-yl)-N-(5-(3,5-dimethoxyphenyl)pyridin-2-yl)-2-methylpropanamide 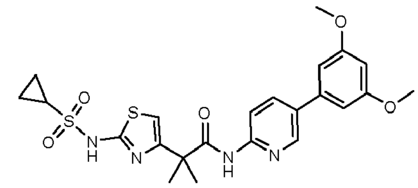 | Method 2 using INTB55, [UPLC acidic], 503, (1.38) | 12.59 (s, 1H), 9.95 (s, 1H), 8.68 (s, 1H), 8.18 - 8.06 (m, 2H), 6.85 (d, J = 2.2 Hz, 2H), 6.58 (s, 1H), 6.53 (t, J = 2.2 Hz, 1H), 3.82 (s, 6H), 2.65 2.53 (m, 1H), 1.61 (s, 6H), 0.96 - 0.82 (m, 4H). |
| T235 | 2-(2-(cyclopropanesulfonamido)thiazol-4-yl)-2-methyl-N-(5-(3-(trifluoromethyl)phenyl)pyridin-2-yl) propanamide 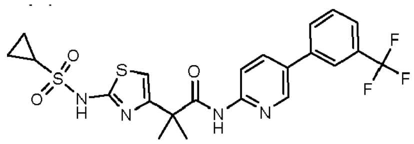 | Method 2 using INTB55, [UPLC acidic], 511, (1.54) | 12.58 (s, 1H), 10.04 (s, 1H), 8.77 (s, 1H), 8.25 (dd, J = 8.6, 2.6 Hz, 1H), 8.15 (d, J = 8.8 Hz, 1H), 8.09 - 8.02 (m, 2H), 7.80 - 7.66 (m, 2H), 6.57 (s, 1H), 2.63 - 2.54 (m, 1H), 1.61 (s, 6H), 0.95 - 0.83 (m, 4H). |
| T236 | 2-(2-(cyclopropanesulfonamido)thiazol-4-yl)-2-methyl-N-(5-(3-(trifluoromethoxy)phenyl)pyridin-2-yl) propanamide 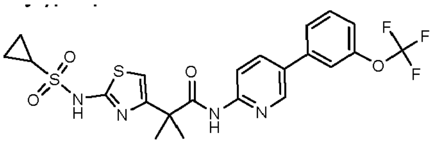 | Method 2 using INTB55, [UPLC acidic], 527, (1.57) | 12.56 (s, 1H), 10.01 (s, 1H), 8.73 (d, J = 2.5 Hz, 1H), 8.22 - 8.16 (m, 1H), 8.13 (dd, J = 8.7, 0.8 Hz, 1H), 7.82 - 7.76 (m, 1H), 7.75 (s, 1H), 7.62 (t, J = 8.0 Hz, 1H), 7.42 - 7.36 (m, 1H), 6.59 (s, 1H), 2.65 - 2.54 (m, 1H), 1.60 (s, 6H), 0.94 - 0.74 (m, 4H). |
| T237 | 2-(2-(cyclopropanesulfonamido)thiazol-4-yl)-N-(5-(3-(2-hydroxypropan-2-yl)phenyl)pyridin-2-yl)-2-methylpropanamide 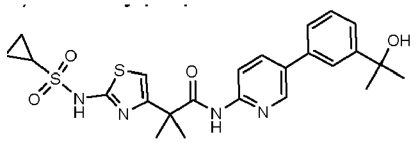 | Method 2 using INTB55, [UPLC acidic], 501, (1.22) | 12.59 (s, 1H), 9.95 (s, 1H), 8.65 (s, 1H), 8.15 - 8.09 (m, 2H), 7.78 (t, J = 1.8 Hz, 1H), 7.56 - 7.50 (m, 1H), 7.50 - 7.47 (m, 1H), 7.45 - 7.39 (m, 1H), 6.59 (s, 1H), 2.64 - 2.55 (m, 1H), 1.61 (s, 6H), 1.48 (s, 6H), 0.97 - 0.83 (m, 4H), O-H not observed. |
| T238 | 2-(2-(cyclopropanesulfonamido)thiazol-4-yl)-2-methyl-N-(5-(3-morpholinophenyl)pyridin-2-yl)propanamide 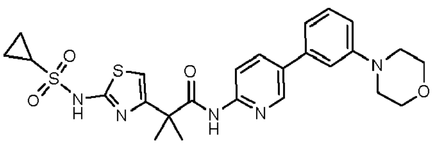 | Method 2 using INTB55, [UPLC acidic], 528, (1.3) | 12.59 (s, 1H), 9.93 (s, 1H), 8.65 (s, 1H), 8.16 - 8.03 (m, 2H), 7.34 (t, J = 7.9 Hz, 1H), 7.22 (d, J = 2.1 Hz, 1H), 7.14 (d, J = 7.6 Hz, 1H), 7.01 6.93 (m, 1H), 6.58 (s, 1H), 3.80 - 3.73 (m, 4H), 3.23 - 3.16 (m, 4H), 2.63 - 2.56 (m, 1H), 1.61 (s, 6H), 0.98 - 0.80 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T239 | 2-(2-(cyclopropanesulfonamido)thiazol-4-yl)-2-methyl-N-(6-phenylpyridin-3-yl)propanamide 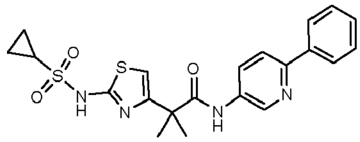 | Method 1, using INTB37, [UPLC acidic], 443, (1.14) | 12.61 (s, 1H), 9.64 (s, 1H), 8.88 (d, J = 2.5 Hz, 1H), 8.14 (dd, J = 8.7, 2.5 Hz, 1H), 8.09 - 8.02 (m, 2H), 7.98 (d, J = 8.7 Hz, 1H), 7.53 - 7.37 (m, 3H), 6.60 (s, 1H), 2.60 (s, 1H), 1.59 (s, 6H), 0.95 - 0.85 (m, 4H). |
| T240 | 2-(2-(cyclopropanesulfonamido)thiazol-4-yl)-N-(4-(2-fluoropyridin-3-yl)phenyl)-2-methylpropanamide 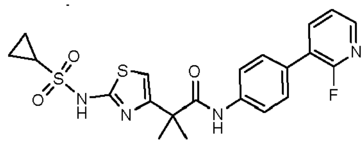 | Method 2 using INTB53, [UPLC acidic], 461, (1.2) | 12.58 (s, 1H), 9.46 (s, 1H), 8.24-8.19 (m, 1H), 8.15-8.08 (m, 1H), 7.81 - 7.73 (m, 2H), 7.59 (d, J = 8.0 Hz, 2H), 7.49-7.43 (m, 1H), 6.55 (s, 1H), 2.48 - 2.42 (m, 1H), 1.58 (s, 6H), 0.94 - 0.87 (m, 4H). |
| T241 | 2-(2-(cyclopropanesulfonamido)thiazol-4-yl)-N-(4-(5-(hydroxymethyl)pyridin-3-yl)phenyl)-2-methylpropanamide 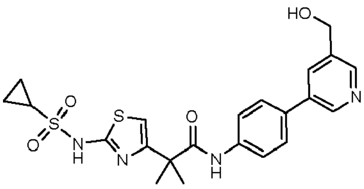 | Method 2 using INTB53, [UPLC acidic], 473, (0.69) | 12.51 (s, 1H), 9.37 (s, 1H), 8.72 (d, J = 2.3 Hz, 1H), 8.44 (d, J = 1.9 Hz, 1H), 7.95 - 7.90 (m, 1H), 7.74 - 7.69 (m, 2H), 7.69 - 7.63 (m, 2H), 6.50 (s, 1H), 5.32 (t, J = 5.7 Hz, 1H), 4.56 (d, J = 5.7 Hz, 2H), 2.52 - 2.47 (m, 1H), 1.53 (s, 6H), 0.88 - 0.84 (m, 4H). |
| T242 | 2-(2-(cyclopropanesulfonamido)thiazol-4-yl)-N-(4-(2-methoxypyrimidin-5-yl)phenyl)acetamide 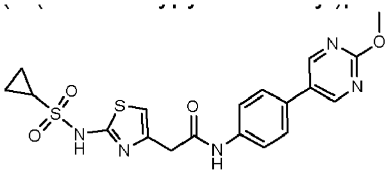 | Method 2 using INTB73, [HPLC acidic], 446, (1.53) | 12.56 (s, 1H), 10.34 (s, 1H), 8.93 (s, 2H), 7.72 (s, 4H), 6.57 (s, 1H), 3.96 (s, 3H), 3.67 (s, 2H), 2.63 - 2.55 (m, 1H), 0.95 - 0.69 (m, 4H). |
| T243 | N-(4'-(tert-butyl)-[1,1'-biphenyl]-4-yl)-2-(2-(cyclopropanesulfonamido)thiazol-4- 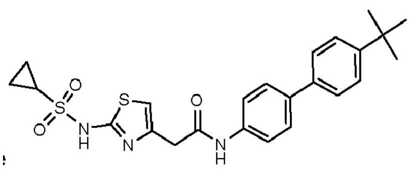 yl)acetamide | Method 2, using INTB73 [HPLC acidic], 470, (2.48) | 12.56 (s, 1H), 10.27 (s, 1H), 7.71 - 7.65 (m, 2H), 7.65 - 7.60 (m, 2H), 7.60 - 7.55 (m, 2H), 7.49 - 7.43 (m, 2H), 6.56 (s, 1H), 3.66 (s, 2H), 2.63 - 2.54 (m, 1H), 1.31 (s, 9H), 0.94 - 0.82 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T244** | 2-(2-(cyclopropanesulfonamido)thiazol-4-yl)-N-(4-(pyridin-3-yl)phenyl)acetamide 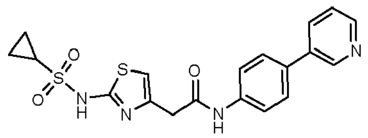 | Method 2, using INTB73 [HPLC acidic], 415, (1.02) | (Methanol-d4) 8.81 (dd, J = 2.4, 0.9 Hz, 1H), 8.51 (dd, J = 4.9, 1.6 Hz, 1H), 8.11 (ddd, J = 8.0, 2.4, 1.6 Hz, 1H), 7.80 - 7.74 (m, 2H), 7.72 - 7.65 (m, 2H), 7.56 - 7.51 (m, 1H), 6.53 (d, J = 1.0 Hz, 1H), 3.73 (d, J = 1.0 Hz, 2H), 2.63 tt, J = 8.1, 4.8 Hz, 1H), 1.13 - 1.07 (m, 2H), 0.99 - 0.92 (m, 2H). |
| **T245** | 2-(2-(cyclopropanesulfonamido)thiazol-4-yl)-N-(4-(pyridin-4-yl)phenyl)acetamide 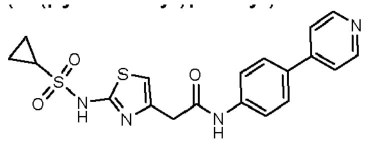 | Method 2, using INTB73 [HPLC acidic], 415, (0.94) | (Methanol-d4) 8.60 - 8.52 (m, 2H), 7.86 - 7.71 (m, 6H), 6.47 (d, J = 1.0 Hz, 1H), 3.66 (d, J = 1.0 Hz, 2H), 2.74 - 2.63 (m, 1H), 1.14 - 1.04 (m, 2H), 0.93 - 0.78 (m,2H). |
| **T246** | 2-(2-(cyclopropanesulfonamido)thiazol-4-yl)-N-(2'-methoxy-[1,1'-biphenyl]-4-yl)acetamide 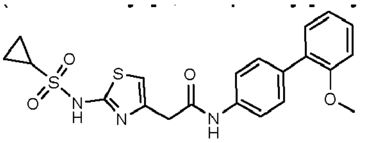 | Method 2, using INTB73 [HPLC acidic], 444, (2.02) | 12.57 (s, 1H), 10.25 (s, 1H), 7.65 - 7.59 (m, 2H), 7.47 - 7.40 (m, 2H), 7.36 - 7.23 (m, 2H), 7.10 (dd, J = 8.3, 1.1 Hz, 1H), 7.02 (td, J = 7.5, 1.1 Hz, 1H), 6.56 (s, 1H), 3.76 (s, 3H), 3.65 (s, 2H), 2.63 - 2.54 (m, 1H), 0.94 - 0.83(m, 4H). |
| **T247** | 2-(2-(cyclopropanesulfonamido)thiazol-4-yl)-N-(4-(pyrimidin-5-yl)phenyl)acetamide 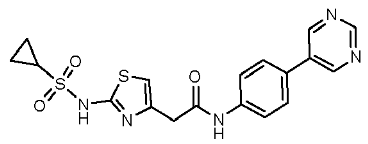 | Method 2, using INTB73 [UPLC acidic], 416, (0.86) | 12.56 (s, 1H), 10.40 (s, 1H), 9.16 (s, 1H), 9.14 (s, 2H), 7.84 - 7.80 (m, 2H), 7.79 - 7.73 (m, 2H), 6.57 (s, 1H), 3.68 (s, 2H), 2.63 - 2.54 (m, 1H), 0.96 - 0.82 (m, 4H). |
| **T248** | 2-(2-(cyclopropanesulfonamido)thiazol-4-yl)-2-methyl-N-(4-(2-(trifluoromethyl)pyridin-3-yl)phenyl) propanamide 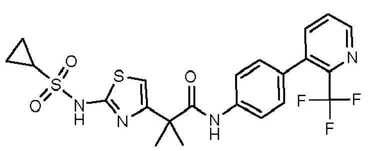 | Method 2 using INTB53, [UPLC acidic], 511, (1.3) | 12.56 (s, 1H), 9.43 (s, 1H), 8.78 - 8.71 (m, 1H), 7.93 - 7.86 (m, 1H), 7.80 - 7.67 (m, 3H), 7.30 (d, J = 8.4 Hz, 2H), 6.54 (s, 1H), 2.60 - 2.55 (m, 1H), 1.57 (s, 6H), 0.92 - 0.85 (m, 4H). |
| **T249** | 2-(2-(cyclopropanesulfonamido)thiazol-4-yl)-2-methyl-N-(5'-methyl-[3,3'-bipyridin]-6-yl)propanamide 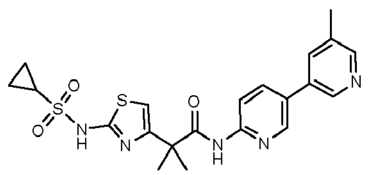 | Method 2 using INTB55, [UPLC acidic], 458, (0.77) | 12.54 (s, 1H), 9.99 (s, 1H), 8.73 (dd, J = 7.8, 2.3 Hz, 2H), 8.43 (d, J = 2.0 Hz, 1H), 8.20 (dd, J = 8.8, 2.5 Hz, 1H), 8.13 (d, J = 8.7 Hz, 1H), 7.97 (d, J = 2.5 Hz, 1H), 6.59 (s, 1H), 2.62 - 2.57 (m, 1H), 2.37 (s, 3H), 1.60 (s, 6H), 0.94 - 0.86 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T250** | 2-(2-(cyclopropanesulfonamido)thiazol-4-yl)-N-(4-(2-methoxy-4-methylpyridin-3-yl)phenyl)-2-methylpropanamide 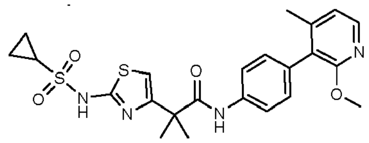 | Method 2 using INTB53, [UPLC acidic], 487, (1.3) | 12.56 (s, 1H), 9.33 (s, 1H), 8.00 (d, J = 5.2 Hz, 1H), 7.69 - 7.61 (m, 2H), 7.15 (d, J = 8.5 Hz, 2H), 6.96 - 6.90 (m, 1H), 6.52 (d, J = 12.8 Hz, 1H), 3.73 (s, 3H), 2.61 -2.56 (m, 1H), 2.05 (s, 3H), 1.56 (s, 6H), 0.93 - 0.74 (m, 4H). |
| **T251** | 2-(2-(cyclopropanesulfonamido)thiazol-4-yl)-N-(4-(6-methoxy-5-methylpyridin-3-yl)phenyl)-2-methylpropanamide 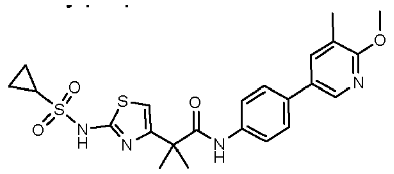 | Method 2 using INTB53, [UPLC acidic], 487, (1.4) | 12.57 (s, 1H), 9.37 (s, 1H), 8.30 (d, J = 2.4 Hz, 1H), 7.87 (d, J = 2.4 Hz, 1H), 7.71 (d, J = 8.6 Hz, 2H), 7.62 (d, J = 8.6 Hz, 2H), 6.54 (s, 1H), 3.92 (s, 3H), 2.52 (s, 1H), 2.21 (s, 3H), 1.58 (s, 6H), 0.91 (s, 4H). |
| **T252** | N-(4-(5-chloropyridin-3-yl)phenyl)-2-(2-(cyclopropanesulfonamido)thiazol-4-yl)-2-methylpropanamide 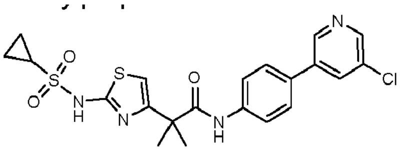 | Method 2 using INTB53, [UPLC acidic], 477, (1.3) | (Methanol-d4) 8.76 (d, J = 2.0 Hz, 1H), 8.52 (d, J = 2.3 Hz, 1H), 8.15 (d, J = 2.2 Hz, 1H), 7.77 (d, J = 8.4 Hz, 2H), 7.68 (d, J = 8.5 Hz, 2H), 6.61 (s, 1H), 3.37 (s, 1H), 1.67 (s, 6H), 1.11 (s, 2H), 0.96 (d, J = 6.4 Hz, 2H). 2 exchangable protons not observed |
| **T253** | 2-(2-(cyclopropanesulfonamido)thiazol-4-yl)-N-(4-(5-fluoropyridin-3-yl)phenyl)-2-methylpropanamide 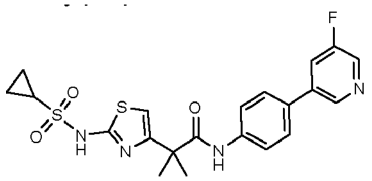 | Method 2 using INTB53, [UPLC acidic], 461, (1.2) | 12.56 (s, 1H), 9.44 (s, 1H), 8.80 (d, J = 2.0 Hz, 1H), 8.52 (d, J = 2.7 Hz, 1H), 8.05 (dt, J = 10.5, 2.3 Hz, 1H), 7.77 (d, J = 1.8 Hz, 4H), 6.53 (s, 1H), 2.58 (s, 1H), 1.57 (s, 6H), 0.92 - 0.85 (m, 4H). |
| **T254** | 2-(2-(cyclopropanesulfonamido)thiazol-4-yl)-2-methyl-N-(4-(4-methylpyridin-3-yl)phenyl)propanamide 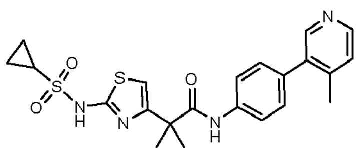 | Method 2 using INTB53, [UPLC acidic], 457, (0.7) | 12.56 (s, 1H), 9.42 (s, 1H), 8.44 - 8.33 (m, 2H), 7.78 - 7.69 (m, 2H), 7.40 - 7.29 (m, 3H), 6.57 (s, 1H), 2.66 - 2.54 (m, 1H), 2.28 (s, 3H), 1.59 (s, 6H), 0.96 - 0.85 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T255** | 2-(2-(cyclopropanesulfonamido)thiazol-4-yl)-2-methyl-N-(4-(4-(trifluoromethyl)pyridin-3-yl)phenyl) propanamide 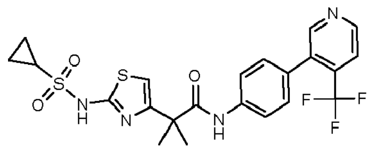 | Method 2 using INTB53, [UPLC acidic], 511, (1.3) | (Methanol-d4) δ 8.78 (s, 1H), 8.62 (s, 1H), 7.79 (d, J = 5.2 Hz, 1H), 7.74 - 7.67 (m, 2H), 7.33 (d, J = 8.3 Hz, 2H), 6.60 (s, 1H), 2.79 - 2.44 (m, 1H), 1.66 (s, 6H), 1.14 - 1.05 (m, 2H), 1.00 - 0.83 (m, 2H). |
| **T256** | N-(4-(5-chloropyridin-3-yl)-2-methoxyphenyl)-2-(2-(cyclopropanesulfonamido)thiazol-4-yl)-2-methylpropanamide 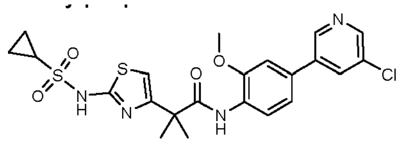 | Method 2 using INTB54, [HPLC acidic], 507, (2.04) | 12.65 (s, 1H), 8.92 (d, J = 2.0 Hz, 1H), 8.60 (d, J = 2.3 Hz, 1H), 8.39 (s, 1H), 8.31 (t, J = 2.2 Hz, 1H), 8.07 (d, J = 8.4 Hz, 1H), 7.49 - 7.37 (m, 2H), 6.76 (s, 1H), 3.93 (s, 3H), 2.64 - 2.59 (m, 1H), 1.57 (s, 6H), 0.96 - 0.88 (m, 4H). |
| **T257** | 2-(2-(cyclopropanesulfonamido)thiazol-4-yl)-N-(4-(5-(dimethylamino)pyridin-3-yl)phenyl)-2-methylpropanamide 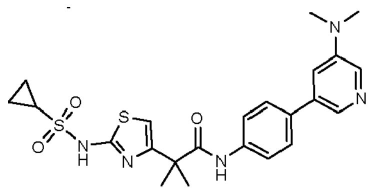 | Method 2 using INTB53, [UPLC acidic], 486, (0.76) | 12.55 (s, 1H), 9.41 (s, 1H), 8.16 (d, J = 1.8 Hz, 1H), 8.07 (d, J = 2.8 Hz, 1H),7.76-7.66 (m, 4H), 7.23 (t, J = 2.3 Hz, 1H), 6.54 (s, 1H), 2.99 (s, 6H), 2.63 - 2.54 (m, 1H), 1.56 (s, 6H), 0.93 - 0.83 (m, 4H). |
| **T258** | 2-(2-(cyclopropanesulfonamido)thiazol-4-yl)-N-(2-methoxy-4-(5-methylpyridin-3-yl)phenyl)-2-methylpropanamide 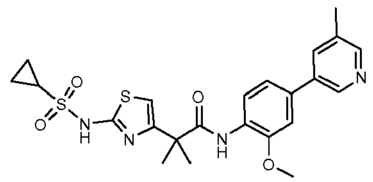 | Method 2 using INTB54, [UPLC acidic], 487, (0.86) | (Methanol-d4) 8.59 (d, J = 2.1 Hz, 1H), 8.37 - 8.31 (m, 1H), 8.13 (d, J = 8.2 Hz, 1H), 7.92 (m, 1H), 7.29 - 7.20 (m, 2H), 6.75 (s, 1H), 3.95 (s, 3H), 2.69 - 2.58 (m, 1H), 2.43 (d, J = 0.7 Hz, 3H), 1.65 (s, 6H), 1.17 - 1.02 (m, 2H), 1.05 - 0.90 (m, 2H). 2 exchangable protons not observed |
| **T259** | 2-(2-(cyclopropanesulfonamido)thiazol-4-yl)-N-(2-methoxy-4-(5-(trifluoromethyl)pyridin-3-yl)phenyl)-2-methylpropanamide 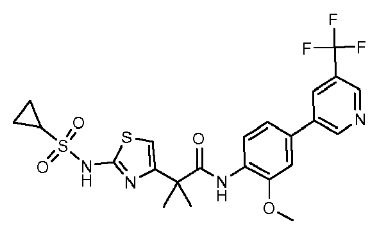 | Method 2 using INTB54, [UPLC acidic], 541, (1.43) | 12.69 (s, 1H), 9.26 (d, J = 2.1 Hz, 1H), 8.94 (d, J = 1.0 Hz, 1H), 8.51 (m, 1H), 8.40 (s (br), 1H), 8.07 (s (br), 1H), 7.55 - 7.43 (m, 2H), 6.74 (s (br), 1H), 3.94 (s, 3H), 2.71 - 2.55 (m, 1H), 1.56 (s, 6H), 0.92 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T260** | 2-(2-(cyclopropanesulfonamido)thiazol-4-yl)-N-(4-(6-methoxypyridin-3-yl)phenyl)acetamide 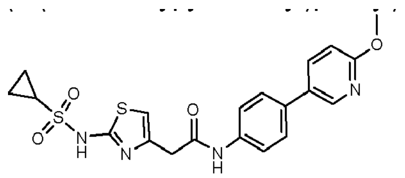 | Method 2, using INTB73 [UPLC acidic], 445, (1.13) | 12.52 (s, 1H), 10.27 (s, 1H), 8.46 (dd, J = 2.6, 0.8 Hz, 1H), 7.99 (dd, J = 8.7, 2.6 Hz, 1H), 7.70 - 7.66 (m, 2H), 7.63 (d, J = 6.9 Hz, 1H), 6.89 (dd, J = 8.6, 0.7 Hz, 1H), 6.54 (s, 1H), 3.88 (s, 3H), 3.65 (s, 2H), 2.60 - 2.55 (m, 1H), 0.92 - 0.83 (m, 4H). |
| **T261** | 2-(2-(cyclopropanesulfonamido)thiazol-4-yl)-N-(5'-fluoro-[3,3'-bipyridin]-6-yl)-2-methylpropanamide 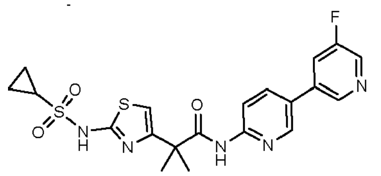 | Method 2 using INTB53, [UPLC acidic], 462, (1.1) | 12.57 (s, 1H), 10.07 (s, 1H), 8.87 (d, J = 1.9 Hz, 1H), 8.81 (d, J = 2.5 Hz, 1H), 8.60 (d, J = 2.7 Hz, 1H), 8.28 (dd, J = 8.7, 2.6 Hz, 1H), 8.22 - 8.11 (m, 2H), 6.57 (s, 1H), 2.62 - 2.57 (m, 1H), 1.61 (s, 6H), 0.94 - 0.87 (m, 4H). |
| **T262** | N-(5-(6-chloropyrazin-2-yl)pyridin-2-yl)-2-(2-(cyclopropanesulfonamido)thiazol-4-yl)-2-methylpropanamide 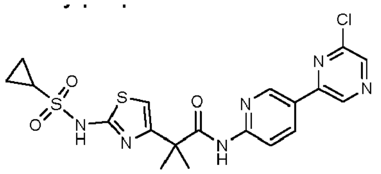 | Method 1b using INTB37 and INTA64, [HPLC basic], 479, (1.8) | 12.56 (s, 1H), 10.42 (s, 1H), 9.33 (s, 1H), 9.10 (s, 1H), 8.77 (s, 1H), 8.53 (dd, J = 8.7, 2.4 Hz, 1H), 8.23 (dd, J = 8.9, 0.8 Hz, 1H), 6.55 (s, 1H), 2.62-2.55 (m, 1H), 1.59 (s, 6H), 0.94 - 0.79 (m, 4H). |
| **T263** | N-(5-(6-cyanopyrazin-2-yl)pyridin-2-yl)-2-(2-(cyclopropanesulfonamido)thiazol-4-yl)-2-methvlpropanamide 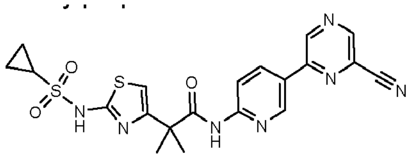 | Method 1b using INTB37 and INTA63, [HPLC basic], 470, (1.7) | 12.59 (s, 1H), 10.27 (s, 1H), 9.64 (s, 1H), 9.19 (s, 1H), 9.16 (d, J = 2.5 Hz, 1H), 8.60 (dd, J = 8.8, 2.5 Hz, 1H), 8.24 (d, J = 8.8 Hz, 1H), 6.59 (s, 1H), 2.64-2.55 (m, 1H), 1.62 (s, 6H), 0.96 - 0.84 (m, 4H) |
| **T264** | 2-(2-(cyclopropanesulfonamido)thiazol-4-yl)-2-methyl-N-(5-(pyrimidin-5-yl)pyridin-2-yl)propanamide 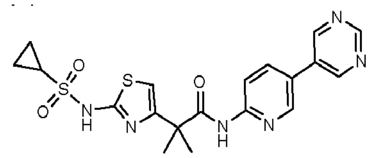 | Method 1b using INTB37, [UPLC acidic], 445, (0.92) | (Methanol-d4) 9.18 (s, 1H), 9.13 (s, 2H), 8.69 (d, J = 2.5 Hz, 1H), 8.32 (d, J = 8.7 Hz, 1H), 8.21 (dd, J = 8.7, 2.5 Hz, 1H), 6.72 (s, 1H), 2.68 (s, 1H), 1.69 (s, 6H), 1.16 - 1.08 (m, 2H), 1.03 - 0.93 (m, 2H), 2 exchangable protons not observed |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T265 | 2-(2-(cyclopropanesulfonamido)thiazol-4-yl)-N-(4-(6-methoxypyrazin-2-yl)phenyl)-2-methylpropanamide 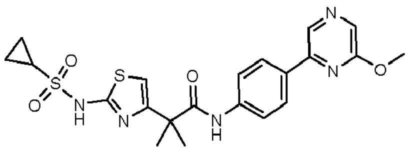 | Method 2b using INTB56, [HPLC acidic], 474, (1.93) | 12.60 (s, 1H), 9.52 (s, 1H), 8.80 (s, 1H), 8.22 (s, 1H), 8.17 - 8.08 (m, 2H), 7.87 - 7.72 (m, 2H), 6.55 (s, 1H), 4.02 (s, 3H), 2.66 - 2.54 (m, 1H), 1.59 (s, 6H), 0.97 - 0.82 (m, 4H). |
| T266 | 2-(2-(cyclopropanesulfonamido)thiazol-4-yl)-2-methyl-N-(4-(6-methylpyrazin-2-yl)phenyl)propanamide 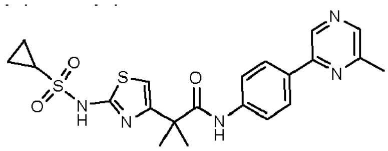 | Method 2b using INTB56, [HPLC acidic], 458, (2.13) | 12.65 (s, 1H), 9.56 (s, 1H), 9.09 (s, 1H), 8.52 (s, 1H), 8.24 - 8.12 (m, 2H), 7.91 - 7.78 (m, 2H), 6.62 (s, 1H), 2.69 - 2.63 (m, 1H),2.62 (s, 3H), 1.65 (s, 6H), 1.02 - 0.90 (m, 4H). |
| T267 | 2-(2-(cyclopropanesulfonamido)thiazol-4-yl)-2-methyl-N-(4-(6-(trifluoromethyl)pyrazin-2-yl)phenyl) propanamide 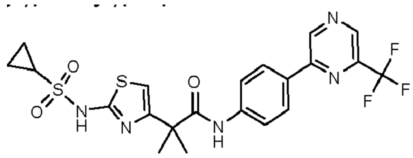 | Method 2b using INTB56, [HPLC acidic], 512, (2.65) | 12.60 (s, 1H), 9.60 (s, 2H), 9.09 (s, 1H), 8.28 - 8.15 (m, 2H), 7.94 - 7.81 (m, 2H), 6.57 (s, 1H), 2.64 - 2.55 (m, 1H), 1.59 (s, 6H), 0.97 - 0.86 (m, 4H). |
| T268 | N-(4-(6-chloropyridin-2-yl)phenyl)-2-(2-(cyclopropanesulfonamido)thiazol-4-yl)-2-methylpropanamide 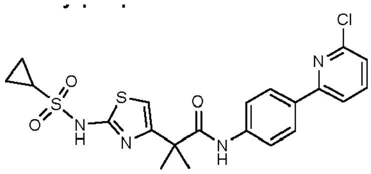 | Method 2b using INTB56, [HPLC acidic], 477, (2.1) | (Methanol-d4) 8.05 - 7.98 (m, 2H), 7.87 - 7.78 (m, 2H), 7.75 - 7.70 (m, 2H), 7.33 (dd, J = 6.6, 2.0 Hz, 1H), 6.62 (s, 1H), 2.70 - 2.59 (m, 1H), 1.67 (s, 6H), 1.14 - 1.07 (m, 2H), 1.02 - 0.92 (m, 2H), 2 exchangable protons not observed |
| T269 | 2-(2-(cyclopropanesulfonamido)thiazol-4-yl)-N-(4-(6-methoxypyridin-2-yl)phenyl)-2-methylpropanamide 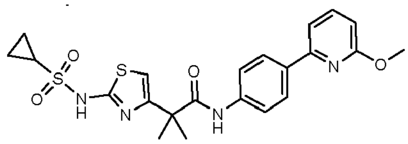 | Method 2b using INTB56, [HPLC acidic], 473, (2.11) | 12.57 (s, 1H), 9.44 (s, 1H), 8.13 - 8.05 (m, 2H), 7.80 - 7.71 (m, 3H), 7.56 - 7.49 (m, 1H), 6.78 - 6.70 (m, 1H), 6.54 (s, 1H), 3.96 (s, 3H), 2.65 - 2.56 (m, 1H), 1.58 (s, 6H), 0.95 - 0.83 (m, 4H) |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T270** | 2-(2-(cyclopropanesulfonamido)thiazol-4-yl)-2-methyl-N-(4-(6-(trifluoromethyl)pyridin-2-yl)phenyl) propanamide 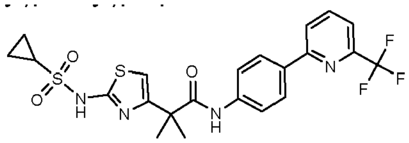 | Method 2b using INTB56, [HPLC acidic], 511, (2.24) | 12.58 (s, 1H), 9.51 (s, 1H), 8.27 (d, J = 8.1 Hz, 1H), 8.18 - 8.08 (m, 3H), 7.87 - 7.77 (m, 3H), 6.55 (s, 1H), 2.64 - 2.56 (m, 1H), 1.59 (s, 6H), 0.97 - 0.84 (m, 4H). |
| **T271** | 2-(2-(cyclopropanesulfonamido)thiazol-4-yl)-N-(4-(4-methoxypyridin-2-yl)phenyl)-2-methylpropanamide 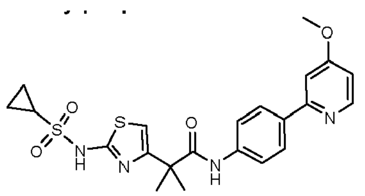 | Method 2b using INTB56, [HPLC acidic], 473, (1.15) | 12.55 (s, 1H), 9.42 (s, 1H), 8.44 (d, J = 5.6 Hz, 1H), 8.12 - 8.06 (m, 2H), 7.80 - 7.69 (m, 2H), 7.46 (d, J = 2.4 Hz, 1H), 6.91 (dd, J = 5.7, 2.3 Hz, 1H), 6.56 (s, 1H), 3.91 (s, 3H), 2.65 - 2.54 (m, 1H), 1.58 (s, 6H), 0.97 - 0.85 (m, 4H). |
| **T272** | 2-(2-(cyclopropanesulfonamido)thiazol-4-yl)-N-(4-(6-isopropoxypyrazin-2-yl)phenyl)-2-methylpropanamide 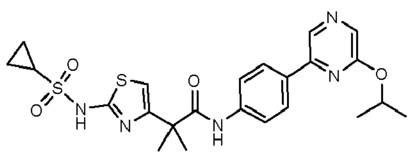 | Method 2b using INTB56, [HPLC basic], 502, (1.9) | 12.58 (s, 1H), 9.50 (s, 1H), 8.75 (s, 1H), 8.13 (s, 1H), 8.11 - 8.06 (m, 2H), 7.85 - 7.73 (m, 2H), 6.55 (s, 1H), 5.49 - 5.35 (m, 1H), 2.64 - 2.55 (m, 1H), 1.59 (s, 6H), 1.39 (d, J = 6.2 Hz, 6H), 0.97 - 0.84 (m, 4H). |
| **T273** | 2-(2-(cyclopropanesulfonamido)thiazol-4-yl)-N-(4-(6-cyclopropoxypyrazin-2-yl)phenyl)-2-methylpropanamide 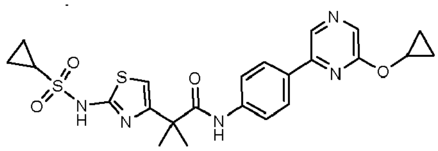 | Method 2b, [UPLC basic], 500, (1.2) | 12.58 (s, 1H), 9.50 (s, 1H), 8.85 (s, 1H), 8.24 (s, 1H), 8.15 - 8.11 (m, 2H), 7.83 - 7.78 (m, 2H), 6.55 (s, 1H), 4.45-4.39 (m, 1H), 2.65 - 2.54 (m, 1H), 1.59 (s, 6H), 0.95 - 0.75 (m, 8H). |
| **T274** | 2-(2-(cyclopropanesulfonamido)thiazol-4-yl)-N-(2-fluoro-4-(pyrazin-2-yl)phenyl)-2-methylpropanamide 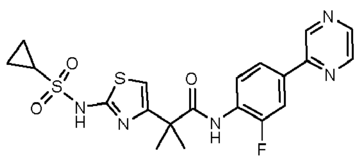 | Method 2b using INTB57, [UPLC acidic], 462, (1.1) | (Methanol-d4) 10.68 (d, J = 1.5 Hz, 1H), 10.23 (dd, J = 2.6, 1.5 Hz, 1H), 10.09 (d, J = 2.5 Hz, 1H), 9.55 - 9.40 (m, 3H), 8.23 (s, 1H), 4.26 - 4.11 (m, 1H), 3.22 (s, 6H), 2.68 - 2.61 (m, 2H), 2.58 - 2.46 (m, 2H). v |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T275** | 2-(2-(cyclopropanesulfonamido)thiazol-4-yl)-N-(2-fluoro-4-(6-methoxypyrazin-2-yl)phenyl)-2-methylpropanamide 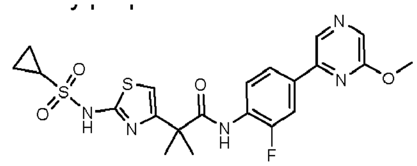 | Method 2b using INTB57, [UPLC acidic], 492, (1.3) | 12.62 (s, 1H), 9.28 (s, 1H), 8.88 (s, 1H), 8.29 (s, 1H), 8.10 - 7.98 (m, 2H), 7.72 (s, 1H), 6.61 (s, 1H), 4.04 (s, 3H), 2.62 - 2.57 (m, 1H), 1.59 (s, 6H), 1.07 - 0.73 (m, 4H). |
| **T276** | N-(4-(6-chloro-3-methylpyrazin-2-yl)phenyl)-2-(2-(cyclopropanesulfonamido)thiazol-4-yl)-2-methylpropanamide 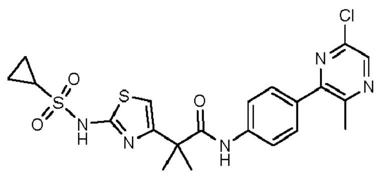 | Method 2b using INTB56, [HPLC basic], 493, (1.73) | 12.59 (s, 1H), 8.62 (s, 1H), 7.90 - 7.75 (m, 2H), 7.71 - 7.54 (m, 2H), 6.44 (s, 1H), 2.60 (s, 4H), 1.54 (s, 6H), 0.99 - 0.74 (m, 4H) 1 exchangable protons not observed |
| **T277** | N-(4-(6-chloro-5-methylpyrazin-2-yl)phenyl)-2-(2-(cyclopropanesulfonamido)thiazol-4-yl)-2-methylpropanamide 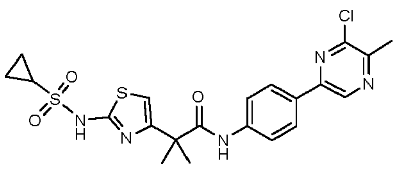 | Method 2b using INTB56, [HPLC basic], 493, (1.85) | 12.57 (s, 1H), 9.55 (s, 1H), 9.12 (s, 1H), 8.15 - 8.04 (m, 2H), 7.91 - 7.72 (m, 2H), 6.63 - 6.42 (m, 1H), 2.66 - 2.56 (m, 4H), 1.58 (s, 6H), 0.97 - 0.82 (m, 4H) |
| **T278** | 2-(2-(cyclopropanesulfonamido)thiazol-4-yl)-2-methyl-N-(4-(6-(pyrrolidin-1-yl)pyrazin-2-yl)phenyl) propanamide 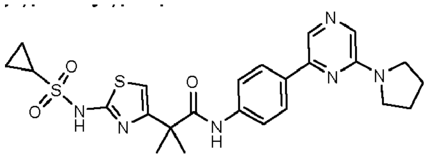 | Method 2b using INTB56, [HPLC basic], 513, (1.86) | 12.56 (s, 1H), 9.45 (s, 1H), 8.34 (s, 1H), 8.10 - 8.00 (m, 2H), 7.88 (s, 1H), 7.79 - 7.70 (m, 2H), 6.56 (s, 1H), 3.62 - 3.42 (m, 4H), 2.59 (d, J = 6.0 Hz, 1 H), 2.06 - 1.93 (m, 4H), 1.59 (s, 6H), 0.99 - 0.82 (m, 4H). |
| **T279** | 2-(2-(cyclopropanesulfonamido)thiazol-4-yl)-N-(4-(6-(2-(dimethylamino)ethoxy)pyrazin-2-yl) phenyl)-2-methylpropanamide 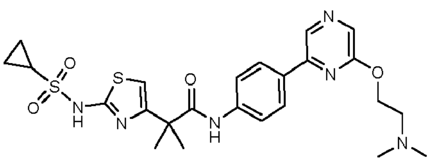 | Method 2b using INTB56, [HPLC basic], 531, (1.57) | 11.6 (s, 1H), 10.07 (s, 1H), 8.81 (s, 1H), 8.20 (s, 1H), 8.17 - 8.07 (m, 2H), 7.88 - 7.79 (m, 2H), 6.46 (s, 1H), 4.60 (t, J = 5.5 Hz, 2H), 3.01-2.93 (m, 2H), 2.65-2.57 (m, 1H), 2.46 (s, 6H), 1.55 (s, 6H), 0.96 - 0.75 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T280** | 2-(2-(cyclopropanesulfonamido)thiazol-4-yl)-2-methyl-N-(4-(3-methylpyrazin-2-yl)phenyl)propanamide 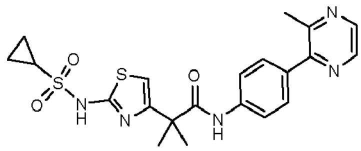 | Method 2b using INTB56, [HPLC basic], 458, (1.46) | 12.59 (s, 1H), 9.47 (s, 1H), 8.54 (d, J = 2.5 Hz, 1H), 8.50 (d, J = 2.5 Hz, 1H), 7.80 - 7.75 (m, 2H), 7.66 - 7.61 (m, 2H), 6.56 (s, 1H), 2.64-2.57 (m, 4H), 1.59 (s, 6H), 0.91 (s, 4H). |
| **T281** | N-(4-(6-acetamidopyrazin-2-yl)phenyl)-2-(2-(cyclopropanesulfonamido)thiazol-4-yl)-2-methylpropanamide 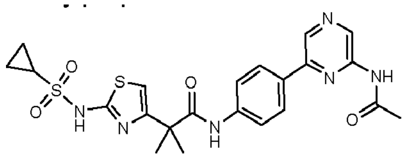 | Method 2b using INTB56, [HPLC acidic], 501, (1.65) | 12.57 (s, 1H), 10.78 (s, 1H), 9.54 - 9.43 (m, 1H), 9.21 (s, 1H), 8.91 (s, 1H), 8.12 - 8.06 (m, 2H), 7.84 - 7.77 (m, 2H), 6.55 (s, 1H), 2.65 - 2.53 (m, 1H), 2.16 (s, 3H), 1.58 (s, 6H), 0.96 - 0.84 (m, 4H). |
| **T282** | 2-(2-(cyclopropanesulfonamido)thiazol-4-yl)-N-(4-(5,6-dimethylpyrazin-2-yl)phenyl)-2-methylpropanamide 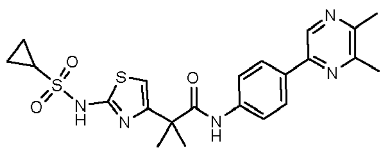 | Method 2b using INTB56, [HPLC acidic], 473, (1.86) | 12.57 (s, 1H), 9.46 (s, 1H), 8.87 (s, 1H), 8.10 - 8.03 (m, 2H), 7.80 - 7.73 (m, 2H), 6.54 (s, 1H), 2.65 - 2.56 (m, 1H), 2.54 (s, 3H), 1.57 (s, 6H), 0.96 - 0.83 (m, 4H). |
| **T283** | 2-(2-(cyclopropanesulfonamido)thiazol-4-yl)-N-(4-(6-(hydroxymethyl)pyrazin-2-yl)phenyl)-2-methylpropanamide 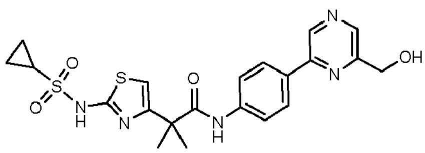 | Method 2b using INTB56, [HPLC acidic], 474, (1.54) | 12.58 (s, 1H), 9.50 (s, 1H), 9.11 (s, 1H), 8.62 (s, 1H), 8.17 - 8.10 (m, 2H), 7.84 - 7.78 (m, 2H), 6.56 (s, 1H), 5.62 (t, J = 5.9 Hz, 1H), 4.70 (d, J = 5.8 Hz, 2H), 2.59 (s, 1H), 1.58 (s, 6H), 1.00 - 0.83 (m, 4H). |
| **T284** | 2-(2-(cyclopropanesulfonamido)thiazol-4-yl)-N-(4-(3,6-dimethylpyrazin-2-yl)phenyl)-2-methylpropanamide 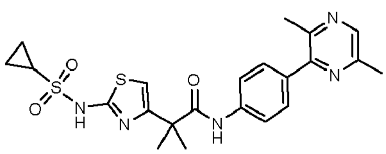 | Method 2b using INTB56, [HPLC acidic], 472, (1.72) | 12.58 (s, 1H), 9.45 (s, 1H), 8.37 (s, 1H), 7.78 - 7.72 (m, 2H), 7.62 - 7.56 (m, 2H), 6.55 (s, 1H), 2.63 - 2.54 (m, 1H), 2.52 (s, 3H), 1.58 (s, 6H), 0.95 - 0.85 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T285 | 2-(2-(cyclopropanesulfonamido)thiazol-4-yl)-N-(4-(5-methoxypyridin-3-yl)-2-methylphenyl)-2-methylpropanamide 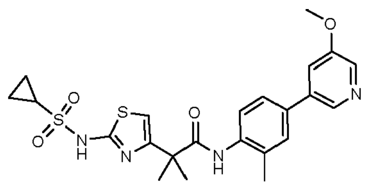 | Method 2b using INTB60, [HPLC acidic], 487, (1.58) | 12.58 (s, 1H), 8.95 (s, 1H), 8.49 (d, J = 1.8 Hz, 1H), 8.27 (d, J = 2.7 Hz, 1H), 7.67 - 7.61 (m, 2H), 7.59 (dd, J = 8.2, 2.2 Hz, 1H), 7.45 - 7.34 (m, 1H), 6.63 (s, 1H), 3.92 (s, 3H), 2.64 - 2.54 (m, 1H), 2.23 (s, 3H), 1.59 (s, 6H), 0.97 - 0.86 (m, 4H). |
| T286 | N-(4-(5-cyanopyridin-3-yl)-2-methylphenyl)-2-(2-(cyclopropanesulfonamido)thiazol-4-yl)-2-methylpropanamide 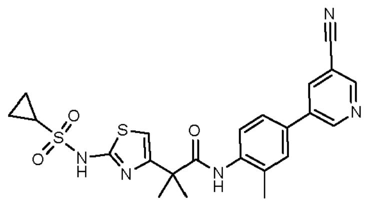 | Method 2b using INTB60, [HPLC acidic], 482, (1.6) | 12.60 (s, 1H), 9.21 (d, J = 2.3 Hz, 1H), 9.03 - 8.91 (m, 2H), 8.67 (t, J = 2.1 Hz, 1H), 7.78 - 7.72 (m, 1H), 7.68 (dd, J = 8.3, 2.3 Hz, 1H), 7.47 7.37 (m, 1H), 6.62 (s, 1H), 2.64 - 2.54 (m, 1H), 2.24 (s, 3H), 1.59 (s, 6H), 0.99 - 0.83 (m, 4H). |
| T287 | 2-(2-(cyclopropanesulfonamido)thiazol-4-yl)-N-(4-(5-fluoropyridin-3-yl)-2-methylphenyl)-2-methylpropanamide 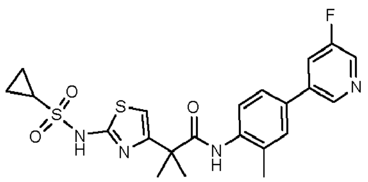 | Method 2b using INTB60, [UPLC acidic], 475, (1.18) | 12.60 (s, 1H), 8.96 (s, 1H), 8.82 (t, J = 1.9 Hz, 1H), 8.56 (d, J = 2.7 Hz, 1H), 8.08 (ddd, J = 10.5, 2.7, 1.8 Hz, 1H), 7.70 (d, J = 2.2 Hz, 1H), 7.64 (dd, J = 8.2, 2.3 Hz, 1H), 7.42 (d, J = 8.2 Hz, 1H), 6.62 (s, 1H), 2.59 (s, 1H), 2.23 (s, 3H), 1.59 (s, 6H), 0.91 (d, J = 8.1 Hz, 4H). |
| T288 | N-(4-(5-chloropyridin-3-yl)-3-methylphenyl)-2-(2-(cyclopropanesulfonamido)thiazol-4-yl)-2-methylpropanamide 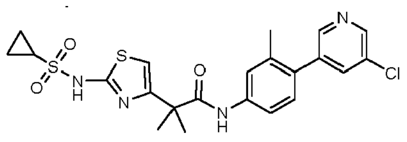 | Method 2 using INTB59, [UPLC acidic], 491, (1.33) | 12.57 (s, 1H), 9.36 (s, 1H), 8.62 (d, J = 2.3 Hz, 1H), 8.52 (d, J = 1.9 Hz, 1H), 7.98 - 7.93 (m, 1H), 7.61 - 7.55 (m, 2H), 7.27 - 7.21 (m, 1H), 6.53 (s, 1H), 2.63 - 2.54 (m, 1H), 2.24 (s, 3H), 1.57 (s, 6H), 0.95 - 0.85 (m, 4H). |
| T289 | N-(4-(5-cyanopyridin-3-yl)-3-ethoxyphenyl)-2-(2-(cyclopropanesulfonamido)thiazol-4-yl)-2-methylpropanamide 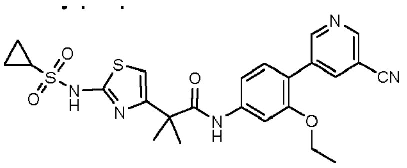 | Method 2, using INTB71 [UPLC acidic], 512, (1.29) | 12.58 (s, 1H), 9.44 (s, 1H), 9.03 - 8.99 (m, 1H), 8.94 - 8.90 (m, 1H), 8.44 - 8.39 (m, 1H), 7.56 (s, 1H), 7.46 - 7.36 (m, 2H), 6.54 (s, 1H), 4.10 - 3.98 (m, 2H), 2.61 - 2.57 (m, 1H), 1.58 (s, 6H), 1.31 (t, J = 6.9, 1.1 Hz, 3H), 0.93 - 0.89 (m, 4H). |

(continued)

| T# | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| **T290** | 2-(2-(cyclopropanesulfonamido)thiazol-4-yl)-N-(4-(5-ethoxypyridin-3-yl)phenyl)-2-methylpropanamide 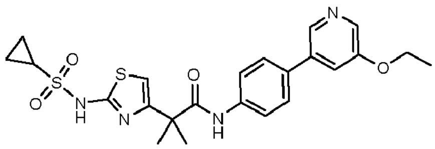 | Method 2b using INTB56, [HPLC acidic], 487, (1.6) | 12.55 (s, 1H), 9.43 (s, 1H), 8.49 (d, J = 1.8 Hz, 1H), 8.25 (d, J = 2.7 Hz, 1H), 7.75 (s, 4H), 7.62 (m, 1H), 6.57 (s, 1H), 4.21 (q, J = 7.0 Hz, 2H), 2.59 (m, |
| **T291** | 2-(2-(cyclopropanesulfonamido)thiazol-4-yl)-N-(4-(6-cyclopropylpyrazin-2-yl)phenyl)butanamide 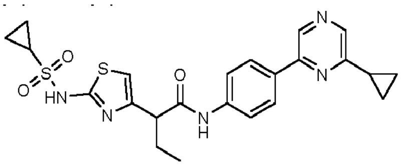 | Method 2c, using INTB38 [UPLC acidic], 484, (1.37) | 12.62 (s, 1H), 10.36 (s, 1H), 8.94 (s, 1H), 8.52 (s, 1H), 8.10 - 8.04 (m, 2H), 7.80 - 7.71 (m, 2H), 6.55 (s, 1H), 3.61 (t, J = 7.4 Hz, 1H), 2.64 - 2.56 (m, 1H), 2.29 - 2.18 (m, 1H), 2.03 - 1.82 (m, 2H), 1.11 - 1.03 (m, 4H), 0.94 - 0.85 (m, 7H). |
| **T292** | 2-(2-(cyclopropanesulfonamido)thiazol-4-yl)-N-(5-(5-methoxypyridin-3-yl)pyrimidin-2-yl)-2-methylpropanamide 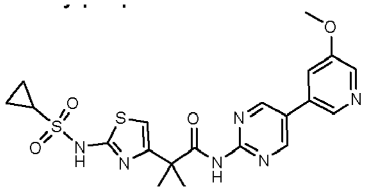 | Method 3 using INTB53, [UPLC acidic], 475, (0.82) | 12.58 (s, 1H), 10.25 (s, 1H), 9.11 (s, 2H), 8.59 (d, J = 1.8 Hz, 1H), 8.35 (d, J = 2.8 Hz, 1H), 7.80 (t, J = 2.3 Hz, 1H), 6.57 (s, 1H), 3.91 (s, 3H), 2.59 (td, J = 7.3, 3.4 Hz, 1H), 1.59 (s, 6H), 0.90 (dd, J = 6.3, 3.3 Hz, 4H). |
| **T293** | 2-(2-(cyclopropanesulfonamido)thiazol-4-yl)-N-(5-(5-fluoropyridin-3-yl)pyrimidin-2-yl)-2-methylpropanamide 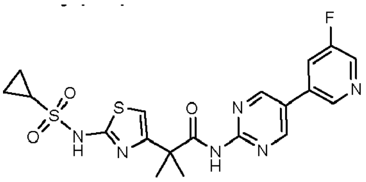 | Method 3 using INTB53, [UPLC acidic], 463, (0.9) | 12.63 (s, 1H), 10.34 (s, 1H), 9.15 (s, 2H), 8.93 (t, J = 1.8 Hz, 1H), 8.64 (d, J = 2.7 Hz, 1H), 8.27 (dt, J = 10.3, 2.3 Hz, 1H), 6.55 (s, 1H), 2.61 - 2.56 (m, 1H), 1.59 (s, 6H), 0.93 - 0.86 (m, 4H). |
| **T294** | 2-(2-(cyclopropanesulfonamido)thiazol-4-yl)-2-methyl-N-(5-(5-(trifluoromethyl)pyridin-3-yl)pyrimidin-2-yl) propanamide 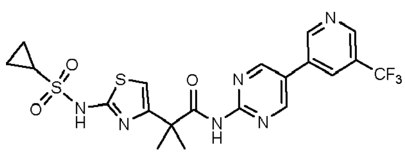 | Method 3 using INTB53, [UPLC acidic], 513, (1.1) | 12.62 (s, 1H), 10.34 (s, 1H), 9.32 (d, J = 2.1 Hz, 1H), 9.20 (s, 2H), 9.03 (d, J = 2.1 Hz, 1H), 8.70 - 8.62 (m, 1H), 6.56 (s, 1H), 2.61 - 2.56 (m, 1H), 1.59 (s, 6H), 0.93 - 0.88 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T295** | N-(5-(6-ethoxypyrazin-2-yl)pyridin-2-yl)-2-methyl-2-(2-((2-methylpropyl)sulfonamido)thiazol-4-yl) propanamide 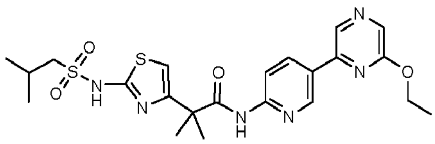 | Method 1b using INTB52 and INTA72, [UPLC acidic], 506, (1.45) | 12.51 (s, 1H), 10.08 (s, 1H), 9.07 (s, 1H), 8.85 (s, 1H), 8.51 (d, J = 9.3 Hz, 1H), 8.25 (s, 1H), 8.18 (dd, J = 8.8, 0.8 Hz, 1H), 6.65 - 6.49 (m, 1H), 4.48 (q, J = 7.1 Hz, 2H), 2.95 - 2.83 (m, 2H), 2.16 - 2.00 (m, 1H), 1.59 (s, 6H), 1.40 (t, J = 7.0 Hz, 3H), 1.05 - 0.95 (m, 6H). |
| **T296** | N-(4-(6-ethoxypyrazin-2-yl)phenyl)-2-methyl-2-(2-((trifluoromethyl)sulfonamido)thiazol-4-yl) propanamide 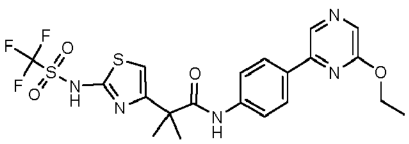 | Method 1b using INTB48 and INTA38, [UPLC acidic], 516, (1.59) | 13.93 (v. br. s, 1H), 9.69 (v. br. s, 1H), 8.77 (s, 1H), 8.17 (s, 1H), 8.13 - 8.04 (m, 2H), 7.82 - 7.67 (m, 2H), 6.83 (s, 1H), 4.47 (q, J = 7.1 Hz, 2H), 1.57 (s, 6H), 1.40 (t, J = 7.0 Hz, 3H). |
| **T297** | 2-methyl-2-(2-((1-methylethyl)sulfonamido)thiazol-4-yl)-N-(4-(pyridin-3-yl)phenyl)propanamide 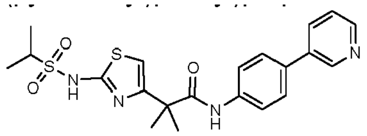 | Method 1b using INTB49, [HPLC acidic], 445, (1.22) | 12.51 (s, 1H), 9.41 (s, 1H), 8.89 (d, J = 2.4 Hz, 1H), 8.59 - 8.41 (m, 1H), 8.10 - 8.00 (m, 1H), 7.78 - 7.73 (m, 2H), 7.73 - 7.69 (m, 2H), 7.46 (dd, J = 8.0, 4.7 Hz, 1H), 6.54 (s, 1H), 3.15 - 3.07 (m, 1H), 1.57 (s, 6H), 1.23 (d, J = 6.8 Hz, 6H). |
| **T298** | N-(4-(6-ethoxypyrazin-2-yl)phenyl)-2-methyl-2-(2-((1-methylethyl)sulfonamido)thiazol-4-yl)propanamide 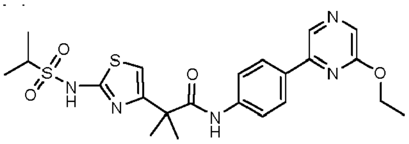 | Method 1b using INTB49 and INTA38, [HPLC acidic], 490, (2.1) | 12.54 (s, 1H), 9.48 (s, 1H), 8.77 (s, 1H), 8.18 (s, 1H), 8.14 - 8.07 (m, 2H), 7.81 - 7.75 (m, 2H), 6.52 (s, 1H), 4.47 (q, J = 7.1 Hz, 2H), 3.16 - 3.04 (m, 1H), 1.57 (s, 6H), 1.40 (t, J = 7.0 Hz, 3H), 1.22 (d, J = 6.8 Hz, 6H). |
| **T299** | 2-methyl-2-(2-((1-methylcyclopropane)-1-sulfonamido)thiazol-4-yl)-N-(4-(pyridin-3-yl)phenyl) propanamide 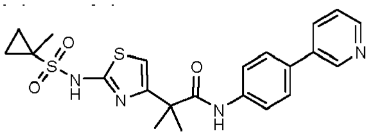 | Method 1b using INTB50, [HPLC acidic], 457, (1.26) | 12.56 (s, 1H), 9.42 (s, 1H), 8.89 (dd, J = 2.4, 0.9 Hz, 1H), 8.53 (dd, J = 4.8, 1.6 Hz, 1H), 8.06 (ddd, J = 8.0, 2.4, 1.6 Hz, 1H), 7.77 - 7.74 (m, 2H), 7.74 - 7.68 (m, 2H), 7.46 (ddd, J = 7.9, 4.8, 0.9 Hz, 1H), 6.53 (s, 1H), 1.57 (s, 6H), 1.40 (s, 3H), 1.20 - 1.11 (m, 2H), 0.81 - 0.67 (m, 2H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **T300** | N-(4-(5-chloropyridin-3-yl)phenyl)-2-methyl-2-(2-((1-methylcyclopropane)-1-sulfonamido)thiazol-4-yl) propanamide 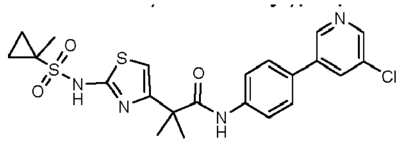 | Method 1b using INTB50 and INTA18, [HPLC acidic], 491, (2.05) | 12.59 (s, 1H), 9.45 (s, 1H), 8.87 (d, J = 2.1 Hz, 1H), 8.58 (d, J = 2.3 Hz, 1H), 8.24 (t, J = 2.2 Hz, 1H), 7.86 - 7.72 (m, 4H), 6.51 (s, 1H), 1.66 - 1.49 (m, 6H), 1.40 (s, 3H), 1.23 - 1.07 (m, 2H), 0.77 - 0.66 (m, 2H). |
| **T301** | N-(4-(6-ethoxypyrazin-2-yl)phenyl)-2-methyl-2-(2-((1-methylcyclopropane)-1-sulfonamido)thiazol-4-yl) propanamide 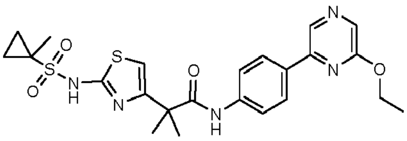 | Method 1b using INTB50 and INTA38, [HPLC acidic], 502, (2.16) | 12.59 (s, 1H), 9.49 (s, 1H), 8.77 (s, 1H), 8.18 (s, 1H), 8.11 - 8.03 (m, 2H), 7.79 (d, J = 8.7 Hz, 2H), 6.52 (s, 1H), 4.47 (q, J = 7.1 Hz, 2H), 1.58 (s, 6H), 1.51 - 1.36 (m, 6H), 1.22 - 1.13 (m, 2H), 0.81 - 0.70 (m, 2H). |
| **T302** | 2-methyl-2-(2-((1-methylcyclopropane)-1-sulfonamido)thiazol-4-yl)-N-(4-(6-(trifluoromethyl) pyrazin-2-yl)phenyl)propanamide 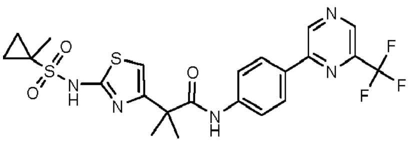 | Method 1b using INTB50 and INTA39, [HPLC acidic], 526, (2.22) | 12.60 (s, 1H), 9.59 (s, 1H), 9.58 (s, 1H), 9.08 (s, 1H), 8.23 - 8.17 (m, 2H), 7.89 - 7.84 (m, 2H), 6.53 (s, 1H), 1.58 (s, 6H), 1.40 (s, 3H), 1.23 - 1.10 (m, 2H), 0.81 - 0.70 (m, 2H). |
| **T303** | 2-(2-((1,1-dimethylethyl)sulfonamido)thiazol-4-yl)-2-methyl-N-(4-(pyridin-3-yl)phenyl)propanamide 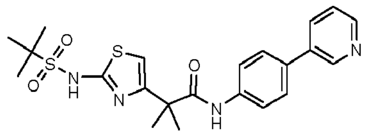 | Method 1b using INTB51, [HPLC acidic], 459, (1.31) | 12.51 (s, 1H), 9.45 (s, 1H), 8.89 (dd, J = 2.5, 0.9 Hz, 1H), 8.53 (dd, J = 4.7, 1.6 Hz, 1H), 8.06 (ddd, J = 8.0, 2.5, 1.6 Hz, 1H), 7.77 - 7.73 (m, 2H), 7.73 - 7.69 (m, 2H), 7.46 (ddd, J = 8.0, 4.7, 0.9 Hz, 1H), 6.50 (s, 1H), 1.57 (s, 6H), 1.28 (s, 9H). |
| **T304** | 2-(2-((1,1-dimethylethyl)sulfonamido)thiazol-4-yl)-N-(4-(6-ethoxypyrazin-2-yl)phenyl)-2-methylpropanamide 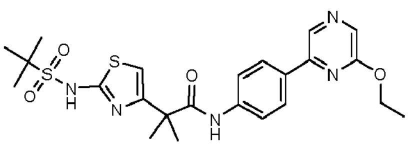 | Method 1b using INTB51 and INTA38, [HPLC acidic], 504, (2.2) | 12.51 (s, 1H), 9.48 (s, 1H), 8.77 (d, J = 0.6 Hz, 1H), 8.18 (d, J = 0.5 Hz, 1H), 8.14 - 8.07 (m, 2H),7.81 - 7.73 (m, 2H), 6.51 (s, 1H), 4.47 (q, J = 7.1 Hz, 2H), 1.58 (s, 6H), 1.40 (t, J = 7.0 Hz, 3H), 1.28 (s, 9H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| T305 | 2-(2-((1,1-dimethylethyl)sulfonamido)thiazol-4-yl)-2-methyl-N-(4-(6-(trifluoromethyl)pyrazin-2-yl)phenyl) propanamide 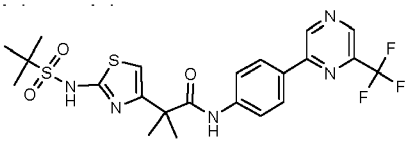 | Method 1 b using INTB51 and INTA39, [HPLC acidic], 528, (2.26) | 12.52 (s, 1H), 9.59 (s, 1H), 9.57 (s, 1H), 9.08 (s, 1H), 8.22 - 8.17 (m, 2H), 7.89 - 7.85 (m, 2H), 6.52 (s, 1H), 1.58 (s, 6H), 1.28 (s, 9H). |
| T306 | 2-(2-(cyclobutanesulfonamido)thiazol-4-yl)-2-methyl-N-(4-(pyridin-3-yl)phenyl)propanamide 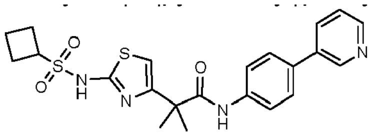 | Method 1 b using INTB47, [HPLC acidic], 457, (1.26) | 12.53 (s, 1H), 9.40 (s, 1H), 8.89 (dd, J = 2.4, 0.9 Hz, 1H), 8.53 (dd, J = 4.8, 1.6 Hz, 1H), 8.06 (ddd, J = 8.0, 2.5, 1.6 Hz, 1H), 7.77 - 7.73 (m, 2H), 7.73 - 7.69 (m, 2H), 7.48 - 7.44 (m, 1H), 6.54 (s, 1H), 3.90 - 3.71 (m, 1H), 2.33 - 2.11 (m, 4H), 1.94 - 1.78 (m, 2H), 1.57 (s, 6H). |
| T307 | 2-(2-(cyclobutanesulfonamido)thiazol-4-yl)-N-(4-(6-ethoxypyrazin-2-yl)phenyl)-2-methylpropanamide 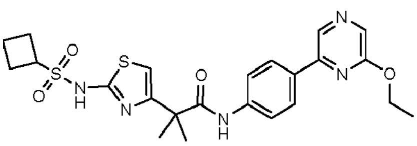 | Method 1 b using INTB47 and INTA38, [HPLC acidic], 502, (2.14) | 12.56 (s, 1H), 9.48 (s, 1H), 8.77 (d, J = 0.6 Hz, 1H), 8.22 - 8.14 (m, 1H), 8.14 - 8.04 (m, 2H), 7.83-7.73 (m, 2H), 6.53 (s, 1H), 4.47 (q, J = 7.1 Hz, 2H), 3.84 - 3.76 (m, 1H), 2.39 - 2.13 (m, 4H), 1.96 - 1.77 (m, 2H), 1.57 (s, 6H), 1.40 (t, J = 7.0 Hz, 3H). |
| T308 | 2-(2-(cyclobutanesulfonamido)thiazol-4-yl)-2-methyl-N-(4-(6-(trifluoromethyl)pyrazin-2-yl)phenyl) propanamide 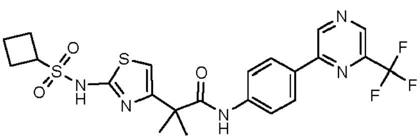 | Method 1 b using INTB47 and INTA39, [HPLC acidic], 526, (2.21) | 12.58 (s, 1H), 9.60 (s, 1H), 9.57 (s, 1H), 9.09 (s, 1H), 8.22 - 8.13 (m, 2H), 7.92 - 7.80 (m, 2H), 6.55 (s, 1H), 3.85 - 3.70 (m, 1H), 2.37-2.11 (m, 4H), 1.98 - 1.75 (m, 2H), 1.58 (s, 6H). |
| T309 | N-(4-(5-cyanopyridin-3-yl)phenyl)-2-(2-(cyclopropanesulfonamido)thiazol-4-yl)-N,2-dimethylpropanamide 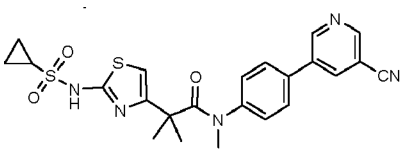 | Method 1 b using INTB37 and INTA67, [HPLC acidic], 482, (1.72) | 12.55 (s, 1H), 9.14 (d, J = 2.3 Hz, 1H), 9.04 (d, J = 1.9 Hz, 1H), 8.61 - 8.56 (m, 1H), 7.70 - 7.64 (m, 2H), 7.23 (d, J = 7.9 Hz, 2H), 5.76 (s, 1H), 3.15 (s, 3H), 2.61 - 2.57 (m, 1H), 1.42 (s, 6H), 0.95 - 0.89 (m, 4H). |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T310** | 2-(2-(cyclopropanesulfonamido)thiazol-4-yl)-N,2-dimethyl-N-(4-(6-(trifluoromethyl)pyrazin-2-yl)phenyl) propanamide 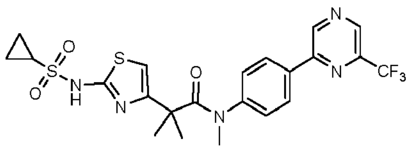 | Method 1b using INTB37 and INTA65, [UPLC acidic], 526, (1.31) | 12.59 (s, 1H), 9.57 (s, 1H), 9.17 (s, 1H), 8.13 - 8.07 (m, 2H), 7.30 (d, J = 8.1 Hz, 2H), 5.83 (s, 1H), 3.15 (s, 3H), 2.64 - 2.60 (m, 1H), 1.42 (s, 6H), 1.02 - 0.92 (m, 4H). |
| **T311** | 2-methyl-2-(2-((2-methylpropyl)sulfonamido)thiazol-4-yl)-N-(4-(pyridin-3-yl)phenyl)propanamide 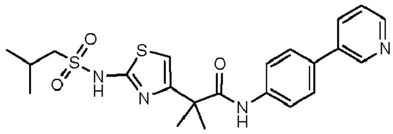 | Method 1b using INTB52, [UPLC acidic], 459, (0.88) | 12.50 (s, 1H), 9.40 (s, 1H), 8.88 (dd, J = 2.4, 0.9 Hz, 1H), 8.53 (dd, J = 4.8, 1.6 Hz, 1H), 8.06 (ddd, J = 8.0, 2.5, 1.6 Hz, 1H), 7.80 - 7.73 (m, 2H), 7.73 - 7.67 (m, 2H), 7.46 (ddd, J = 8.0, 4.7, 0.9 Hz, 1H), 6.57 (s, 1H), 2.91 (d, J = 6.5 Hz, 2H), 2.17 - 2.01 (m, 1H), 1.57 (s, 6H), 1.01 (d, J = 6.7 Hz, 6H). |
| **T312** | N-(4-(6-ethoxypyrazin-2-yl)phenyl)-2-methyl-2-(2-((2-methylpropyl)sulfonamido)thiazol-4-yl)propanamide 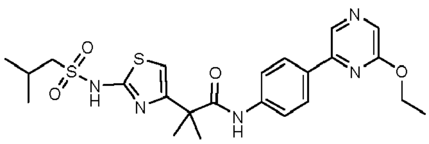 | Method 1b using INTB52 and INTA38, [UPLC acidic], 504, (1.48) | 12.53 (s, 1H), 9.48 (s, 1H), 8.77 (s, 1H), 8.17 (s, 1H), 8.14 - 8.04 (m, 2H), 7.81 - 7.63 (m, 2H), 6.54 (s, 1H), 4.47 (q, J = 7.0 Hz, 2H), 2.95 - 2.85 (m, 2H), 2.16 - 2.04 (m, 1H), 1.56 (s, 6H), 1.40 (d, J = 7.1 Hz, 3H), 1.00 (d, J = 6.7 Hz, 6H). |
| **T313** | 2-methyl-2-(2-((2-methylpropyl)sulfonamido)thiazol-4-yl)-N-(4-(6-(trifluoromethyl)pyrazin-2-yl)phenyl) propanamide 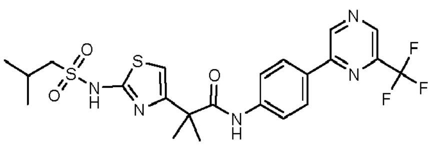 | Method 1b using INTB52 and INTA39, [UPLC acidic], 528, (1.52) | 12.52 (s, 1H), 9.58 (s, 1H), 9.07 (s, 1H), 8.27 - 8.13 (m, 2H), 7.95 - 7.78 (m, 2H), 6.52 (s, 1H), 2.98 - 2.86 (m, 2H), 2.15 - 1.99 (m, 1H), 1.55 (s, 6H), 1.00 (d, J = 6.7 Hz, 6H), 1 x N-H not observed. |
| **T314** | N-(4-(5-chloropyridin-3-yl)phenyl)-2-(2-(cyclopropanesulfonamido)-5-methylthiazol-4-yl)-2-methylpropanamide 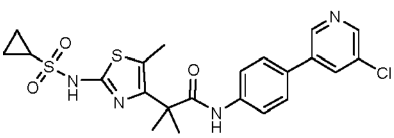 | Method 1b using INTB42 and INTA18, [UPLC acidic], 491, (1.2) | 12.54 (s, 1H), 8.81 (d, J = 1.9 Hz, 1H), 8.64 (d, J = 2.3 Hz, 1H), 8.20 - 8.15 (m, 1H), 7.64 - 7.59 (m, 2H), 7.19 (d, J = 7.9 Hz, 2H), 5.74 (s, 1H), 3.13 (s, 3H), 2.60 - 2.56 (m, 1H), 1.40 (s, 6H), 0.92 - 0.88 (m, |

(continued)

| T# | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **T315** | 2-(2-(cyclopropanesulfonamido)thiazol-4-yl)-N-methyl-N-(4-(pyridin-3-yl)phenyl)butanamide 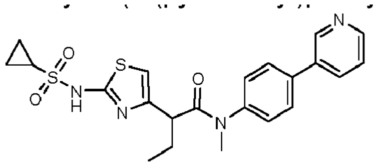 | Method 1c using INTB38 and INTA68, [UPLC acidic], 457, (0.85) | 12.23 (s, 1H), 8.97 - 8.92 (m, 1H), 8.59 (dd, J = 4.8, 1.6 Hz, 1H), 8.13 (d, J = 8.0 Hz, 1H), 7.85 (d, J = 8.0 Hz, 2H), 7.50 (dd, J = 8.0, 4.7 Hz, 1H), 7.41 - 7.36 (m, 2H), 6.37 (s, 1H), 3.47 - 3.43 (m, 1H), 3.22 (s, 3H), 2.59 - 2.51 (m, 1H), 1.85 - 1.78 (m, 1H), 1.72 - 1.66 (m, 1H), 0.89 - 0.82 (m, 4H), 0.73 (t, J = 7.3 Hz, 3H). |
| **T316** | N-(4-(5-cyanopyridin-3-yl)phenyl)-2-(2-(cyclopropanesulfonamido)thiazol-4-yl)-N-methylbutanamide 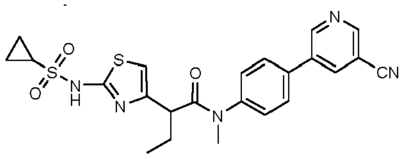 | Method 1c using INTB38 and INTA67, [UPLC acidic], 482, (1.17) | 12.25 (s, 1H), 9.26 (d, J = 2.3 Hz, 1H), 9.05 (d, J = 1.9 Hz, 1H), 8.74 - 8.69 (m, 1H), 7.96 (d, J = 7.8 Hz, 2H), 7.45 (d, J = 8.2 Hz, 2H), 6.37 (s, 1H), 3.47 - 3.43 (m, 1H), 3.24 (s, 3H), 2.58 - 2.52 (m, 1H), 1.85 - 1.81 (m, 1H), 1.73 - 1.69 (m, 1H), 0.91 - 0.86 (m, 4H), 0.74 (s, 3H). |
| **T317** | 2-(2-(cyclopropanesulfonamido)-5-methylthiazol-4-yl)-N-(4-(6-ethoxypyrazin-2-yl)phenyl)-2-methylpropanamide 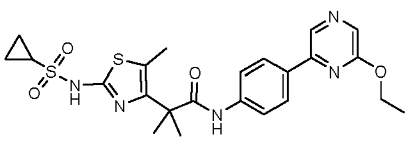 | Method 1e using INTB42 and INTA38, [HPLC acidic], 502, (2.25) | 12.04 (s, 1H), 9.90 (s, 1H), 8.76 (s, 1H), 8.19 (s, 1H), 8.13 - 8.04 (m, 2H), 7.84 - 7.73 (m, 2H), 4.48 (q, J = 7.0 Hz, 2H), 2.62-2.55 (m, 1H), 1.95 (s, 3H), 1.53 (s, 6H), 1.40 (t, J = 7.0 Hz, 3H), 0.95-0.85 (m, 4H). |
| **T318** | N-(4-(5-chloropyridin-3-yl)phenyl)-2-(2-(cyclopropanesulfonamido)thiazol-4-yl)-N,2-dimethylpropanamide 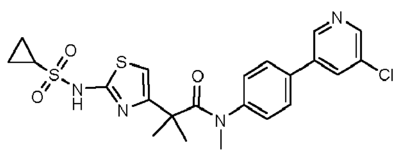 | Method 1e using INTB37 and INTA66, [HPLC acidic], 491, (2.157) | 12.04 (s, 1H), 9.86 (s, 1H), 8.86 (d, J = 2.0 Hz, 1H), 8.58 (d, J = 2.3 Hz, 1H), 8.23 (t, J = 2.2 Hz, 1H), 7.81-7.73 (m, 4H), 2.62-2.53 (m, 1H), 1.95 (s, 3H), 1.52 (s, 6H), 0.98-0.87 (m, 4H). |
| **T319** | 2-(2-(cyclopropanesulfonamido)-5-methylthiazol-4-yl)-2-methyl-N-(4-(pyridin-3-yl)phenyl)propanamide 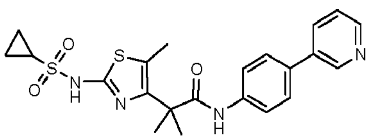 | Method 1e using INTB42, [HPLC acidic], 457, (1.377) | 12.02 (s, 1H), 9.83 (s, 1H), 8.88 (dd, J = 2.4, 0.9 Hz, 1H), 8.54 (dd, J = 4.7, 1.6 Hz, 1H), 8.09-8.00 (m, 1H), 7.79 - 7.65 (m, 4H), 7.49-7.42 (m, 1H), 2.63-2.54 (m, 1H), 1.96 (s, 3H), 1.52 (s, 6H), 0.95 - 0.90 (m, 4H). |

(continued)

| T# | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| T320 | N-(4-(5-cyanopyridin-3-yl)-2,6-dimethylphenyl)-2-(2-(cyclopropanesulfonamido)thiazol-4-yl)-2-methylpropanamide 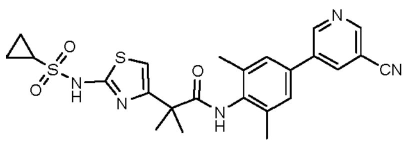 | Method 2 using INTB70, [UPLC acidic], 496, (1.16) | 12.57 (s, 1H), 9.19 (d, J = 2.3 Hz, 1H), 8.98 (d, J = 1.9 Hz, 1H), 8.67 - 8.62 (m, 1H), 7.56 (s, 2H), 6.59 (s, 1H), 2.58 - 2.54 (m, 1H), 2.19 (s, 6H), 1.61 (s, 6H), 0.92 - 0.88 (m, 4H). 1 exchangeable proton missing. |
| T321 | N-(4-(5-chloropyridin-3-yl)-2,6-dimethylphenyl)-2-(2-(cyclopropanesulfonamido)thiazol-4-yl)-2-methylpropanamide 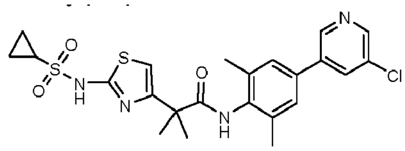 | Method 2 using INTB70, [UPLC acidic], 505, (1.28) | 12.56 (s, 1H), 8.95 (s, 1H), 8.86 (d, J = 2.0 Hz, 1H), 8.59 (d, J = 2.3 Hz, 1H), 8.26 - 8.21 (m, 1H), 7.52 (s, 2H), 6.59 (s, 1H), 2.61 - 2.48 (m, 1H), 2.18 (s, 6H), 1.60 (s, 6H), 0.91 - 0.87 (m, 4H). |
| T322 | N-(4-(5-cyanopyridin-3-yl)-3-methylphenyl)-2-(2-(cyclopropanesulfonamido)thiazol-4-yl)-2-methylpropanamide 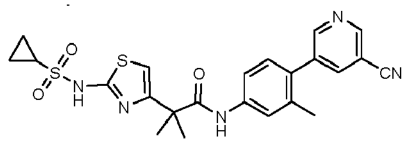 | Method 2 using INTB60, [UPLC acidic], 482, (1.2) | 12.56 (s, 1H), 9.37 (s, 1H), 9.00 (d, J = 2.0 Hz, 1H), 8.84 (d, J = 2.2 Hz, 1H), 8.38 - 8.33 (m, 1H), 7.62 - 7.56 (m, 2H), 7.29 - 7.23 (m, 1H), 6.52 (s, 1H), 2.59 - 2.55 (m, 1H), 2.23 (s, 3H), 1.56 (s, 6H), 0.92 - 0.87 (m, 4H). |

## Biological Examples

### Biological Example 1 - Human CTPS1 Enzyme Inhibition

The enzyme inhibitory activities of compounds invented against the target of interest were determined using the ADP-Glo™ Max assay (Promega, UK). Assays for human CTPS1 were performed in 1x assay buffer containing 50mM Tris, 10mM $MgCl_2$, 0.01% Tween-20, pH to 8.0 accordingly. Finally, immediately before use, L-cysteine was added to the 1x assay buffer to a final concentration of 2mM. All reagents are from Sigma-Aldrich unless specified otherwise. Human full length active C-terminal FLAG-$His_8$-tag CTPS1 (UniProtKB - P17812, CTPS[1-591]-GGDYKDDDDKGGHHHHHHHH) was obtained from Proteros biostructures GmbH.

Assay Procedure.

3x human CTPS1 protein was prepared in 1x assay buffer to the final working protein concentration required for the reaction. A 2uL volume per well of 3x human CTPS1 protein was mixed with 2uL per well of 3x test compound (compound prepared in 1x assay buffer to an appropriate final 3x compound concentration respective to the concentration response curve designed for the compounds under test) for 10 minutes at 25°C. The enzymatic reaction was then initiated by addition of a 2uL per well volume of a pre-mixed substrate mix (UltraPure ATP from ADP-Glo™ Max kit (0.31mM), GTP (0.034mM), UTP (0.48mM) and L-glutamine (0.186mM)) and the mixture was incubated for an appropriate amount of time within the determined linear phase of the reaction at 25°C under sealed plate conditions with constant agitation at 500 revolutions per minute (rpm). ADP-Glo™ Max reagent was added for 60 minutes (6μL per well) and subsequently ADP-Glo™ Max development reagent was added for 60 minutes (12uL per well) prior to signal detection

in a microplate reader (EnVision® Multilabel Reader, Perkin Elmer). Following each reagent addition over the course of the assay, assay plates were pulse centrifuged for 30 seconds at 500rpm.

In all cases, the enzyme converts ATP to ADP and the ADP-Glo™ Max reagent subsequently depletes any remaining endogenous ATP in the reaction system. The ADP-Glo™ Max detection reagent converts the ADP that has been enzymatically produced back into ATP and using ATP as a substrate together with luciferin for the enzyme luciferase, light is generated which produces a detectable luminescence. The luminescent signal measured is directly proportional to the amount of ADP produced by the enzyme reaction and a reduction in this signal upon compound treatment demonstrates enzyme inhibition. The percentage inhibition produced by each concentration of compound was calculated using the equation shown below:

$$\%\,Inhibition = 1 - \frac{(Mean_{Min} - Mean_{Inh})}{(Mean_{Min} - Mean_{Max})}\ x\ 100$$

Percentage inhibition was then plotted against compound concentration, and the 50% inhibitory concentration ($IC_{50}$) was determined from the resultant concentration-response curve.

**Table 7**: *Human CTPS1 Enzyme Inhibition data grouped by potency range* (+ indicates $IC_{50}$ in the range >1 to 10 micromolar, ++ indicates $IC_{50}$ in the range >0.1 to 1 micromolar, +++ indicates $IC_{50}$ of $\leq$0.1 micromolar)

| **T #** | **CTPS1** | **T #** | **CTPS1** | **T #** | **CTPS1** | **T #** | **CTPS1** |
|---|---|---|---|---|---|---|---|
| T1 | + | T18 | +++ | T35 | +++ | T52 | +++ |
| T2 | ++ | T19 | ++ | T36 | ++ | T53 | + |
| T3 | ++ | T20 | ++ | T37 | ++ | T54 | ++ |
| T4 | + | T21 | + | T38 | +++ | T55 | +++ |
| T5 | +++ | T22 | +++ | T39 | ++ | T56 | +++ |
| T6 | ++ | T23 | ++ | T40 | +++ | T57 | +++ |
| T7 | +++ | T24 | ++ | T41 | ++ | T58 | +++ |
| T8 | +++ | T25 | + | T42 | +++ | T59 | +++ |
| T9 | +++ | T26 | ++ | T43 | ++ | T60 | ++ |
| T10 | +++ | T27 | ++ | T44 | ++ | T61 | +++ |
| T11 | ++ | T28 | ++ | T45 | ++ | T62 | ++ |
| T12 | ++ | T29 | +++ | T46 | + | T63 | ++ |
| T13 | ++ | T30 | +++ | T47 | +++ | T64 | ++ |
| T14 | ++ | T31 | + | T48 | +++ | T65 | ++ |
| T15 | ++ | T32 | +++ | T49 | ++ | T66 | ++ |
| T16 | ++ | T33 | +++ | T50 | +++ | T67 | ++ |
| T17 | ++ | T34 | ++ | T51 | +++ | T68 | ++ |

| T # | CTPS 1 | T # | CTPS 1 | T # | CTPS 1 | T # | CTPS 1 |
|---|---|---|---|---|---|---|---|
| T69 | + | T102 | +++ | T135 | ++ | T168 | |
| T70 | ++ | T103 | ++ | T136 | +++ | T169 | ++ |
| T71 | +++ | T104 | ++ | T137 | +++ | T170 | ++ |
| T72 | +++ | T105 | + | T138 | +++ | T171 | +++ |
| T73 | +++ | T106 | +++ | T139 | +++ | T172 | +++ |
| T74 | ++ | T107 | +++ | T140 | ++ | T173 | +++ |
| T75 | +++ | T108 | +++ | T141 | ++ | T174 | +++ |
| T76 | +++ | T109 | +++ | T142 | +++ | T175 | +++ |
| T77 | +++ | T110 | ++ | T143 | +++ | T176 | +++ |
| T78 | +++ | T111 | +++ | T144 | ++ | T177 | +++ |
| T79 | ++ | T112 | +++ | T145 | +++ | T178 | + |
| T80 | +++ | T113 | + | T146 | ++ | T179 | +++ |
| T81 | +++ | T114 | ++ | T147 | +++ | T180 | +++ |
| T82 | +++ | T115 | +++ | T148 | ++ | T181 | ++ |
| T83 | + | T116 | ++ | T149 | +++ | T182 | +++ |
| T84 | ++ | T117 | ++ | T150 | ++ | T183 | +++ |
| T85 | ++ | T118 | +++ | T151 | ++ | T184 | + |
| T86 | + | T119 | +++ | T152 | ++ | T185 | +++ |
| T87 | ++ | T120 | +++ | T153 | ++ | T186 | +++ |
| T88 | ++ | T121 | +++ | T154 | ++ | T187 | +++ |
| T89 | +++ | T122 | +++ | T155 | +++ | T188 | ++ |
| T90 | +++ | T123 | +++ | T156 | +++ | T189 | +++ |
| T91 | +++ | T124 | +++ | T157 | +++ | T190 | +++ |
| T92 | +++ | T125 | +++ | T158 | +++ | T191 | +++ |
| T93 | +++ | T126 | +++ | T159 | +++ | T192 | +++ |
| T94 | +++ | T127 | +++ | T160 | +++ | T193 | +++ |
| T95 | +++ | T128 | +++ | T161 | ++ | T194 | +++ |
| T96 | +++ | T129 | +++ | T162 | +++ | T195 | +++ |
| T97 | ++ | T130 | +++ | T163 | ++ | T196 | +++ |
| T98 | ++ | T131 | +++ | T164 | + | T197 | +++ |
| T99 | +++ | T132 | ++ | T165 | ++ | T198 | +++ |
| T100 | ++ | T133 | +++ | T166 | +++ | T199 | +++ |
| T101 | + | T134 | +++ | T167 | + | T200 | +++ |

| T # | CTPS 1 |
|---|---|
| T201 | +++ |
| T202 | +++ |
| T203 | +++ |
| T204 | +++ |
| T205 | +++ |
| T206 | +++ |
| T207 | +++ |
| T208 | +++ |
| T209 | +++ |
| T210 | +++ |
| T211 | +++ |
| T212 | +++ |
| T213 | ++ |
| T214 | +++ |
| T215 | +++ |
| T216 | ++ |
| T217 | +++ |
| T218 | ++ |
| T219 | +++ |
| T220 | +++ |
| T221 | +++ |
| T222 | ++ |
| T223 | +++ |
| T224 | +++ |
| T225 | +++ |
| T226 | +++ |
| T227 | ++ |
| T228 | +++ |
| T229 | +++ |
| T230 | +++ |
| T231 | + |
| T232 | ++ |
| T233 | + |

| T # | CTPS 1 |
|---|---|
| T234 | + |
| T235 | ++ |
| T236 | ++ |
| T237 | + |
| T238 | + |
| T239 | ++ |
| T240 | ++ |
| T241 | ++ |
| T242 | + |
| T243 | + |
| T244 | ++ |
| T245 | + |
| T246 | ++ |
| T247 | ++ |
| T248 | + |
| T249 | +++ |
| T250 | + |
| T251 | + |
| T252 | +++ |
| T253 | +++ |
| T254 | ++ |
| T255 | + |
| T256 | +++ |
| T257 | ++ |
| T258 | ++ |
| T259 | +++ |
| T260 | + |
| T261 | +++ |
| T262 | +++ |
| T263 | + |
| T264 | ++ |
| T265 | +++ |
| T266 | +++ |

| T # | CTPS 1 |
|---|---|
| T267 | +++ |
| T268 | ++ |
| T269 | ++ |
| T270 | ++ |
| T271 | + |
| T272 | +++ |
| T273 | +++ |
| T274 | +++ |
| T275 | +++ |
| T276 | ++ |
| T277 | ++ |
| T278 | +++ |
| T279 | + |
| T280 | + |
| T281 | ++ |
| T282 | ++ |
| T283 | ++ |
| T284 | + |
| T285 | +++ |
| T286 | +++ |
| T287 | +++ |
| T288 | ++ |
| T289 | ++ |
| T290 | +++ |
| T291 | +++ |
| T292 | ++ |
| T293 | ++ |
| T294 | ++ |
| T295 | ++ |
| T296 | ++ |
| T297 | + |
| T298 | +++ |
| T299 | ++ |

| T # | CTPS 1 |
|---|---|
| T300 | ++ |
| T301 | +++ |
| T302 | ++ |
| T303 | + |
| T304 | ++ |
| T305 | ++ |
| T306 | ++ |
| T307 | +++ |
| T308 | ++ |
| T309 | + |
| T310 | + |
| T311 | + |
| T312 | ++ |
| T313 | ++ |
| T314 | + |
| T315 | ++ |
| T316 | ++ |
| T317 | ++ |
| T318 | ++ |
| T319 | + |
| T320 | ++ |
| T321 | +++ |
| T322 | ++ |

All compounds of the invention which have been tested were found to demonstrate inhibition of CTPS1 enzyme in this assay. Consequently, these compounds may be expected to have utility in the inhibition of CTPS1.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the claims which follow.

All publications, including but not limited to patents and patent applications, cited in this specification are herein incorporated by reference as if each individual publication were specifically and individually indicated to be incorporated by reference herein as though fully set forth.

**Clauses of the invention:**

Clause 1 - A compound of formula (I):

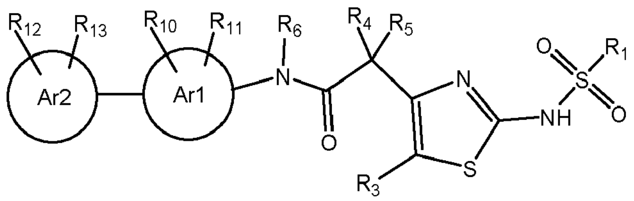

(I)

wherein

$R_1$ is $C_{1-5}$alkyl, $C_{0-2}$alkylene$C_{3-5}$cycloalkyl which cycloalkyl is optionally substituted by $CH_3$, or $CF_3$;

$R_3$ is H, halo, $CH_3$ or $CF_3$,

or $R_3$ together with $R_5$ forms a 5- or 6-membered cycloalkyl;

$R_4$ and $R_5$ are each independently H, F, $C_{1-6}$alkyl, $C_{0-2}$alkylene$C_{3-6}$cycloalkyl, $OC_{1-6}$alkyl, $OC_{0-2}$alkylene$C_{3-6}$cycloalkyl, $C_{1-3}$alkylene$OC_{1-3}$alkyl, $C_{1-6}$haloalkyl or $OC_{1-6}$haloalkyl,

or $R_4$ is H and $R_5$ together with $R_3$ form a 5- or 6-membered cycloalkyl,

or $R_4$ and $R_5$ together with the carbon atom to which they are attached form a $C_{3-6}$spirocycloalkyl,

or $R_4$ is O and $R_5$ is absent;

$R_6$ is H or $C_{1-3}$alkyl,

or $R_6$ together with $R_{11}$ in the ortho-position to the amide are a $C_2$alkylene chain forming a 5-membered ring;

Ar1 is 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $C_{1-2}$haloalkyl, $OC_{1-2}$haloalkyl or CN;

$R_{11}$ is H, F, Cl, $CH_3$, $OCH_3$, $CF_3$, $OCF_3$ or CN,

or $R_{11}$, when in the ortho-position to the amide, together with $R_6$ are a $C_2$alkylene chain forming a 5-membered

ring;

$R_{12}$ is attached to Ar2 in the ortho or meta position relative to Ar1 and $R_{12}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, $C_{1-4}$alkylOH, CN, $C_{1-3}$alkylene$OC_{1-3}$alkyl, $C_{1-4}$haloalkyl, $OC_{1-4}$haloalkyl, $N(CH_3)_2$, $SO_2CH_3$, $C(O)N(CH_3)_2$, $NHC(O)C_{1-3}$alkyl or a $C_{3-6}$heterocycloalkyl comprising one nitrogen located at the point of attachment to Ar2; and

$R_{13}$ is H, F, $CH_3$ or $OCH_3$;

or a salt and/or solvate thereof and/or derivative thereof.

Clause 2 - The compound according to clause 1 wherein $R_1$ is $C_{1-5}$alkyl.

Clause 3 - The compound according to clause 2 wherein $R_1$ is $CH_3$.

Clause 4 - The compound according to clause 1 wherein $R_1$ is $C_{0-2}$alkylene$C_{3-5}$cycloalkyl which cycloalkyl is optionally substituted by $CH_3$.

Clause 5 - The compound according to clause 4, wherein $R_1$ is $C_{0-2}$alkylene$C_{3-5}$cycloalkyl.

Clause 6 - The compound according to clause 4, wherein $R_1$ is $C_{0-2}$alkylene$C_{3-5}$cycloalkyl which cycloalkyl is substituted by $CH_3$.

Clause 7 - The compound according to any one of clauses 4 to 6, wherein $R_1$ is $C_{3-5}$cycloalkyl, optionally substituted by $CH_3$.

Clause 8 - The compound according to any one of clauses 4 to 6, wherein $R_1$ is $C_1$alkylene$C_{3-5}$cycloalkyl which cycloalkyl is optionally substituted by $CH_3$.

Clause 9 - The compound according to any one of clauses 4 to 6, wherein $R_1$ is $C_2$alkylene$C_{3-5}$cycloalkyl which cycloalkyl is optionally substituted by $CH_3$.

Clause 10 - The compound according to any one of clauses 4 to 9, wherein $R_1$ is $C_{0-2}$alkylene$C_3$cycloalkyl which cycloalkyl is optionally substituted by $CH_3$.

Clause 11 - The compound according to any one of clauses 4 to 9 wherein $R_1$ is $C_{0-2}$alkylene$C_4$cycloalkyl which cycloalkyl is optionally substituted by $CH_3$.

Clause 12 - The compound according to any one of clauses 4 to 9 wherein $R_1$ is $C_{0-2}$alkylene$C_5$cycloalkyl which cycloalkyl is optionally substituted by $CH_3$.

Clause 13 - The compound according to any one of clauses 1 to 12 wherein $R_1$ is cyclopropyl, cyclopropyl substituted by $CH_3$ at the point of attachment, cyclobutyl, $CH_3$, isopropyl, sec-butyl or tert-butyl.

Clause 14 - The compound according to any clause 13 wherein $R_1$ is cyclopropyl, cyclopropyl substituted by $CH_3$ at the point of attachment, cyclobutyl or isopropyl.

Clause 15 - The compound according to any one of clauses 1 to 14 wherein $R_3$ is H.

Clause 16 - The compound according to any one of clauses 1 to 14 wherein $R_3$ is chloro or fluoro.

Clause 17 - The compound according to any one of clauses 1 to 14 wherein $R_3$ is $CH_3$.

Clause 18 - The compound according to any one of clauses 1 to 14 wherein $R_3$ is $CF_3$.

Clause 19 - The compound according to any one of clauses 1 to 14 wherein $R_3$ together with $R_5$ forms a 5- or 6-membered cycloalkyl.

Clause 20 - The compound according to clause 19 wherein $R_3$ together with $R_5$ forms a 5-membered cycloalkyl.

Clause 21 - The compound according to any one of clauses 1 to 20 wherein $R_4$ together with $R_5$ form a $C_{3-6}$spirocycloalkyl.

Clause 22 - The compound according to clause 21 wherein $R_4$ together with $R_5$ form spirocyclopropyl or spirocyclopentyl.

Clause 23 - The compound according to any one of clauses 1 to 20, wherein $R_4$ is $C_{1-6}$alkyl, in particular $C_{1-4}$alkyl such as methyl, ethyl or propyl (n-propyl or isopropyl).

Clause 24 - The compound according to any one of clauses 1 to 20, wherein $R_4$ is $C_{0-2}$alkylene$C_{3-6}$cycloalkyl, such as $C_{3-6}$cycloalkyl, $C_1$alkylene$C_{3-6}$cycloalkyl or $C_2$alkylene$C_{3-6}$cycloalkyl.

Clause 25 - The compound according to any one of clauses 1 to 20, wherein $R_4$ is $OC_{1-6}$alkyl, in particular $OC_{1-4}$alkyl, such as methoxy or isopropoxy.

Clause 26 - The compound according to any one of clauses 1 to 20, wherein $R_4$ is $OC_{0-2}$alkylene$C_{3-6}$cycloalkyl such as $OC_{3-6}$cycloalkyl, $OC_1$alkylene$C_{3-6}$cycloalkyl or $OC_2$alkylene$C_{3-6}$cycloalkyl.

Clause 27 - The compound according to any one of clauses 1 to 20, wherein $R_4$ is $C_{1-3}$alkylene$OC_{1-3}$alkyl, in particular $C_{1-2}$alkylene$OC_{1-2}$alkyl, such as $CH_2CH_2OCH_3$.

Clause 28 - The compound according to any one of clauses 1 to 20, wherein $R_4$ is $C_{1-6}$haloalkyl, in particular $C_{1-4}$haloalkyl.

Clause 29 - The compound according to any one of clauses 1 to 20, wherein $R_4$ is $OC_{1-6}$haloalkyl, in particular $OC_{1-4}$haloalkyl.

Clause 30 - The compound according to clause 1 to 20 wherein $R_4$ is H.

Clause 31 - The compound according to clause 1 to 20 wherein $R_4$ is F.

Clause 32 - The compound according to any one of clauses 1 to 18 and 23 to 31, wherein $R_5$ is H, methyl, ethyl or fluoro e.g. H, methyl or ethyl.

Clause 33 - The compound according to clause 32, wherein $R_5$ is H.

Clause 34 - The compound according to any one of clauses 1 to 32 wherein $R_4$ and $R_5$ are methyl.

Clause 35 - The compound according to any one of clauses 1 to 32 wherein $R_4$ and $R_5$ are ethyl.

Clause 36 - The compound according to any one of clauses 1 to 32 wherein $R_4$ is ethyl and $R_5$ is H and the groups are arranged in the S configuration.

Clause 37 - The compound according to any one of clauses 1 to 36 wherein $R_6$ is H.

Clause 38 - The compound according to any one of clauses 1 to 36 wherein $R_6$ together with $R_{11}$ in the ortho-position to the amide are a $C_2$alkylene chain forming a 5-membered ring.

Clause 39 - The compound according to any one of clauses 1 to 38 wherein Ar1 is phenyl.

Clause 40 - The compound according to any one of clauses 1 to 38 wherein Ar1 is pyridyl.

Clause 41 - The compound according to clause 40 wherein Ar1 is 2-pyridyl.

Clause 42 - The compound according to any one of clauses 1 to 38 wherein Ar1 is pyridazinyl.

Clause 43 - The compound according to any one of clauses 1 to 38 wherein Ar1 is pyrimidinyl.

Clause 44 - The compound according to any one of clauses 1 to 38 wherein Ar1 is pyrazinyl.

Clause 45 - The compound according to any one of clauses 1 to 44 wherein $R_{10}$ is H.

Clause 46 - The compound according to any one of clauses 1 to 44 wherein $R_{10}$ is chloro or fluoro, such as fluoro.

Clause 47 - The compound according to any one of clauses 1 to 44 wherein $R_{10}$ is methoxy or ethoxy.

Clause 48 - The compound according to any one of clauses 1 to 44 wherein $R_{10}$ is methyl.

Clause 49 - The compound according to any one of clauses 1 to 44 wherein $R_{10}$ is $OCF_3$.

Clause 50 - The compound according to any one of clauses 1 to 49 wherein $R_{11}$ is H.

Clause 51 - The compound according to any one of clauses 1 to 49 wherein $R_{11}$ is fluoro.

Clause 52 - The compound according to any one of clauses 1 to 49 wherein $R_{11}$ is methyl.

Clause 53 - The compound according to any one of clauses 1 to 52 wherein Ar2 is phenyl.

Clause 54 - The compound according to any one of clauses 1 to 52 wherein Ar2 is pyridyl.

Clause 55 - The compound according to clause 54 wherein Ar2 is 3-pyridyl.

Clause 56 - The compound according to any one of clauses 1 to 52 wherein Ar2 is pyridazinyl.

Clause 57 - The compound according to any one of clauses 1 to 52 wherein Ar2 is pyrimidinyl.

Clause 58 - The compound according to any one of clauses 1 to 52 wherein Ar2 is pyrazinyl.

Clause 59 - The compound according to any one of clauses 1 to 58 wherein $R_{12}$ is H.

Clause 60 - The compound according to any one of clauses 1 to 58 wherein $R_{12}$ is halo, such as F or Cl.

Clause 61 - The compound according to any one of clauses 1 to 58 wherein $R_{12}$ is $C_{1-4}$alkyl, such as methyl.

Clause 62 - The compound according to any one of clauses 1 to 58 wherein $R_{12}$ is $C_{2-4}$alkynyl, such as C=CH.

Clause 63 - The compound according to any one of clauses 1 to 58 wherein $R_{12}$ is $C_{0-2}$alkylene$C_{3-5}$cycloalkyl, such as cyclopropyl.

Clause 64 - The compound according to any one of clauses 1 to 58 wherein $R_{12}$ is $OC_{1-4}$alkyl such as methoxy, ethoxy, isopropoxy or n-propoxy.

Clause 65 - The compound according to any one of clauses 1 to 58 wherein $R_{12}$ is $OC_{0-2}$alkylene$C_{3-5}$cycloalkyl, such as cyclopropoxyl or cyclobutoxy.

Clause 66 - The compound according to any one of clauses 1 to 58 wherein $R_{12}$ is $OCH_2CH_2N(CH_3)_2$

Clause 67 - The compound according to any one of clauses 1 to 58 wherein $R_{12}$ is $C_{1-4}$alkylOH, such as $CH_2OH$ or $C(CH_3)_2OH$.

Clause 68 - The compound according to any one of clauses 1 to 58 wherein $R_{12}$ is CN.

Clause 69 - The compound according to any one of clauses 1 to 58 wherein $R_{12}$ is $C_{1-3}$alkylene$OC_{1-3}$alkyl.

Clause 70 - The compound according to any one of clauses 1 to 58 wherein $R_{12}$ is $C_{1-4}$haloalkyl, such as $CF_3$.

Clause 71 - The compound according to any one of clauses 1 to 58 wherein $R_{12}$ is $OC_{1-4}$haloalkyl, such as $OCF_3$, $OCHF_2$ or $OCH_2CF_3$.

Clause 72 - The compound according to any one of clauses 1 to 58 wherein $R_{12}$ is $N(CH_3)_2$.

Clause 73 - The compound according to any one of clauses 1 to 58 wherein $R_{12}$ is $SO_2CH_3$.

Clause 74 - The compound according to any one of clauses 1 to 58 wherein $R_{12}$ is $C(O)N(CH_3)_2$.

Clause 75 - The compound according to any one of clauses 1 to 58 wherein $R_{12}$ is $NHC(O)C_{1-3}$alkyl such as $NHC(O)CH_0$.

Clause 76 - The compound according to any one of clauses 1 to 58 wherein $R_{12}$ is $C_{3-6}$heterocycloalkyl comprising one nitrogen located at the point of attachment to Ar2 such as a $C_5$heterocycloalkyl or $C_6$heterocycloalkyl, and in particular pyrrolidinyl.

Clause 77 - The compound according to any one of clauses 1 to 76 wherein $R_{12}$ is in the meta position of Ar2.

Clause 78 - The compound according to any one of clauses 1 to 76 wherein $R_{12}$ is in the ortho position of Ar2.

Clause 79 - The compound according to any one of clauses 1 to 78 wherein $R_{13}$ is H.

Clause 80 - The compound according to any one of clauses 1 to 78 wherein $R_{13}$ is methyl.

Clause 81 - A compound of the examples T1 to T322.

Clause 82 - A compound of the formula:

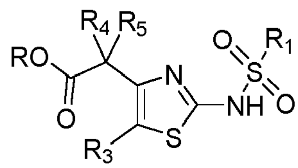

wherein $R_1$, $R_3$, $R_4$ and $R_5$ are as defined in any preceding clause and R is H, $C_{1-6}$alkyl (e.g. methyl and ethyl) or benzyl.

Clause 83 - A compound of formula (III):

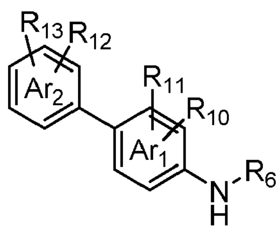

wherein Ar1, Ar2, $R_6$, $R_{10}$, $R_{11}$, $R_{12}$ and $R_{13}$ are as defined in any preceding clause.

Clause 84 - A compound of formula (X):

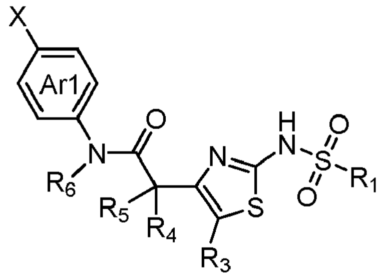

X = Cl, Br, $B(OH)_2$, $B(pin)_2$ ;
wherein Ar1, $R_1$, $R_3$, $R_4$, $R_5$ and $R_6$ are as defined in any preceding clause.

Clause 85 - A compound of formula (XII):

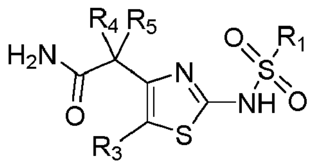

wherein $R_1$, $R_3$, $R_4$ and $R_5$ are as defined in any preceding clause.

Clause 86 - A compound of formula (XIII):

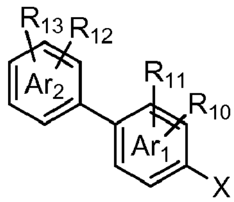

wherein Ar1, Ar2, $R_{10}$, $R_{11}$, $R_{12}$ and $R_{13}$ are as defined in any preceding clause and X is halo such as Cl.

Clause 87 - A compound according to any one of clauses 1 to 81, for use as a medicament.

Clause 88 - The compound according to clause 87, for use in the inhibition of CTPS1 in a subject.

Clause 89 - The compound according to clause 87, for use in the reduction of T-cell and/or B-cell proliferation in a subject.

Clause 90 - The compound according to clause 87, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

Clause 91 - A method for the inhibition of CTPS1 in a subject, which comprises administering to the subject an effective amount of a compound according to any one of clauses 1 to 81.

Clause 92 - Use of a compound according to any one of clauses 1 to 81, in the manufacture of a medicament for the inhibition of CTPS1 in a subject.

Clause 93 - A pharmaceutical composition comprising a compound according to any one of clauses 1 to 81.

Clause 94 - The compound, method or use according to any one of clauses 87 to 92, for administration to a human subject.

Clause 95 - The compound, method, use or composition according to any one of clauses 87 to 94, for administration in conjunction with a further pharmaceutically acceptable active ingredient or ingredients.

Clause 96 - The compound according to any one of clauses 1 to 95, which is in natural isotopic form.

REFERENCES

Cheng, D. et al. Discovery of Pyridinyl Acetamide Derivatives as Potent, Selective, and Orally Bioavailable Porcupine Inhibitors. Medicinal Chemistry Letters, 7(7), 676-680; 2016

Evans, D. R. & Guy, H. I. Mammalian pyrimidine biosynthesis: fresh insights into an ancient pathway. J. Biol. Chem. 279, 33035-33038 (2004).

Fairbanks, L. D., Bofill, M., Ruckemann, K. & Simmonds, H. A. Importance of ribonucleotide availability to proliferating T-lymphocytes from healthy humans. Disproportionate expansion of pyrimidine pools and contrasting effects of de novo synthesis inhibitors. J. Biol. Chem. 270, 29682-29689 (1995).

Higgins, M. J., Graves, P. R. & Graves, L. M. Regulation of human cytidine triphosphate synthetase 1 by glycogen synthase kinase 3. J. Biol. Chem. 282, 29493-29503 (2007).

Kursula, P., Flodin, S., Ehn, M., Hammarström, M., Schüler, H., Nordlund, P. and Stenmarka, P. Structure of the synthetase domain of human CTP synthetase, a target for anticancer therapy. Acta Crystallogr Sect F Struct Biol Cryst Commun. 62 (Pt7): 613-617 (2006).

Lieberman I. Enzymatic amination of uridine triphosphate to cytidine triphosphate. The J. Biol. Chem. 222 (2): 765-75 (1956).

Lübbers, T et al. Aminothiazoles as γ-secretase modulators. Bioorganic & Medicinal Chemistry Letters, 21(21), 6554-6558; 2011

Martin E. et al. ; CTP synthase 1 deficiency in humans reveals its central role in lymphocytes proliferation. Nature. Jun 12; 510(7504):288-92 (2014). Erratum in: Nature. Jul 17; 511(7509):370 (2014).

Ostrander, D. B., O'Brien, D. J., Gorman, J. A. & Carman, G. M. Effect of CTP synthetase regulation by CTP on phospholipid synthesis in Saccharomyces cerevisiae. J. Biol. Chem. 273, 18992-19001 (1998).

Sakamoto K, Ishibashi Y, Adachi R, et al. Identification of cytidine-5-triphosphate synthase1-selective inhibitory peptide from random peptide library displayed on T7 phage. Peptides. 2017; 94:56-63 (2017).

van den Berg, A. A. et al. Cytidine triphosphate (CTP) synthetase activity during cell cycle progression in normal and malignant T-lymphocytic cells. Eur. J. Cancer 31, 108-112 (1995).

van Kuilenburg, A.B.P, Meinsma, R., Vreken, P., Waterham, H.R., van Gennip, A.H. Identification of a cDNA encoding an isoform of human CTP synthetase. Biochimica et Biophysica Acta 1492548-552 (2000).

Xing-Li F. et al. Efficient Diphosphane-Based Catalyst for the Palladium-Catalyzed Suzuki Cross-Coupling Reaction of 3-Pyridylboronic Acids. European Journal of Organic Chemistry, (13), 2051-2054; 2009


## Claims

**1.** A compound of formula (I):

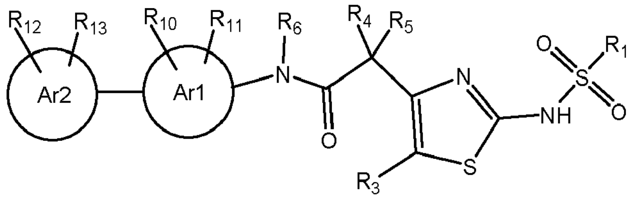

(I)

wherein

$R_1$ is $C_{1-5}$alkyl, $C_{0-2}$alkylene$C_{3-5}$cycloalkyl which cycloalkyl is optionally substituted by $CH_3$, or $CF_3$;
$R_3$ is H, halo, $CH_3$ or $CF_3$,
or $R_3$ together with $R_5$ forms a 5- or 6-membered cycloalkyl;
$R_4$ and $R_5$ are each independently H, F, $C_{1-6}$alkyl, $C_{0-2}$alkylene$C_{3-6}$cycloalkyl, $OC_{1-6}$alkyl, $OC_{0-2}$alkylene$C_{3-6}$cycloalkyl, $C_{1-3}$alkylene$OC_{1-3}$alkyl, $C_{1-6}$haloalkyl or $OC_{1-6}$haloalkyl,
or $R_4$ is H and $R_5$ together with $R_3$ form a 5- or 6-membered cycloalkyl,
or $R_4$ and $R_5$ together with the carbon atom to which they are attached form a $C_{3-6}$spirocycloalkyl,
or $R_4$ is O and $R_5$ is absent;
$R_6$ is H or $C_{1-3}$alkyl,
or $R_6$ together with $R_{11}$ in the ortho-position to the amide are a $C_2$alkylene chain forming a 5-membered ring;
Ar1 is 6-membered aryl or heteroaryl;
Ar2 is a 6-membered aryl or heteroaryl and is attached to Ar1 in the para position relative to the amide;
$R_{10}$ is H, halo, $C_{1-2}$alkyl, $OC_{1-2}$alkyl, $C_{1-2}$haloalkyl, $OC_{1-2}$haloalkyl or CN;
$R_{11}$ is H, F, Cl, $CH_3$, $OCH_3$, $CF_3$, $OCF_3$ or CN,
or $R_{11}$, when in the ortho-position to the amide, together with $R_6$ are a $C_2$alkylene chain forming a 5-membered ring;
$R_{12}$ is attached to Ar2 in the ortho or meta position relative to Ar1 and $R_{12}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, $C_{1-4}$alkylOH, CN, $C_{1-3}$alkylene$OC_{1-3}$alkyl, $C_{1-4}$haloalkyl, $OC_{1-4}$haloalkyl. , $N(CH_3)_2$, $SO_2CH_3$, $C(O)N(CH_3)_2$, $NHC(O)C_{1-3}$alkyl or a $C_{3-6}$heterocycloalkyl comprising one nitrogen located at the point of attachment to Ar2; and
$R_{13}$ is H, F, $CH_3$ or $OCH_3$;

or a salt and/or solvate thereof and/or derivative thereof.

2. The compound according to claim 1, wherein $R_1$ is $C_{3-5}$cycloalkyl, optionally substituted by $CH_3$.

3. The compound according to claim 1 or 2, wherein $R_4$ is $C_{1-6}$alkyl, in particular $C_{1-4}$alkyl such as methyl, ethyl or propyl (n-propyl or isopropyl).

4. The compound according to any one of claims 1 to 3, wherein $R_5$ is H, methyl, ethyl or fluoro e.g. H, methyl or ethyl.

5. The compound according to any one of claims 1 to 4 wherein $R_6$ is H.

6. The compound according to any one of claims 1 to 5 wherein Ar1 is phenyl or 2-pyridyl.

7. The compound according to any one of claims 1 to 6 wherein $R_{10}$ is H, F, Cl, $CH_3$, $OCH_3$ or $OCF_3$ and $R_{11}$ is H, F or $CH_3$.

8. The compound according to any one of claims 1 to 7 wherein Ar2 is 3-pyridyl or pyrazinyl.

9. The compound according to any one of claims 1 to 8 wherein $R_{12}$ is H, fluoro, chloro, $CH_3$, cyclopropyl, C=CH, $OCH_3$, ethoxy, n-propoxy, isopropoxy, cyclopropoxy, CN, $CF_3$, $OCHF_2$, $OCH_2CF_3$ or pyrrolidinyl.

10. A compound according to any one of claims 1 to 9, for use as a medicament.

11. The compound according to claim 10, for use in the reduction of T-cell and/or B-cell proliferation in a subject.

12. The compound according to claim 10, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

**13.** A compound of the formula:

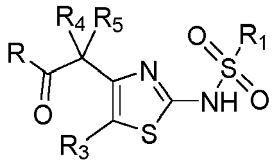

wherein $R_1$, $R_3$, $R_4$ and $R_5$ are as defined in any preceding claim and R is OH, $OC_{1-6}$alkyl (e.g. Omethyl and Oethyl) or Obenzyl, or R is $NH_2$.

**14.** A compound of the formula:

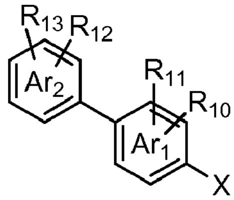

wherein Ar1, Ar2, $R_{10}$, $R_{11}$, $R_{12}$ and $R_{13}$ are as defined in any preceding claim and X is halo or $NHR_6$ and $R_6$ is defined in any preceding claim.

**15.** A compound of the formula:

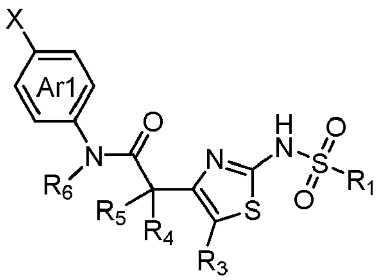

X = Cl, Br, $B(OH)_2$, $B(pin)_2$ ;
wherein Ar1, $R_1$, $R_3$, $R_4$, $R_5$ and $R_6$ are as defined in any preceding claim.

Europäisches Patentamt
European Patent Office
Office européen des brevets

# EUROPEAN SEARCH REPORT

Application Number
EP 17 20 4796

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2014/170435 A2 (INSERM INST NAT DE LA SANTÉ ET DE LA RECH MÉDICALE [FR]; FOND IMAGINE) 23 October 2014 (2014-10-23)<br>* page 5, line 24 - page 6, line 9; claim 1 *<br>----- | 1-15 | INV.<br>C07D241/12<br>C07D401/04<br>C07D417/12<br>C07D417/14<br>C07D277/52<br>C07D213/22<br>C07D213/38<br>C07C211/45<br>A61P35/00<br>A61P37/00<br>A61K31/425 |
| A | WO 2014/090715 A1 (HOFFMANN LA ROCHE [CH]; HOFFMANN LA ROCHE [US])<br>19 June 2014 (2014-06-19)<br>* page 1, line 1 - page 4, line 3; claim 1 *<br>----- | 1-15 | |
| A | US 2008/139557 A1 (BLOMGREN PETER A [US] ET AL) 12 June 2008 (2008-06-12)<br>* paragraph [0002] - paragraph [0026]; claim 1 *<br>----- | 1-15 | |
| X | GB 1 555 007 A (FUJISAWA PHARMACEUTICAL CO) 7 November 1979 (1979-11-07)<br>* example 1 *<br>----- | 13 | |
| X | KLAPERS A ET AL: "Copper-catalyzed halogen exchange in aryl halides: an aromatic Finkelstein Reaction",<br>JOURNAL OF THE AMERICAN CHEMICAL SOCIETY, AMERICAN CHEMICAL SOCIETY, US,<br>vol. 124, 1 January 2002 (2002-01-01), pages 14844-14845, XP002337383,<br>ISSN: 0002-7863, DOI: 10.1021/JA028865V<br>* compound 4o *<br>----- | 14 | TECHNICAL FIELDS SEARCHED (IPC)<br>C07D<br>A61P<br>C07C |
| X | US 2016/152583 A1 (ARISAWA MITSUHIRO [JP] ET AL) 2 June 2016 (2016-06-02)<br>* paragraph [0110] *<br>-----<br>-/-- | 14 | |

1

The present search report has been drawn up for all claims

| Place of search | Date of completion of the search | Examiner |
|---|---|---|
| Munich | 23 February 2018 | Seelmann, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 20 4796

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/010751 A1 (APPLIED RESEARCH SYSTEMS [NL]; SWINNEN DOMINIQUE [FR]; BOMBRUN AGNES [) 2 February 2006 (2006-02-02) * page 84, line 8 * ----- | 14 | |
| X | EP 2 292 603 A1 (SAMSUNG MOBILE DISPLAY CO LTD [KR]) 9 March 2011 (2011-03-09) * paragraph [0085] * ----- | 14 | |

TECHNICAL FIELDS SEARCHED (IPC)

1

The present search report has been drawn up for all claims

| Place of search | Date of completion of the search | Examiner |
|---|---|---|
| Munich | 23 February 2018 | Seelmann, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 20 4796

This annex lists the patent family members relating to the patent documents cited in the above-mentioned European search report. The members are as contained in the European Patent Office EDP file on
The European Patent Office is in no way liable for these particulars which are merely given for the purpose of information.

23-02-2018

| Patent document cited in search report | Publication date | Patent family member(s) | Publication date |
|---|---|---|---|
| WO 2014170435 A2 | 23-10-2014 | CA 2909434 A1 | 23-10-2014 |
| | | CN 105473136 A | 06-04-2016 |
| | | EP 2986287 A2 | 24-02-2016 |
| | | JP 2016523818 A | 12-08-2016 |
| | | US 2016051674 A1 | 25-02-2016 |
| | | WO 2014170435 A2 | 23-10-2014 |
| WO 2014090715 A1 | 19-06-2014 | CA 2893637 A1 | 19-06-2014 |
| | | CN 104837837 A | 12-08-2015 |
| | | EP 2931721 A1 | 21-10-2015 |
| | | ES 2607635 T3 | 03-04-2017 |
| | | HK 1212975 A1 | 24-06-2016 |
| | | JP 6154482 B2 | 28-06-2017 |
| | | JP 2016502988 A | 01-02-2016 |
| | | KR 20150093238 A | 17-08-2015 |
| | | RU 2015126293 A | 19-01-2017 |
| | | US 2015353534 A1 | 10-12-2015 |
| | | WO 2014090715 A1 | 19-06-2014 |
| US 2008139557 A1 | 12-06-2008 | AR 063706 A1 | 11-02-2009 |
| | | AU 2007296559 A1 | 20-03-2008 |
| | | BR PI0716918 A2 | 05-11-2013 |
| | | CA 2661654 A1 | 20-03-2008 |
| | | CA 2941688 A1 | 20-03-2008 |
| | | CL 2007002640 A1 | 20-06-2008 |
| | | CN 101679361 A | 24-03-2010 |
| | | CO 6160313 A2 | 20-05-2010 |
| | | CR 10710 A | 14-07-2009 |
| | | EC SP099239 A | 31-07-2009 |
| | | EP 2081923 A1 | 29-07-2009 |
| | | ES 2576478 T3 | 07-07-2016 |
| | | JP 5295961 B2 | 18-09-2013 |
| | | JP 2010504287 A | 12-02-2010 |
| | | KR 20090074762 A | 07-07-2009 |
| | | MA 30782 B1 | 01-10-2009 |
| | | PE 08392008 A1 | 23-08-2008 |
| | | RU 2009113693 A | 20-10-2010 |
| | | TW 200829579 A | 16-07-2008 |
| | | US 2008139557 A1 | 12-06-2008 |
| | | WO 2008033854 A1 | 20-03-2008 |
| | | ZA 200901633 B | 30-12-2009 |
| GB 1555007 A | 07-11-1979 | NONE | |
| US 2016152583 A1 | 02-06-2016 | CN 105408021 A | 16-03-2016 |
| | | EP 2985078 A2 | 17-02-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 17 20 4796

This annex lists the patent family members relating to the patent documents cited in the above-mentioned European search report. The members are as contained in the European Patent Office EDP file on
The European Patent Office is in no way liable for these particulars which are merely given for the purpose of information.

23-02-2018

| Patent document cited in search report | Publication date | Patent family member(s) | Publication date |
|---|---|---|---|
| | | JP 6005259 B2 | 12-10-2016 |
| | | JP 2016028795 A | 03-03-2016 |
| | | JP WO2014157677 A1 | 16-02-2017 |
| | | KR 20160013008 A | 03-02-2016 |
| | | US 2016152583 A1 | 02-06-2016 |
| | | WO 2014157677 A2 | 02-10-2014 |
| WO 2006010751 A1 | 02-02-2006 | AR 050435 A1 | 25-10-2006 |
| | | AT 430133 T | 15-05-2009 |
| | | AU 2005266313 A1 | 02-02-2006 |
| | | BR PI0513878 A | 20-05-2008 |
| | | CA 2570903 A1 | 02-02-2006 |
| | | CN 1989106 A | 27-06-2007 |
| | | CY 1109194 T1 | 02-07-2014 |
| | | DK 1771421 T3 | 13-07-2009 |
| | | EA 200700037 A1 | 31-08-2007 |
| | | EP 1771421 A1 | 11-04-2007 |
| | | ES 2325836 T3 | 21-09-2009 |
| | | HR P20090360 T1 | 31-08-2009 |
| | | IL 180682 A | 30-11-2011 |
| | | JP 5226307 B2 | 03-07-2013 |
| | | JP 2008507575 A | 13-03-2008 |
| | | KR 20070046873 A | 03-05-2007 |
| | | PT 1771421 E | 25-05-2009 |
| | | RS 50980 B | 31-10-2010 |
| | | SI 1771421 T1 | 31-10-2009 |
| | | US 2008021028 A1 | 24-01-2008 |
| | | US 2011263628 A1 | 27-10-2011 |
| | | WO 2006010751 A1 | 02-02-2006 |
| EP 2292603 A1 | 09-03-2011 | CN 101993410 A | 30-03-2011 |
| | | EP 2292603 A1 | 09-03-2011 |
| | | JP 5211104 B2 | 12-06-2013 |
| | | JP 2011037826 A | 24-02-2011 |
| | | KR 20110016031 A | 17-02-2011 |
| | | US 2011031483 A1 | 10-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHENG, D. et al.** Discovery of Pyridinyl Acetamide Derivatives as Potent, Selective, and Orally Bioavailable Porcupine Inhibitors. *Medicinal Chemistry Letters,* 2016, vol. 7 (7), 676-680
- **EVANS, D. R. ; GUY, H. I.** Mammalian pyrimidine biosynthesis: fresh insights into an ancient pathway. *J. Biol. Chem.,* 2004, vol. 279, 33035-33038
- **FAIRBANKS, L. D. ; BOFILL, M. ; RUCKEMANN, K. ; SIMMONDS, H. A.** Importance of ribonucleotide availability to proliferating T-lymphocytes from healthy humans. Disproportionate expansion of pyrimidine pools and contrasting effects of de novo synthesis inhibitors. *J. Biol. Chem.,* 1995, vol. 270, 29682-29689
- **HIGGINS, M. J. ; GRAVES, P. R. ; GRAVES, L. M.** Regulation of human cytidine triphosphate synthetase 1 by glycogen synthase kinase 3. *J. Biol. Chem.,* 2007, vol. 282, 29493-29503
- **KURSULA, P. ; FLODIN, S. ; EHN, M. ; HAMMARSTRÖM, M. ; SCHÜLER, H. ; NORDLUND, P. ; STENMARKA, P.** Structure of the synthetase domain of human CTP synthetase, a target for anticancer therapy. *Acta Crystallogr Sect F Struct Biol Cryst Commun,* 2006, vol. 62, 613-617
- **LIEBERMAN I.** Enzymatic amination of uridine triphosphate to cytidine triphosphate. *The J. Biol. Chem.,* 1956, vol. 222 (2), 765-75
- **LÜBBERS, T et al.** Aminothiazoles as γ-secretase modulators. *Bioorganic & Medicinal Chemistry Letters,* 2011, vol. 21 (21), 6554-6558
- **MARTIN E. et al.** CTP synthase 1 deficiency in humans reveals its central role in lymphocytes proliferation. *Nature,* 12 June 2014, vol. 510 (7504), 288-92
- *Erratum in: Nature,* 17 July 2014, vol. 511 (7509), 370
- **OSTRANDER, D. B. ; O'BRIEN, D. J. ; GORMAN, J. A. ; CARMAN, G. M.** Effect of CTP synthetase regulation by CTP on phospholipid synthesis in Saccharomyces cerevisiae. *J. Biol. Chem.,* 1998, vol. 273, 18992-19001
- **SAKAMOTO K ; ISHIBASHI Y ; ADACHI R et al.** Identification of cytidine-5-triphosphate synthase1-selective inhibitory peptide from random peptide library displayed on T7 phage. *Peptides,* 2017, vol. 94, 56-63
- **VAN DEN BERG, A. A. et al.** Cytidine triphosphate (CTP) synthetase activity during cell cycle progression in normal and malignant T-lymphocytic cells. *Eur. J. Cancer,* 1995, vol. 31, 108-112
- **VAN KUILENBURG, A.B.P ; MEINSMA, R. ; VREKEN, P. ; WATERHAM, H.R. ; VAN GENNIP, A.H.** Identification of a cDNA encoding an isoform of human CTP synthetase. *Biochimica et Biophysica Acta,* 2000, 1492548-552
- **XING-LI F. et al.** Efficient Diphosphane-Based Catalyst for the Palladium-Catalyzed Suzuki Cross-Coupling Reaction of 3-Pyridylboronic Acids. *European Journal of Organic Chemistry,* 2009, vol. 13, 2051-2054